(19) Europäisches Patentamt
European Patent Office
Office européen des brevets

(11) Publication number: **0 095 925 B2**

(12) **NEW EUROPEAN PATENT SPECIFICATION**

(45) Date of publication of the new patent specification: **07.12.94**

(51) Int. Cl.5: **C07D 403/12**, C07D 417/12, A01N 47/36, //C07D231/18, C07D277/36,C07D277/56, C07D233/68,C07D275/02, C07D233/84

(21) Application number: **83303105.7**

(22) Date of filing: **31.05.83**

(54) **Herbicidal imidazole, pyrazole, thiazole and isothiazole derivatives.**

(30) Priority: **01.06.82 US 384043**
**25.04.83 US 486092**
**04.04.83 US 479363**
**30.03.83 US 478146**

(43) Date of publication of application:
**07.12.83 Bulletin 83/49**

(45) Publication of the grant of the patent:
**22.03.89 Bulletin 89/12**

(45) Mention of the opposition decision:
**07.12.94 Bulletin 94/49**

(84) Designated Contracting States:
**AT BE CH DE FR GB IT LI LU NL SE**

(56) References cited:
**EP-A- 0 001 485**
**EP-A- 0 009 419**
**EP-A- 0 023 440**
**EP-A- 0 030 142**
**EP-A- 0 035 893**
**EP-A- 0 045 196**
**EP-A- 0 087 780**
**EP-A- 0 005 986**
**EP-A- 0 013 480**
**EP-A- 0 030 140**
**EP-A- 0 030 433**
**EP-A- 0 039 239**
**EP-A- 0 046 677**
**EP-A- 0 096 003**

(73) Proprietor: **E.I. DU PONT DE NEMOURS AND COMPANY**
**1007 Market Street**
**Wilmington Delaware 19898 (US)**

(72) Inventor: **Hay, James Volney**
**36 Stature Drive**
**Newark Delaware 19713 (US)**
Inventor: **Shapiro, Rafael**
**2221 F Prior Road**
**Wilmington Delaware 19809 (US)**
Inventor: **Wolf, Anthony David**
**151 Kirkcaldy Drive**
**Elkton Maryland 21921 (US)**

(74) Representative: **Hildyard, Edward Martin et al**
**Frank B. Dehn & Co.**
**European Patent Attorneys**
**Imperial House**
**15-19 Kingsway**
**London WC2B 6UZ (GB)**

EP 0 095 925 B2

## Description

Background of the Invention

This invention relates to novel N-[(heterocyclic)aminocarbonyl]imidazolesulfonamides, pyrazolesulfonamides, thiazolesulfonamides and isothiazolesulfonamides, to herbicidal compositions containing them and to methods of using them to control the growth of undesired vegetation.

New compounds effective for controlling the growth of undesired vegetation are in constant demand. In the most common situation, such compounds are sought to selectively control the growth of weeds in useful crops such as cotton, rice, corn, wheat and soybeans, to name a few. Unchecked weed growth in such crops can cause significant losses, reducing profit to the farmer and increasing costs to the consumer. In other situations, herbicides are desired which will control all plant growth. Examples of areas in which complete control of all vegetation is desired are areas around fuel storage tanks, ammunition depots and industrial storage areas. There are many products commercially available for these purposes, but the search continues for products which are more effective, less costly and environmentally safe.

A number of different types of N-[(heterocyclic)-aminocarbonyl]arylsulfonides are known as herbicides. Two of the first patents to issue on such compounds are U.S. Patents 4,169,719 and 4,127,405, issued on October 2, 1979 and November 28, 1978, respectively. These patents disclose compounds of the general formula

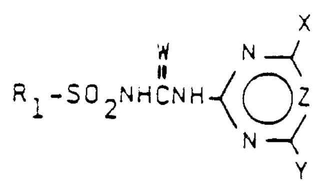

where

W can be O or S,

Z can be N or CH, and

R is optionally substituted benzene, optionally substituted thiopene, optionally substituted furan or naphthalene.

Later publications have disclosed similar compounds were $R_1$ is a thiophene or pyrrole. See, for example, EP-A-41,404; EP-A-64,804; EP-A-30,142; and EP-A-39,239. Nowhere in the art is there a disclosure of N-[(heterocyclic)-aminocarbonyl]imidazole-, pyrazole, thiazole, or isothiazolesulfonamides of any indication that such novel compounds would be useful as herbicides. However, EP-A-87,780 and EP-A-96,003 are relevant merely to the novelty of this invention in some designated states, and for this reason the appended claims for such states contain some additional limitations.

Summary of the Invention

It has now been found that the compounds of Formula I have utility as plant growth regulants and/or herbicides.

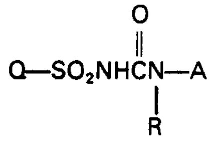

(I)

where

R is H or $CH_3$;

Q is

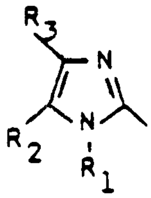 , 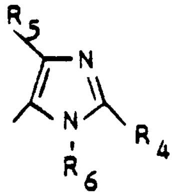 , 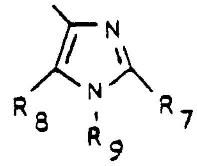 ,

Q-1 Q-2 Q-3

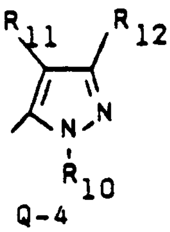 , 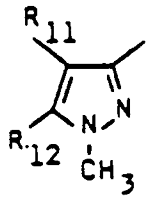 or 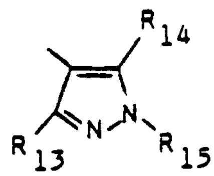 ;

Q-4 Q-5 Q-6

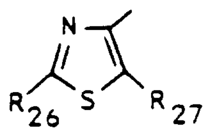 , 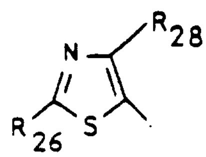 ,

Q-7 Q-8

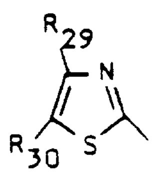 or 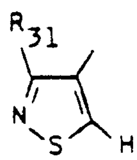 ;

Q-9 Q-10

$R_1$ is H, $C_1$-$C_8$ alkyl, $C_3$-$C_6$ alkenyl, $C_5$-$C_6$ cycloalkyl, $C_5$-$C_6$ cycloalkenyl, $C_3$-$C_6$ alkynyl, $C_4$-$C_7$ cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)$-$_nSO_2NR_{20}R_{21}$., $(R_{17}CH)_nSO_2R_{22}$,

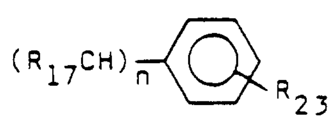 ;

or C,-$C_6$, alkyl
substituted either with
a) 1-3 atoms of F, Br or Cl; or
b) $OR_{16}$;
$R_2$, $R_3$ and $R_4$ are independently H or $CH_3$;
$R_5$ is H, $C_1$-$C_4$ alkyl, -$OR_6$, $NO_2$, F, Cl, Br, $CO_2R_{24}$, $S(O)_mR_{25}$ or $SO_2NR_{20}R_{21}$;
$R_6$ is H, $C_1$-$C_4$ alkyl, $C_3$-$C_4$ alkenyl, $C_3$-$C_4$ alkynyl, $CO_2R_{18}$, $SO_2NR_{20}R_{21}$, $SO_2R_{22}$ or $C_1$-$C_4$ alkyl substituted with a) 1-3 atoms of F, Cl or Br; or b) $OCH_3$;
$R_7$ is H or $CH_3$;
$R_8$ is H, $C_1$-$C_4$ alkyl, -$OR_{16}$, $NO_2$, F, Cl, Br, $CO_2R_{24}$, $S(O)_mR_{25}$ or $SO_2NR_{20}R_{21}$;
$R_9$ is $CH_3$ or $C_2H_5$;
$R_{10}$ is H, $C_1$-$C_4$ alkyl, $C_3$-$C_4$ alkenyl, $C_3$-$C_4$ alkynyl, $CO_2R_{24}$, $SO_2NR2_0R_{21}$ or $SO_2R_{22}$;

$R_{11}$ is H, $C_1$-$C_3$ alkyl, F, Cl, Br, $NO_2$, $-OR_{16}$, $CO_2R_{24}$, $S(O)_mR_{25}$ or $SO_2NR_{20}R_{21}$;
$R_{12}$ is H or $CH_3$;
$R_{13}$ and $R_{14}$ are independently H, $C_1$-$C_3$ alkyl $-OR_{16}$, F, Cl, Br, $NO_2$, $CO_2R_{24}$, $S(O)_mR_{25}$ or $SO_2NR_{20}R_{21}$;
$R_{15}$ is H or $CH_3$;
$R_{16}$ is $C_1$-$C_3$ alkyl;
$R_{17}$ is H or $CH_3$;
$R_{18}$ is $C_1$-$C_4$ alkyl, $C_3$-$C_4$ alkenyl, $C_3$-$C_4$ alkynyl, $CH_2CH_2Cl$ or $CH_2CH_2OCH_3$;
$R_{19}$ is $C_1$-$C_4$ alkyl, $C_3$-$C_4$ alkenyl, $C_3$-$C_4$ alkynyl or $CH_2CH_2OCH_3$;
$R_{20}$ and $R_{21}$ are independently $CH_3$ or $C_2H_5$;
$R_{22}$ is $C_1$-$C_3$ alkyl or $CF_3$;
$R_{23}$ is H, Cl, Br, $CH_3$, F, $CF_3$, $OCH_3$ or $NO_2$;
$R_{24}$ is $C_1$-$C_3$ alkyl or allyl;
$R_{25}$ is $C_1$-$C_3$ alkyl;
m is 0, 1 or 2;
n is 0 or 1;
$R_{26}$ is H, $C_1$-$C_3$ alkyl or $CH_3C(O)NH$;
$R_{27}$ is H, $C_1$-$C_3$ alkyl, $C_1$-$C_3$ alkoxy, $C_1$-$C_3$ alkylthio or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
$R_{31}$ is Cl, $C_1$-$C_3$ alkoxycarbonyl or $C_1$-$C_3$ alkyl;
A is

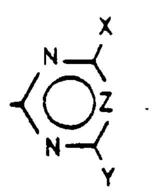 ,
A-1

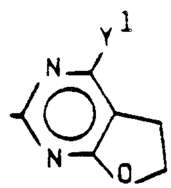 ,
A-2

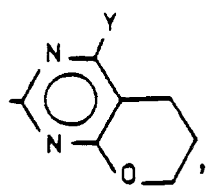 ,
A-3

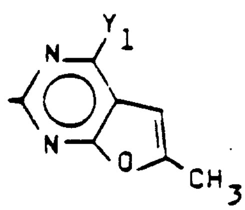 ,
A-4

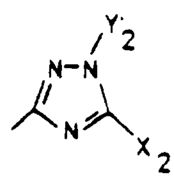 or
A-5

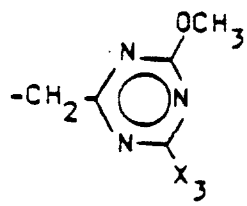 ;
A-6

X is $CH_3$, $OCH_3$, Cl, F, $OCF_2H$ or $SCF_2H$;
Y is $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ or $GCF_2T$ where G is O or S and T is H, CHClF, CHBrF, $CF_2H$ or $CHFCF_3$;
Z is CH or N;
$Y_1$ is H, Cl, $CH_3$ $OCH_3$ or $OCF_2H$;
$X_2$ is $OCH_3$, $CH_3$, $CH_2CH_3$, $OCH_2CH_3$, $SCH_3$ or $SCH_2CH_3$;
$Y_2$ is $CH_3$, $CH_2CH_3$ or $CH_2CF_3$;
$X_3$ is $OCH_3$ or $CH_3$;
provided that,
a) when Q is Q-7, Q-8, Q-9 or Q-10, then R is H;
b) the total number of carbon atoms in $R_1$ is less than or equal to 8;
c) if $R_1$ is other than $C_1$-$C_3$ alkyl, then $R_3$ must be H;
d) when $R_5$ is other than H, $CH_3$, $OCH_3$ or $NO_2$, then $R_6$ is H or $CH_3$;

e) when $R_6$ is $CO_2R_{18}$, $SO_2NR_{20}R_{21}$ or $SO_2R_{22}$, then $R_5$ is H, $CH_3$, $OCH_3$ and $NO_2$;

f) when $R_{10}$ is other than $C_1$-$C_3$ alkyl, then $R_{11}$ is H, Cl, $OCH_3$, $NO_2$ or $CH_3$;

g) when either of $R_{13}$ or $R_{14}$ is $CO_2R_{24}$, $S(O)_mR_{25}$ or $SO_2NR_{20}R_{21}$, then the other is H, Cl, $CH_3$, $OCH_3$ or $NO_2$;

h) $R_{29}$ and $R_{30}$ are not simultaneously Cl;

i) when X is Cl or F, then Z is CH and Y is $OCH_3$, $NH_2$, $NHCH_3$ or $N(CH_3)_2$;

j) when Q is Q-7, Q-8, Q-9 or Q-10, then A is A-1; X is $CH_3$, $OCH_3$ or $OCF_2H$, Y is $CH_3$, $OCH_3$, $OC_2H_5$, $OCF_2H$ or $CH_2OCH_3$; and

k) when either X or Y is $OCF_2H$ then Z is CH;

and agriculturally suitable salts thereof.

This invention therefore relates to compounds of Formula I as set out in this claims, to herbicidal compositions containing the compounds and to methods of using the compounds to control the growth of undesired vegetation.

Certain groups of compounds are preferred because of their high herbicidal and/or plant growth regulant activity and/or because of the ease with which they may be prepared. These preferred groups are as follows:

1. Compounds of Formula I where Q is Q-1 and R is H.

1a. Compounds of Preferred 1 where A is A-1, and Z is CH.

1b. Compounds of Preferred 1a where X is $CH_3$, Cl, $QCH_3$ or $COCF_2H$ and Y is $CH_3$, $OCH_3$ or $OCF_2H$.

1c. Compounds of Preferred 1b where $R_1$ is H or $C_1$-$C_4$ alkyl, and $R_3$ is H.

2. Compounds of Formula I where Q is Q-2, and R is H.

2a. Compounds of Preferred 2 where A is A-1, Z is CH and $R_4$ is H.

2b. Compounds of Preferred 2a where X is $CH_3$, Cl, $OCH_3$ or $OCF_2H$ and Y is $CH_3$, $OCH_3$ or $OCF_2H$.

2c. Compounds of Preferred 2b where $R_5$ is H, $CH_3$, $OCH_3$, Cl, $NO_2$, $CO_2R_{24}$ or $SO_2NR_{20}R_{21}$ and $R_6$ is H or $C_1$-$C_4$ alkyl.

3. Compounds of Formula I where Q is Q-3 and R is H.

3a. Compounds of Preferred 3 where A is A-1, Z is CH and $R_7$ is H.

3b. Compounds of Preferred 3a where X is $CH_3$, Cl, $OCH_3$ or $OCF_2H$ and Y is $CH_3$, $OCH_3$ or $OCF_2H$.

3c. Compounds of Preferred 3b where $R_8$ is Br, $C_1$-$C_4$ alkyl, $OCH_3$, Cl, $NO_2$, $CO_2R_{24}$, $S(O)_mR_{25}$ or $SO_2NR_{20}R_{21}$.

4. Compounds of Formula I where Q is Q-4 and R is H.

4a. Compounds of Preferred 4 where A is A-1 and Z is CH.

4b. Compounds of Preferred 4a where X is $CH_3$, Cl, $OCH_3$ or $OCF_2H$ and Y is $CH_3$, $OCH_3$ or $OCF_2H$.

4c. Compounds of Preferred 4b where $R_{10}$ is H, $C_1$-$C_3$ alkyl, $CO_2CH_3$, $SO_2CH_3$, or $SO_2N(CH_3)_2$ and $R_{11}$ is H, $CH_3$, $OCH_3$, Cl, Br, $NO_2$, $CO_2CH_3$, $SO_2CH_3$ or $SO_2N(CH_3)_2$.

5. Compounds of Formula I where Q is Q-5 and R is H.

5a. Compounds of Preferred 5 where A is A-1 and Z is CH.

5b. Compounds of Preferred 5a where X is $CH_3$, Cl, $OCH_3$ or $OCF_2H$ and Y is $CH_3$, $OCH_3$ or $OCF_2H$.

5c. Compounds of Preferred 5b where $R_{11}$ is H, $CH_3$, $OCH_3$, Cl, Br, $NO_2$, $CO_2CH_3$, $SO_2CH_3$ or $SO_2N$-$(CH_3)_2$.

6. Compounds of Formula I where Q is Q-6 and R is H.

6a. Compounds of Preferred 6 where A is A-1 and Z is CH.

6b. Compounds of Preferred 6a where X is $CH_3$, Cl, $OCH_3$ or $OCF_2H$ and Y is $CH_3$, $OCH_3$ or $OCF_2H$.

6c. Compounds of Preferred 6b where $R_{13}$ and $R_{14}$ are independently H, $CH_3$, $OCH_3$, Cl, Br, $NO_2$, $CO_2CH_3$, $SO_2CH_3$ or $SO_2N(CH_3)_2$.

7. Compounds of Formula I where Q is Q-7, Q-8, Q-9 or Q-10 and Z is CH.

7a. Compounds of Preferred 7 where Q is Q-9, then $R_{30}$ is H.

7b. Compounds of Preferred 7 where Q is Q-7, Q-8 or Q-10.

7c. Compounds of Preferred 7b where Q is Q-7, $R_{26}$ is H, $CH_3$, and $R_{27}$ is H, $CH_3$, $C_1$-$C_2$ alkoxy or $C_1$-$C_2$ alkoxycarbonyl.

7d. Compounds of Preferred 7b where Q is Q-10 and $R_{31}$ is $CO_2CH_3$.

8. Compounds of Formula I wherein Q is Q-1, Q-2, Q-3, Q-4, Q-5 or Q-6;

A is

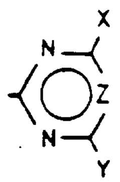 , 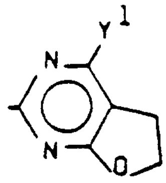 , 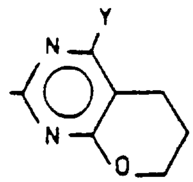 or 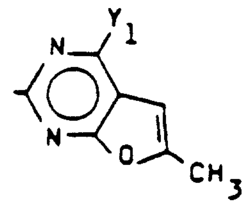 ;

X is $CH_3$, $OCH_3$ or Cl; Y is $CH_3$, $C_2H_5$, $OCH_3$, $OC_2H_5$, $CH_2OCH_3$, $CH(OCH_3)_2$, $NH_2$, $NHCH_3$ or $N(CH_3)_2$; Z is CH or N; $Y_1$ is H, Cl, $CH_3$ or $OCH_3$; $X_2$ is $OCH_3$ or $CH_3$; and $Y_2$ is $CH_3$.

9. Compounds of Formula I wherein Q is Q-7, Q-8 or Q-9; $R_{26}$ is H, $C_1$-$C_3$ alkyl or $CH_3C(O)NH$; $R_{27}$ is H, $C_1$-$C_3$ alkyl, $C_1$-$C_3$ alkoxy, $C_1$-$C_3$ alkylthio, or $C_1$-$C_3$ alkoxycarbonyl; $R_{28}$ is H, $C_1$-$C_3$ alkyl, $C_1$-$C_3$ alkoxy, $C_1$-$C_3$ alkylthio, $C_1$-$C_3$ alkylsulfonyl, $C_1$-$C_3$ alkoxycarbonyl, $NO_2$, Cl, Br, or $CF_3$; $R_{29}$ is H, Cl or $CH_3$; $R_{30}$ is H, Cl or $CH_3$;

A is

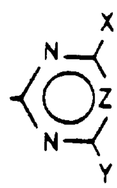 ,

Y is $CH_3$, $OCH_3$, $OCF_2H$, $OC_2H_5$ or $CH_2OCH_3$; X is $CH_3$, $OCH_3$ or $OCH_2H$; and Z is CH or N.

10. Compounds of Formula I wherein Q is Q-10; $R_{31}$ is Cl, $C_1$-$C_3$ alkoxycarbonyl or $C_1$-$C_3$ alkyl;

A is

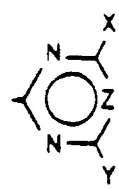 ,

X is $CH_3$, $OCH_3$ or $OCF_2H$; Y is $CH_3$, $OCH_3$, $OC_2H_5$, $OCF_2H$ or $CH_2OCH_3$; and Z is CH or N.

11. Compounds of Formula I wherein Q is Q-4; R is H; $R_{10}$ is $CH_3$, $R_{12}$ is H; $R_{11}$ is $CO_2R_{24}$, $R_{24}$ is $C_1$-$C_3$ alkyl; A is A-1; X and Y are $OCH_3$; and Z is CH.

The following compounds are specifically preferred because of their high herbicidal and/or plant growth regulant activity and/or because of the ease with which they may be synthesized.

N-[(4,6-dimethylpyrimidin-2-yl)aminocarbonyl]-1-(1-methylethyl)-1H-imidazole-2-sulfonamide, m.p. 185-186 °C(d);

N-[(4-methoxy-6-methylpyrimidin-2-yl)aminocarbonyl]-1-(methylethyl-1H-imidazole-2-sulfonamide, m.p. 157-158 °C(d);

N-[(4,6-dimethoxypyrimidin-2-yl)aminocarbonyl]-1-(methylethyl)-1H-imidazole-2-sulfonamide, m.p. 166-167 °C;

N-[(4,6-dimethoxypyrimidin-2-yl)aminocarbonyl]-1-ethyl)-1H-imidazole-2-sulfonamide, m.p. 196-197 °C;

N-[(4-methoxy-6-methylpyrimidin-2-yl)aminocarbonyl]-1-ethyl-1H-imidazole-2-sulfonamide, m.p. 178-179 °C(d);

N-[(4,6-dimethoxypyrimidin-2-yl)aminocarbonyl]-1-ethyl-1H-imidazole-2-sulfonamide, m.p. 186-187 °C;

N-[(4,6-dimethoxypyridin-2-yl)aminocarbonyl]-5-bromo-1-methyl-1H-imidazole-4-sulfonamide, m.p. 205-206 °C;

N-[(4-methoxy-6-methylpyrimidin-2-yl)aminocarbonyl]4-thiazolesulfonamide, m.p. 196-200 ° (d);

N-[(4,6-dimethoxypyrimidin-2-yl)aminocarbonyl]-4-thiazolesulfonamide, m.p. 201-203 °C;

Methyl 4-[[(4-methoxy-6-methylpyrimidin-2-yl)aminocarbonyl]aminosulfonyl]-3-isothiazolecarboxylate, m.p. 128 °C(d); and

Methyl 4-[[(4,6-dimethoxypyrimidin-2-yl)aminocarbonyl]aminosulfonyl)]-3-isothiazolecarboxylate, m.p. 161 °C(d).

Detailed Description of the Invention

Synthesis

*a. Compounds of the Invention*

The sulfonylureas of this invention can be prepared by a number of methods. These methods, and the publications or sources describing them are as follows:

Equation 1

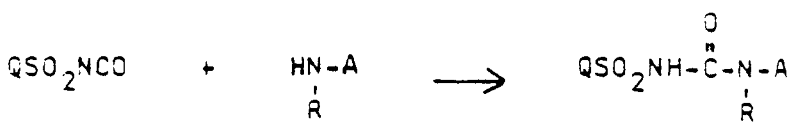

(a) (b) . I

As shown in Equation 1, an appropriately substituted sulfonyl isocyanate (a) is reacted with an appropriate aminoheterocycle (b). The reaction is best carried out in an inert aprotic solvent, such as methylene chloride, tetrahydrofuran, acetonitrile, ether or chloroform, at temperatures ranging from about -20 to 50 °C. in some cases, the desired product may crystallize from the reaction medium amd may be filtered. Reaction products which are soluble in the reaction medium may be isolated by evaporation of the solvent, tituration of the residue with solvents such as diethyl ether, ethyl acetate, 1-chlorobutane or hexane. Chromatography (e.g., silica gel) may also be necessary for purification. The process is described in U.S. Patents, 4,127,405, 4,257,802 and 4,221,585.

Equation 2

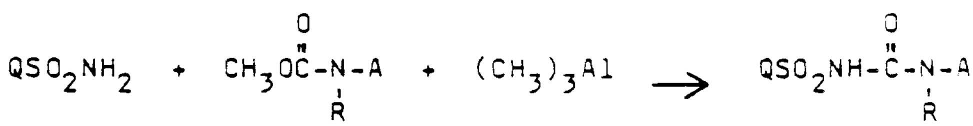

(c) (d) I

As shown in Equation 2, compounds of Formula (I) can also be prepared by reacting a sulfonamide (e) with an appropriate methyl carbamate (d) in the presence of an equimolar amount of trimethylaluminium. The reaction is best run in an inert aprotic solvent such as methylene chloride at about 25° to 40 °C for 10 to 96 hours under a nitrogen atmosphere. The product can be isolated by addition of an aqueous acetic acid solution followed by extraction of the product into methylene chloride, or by filtration of a product of low solubility. The product can be purified by trituration with solvents such as 1-chlorobutane, ethyl acetate or ethyl ether or by column chromatography on silica gel.

Equation 3

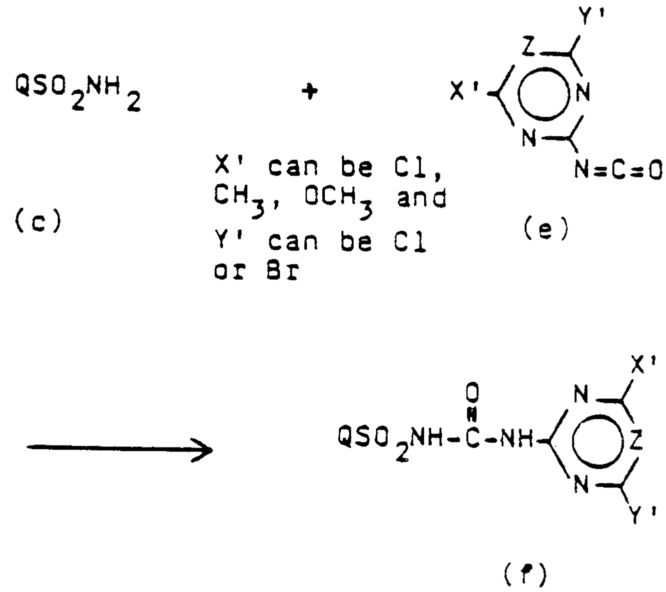


Equation 4

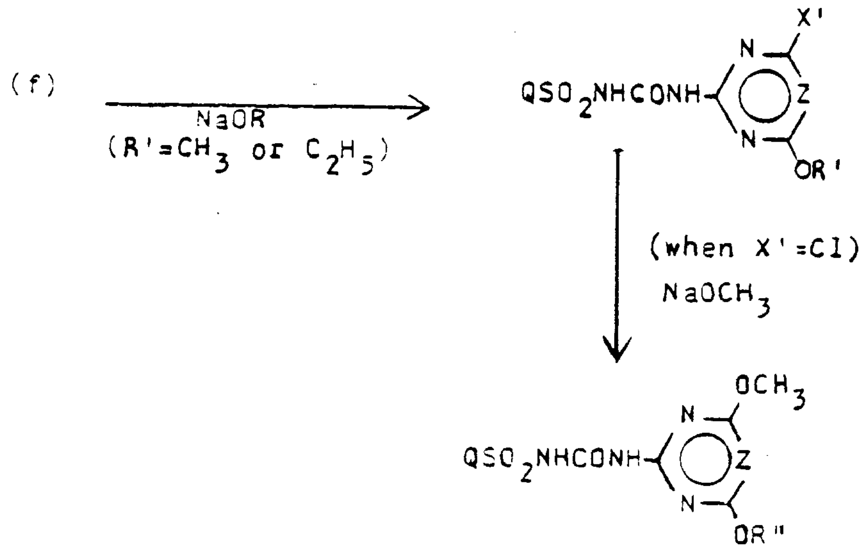


Examples of other methods for preparing compounds of this invention are illustrated in Equations 3 and 4 and described in our EP-A-30,140. The first step of this two-step process (Equation 3) involves reacting of a diazole sulfonamide and a heterocyclic isocyanate to yield a N-(haloheterocyclicaminocarbonyl)-aromatic sulfonamide (f). This reaction is best run in an inert organic solvent such as acetonitrile, tetrahydrofuran, toluene, acetone or butanone, preferably at a temperature of about 25 to 110 °C. The product can then be further reacted with selected nucleophiles, for example alkoxide -OR', (Equation 4) to yield herbicidal N-(substituted heterocyclicaminocarbonyl)-aromatic sulfonamides of this invention.

Equation 5

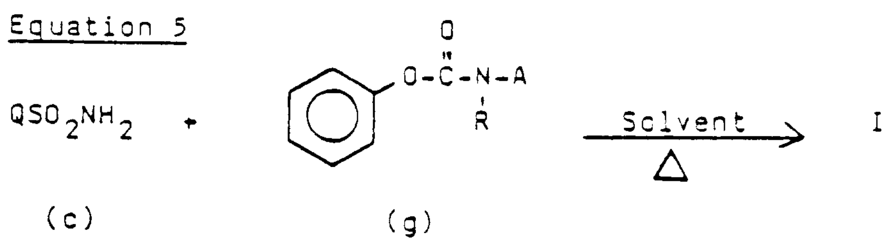

European Patent Application 81810282.4, published January 27, 1982

Equation 6

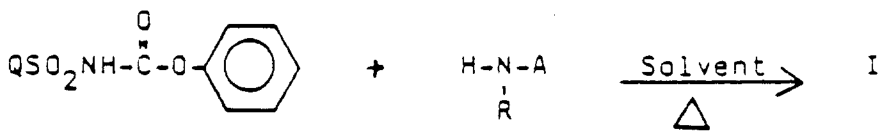


(h) (b)

European Patent Application 81810282.4, published January 27, 1982

Equations 5 to 6 illustrate two additional methods for preparing the compounds of this invention. As shown in Equation 5, a diazole sulfonamide (e) is reacted with an N-heterocyclic carbamate (g) in the presence of a base. Alternatively, in Equation 6, an N-phenylsulfonylcarbamate (h) is reacted with an amine (b). Both of these processes are described in our EP-A-44,807. Both reactions are best carried out in aprotic, inert organic solvents such as methylene chloride, tetrahydrofuran, acetonitrile, dioxane and toluene, at temperatures between -20° and 120°C. Suitable bases for use in the process of Equation 5 are organic bases such as amines and inorganic bases such as hydrides, hydroxides, carbonates and bicarbonates.

Compounds of Formula I in which Q is Q-1, $R_1$ is as defined above except that n must be 1, and R, $R_2$, $R_3$ and A are as defined previously, are best prepared by the procedures outlined in Equations 2, 5 or 6.

Compounds of Formula I in which Q is Q-2, Q-3, Q-4, Q-5 or Q-6, m is 0 or 2 and all other substituents are as previously defined are best prepared by the procedures of Equation 2, 5 or 6. Many of these compounds may also be prepared by the procedure of Equation 1. When m is 1, the compounds can be prepared by oxidation of the corresponding sulfide (m = 0) with *m*-chloroperbenzoic acid as described in our EP-A-35,893.

Compounds of Formula I in which Q is Q-7 or Q-8, $E_{26}$ is $CH_3C(O)NH$, and $R_{27}$ and $R_{28}$ are other than $C_1$-$C_3$ alkoxycarbonyl, are best prepared by the procedures of Equations 2, 5 or 6.

Compounds of this invention represented below by Formula 4 (Q of Formula I is Q-1, $R_1$ is $(R_{17}CH)_nCOR_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, or $(R_{17}CH)SO_2R_{22}$, and R, $R_2$, $R_3$ and A are as previously defined) may be prepared by the procedures outlined in Equations 7 or 8.

Equation 7

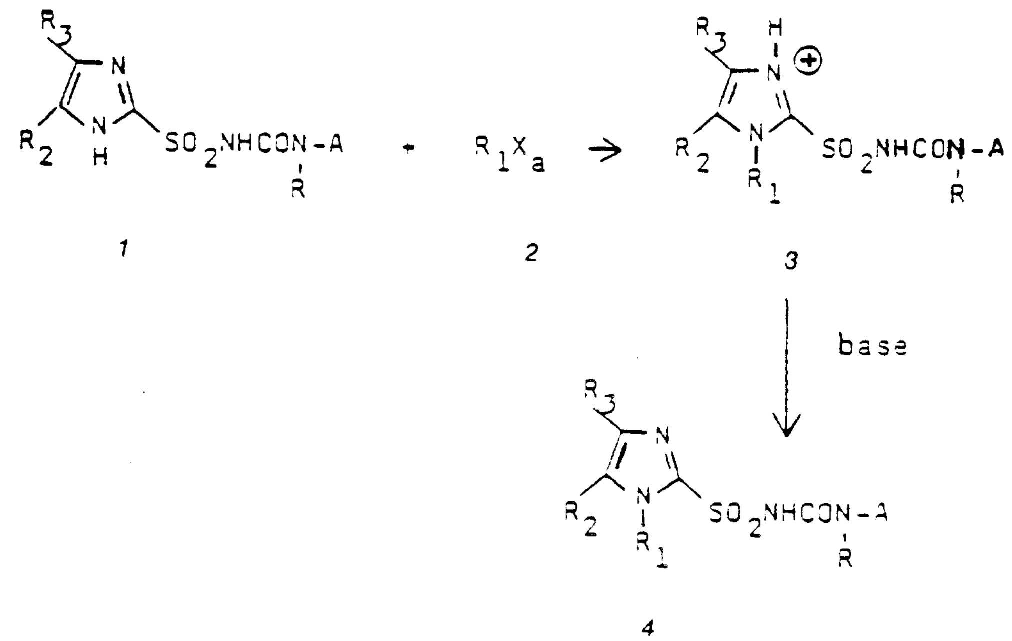


1 2 3

4

Equation 8

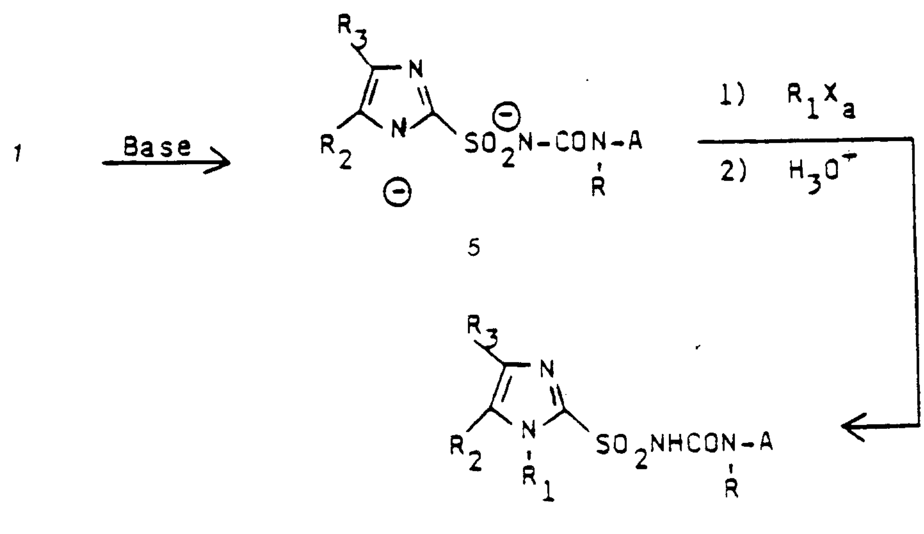


1 5 4

In both Equations 7 and 8, $X_a$ can be Cl, Br or I and, when n is O and $R_1$ is $C(O)R_{16}$, then $X_a$ can also be $-OCOR_{16}$, and when n is O and $R_1$ is $-SO_2R_{22}$, then $X_a$ can also be $-OSO_2R_{22}$.

In Equation 7, a sulfonylurea *1* is reacted with an appropriate alkylating or acylating agent $R_1X_a$ , in a suitable solvent such as $CH_2Cl_2$, xylene or tetrahydrofuran, for periods of 1 to 50 days at temperatures from ambient to the reflux temperature to the solvent. The salt *3* is neutralized to the product *4* by pouring the reaction mixture into water and reacting it with an appropriate amount of a base such as $NaHCO_3$ or $Na_2CO_3$. The product is isolated by extraction and then purified by the usual techniques of crystallization or chromatography.

In Equation 8, the sulfonylurea *1* is converted to its dianion *5* with two equivalents of a strong base such as NaH, *sec*-butyllithium or potassium *tert*-butoxide, in a suitable solvent such as tetrahydrofuran. The

dianion is treated with an equivalent of an alkyl or acylating agent, $R_1X_a$, to yield the salt of *4*. The reaction mixture is poured into water and acidified to pH 2-3 with a mineral acid such as aqueous hydrochloric acid. The product *4* is isolated and purified as described for Equation 7.

*b. Intermediate Compounds*

Heterocyclic amines of formula (b) are known in the art. For procedures of synthesis see U.K. Patent 4,127,405, U.S. Patent 4,221,585, EP-A-15,683, EP-A-46,677 and South African Patent Application 82/5045.

Heterocyclic carbamates of Formula (d) are prepared by procedures taught in European Patent Application 83300073.0, filed January 6, 1983.

Sulfonyl isocyanates may be prepared by procedures taught in U.S. 4,127,405 and EP-A-21,641.

Sulfonamides of Formula (c) in which Q is Q-1, $R_2$ and $R_3$ are as previously defined, and $R_1$ is H, $C_1$-$C_8$ alkyl; $C_4$-$C_7$ cycloalkylalkyl, $C_1$-$C_6$ alkyl substituted with 1-3 atoms of F, Br or Cl or with one $-OR_{16}$; or

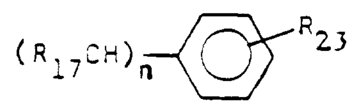

are prepared by procedures known in the art and also outlined by Equations 9, 10 and 10a.

Equation 9

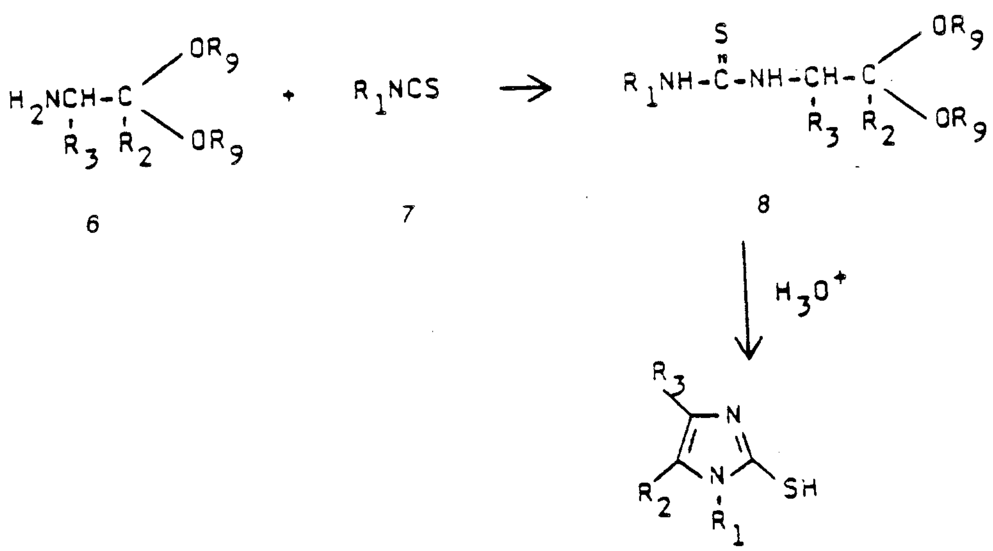


6

7

8

9

Equation 10

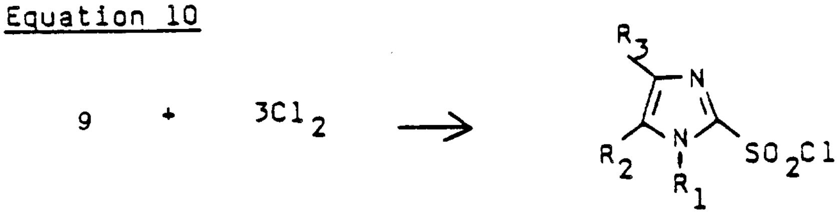


10

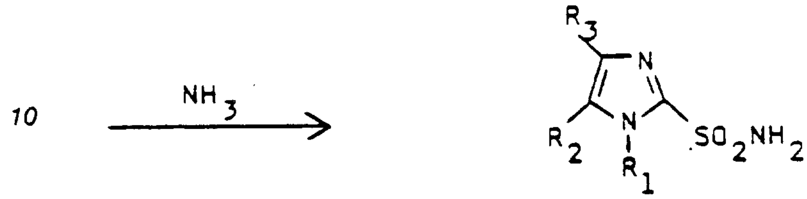


(c-1)

Equation 10a

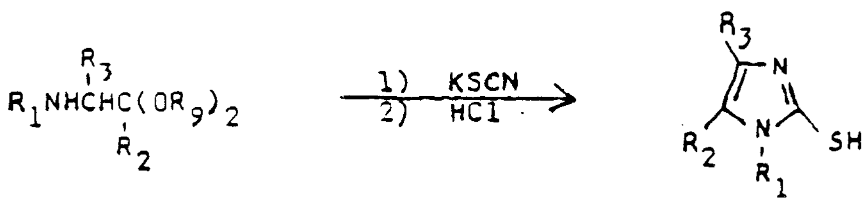


11 9

Mercapto imidazoles *9* are as known in the art. For representative procedures see R. G. Jones, E. C. Kornfield, K. C. McLaughlin and R. C. Anderson, *J. Am. Chem. Soc., 71* 4000 (1949) and references cited therein.

The procedure for converting mercapto imidazoles *9* to sulfonamides (c-l), shown in Equation 10, is known in the art. Details can be found in R. O. Roblin, Jr. and James W. Clapp, *J. Am. Chem. Soc., 72* 4890 (1950). Improved yields of sulfonamides are sometimes obtained when stoichiometric quantities of $Cl_2$ are used in this procedure, rather than an excess as described.

Compounds of Formula (c) in which Q is Q-1, $R_2$ and $R_3$ are as previously defined, and $R_1$ is $C_3$-$C_6$ alkenyl, $C_3$-$C_6$ alkynyl, $COR_{16}$, $CO_2R_{18}$, $COSR_{19}$, $CONR_{20}R_{21}$, $SO_2NR_{20}R_{21}$ or $SO_2R_{22}$ are best prepared by the procedure of Equation 11.

Equation 11

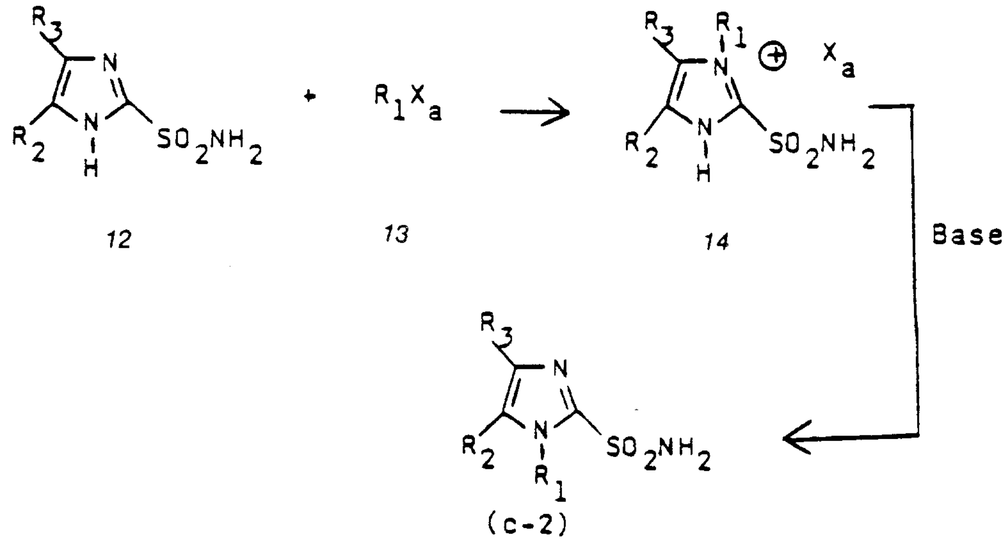


12 13 14

(c-2)

$X_a$ is Cl or Br;

when $R_1$ is $C_3$-$C_6$ alkenyl or $C_3$-$C_6$ alkynyl $X_a$ can also be I;

when $R_1$ is $COR_{16}$, $X_a$ can also be $OCOR_{16}$, and

when $R_1$ is $SO_2R_{22}$, $X_a$ can also be $OSO_2R_{22}$.

This method is known to those skilled in the art and is carried out in a manner analogous to the process outlined in Equation 7.

Many of the sulfonamides of Formula (c) are known in the art or can be prepared from known imidazole intermediates by chemical transformations known to those skilled in the art. General literature references describing the chemistry of imidazoles include:

Elderfield, Chemistry of Heterocyclic Compounds, Vol. V, p. 194-297, 1957, John Wiley and Sons, Inc., New York.

T. Kauffman and R. Werthwein, Angew Chem., Int. Ed. Engl, *10* 20-33 (1971).

M. R. Grimmett, Adv. Heterocycl. Chem. 12. 104-184 (1970).

The preparation of substituted nitro imidazolesulfonamides of Formulas (c-3) and (c-4) (Q is Q-2 or Q-3 and $R_5$ and $R_8$ are $-NO_2$) is shown in Equation 12.

Equation 12

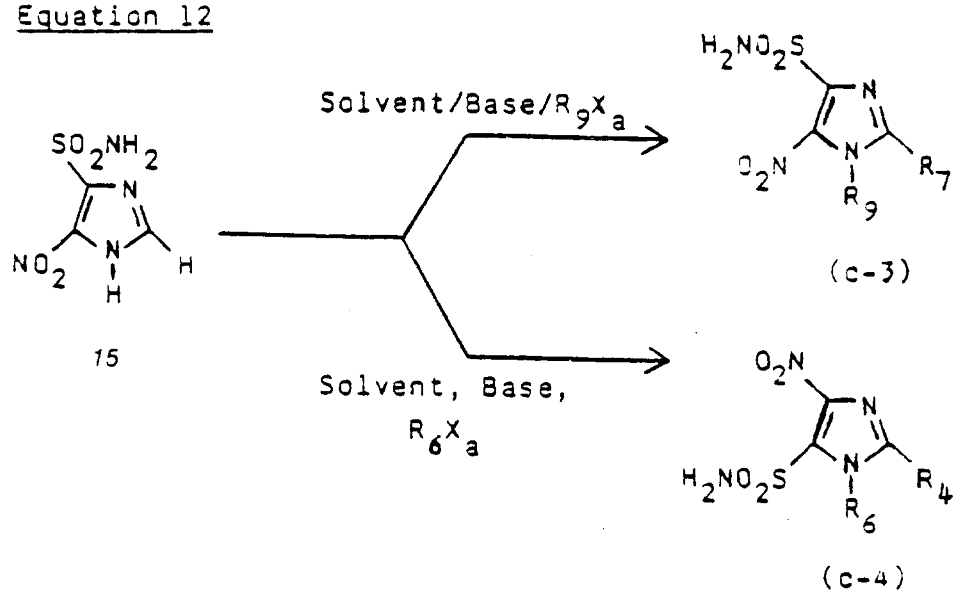


15

(c-3)

(c-4)

where $R_4$ and $R_8$ are H;

$R_6$ and $R_9$ are H, $CH_3$ or $CH_2C_6H_5$;

$X_a$ is Cl, Br or I; and

$R_9$ is $CH_3$ or $CH_3CH_2$.

This process is as described in B. S. Huang. M. J. Lauzon and J. C. Parham, J. Het. Chem. *16*, 811 (1979). Using similar procedures compounds of structure (c-3) in which $R_9$ is $CH_3CH_2$, and $R_7$ is H or $CH_3$ can be prepared. Also, intermediates of structure (c-4) in which $R_4$ is $CH_3$ or H and $R_6$ is $C_2$-$C_4$ alkyl; $C_3$-$C_4$ alkenyl; $C_1$-$C_4$ alkyl substituted with 1-3 atoms of F, Cl, Br or with $OCH_3$; $CO_2R_{18}$; $SO_2NR_{19}R_{20}$; or $SO_2R_{21}$ can be prepared.

The preparation of bromo or chloro imidazole sulfonamides of formula (c-5) and (c-6) (Q is Q-2 or Q-3 and $R_5$ and $R_8$ are Br or Cl) is outlined in Equation 12a.

Equation 12a

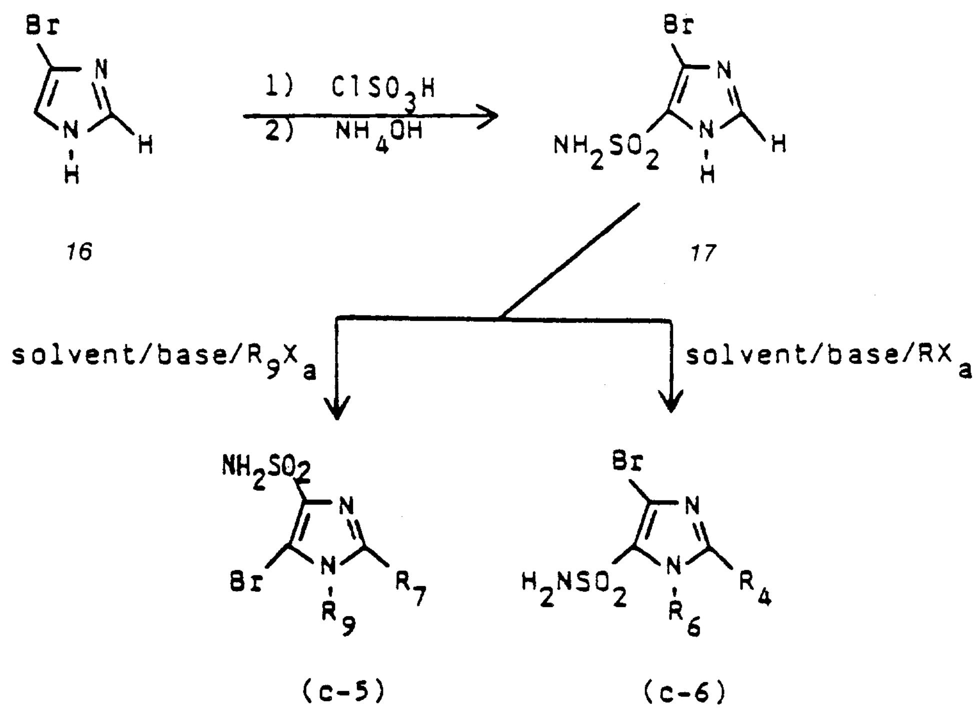


The preparation of 4(5)bromo imidazole *16* is described by I. E. Balaban and F. L. Pyman, *J. Chem. Soc., 121* 947 (1922). The preparation of the 4(5)-bromo-5(4)sulfonamide imidazole *17* is taught in L. L. Bennett and H. T. Baker, *J. Am. Chem. Soc., 79,* 2188 (1956).

The preparation of additional intermediates for use in preparing the compounds of this invention from readily available imidazole intermediates is outlined in Scheme 1.

Scheme 1

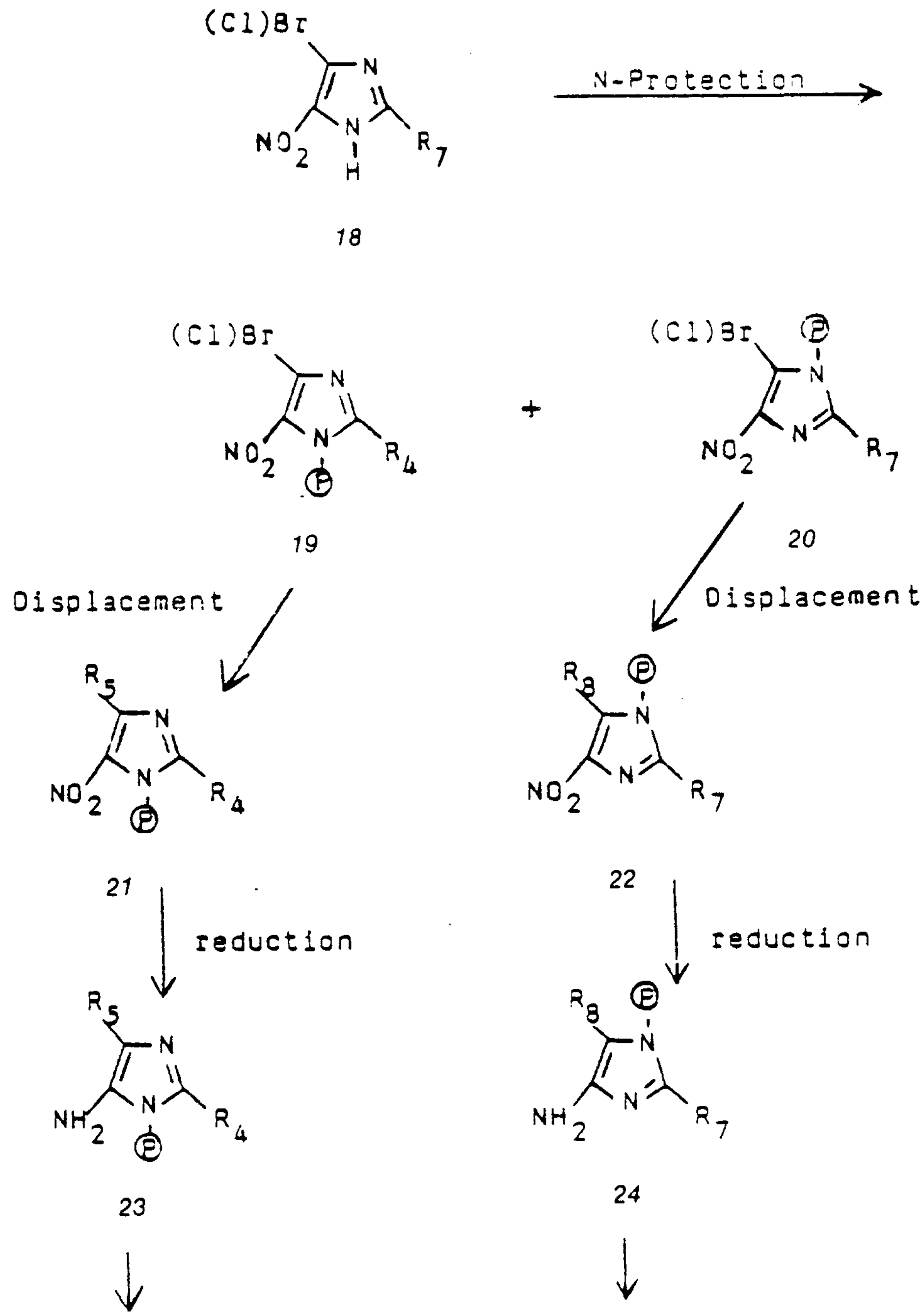


18

19

20

21

22

23

24

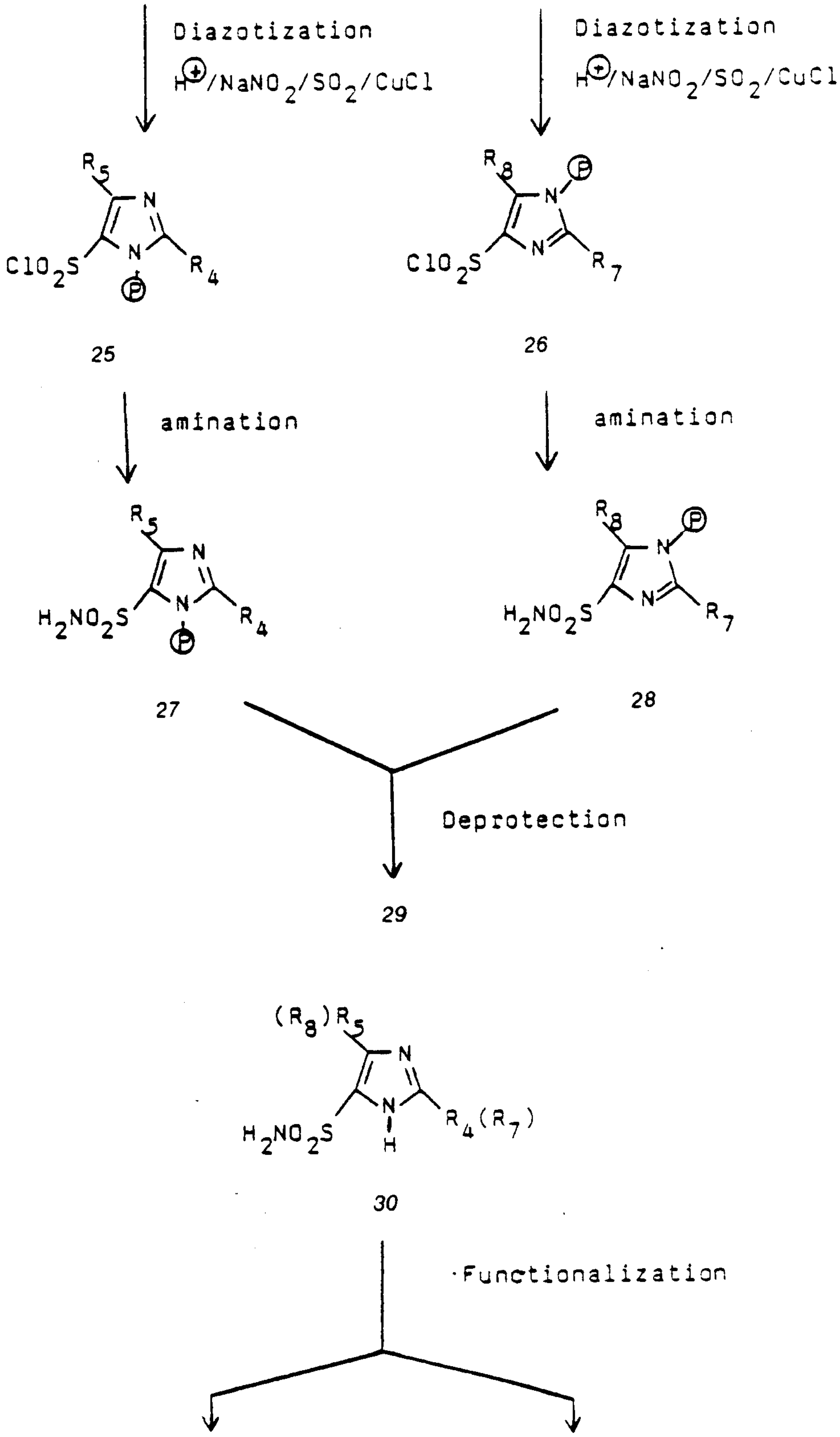


25

26

27

28

29

30

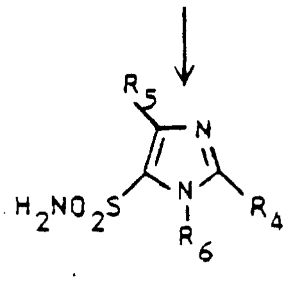

31

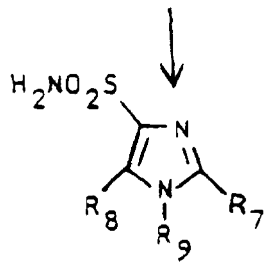

32

The preparation of 4-chloro or bromo-5-nitro-imidazole *18* is accomplished by procedures taught in I. E. Balaban and F. L. Pyman, J. Chem. Soc., *121* 947 (1922).

Protection of the imidazole nitrogen for futher manipulation of the ring is accomplished with such groups as $CH_3OCH_2$- or $CH_3OCH_2CH_2O$-$CH_2$-. These groups can be introduced and removed by procedures taught in C. Tang, D. Davalian, P. Huang, and R. Breslow, J. Am. Chem. Soc. 3918 (1978) and E. J. Corey, J. L. Cras and P. Ulrich, Tet. Let. 809 (1976).

Protection and deprotection can also be accomplished with groups such as

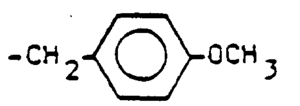

and

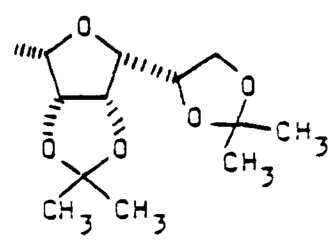

by procedures taught in T. Brown, G., Shaw and G. Durrant, J. C. S. Perkin I 2310, (1980).

Protection of the imidazole as shown in Scheme 1 leads to a mixture of isomers *19* and *20*.

The mixture is used without separation of the isomers in the following steps, since deprotection of either isomer eventually leads to the same product *30*.

Introduction of $R_5$ or $R_8$ as F can be accomplished by procedures taught in U.S. Patent 4,069,262 in which fluoronitrobenzene is produced from chloronitrobenzene by displacement with KF and CsF as catalyst.

Replacement of halogen in *19* and *20* to produce *21* and *22* in which $R_5$ and $R_8$ are -S($C_1$-$C_3$ alkyl) is accomplished by displacement of halogen with the appropriate mercaptide salt as shown in Scheme 1. Details for displacement of halogen by mercaptides in halonitroimidazoles can be found in L. L. Bennett and H.T. Baker, J. Am. Chem. Soc., *79*, 2188, (1957). See this same reference for methods of oxidizing nitroalkylthioimidazoles to nitroalkylsulfonylimidazoles of Formulas *21* and *22* in which $R_5$ and $R_8$ are -$SO_2$-($C_1$-$C_3$ alkyl).

Compounds of Formulas *21* and *22* in which $R_5$ and $R_8$ are -$SO_2NR_{20}R_{21}$ are produced by oxidative chlorination of the corresponding thiol or benzylmercaptide to the sulfonyl chloride. Treatment of the sulfonyl chloride with two equivalents of $R_{20}R_{21}NH$ produces the desired intermediates. This procedure is outlined in Scheme 2.

Scheme 2

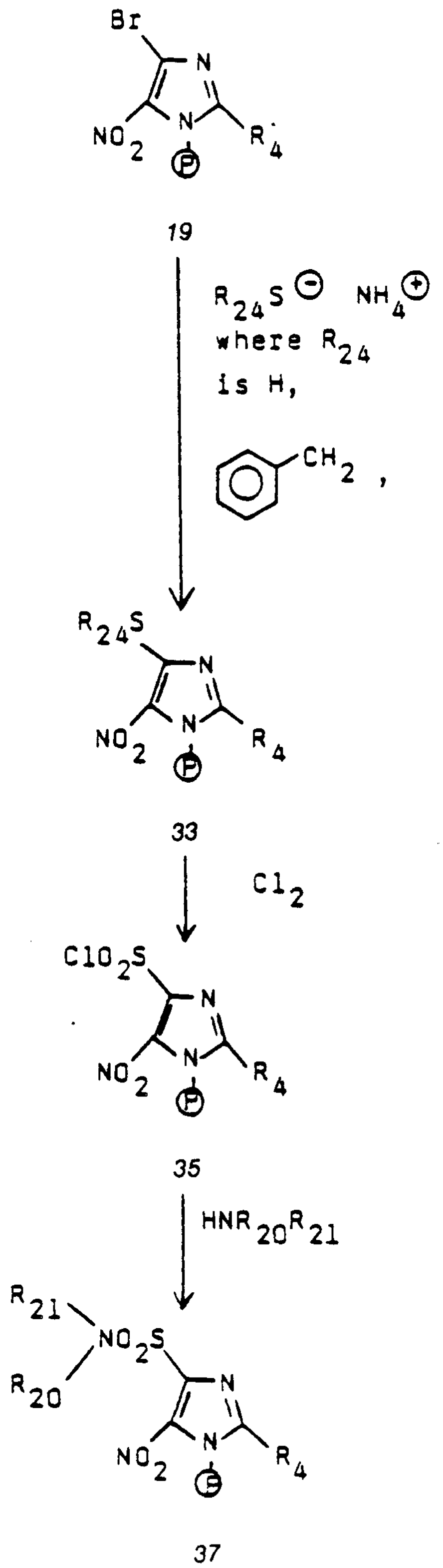


19

33

35

37

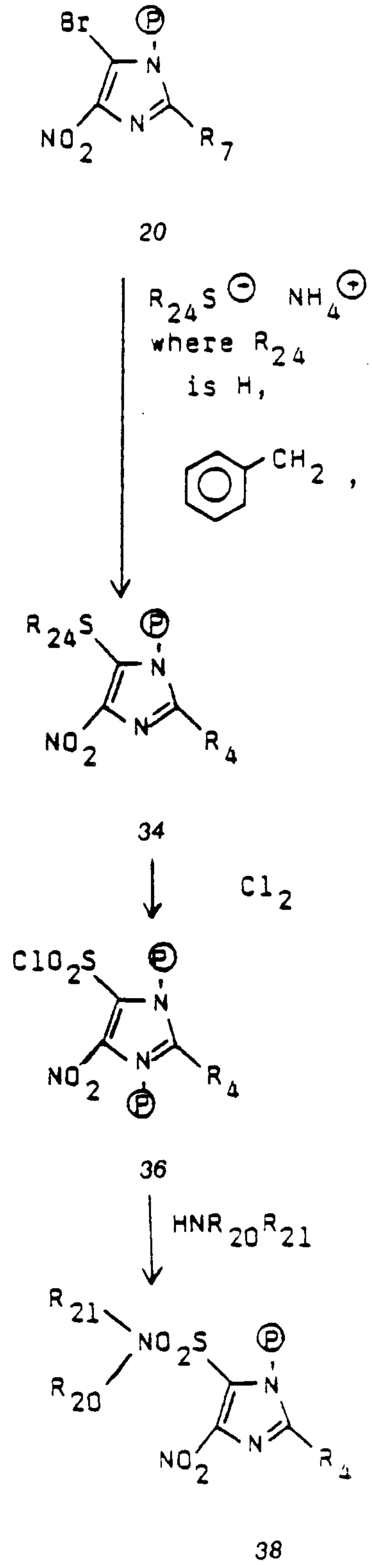


20

34

36

38

Conditions for carrying out the transformations outlined in Scheme 2 are known in the art. For example, see M. H. Fisher, W. H. Nicholson and R. S. Stuart, Can. J. Chem., *39*, 501 (1961), R. O. Roblin and J. W. Clapp, J. Am. Chem. Soc., *72*, 4890 (1950) and U.S. 4,310,346.

Compounds of Formula *21* and *22* in which $R_5$ and $R_8$ and $OR_{16}$ and $R_{16}$ is $C_1$-$C_3$ alkyl are produced by displacement of halogen in *19* and *20* with the appropriate alkoxide. Suitable procedures for replacement of an activated halogen with alkoxide can be found in R. S. Kittila, "Dimethylformamide Chemical Uses", Chapter 15, E. I. du Pont de Nemours and Company, Wilmington, Delaware 19898.

Reduction of nitroimidazoles (shown in Scheme 1) of Formulas *21* and *22* in which $R_4$, $R_5$, $R_7$ and $R_8$ are as defined for Formula I can be accomplished by treatment with Adam's platinum catalyst, hydrogen and ethanol at room temperature and atmospheric pressure. For details see M. H. Fisher, W. H. Nicholson and R. S. Stuart, Can. J. Chem., *39*, 501 (1961). Other suitable procedures which employ Raney nickel catalyst are taught in L. Bennett, Jr, and H. T. Baker, J. Am. Chem. Soc., *79* 2188 (1957). Stannous chloride may also be used. See Fieser and Fieser, "Reagents for Organic Synthesis", Vol. I, p. 1113, J. Wiley and Sons, Inc., New York (1967) for details and references.

Imidazoles of Formula *39*

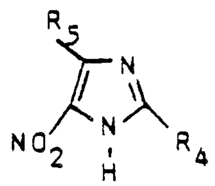

*39*

in which $R_4$ is H or $CH_3$ and $R_5$ is H or $C_1$-$C_4$ alkyl are produced by nitration of the corresponding imidazole *40*

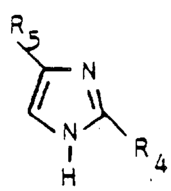

*40*

using procedures well known in imidazole chemistry. For example, see V. K. Bhagwat and F. L. Pyman, J. Chem. Soc., *127*, 1832 (1925).

Conversion of *39* into the sulfonamides *41* in which $R_4$ is H or $CH_3$ and $R_5$ is H or $C_1$-$C_4$ alkyl is accomplished by procedures

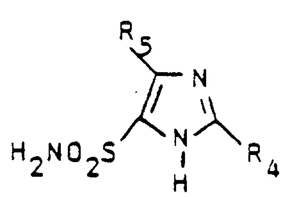

*41*

analogous to those outlined in Scheme 1.

Functionalization of *41* to produce sulfonamides *42* and *43* is outlined in Scheme 3.

Scheme 3

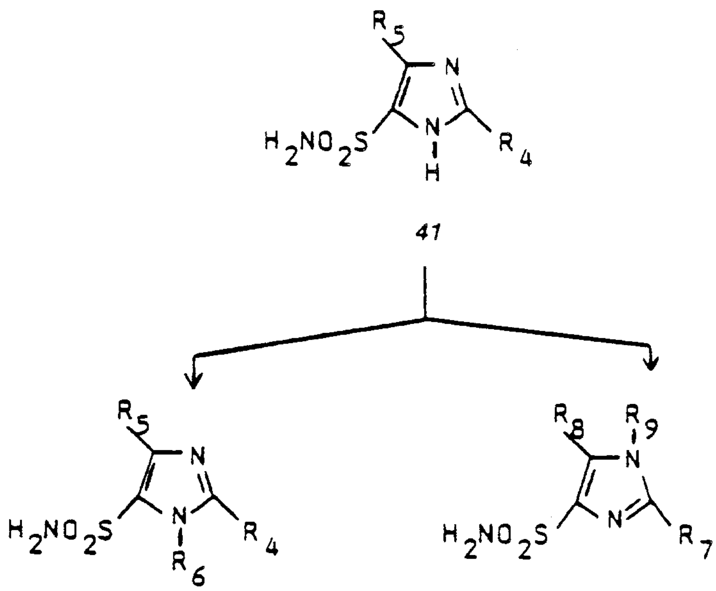

*41*

*42* *43*

This procedure carried out under typical conditions for alkylating or acylating nitrogen, produces the mixture of isomeric sulfonamides *42* and *43* in which $R_6$ and $R_9$ are as defined for Formula I.

Compounds of Formula *44*

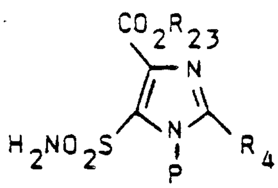

*44*

in which $R_4$ is H or $CH_3$ and $R_{23}$ is $C_1$-$C_3$ alkyl or allyl and P is a suitable protecting group such as *t*-butyl or a glycosyl group such as

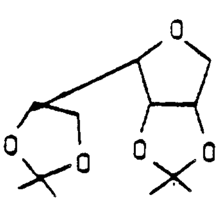

are prepared from the corresponding amino compound by diazotization, followed by coupling with $SO_2$ and HCl in the presence of a copper catalyst. The sulfonyl chlorides produced are converted to the sulfonamides by treatment with ammonia solution. This procedure is outlined in Scheme 4.

Scheme 4

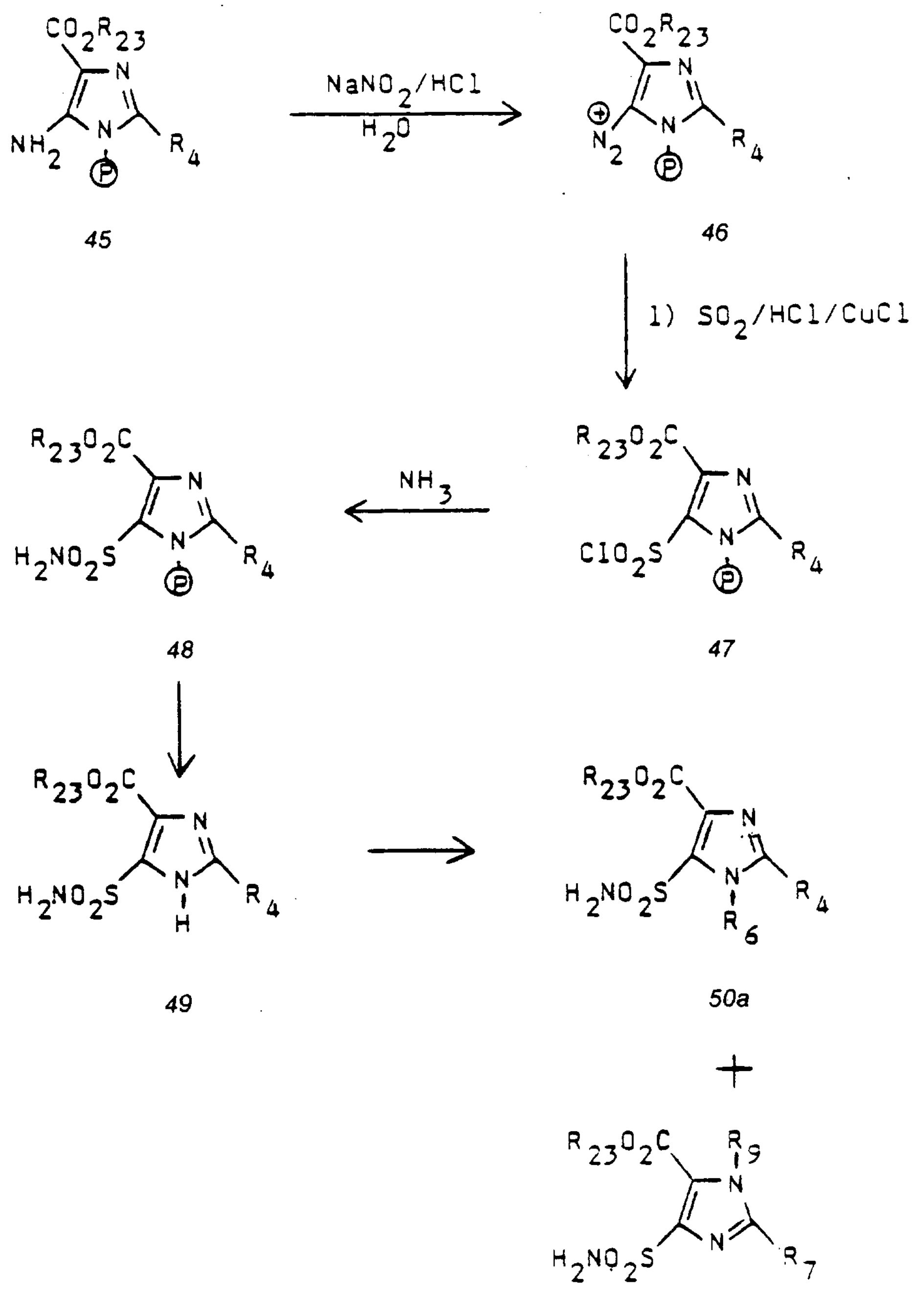


45, 46, 47, 48, 49, 50a, 50b

Protected amino esters *45* and the subsequent diazotization to produce diazonium chlorides *46* is taught in T. Brown, G. Shaw and G. Durant in J. Chem. Soc., Perkins Trans. I, 2304 (1980). The reaction of diazonium chlorides to produce sulfonamides is also outlined in Schemes 1 and 2. See the pertinent references cited there for the necessary details for these transformations.

Intermediates of Formula (c) in which Q is Q-4, Q-5 or Q-6 may be prepared by procedures analogous to those outlined above for the imidazole intermediates. A number of references are available which teach chemical transformations and preparation of pyrazole intermediates needed for this invention. For example:

A. N. Kost and I. I. Groundberg, Advan. Heterocyclic Chem *6*, 347-429 (1966).

T. L. Jacobs, In "Heterocyclic Compounds" (R. C. Elderfield, ed.), Vol. 5, pp. 45-161, Wiley, New York, 1957.

Many of the compounds of Formula (c) in which Q is Q-4 or Q-5, for example *51*, can be made by the procedures outlined in Equation 13 and Scheme 5.

Equation 13

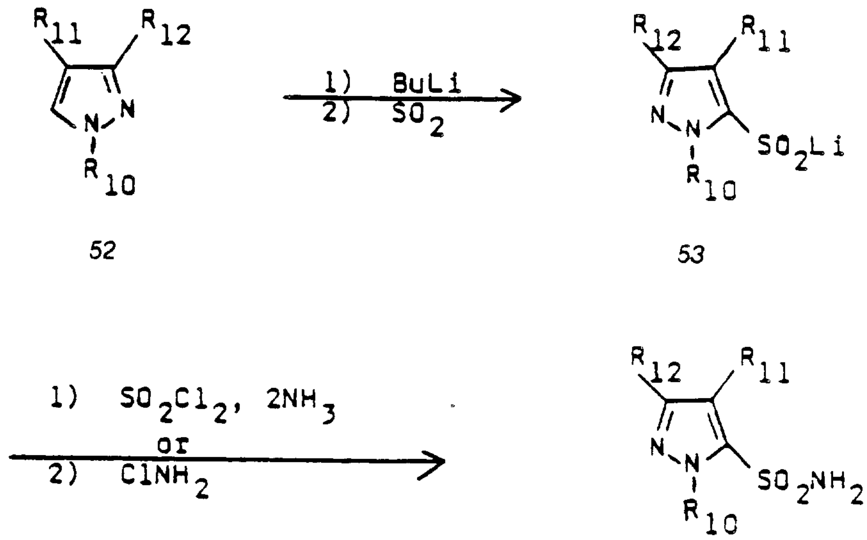


52

53

51

Scheme 5

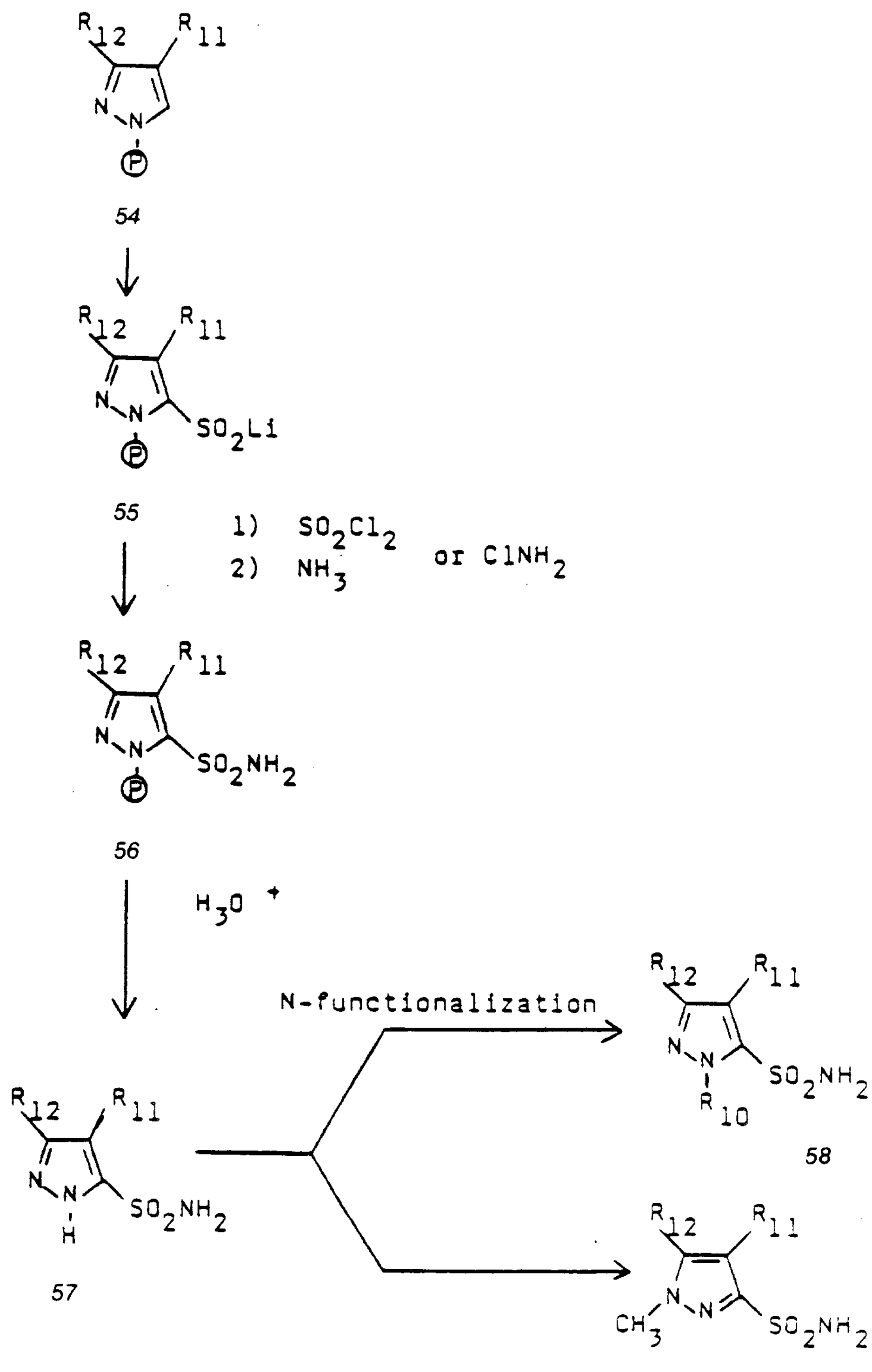


54 55 56 57 58 59

The procedure of Equation 13 is suitable for those substituents which can survive the pyrazole metallation; for example $R_{12}$ is H or $CH_3$, $R_{10}$ is $C_1$-$C_4$ alkyl, $C_3$-$C_4$ alkenyl, $C_3$-$C_4$ alkynyl or $SO_2NR_{20}R_{21}$.

Metallation reactions of pyrazoles are discussed in H. W. Gschwend and H. R. Rodriguez, "Organic Reactions", Vol. 26, p. 23, 24, John Wiley and Sons, Inc., New York, 1979.

For the pyrazoles cited above, as well as pyrazoles with groups sensitive to metallation conditions, the procedure of Scheme 5 can be used. For example, compounds in which $R_{10}$ and $R_{12}$ are as defined for

Formula I and $R_{11}$ is H, $C_1$-$C_3$ alkyl, F, Cl, Br, $OR_{16}$, $S(O)_2NR_{20}R_{21}$ may be prepared, Suitable protecting groups include

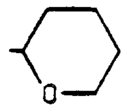 ,

$CH_2OCH_2CH_2OCH_3$ and *t*-butyl.

See the previously cited reference for details.

Compounds of Formulas *58* and *59* in which $R_{12}$ is H or $CH_3$ and $R_{11}$ is F, Cl, Br, $OR_{16}$, $CO_2R_{24}$, $S(O)_mR_{25}$ or $SO_2NR_{20}R_{21}$ and $R_{10}$ is as defined for Example I can also be made from intermediates of Formulas *60* and *61* by using the procedures disclosed for the imidazoles in Schemes 1-4.

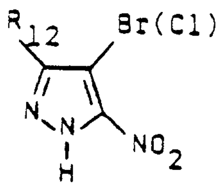

*60*

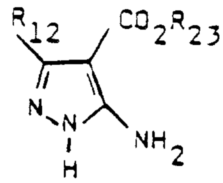

*61*

Compounds of Formula (c) in which *Q* is Q-6 and $R_{13}$ and $R_{14}$ are H, $C_1$-$C_3$ alkyl, $OR_{16}$, F, Cl, Br, $CO_2R_{24}$, $S(O)_mR_{25}$ and $SO_2NR_{20}R_{21}$ and $R_{15}$ is H or $CH_3$ are made by metallation at the appropriate pyrazole intermediate *64* as shown in Scheme 6.

Scheme 6

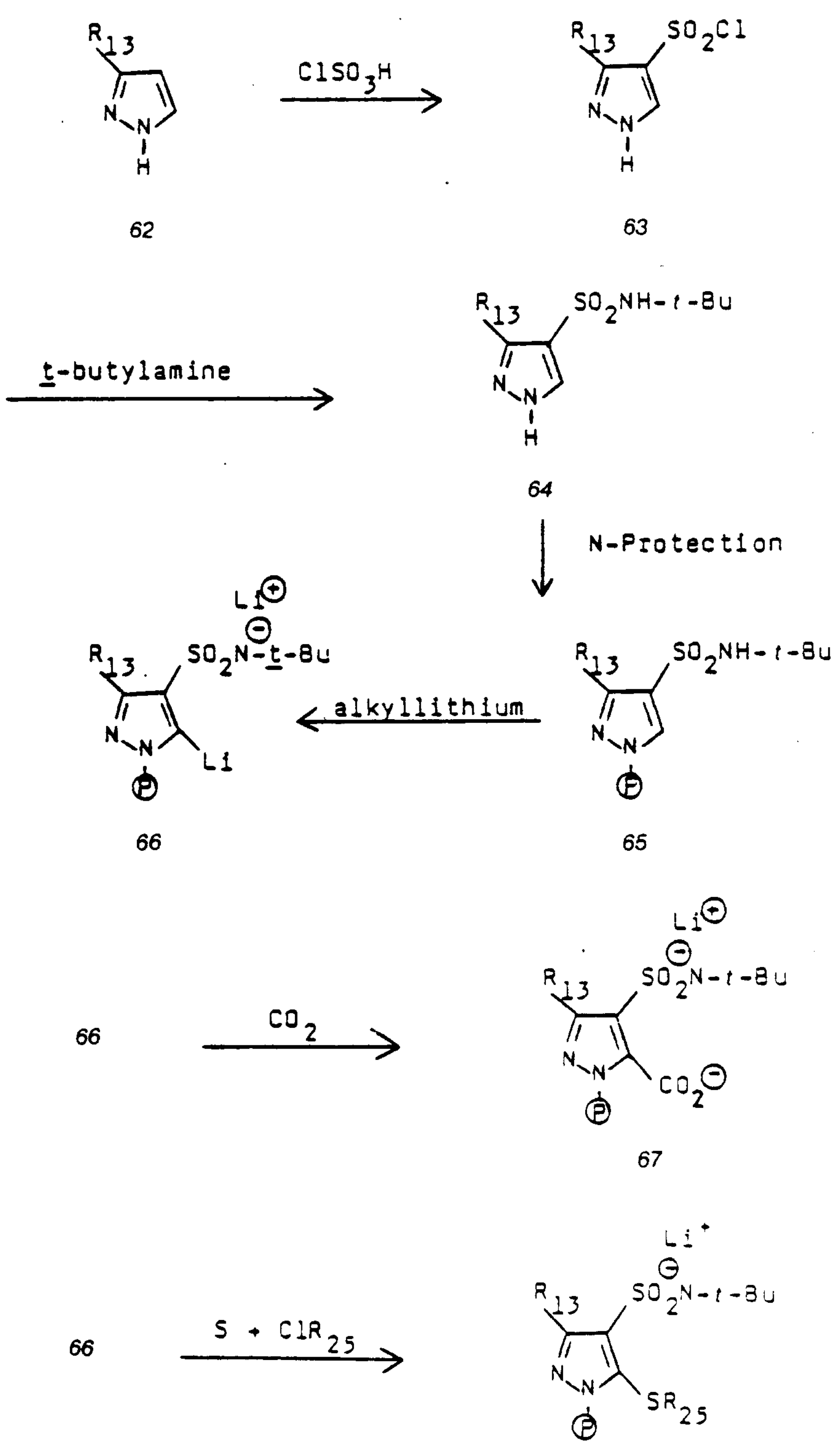


62 63 64 65 66 67 68

66 -

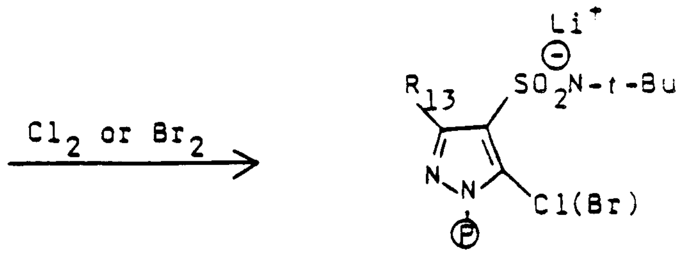


69

66

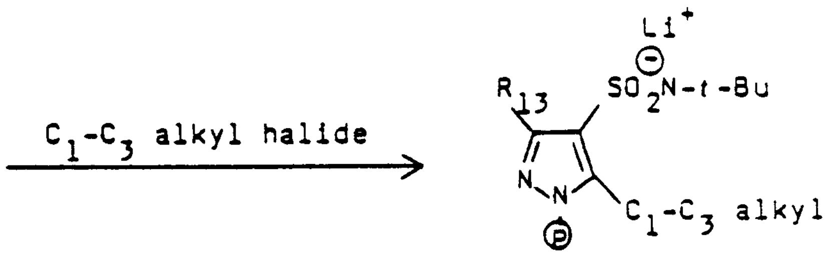


70

66

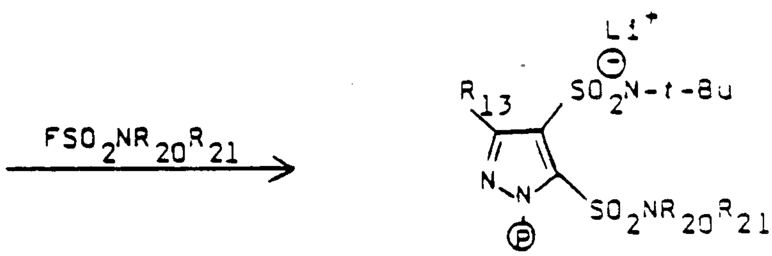


71

The chlorosulfonation of pyrazoles *62* and conversion to sulfonamides *64* in which $R_{13}$ is H, F, Cl, Br or $OR_{16}$ where $R_{16}$ is $C_1$-$C_3$ alkyl is carried out by procedures taught in U.S. Patent 3,665,009.

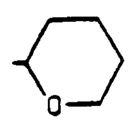 ,

(TPE) or $-CH_2OCH_2CH_2OCH_3$ (MEM).

For details of this procedure with (TPE) see H. W. Gschwend and H. R. Rodriguez "Organic Reactions", Vol. *26*, p. 24, John Wiley and Sons, Inc., New York, 1979. For details of this procedure with (MEM) see E. J. Corey, J. L. Grus and P. Ulrich, Tet. Let., 809 (1976).

The lithiation of protected pyrazoles and subsequent reactions with electrophiles is taught in H. W. Gschwend and H. R. Rodriguez "Organic Reactions", Vol. 26, p. 24, John Wiley and Sons, Inc., New York, 1979.

Further reactions of *67*−*71* or produce the appropriate sulfonamide intermediates of this invention is shown in Equations 14-16.

Equation 14

67

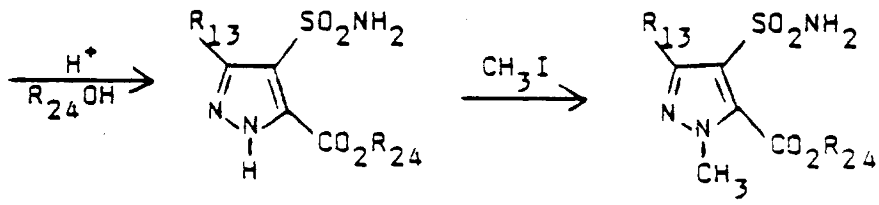


72 73

Deprotection of the sulfonamide and pyrazole is accomplished with an acid such as trifluoroacetic acid. Use of HCl in an alcohol $R_{24}OH$ lead simultaneously to esterification and deprotection to yield sulfonamide *72*. Alkylation of *72* with $CH_3I$ under conditions well known to those skilled in the art leads to additional intermediates *73*.

Alkylthio, alkylsulfinyl and alkylsulfonyl analogs are produced from *68* using the procedures of Equations 15 or 15a. Details of this type of transformation can be found in U.S.S.N. 345,935, filed February 8, 1982.

Equation 15

68

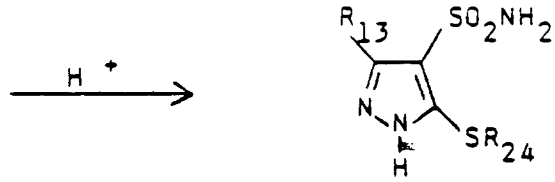


74

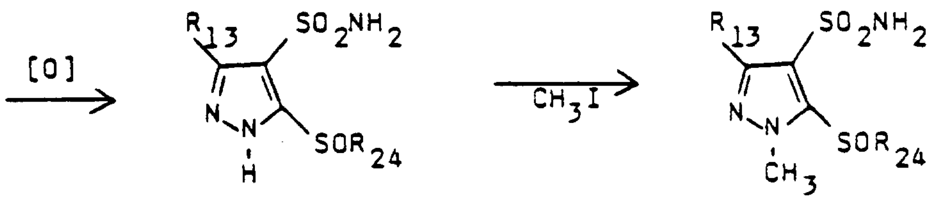


75 76

Equation 15a

68

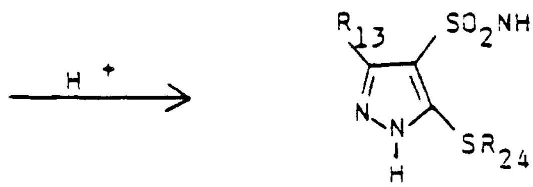


74

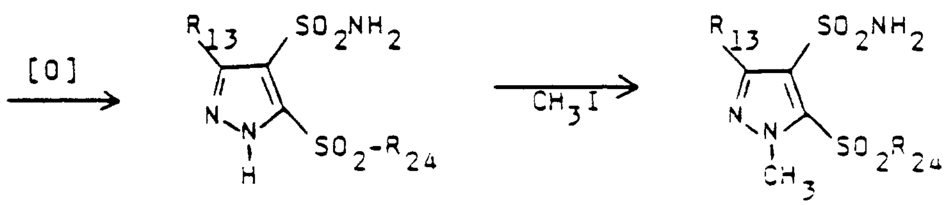


77 78

The halo derivatives *79* and *80* are obtained from *69* by deprotection as shown in Equation 16.

Equation 16

69

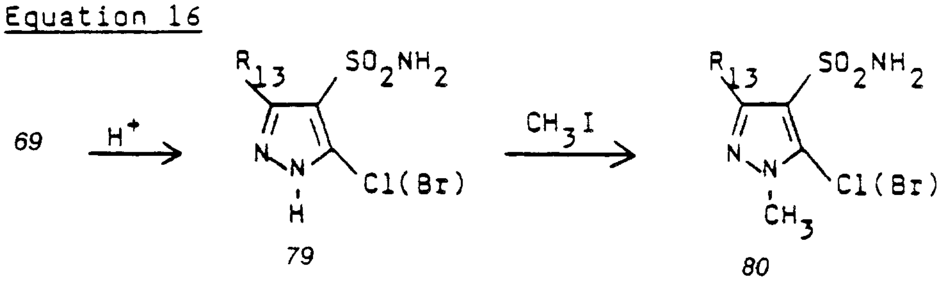


79 80

Treatment of heteroaryllithium reagents with halogens is well known in the art. Details are available in H. W. Gschwend and H. R. Rodriguez "Organic Reactions", Vol. 26, John Wiley and Sons, Inc., New York, 1979. Using similar procedures, the analogous compounds in which $R_{14}$ is $C_1$-$C_3$ alkyl *81* and $SO_2NR_{20}R_{21}$ *82* are produced.

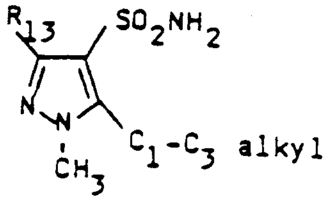

81

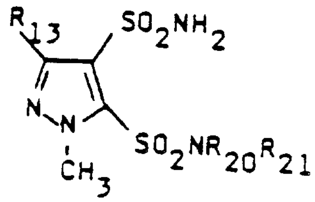

82

Sulfonamides of Formula (c) where Q is Q-7, Q-8, Q-9 or Q-10 can be prepared by amination of the corresponding sulfonyl chlorides of Formula 83 by methods well known in the art. The intermediate sulfonyl chlorides of Formula 83 can be prepared by one of the methods shown in Equation 17.

Equation 17

A. $$\underset{84}{QSCH_2C_6H_5} + Cl_2 \xrightarrow[H_2O]{CH_3CO_2H} \underset{83}{QSO_2Cl}$$

B. $$\underset{85}{QSH} + Cl_2 \xrightarrow[H_2O]{CH_3CO_2H} 83$$

C. $$\underset{86}{QBr} \xrightarrow[2)\ SO_2Cl_2]{1)\ \underline{n}\text{-}C_4H_9Li} 83$$

D. $$\underset{87}{QH} + ClSO_3H \longrightarrow 83$$

E. $$\underset{88}{QNH_2} \xrightarrow[2)\ SO_2,\ CuCl,\ HCl,\ CH_3CO_2H]{1)\ NaNO_2,\ HCl,\ CH_3CO_2H} 83$$

In Equations 17A and 17B, a solution or a suspension of a benzyl sulfide of Formula 84 or a thiol of Formula 85 in aqueous acetic acid is treated with at least three molar equivalents of chlorine at temperatures of about 0 to 15°. The sulfonyl chloride of Formula 83 can be isolated by dilution of the reaction mixture with water, followed by either filtration or extraction with an organic solvent such as ether, methylene chloride or 1-chlorobutane.

In Equation 17C, a bromide of Formula 86 is treated with one molar equivalent of *n*-butyl lithium, in solvents such as ether, tetrahydrofuran or dimethoxyethane, at temperatures of about -78 to -20°. The intermediate lithioheterocycle is added to an excess of sulfuryl chloride in a solvent such as hexane or ether at temperatures of about -20 to 0°. The sulfonyl chloride of Formula 83 can be isolated by addition of water to the reaction mixture, separation of the organic solution, and evaporation of the solvents,

In Equation 17D, a heterocycle of Formula 87 is treated with an excess of chlorosulfonic acid at temperatures of about ambient to the boiling point of chlorosulfonic for periods of from several hours to several days. The sulfonyl chlorides of Formula 83 can be isolated by pouring the reaction mixture into ice

water, and either filtering the product of extracting it into an organic solvent.

In Equation 17E, a heterocyclic amine of Formula 88 is diazotized with sodium nitrite in a mixture of water, concentrated hydrochloric acid and acetic acid at temperatures from about -5 to 10°. The resulting diazonium salt is added to a mixture of sulfur dioxide, cuprous or cupric chloride, concentrated hydrochloric acid and acetic acid, and the reaction mixture is stirred at temperatures from about 0° to ambient. The sulfonyl chloride of Formula 83 can be isolated by diluting the reaction mixture with water and either filtration or extraction with an organic solvent.

Agriculturally suitable salts of compounds of Formula I are also useful herbicides and can be prepared in a number of ways known to the art. For example, metal salts can be made by treating compounds of Formula I with a solution of alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation for another. Cationic exchange can be effected by direct treatment of an aqueous solution of a salt of a compound of Formula I (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium, sodium or calcium salt.

Acid addition salts, useful in this invention, can be obtained by reacting a compound of Formula I or II with a suitable acid, e.g., *p*-toluenesulfonic acid, trichloroacetic acid or the like.

The preparation of the compounds of this invention is further illustrated by the following specific examples. Unless otherwise indicated, temperatures are in degrees centigrade.

Example 1

1-Ethyl-1H-imidazole-2-thiol

52.2 g of Ethylisothiocyanate was added dropwise of 79.8 g of aminoacetaldehyde diethyl acetal in 600 ml of ethanol. After the addition was complete, the mixture was heated to reflux for thirty minutes. The ethanol was removed at reduced pressure on a rotary evaporator. One liter of 10% HCl was added, and the mixture was heated to reflux for thirty minutes and then cooled to room temperature. The pH of the solution was then adjusted to 3 with 50% NAOH. The product was extrated with $CH_2Cl_2$. The extracts were then dried with $MgSO_4$, filtered and concentrated on a rotary evaporator. The crude product was chromatographed on silica with 75% ethyl acetate hexane. 18.4 g of the title compound, a solid with m.p. 71-72°, was obtained.

Example 2

1-Ethyl-1H-imidazole-2-sulfonamide

To 17.0 g 1-ethyl-1H-imidazole-2-thiol in 180 ml 2N HCl cooled to 0 to -8° was added dropwise 19.3 ml of $Cl_2$. The temperature of the reaction was maintained at less than 10° during the addition. The reaction was stirred for about thirty minutes at 0-10° after the addition was complete. Enough 50% NaOH was added dropwise to the reaction while maintaining the temperature, to raise the pH to 4.0. The white precipitate which formed was filtered and added to a mixture of 100 ml concentrated aqueous ammonia cooled to 0 to 10°C. The mixture was stirred for thirty minutes and then concentrated on a rotary evaporator. The concentrate was boiled three times with $CH_3CN$ and the insolubles were filtered and discarded. The $CH_3CN$ extracts were dried over $MgSO_4$, filtered and concentrated on a rotary evaporator.

11.8 g of the title compound, a white solid with m.p. 123-125°, was obtained.

Example 3

1-Ethyl-N-[(4-methoxy-6-methylpyrimidin-2-yl)aminocarbonyl]-1H-imidazole-2-sulfonamide (I; Q = Q-1, $R_1 = C_2H_5$, $R_2 = R_3 = H$; A = A-1, Z = CH, $X = OCH_3$, $Y = CH_3$; R = H)

1.75 g of 1-Ethyl-1H-imidazole-2-sulfonamide in 80 ml of dry $CH_2Cl_2$ at ambient temperature under $N_2$ was treated dropwise with 6.0 ml of a 2.0 M solution in hexane of $(CH_3)_3Al$. The mixture was stirred for a period of fifteen to thirty minutes. 1.97 g of methyl (4-methoxy-6-methylpyrimidin-2-yl)carbamate was then added and the mixture was refluxed for a period of sixteen hours. Enough of a 5% solution of aqueous acetic acid was then added to lower the pH to 3.0. The crude reaction mixture was extracted three times with $CH_2Cl_2$. The extracts were dried with $MgSO_4$, filtered and concentrated on a rotary evaporator. The concentrate was boiled with hot ethyl acetate and the product was filtered to yield 0.8 g of the title compound, a white solid with m.p. 178-179° (d).

Example 4

1-Methylpyrazole-3-sulfonyl chloride

To 120 ml of water and 120 ml of 12N hydrochloric acid was added 45 g of 1-methylpyrazole-3-amine at -10°C followed by the dropwise addition of 33.9 g sodium nitrate dissolved in 75 ml water. The temperature was maintained at -10°C and the mixture was stirred for an additional half hour after the additions were completed.

The above solution was then added portionwise with stirring at -10°C to a mixture containing 348 ml acetic acid, 5.8 g of CuCl and 48 ml (liquified) of sulfur dioxide. After being stirred for one hour at -10°C, the reaction mixture was allowed to warm to 10°C, and was poured into 1500 ml of ice and water. The resultant mixture was extracted three times with 500 ml portions of methylene chloride. The extracts were combined, dried over anhydrous mangesium sulfate, filtered and evaporated *in vacuo* to yield a red oil. This material was used for the preparation of 1-methylpyrazole-3-sulfonamide without further purification.

Example 5

1-Methylpyrazole-3-sulfonamide

A mixture containing chloroform (100 ml), 13.6 g of 1-methylpyrazole-3-sulfonyl chloride and 13.6 g ammonium carbonate was heated to reflux for six hours, cooled, filtered and the solid was washed with water. The water-insoluble solid which was recrystallized from 50% ethanol-water melted at 149-151° and showed absorption peaks in the infrared at 3100 and 3200 $cm^{-1}$, consistent for the $NH_2$ in the desired product.

| | | | | |
|---|---|---|---|---|
| Anal. Calc. for $C_4H_7N_3O_2S$: | C, 29.8; | H, 4.38; | N, 26.07; | S, 19.9. |
| Found: | C, 29.3; | H, 4.6; | N, 25.3; | S, 19.2. |

Example 6

5-Bromo-1-methyl-1H-imidazole-1-sulfonamide.

20 ml of chlorosulfonic acid was added dropwise to 6.0 g of 1H-imidazole-5-bromo-1-methyl with stirring at room temperature. The reaction mixture was heated to 100°C for 16 hours then 140°C for 4 hours. The reaction mixture was cooled to 0°C and poured over cracked ice. The resulting tan solid was collected by filtration and dried under vacuum. This crude sulfonyl chloride (m.p. 84-84°C) was added to 200 ml of anhydrous ammonia and allowed to sit overnight at ambient temperature. Excess ammonia was removed under a flow of nitrogen and the resulting solid was crystallized from 25 ml of water yielding 3.2 g of the desired sulfonamide melting to 204-205.5°C.

Example 7

1,3-Dimethylpyrazole-4-sulfonamide

59 g (0.615 mmol) of 1,3-dimethylpyrazole is added slowly to 300 g (2.5 mmol) of chlorosulfonic acid at 0°C. The reaction is warned to 90°C for 3 hours, cooled and poured onto 500 g of ice. The aqueous solution is extracted with 3 × 100 ml of ether. The organic extract is dried over anhydrous sodium sulfate and filtered. The ether solution is added dropwise to a solution of 55 grams of anhydrous ammonia (2.5 mmol) in 250 ml of ether. After standing overnight, water is added and the layers separated. The aqueous solution is extracted with ethyl acetate, the organic solutions combined and dried over anhydrous sodium sulfate. After removal of the drying agent, the solvent is removed *in vacuo* giving 20.5 g of product, m.p. = 97-105°C.

Example 8

N-[(4-Methoxy-6-methylpyrimidin-2-yl)aminocarbonyl]-1,3-dimethylpyrazol-4-sulfonamide (I; Q=Q-6, $R_{13}=R_{15}=CH_3$, $R_{14}=H$; A=A-1, Z=CH, $X=OCH_3$, $Y=CH_3$; R=H)

1,3-Dimethylpyrazole-4-sulfonamide (1.75 g, 10 mmol) is placed in 100 ml of 1,2-dichloroethane. Trimethylaluminum is toluene (2M, 10 ml) is added. The reaction is brought to reflux and methyl 4-methoxy-6-methyl-2-aminopyrimidine carbamate (2.0 g, 10 mmols) is added. After refluxing overnight, the reaction is cooled, poured into ice water and treated with 60 ml of 10% HCl. The layers are separated, the organic phase extracted with methylene chloride, and the organic solutions combined. The organic solution is dried with anhydrous sodium sulfate and decolorized with charcoal. The solvent is removed *in vacuo*. The solid is triturated with ether and collected giving 1.5 g, m.p. = 207-210°C.

Example 9

4-Chloro-2-thiazolesulfonamide

A. Benzylmercaptan (24.8 g) was added dropwise to a solution of 10.8 g of sodium methoxide in 100 ml ethanol. After stirring 15 minutes, the ethanolic solution of sodium benzylmercaptide was added dropwise to a solution of 30.8 g 2,4-dichlorothiazole (prepared by the method of P. Reynaud et al., *Bull. Soc. Chim. France*, 1735 (1962)], resulting in a temperature increase of 28 to 48°. When the exotherm subsided, the suspension was refluxed 1 hour, then the solvent was evaporated. Cold water (300 ml) was added to the oily residue, and the aqueous mixture was extracted with methylene chloride. The organic solution was washed with water, followed by saturated brine, then dried over magnesium sulfate, filtered and the solvent evaporated to give 43.8 g of 4-chloro-2-phenylmethyl-thiothiazole as a brown oil.

B. A mixture of 43.8 g of 4-chloro-2-phenylthiothiazole, 225 ml acetic acid and 25 ml water was cooled to 4° and 29 ml of liquid chlorine was added dropwise while maintaining the temperature below 7°. When the addition of chlorine was complete, the light yellow solution was stirred 15 minutes at 0° prior to being poured into 1200 ml ice-water. The aqueous mixture was extracted with ether; the organic solution was washed with water, then dried over magnesium sulfate, filtered, and the solvent evaporated. The residual yellow oil was dissolved in 200 ml of tetrahydrofuran, and 50 ml of ammonium hydroxide was added dropwise at 25-30°. The brown reaction mixture was stirred 1 hour at ambient temperature. The reaction mixture was concentrated, diluted with 200 ml water and acidified with acetic acid. The aqueous mixture was extracted with methylene chloride. The organic solution was washed with water, dried over magnesium sulfate, filtered, and the solvent evaporated. Trituration of the resulting oily solid with 300 ml hexane, filtration and washing with hexane gave 7.7 g of the title compound, m.p. 137-141.5°.

NMR ($CDCl_3$/DMSO-$d_6$): $\delta$ 7.5 (s, 1H, 5-CH) and 7.7 (broad s, 2H, $SO_2NH_2$). IR: 3.06 and 3.28 $\mu$ ($SO_2NH_2$).

Example 10

N-[(4,6-Dimethoxypyrimidin-2-yl)aminocarbonyl]-4-chloro-2-thiazolesulfonamide (I; Q = Q-9, $R_{29}$ = Cl, $R_{30}$ = H, Z = CH, X = Y = $OCH_3$)

To a suspension of 1.6 g of 4-chloro-2-thiazolesulfonamide in 100 ml methylene chloride under a nitrogen atmosphere, 4.4 ml of a 2.0*M* solution of trimethylaluminum in toluene was added slowly via a syringe. The reaction mixture was stirred 10 minutes; 1.9 g of methyl N-(4,6-dimethoxypyrimidin-2-yl)-carbamate was added, and the reaction mixture refluxed for 18 hours. The reaction mixture was cooled to 0°. and 55 ml of 5% hydrochloric acid was added dropwise giving a flocculant solid. The reaction mixture was extracted with 100 ml of ethyl acetate. The organic solution was washed with brine, dried over magnesium sulfate, filtered and the solvent evaporated. The resulting tan solid was slurried in l-chlorobutane, collected, washed with hexane and dried giving 1.25 g of the title compound as a white solid, m.p. 182-183° (d). IR: 2.98 and 3.25 μ (NH), 5.80 μ (C = O).

NMR ($CDCl_3$/DMSO-$d_6$): δ 3.90 (s, 6H, $OCH_3$), 5.9 (s, 1H, pyrimidine CH), (s, 1H, thiazole CH), 7.95 (s, 1H thiazole CH) and 11.2 (s, 1H, NH).

| Calcd. for $C_{10}H_{10}ClN_5O_5S_2$: | C-31.62, | H-2.66, | N-18.43. |
|---|---|---|---|
| Found: | C-31.8,<br>C-31.8, | H-2.7,<br>H-2.7, | N-18.5<br>N-18.4. |

Example 11

4-Thiazolesulfonamide

A. Benzylmercaptan (24.8 g) was added slowly to a solution of 10.8 g of sodium methoxide in 100 ml ethanol. After 10 minutes, the reaction mixture was heated to 65° and a solution of 23.8 g of 4-chlorothiazole [prepared by the method of P. Reynaud et al., *Bull. Soc. Chim. France*, 1735 (1962)] in 25 ml ethanol was added dropwise. When addition was complete, the reaction mixture was refluxed 36 hours. The reaction mixture was cooled, and the bulk of the solvent evaporated. Cold water (300 ml) was added to the residue, and the aqueous mixture was extracted with 200 ml ether followed by 200 ml methylene chloride. The combined organic solution was washed with brine, dried over magnesium sulfate, filtered and the solvent evaporated. Distillation of the resulting yellow oil gave 15.6 g of 4-(phenylmethylthio)thiazole, bp 122-134 (0.6 mm).

B. A mixture of 15.6 g of 4-(phenylmethylthio)thiazole in 75 ml acetic and 25 ml water was cooled to -5° and 12.0 ml of liquid chlorine was added dropwise at -5°. When the addition was complete, the yellow solution was stirred 20 minutes at 0°, then poured into 500 ml of ice water. The aqueous mixture was extracted with methylene chloride. The organic solution was washed twice with water, fried over magnesium sulfate, filtered, and the solvent evaporated to give a yellow oil that partially crystallized on cooling. The crude sulfonyl chloride was dissolved in 100 ml of tetrahydrofuran, and 12.0 ml of ammonium hydroxide added dropwise at 5-10°. The resulting brown suspension was allowed to warm to ambient temperature, then concentrated to an oily solid. Addition of 200 ml water and 100 ml methylene chloride to the oily solid gave a solid at the interface. The solid was collected, washed with water followed by hexane and dried giving 3.8 g of the title compound as a brown solid, m.p. 142-146.5°. IR: 3.1 and 3.2 μ ($SO_2NH_2$).

NMR ($CDCl_3$/DMSO-$d_6$): δ 7.15 (broad s, 2H, $SO_2NH_2$), 8.1 (d, 1H, CH) and 8.6 (d, 1H CH).

Example 12

N-[(4-methoxy-6-methylpyrimidin-2-yl)aminocarbonyl]-4-thiazolesulfonamide (I; Q = Q-7, $R_{26}$ = $R_{27}$ = H, Z = CH, X = $OCH_3$, Y = $CH_3$)

By using the procedure described in Example 10, the reaction of 0.98 g of 4-thiazolesulfonamide with 1.3 g of methyl N-(4-methoxy-6-methylpyrimidin-2-yl)-carbamate in the presence of 3.3 ml of a 2.0*M* solution of trimethylaluminum in toluene, there was obtained 0.7 g of the title compound as an off-white solid, m.p. 196-200° (d). IR: 5.89 μ (C = O).

NMR ($CDCl_3/DMSO-d_6$): δ 2.48 (s, 3H, $CH_3$), 3.90 (s, 3H, $OCH_3$), 6.45 (s, 1H, pyrimidine CH), 8.67 (d, 1H, thiazole CH), 9.26 (d, 1H, thiazole CH), 10.5 (s, 1H, NH) and 13.2 (s, 1H, NH).

Example 13

5-Ethoxy-2-methyl-4-thiazolesulfonamide

A solution of 11.1 g of 4-bromo-5-ethoxy-2-methylthiazole [prepared by the method of D. S. Tarbell et al., *J. Amer. Chem. Soc., 72,* 3138 (1950) in 50 ml ether was cooled to -60°, and 35.9 ml of a 1.7*M* solution of *n*-butyl lithium in hexane was added dropwise while maintaining the temperature below -45°. The resulting heavy yellow-brown suspension was diluted with an additional 25 ml ether and stirred 20 minutes at a temperature of -50 to -30°. The ethereal suspension of the lithiothiazole was added via syringe to a solution of 8.0 ml sulfuryl chloride in 50 ml hexane, while maintaining the temperature between -30 and -20°. After the suspension was stirred 15 minutes at -30°, it was allowed to warm to -5° and stirred 3.5 hours. The reaction mixture was poured into 500 ml ice-water. The two-phase suspension was extracted twice with ether. The combined organic solution was washed with water and brine, then dried over magnesium sulfate, filtered, and the solvent evaporated. The resulting oily solid was dissolved in 150 ml of tetrahydrofuran; the solution was cooled to 0°, and 6.7 ml of ammonium hydroxide added dropwise. The brown suspension was allowed to warm to ambient and stirred 3 hours. The solvent was evaporated, and the residual black oil partitioned between methylene chloride and water. The organic solution was washed with water followed by brine, then stirred 0.5 hour with magnesium sulfate and charcoal. The mixture was filtered through Celite® and the solvent was evaporated. Trituration of the resulting brown oily solid with 1-chlorobutane gave 4.2 g of the title compound as a tan solid, m.p. 118-120°. IR: 3.05 and 3.15 μ ($SO_2NH_2$).

NMR ($CDCl_3/DMSO-d_6$): δ 1.25 (t, 3H, $CH_3$), 2.60 (s, 3H, $CH_3$), 4.22 (q, 2H, $OCH_2$) and 6.10 (broad s, 2H, $SO_2NH_2$).

Example 14

5-Ethoxy-N-[(4,6-dimethoxypyrimidin-2-yl)aminocarbonyl]-2-methyl-4-thiazolesulfonamide (I: Q=Q-7, $R_{26}=CH_3$, $R_{27}=OC_2H_5$, Z=CH, X=Y=$OCH_3$)

By employing the procedure described in Example 10, the reaction of 1.33 g of 5-ethoxy-2-methyl-4-thiazolesulfonamide with 1.4 g of methyl N-(4,6-dimethoxypyrimidin-2-yl)carbamate in the presence of 3.3 ml of a 2.0*M* solution of trimethylaluminum, there was obtained 1.2 g of the title compound, m.p. 140-143°. IR: 1710 $cm^{-1}$ (C=O).

NMR ($CDCl_3$): δ 1.5 (t, 3H, $CH_3$), 2.6 (s, 3H, $CH_3$), 3.95 (s, 6H, $OCH_3$), 4.3 (q, 2H, $OCH_2$), 5.8 (s, 1H, pyrimidine $CH_3$), 7.5 (broad s, 1H, NH) and 13.0 (s, 0.67H, NH).

Example 15

Methyl 5-(aminosulfonyl)-4-thiazolecarboxylate

A. A solution of 31.3 g of potassium *t*-butoxide in 280 ml of tetrahydrofuran was cooled to -50° and a solution of 27.7 g of methyl isocyanoacetate in 230 ml of tetrahydrofuran was added dropwise at -50 to -40°. The resulting brown suspension was stirred 15 minutes then cooled to -78°, then a solution of 16.8 ml of carbon disulfide in 225 ml tetrahydrofuran was added dropwise while maintaining the temperature below -50°. The pinkish-brown think suspension was allowed to warm to -15 to 10° over 15 to 20 minutes. To the reaction mixture, a solution of 33.3 ml of benzyl bromide in 140 ml tetrahydrofuran was added dropwise at -15 to -5°. The reaction mixture was stirred at ambient temperature for 30 minutes, refluxed for 20 minutes, then cooled and the bulk of the solvent evaporated. To the oily residue, 1000 ml ice-water was added. After several minutes, the product solidified. The tan solid was collected, washed twice with water, followed by hexane and dried giving 68.9 g of methyl 5-(phenylmethylthio)-4-thiazolecarboxylate, m.p. 90-92°.

B. A solution of 39.8 g of methyl 5-(phenylmethylthio)-4-thiazolecarboxylate in 225 ml acetic acid and 25 ml water was cooled to 3°, and 23.9 ml of liquid chlorine was added dropwise below 10°. The yellow-brown solution was stirred 45 minutes as 5-10° before being poured into 1200 ml of ice-water to give a yellow oil which partially solidified on standing. The crude reaction product was collected, washed with water, then four times with hexane and dried to give 29.4 g of methyl 5-(chlorosulfonyl)-4-thiazolecarbox-

ylate, m.p. 94-97.5°.

C. A solution of 28.9 g of methyl 5-(chlorosulfonyl)-4-thiazolecarboxylate in 150 ml tetrahydrofuran was cooled to 0° and 6.6 ml of liquid ammonia was added dropwise at 0-10°. The suspension was allowed to warm to ambient temperature and stirred 1 hour. The reaction mixture was filtered, the ammonium chloride washed with tetrahydrofuran, and the solvent evaporated from the filtrate. The residual solid was slurried in hexane, collected and dried to give 25.0 g of the title compound, m.p. 138-140°. IR: 3.01 and 3.1 $\mu$ ($SO_2NH_2$) and 5.8 $\mu$ (C=O).

NMR ($CDCl_3$/DMSO-$d_6$): $\delta$ 4.0 (s, 3H, $OCH_3$), 7.2 (broad s, $SO_2NH_2$) and 9.0 (s, 1H, thiazole CH).

Example 16

5-Methoxycarbonyl-4-thiazolesulfonyl isocyanate

A mixture of 11.1 g of 5-(aminosulfonyl)-4-thiazolecarboxylate, 5.6 ml of butyl isocyanate and 0.17 g of diaza[2.2.2]bicyclooctane in 150 ml of xylene was heated to reflux. Phosgene (4.2 ml) was added dropwise in small portions to the reaction mixture, while maintaining the temperature above 130°. When the last 0.5 ml of phosgene was added, the reaction temperature dropped to 124°. The reaction mixture refluxed 1 hour, then the dry-ice condenser replaced with a water condenser, and the mixture fluxed 30 minutes under a slow stream of nitrogen. The reaction mixture cooled, filtered twice under nitrogen, and the solvent evaporated from the filtrate to give the title sulfonyl isocyanate as thick yellow oil. IR: 4.45 $\mu$ ($SO_2NCO$).

The crude sulfonyl isocyanate was dissolved in 50 ml of methylene chloride for reactions with amino heterocycles.

Example 17

Methyl 5-[(4,6-dimethoxypyrimidin-2-yl]aminocarbonyl)aminosulfonyl]-4-thiazolecarboxylate (I; Q=Q-7; $R_{27}=CO_2CH_3$, $R_{26}=H$, Z=CH, X=Y=$OCH_3$).

To a stirred suspension of 0.78 g of 4,6-dimethoxy-2-pyrimidinamine and a crystal of diaza[2.2.2]-bicyclooctane in 5 ml of methylene chloride was added 11 ml of the methylene chloride solution prepared in Example 16. After the addition, a clear solution developed; a precipitate began forming after approximately 30 minutes. The suspension was stirred overnight at ambient temperature. The solid was collected, washed with 1-chlorobutane and dried giving 1.2 g of the title compound, m.p. 172-174°(d). IR: 1721 and 1790 $cm^{-1}$ (2 $\times$ C=O).

NMR ($CDCl_3$/DMSO-$d_6$): $\delta$ 3.97 (s, 3H, $OCH_3$), 4.02 (s, 6H, $OCH_3$), 5.8 (s, 1H, pyrimidine CH), 9.1 (s, 1H thiazole CH), 10.0 (broad s, 1H, NH) and 13.1 (broad s, 1H, NH).

Example 18

Ethyl 4-(aminosulfonyl)-2-methyl-5-thiazolecarboxylate

A. A 5.05 g portion of 50% sodium hydride in mineral oil was washed with hexane and suspended in 100 ml of dimethylformamide. To this suspension, a solution of 19.57 g ethyl 4-hydroxy-2-methyl-5-thiazolecarboxylate in 100 ml of dimethylformamide was added dropwise resulting in the formation of a heavy yellow gel. The reaction mixture was diluted with 75 ml dimethylformamide and stirred for 30 minutes. A solution of 14.23 g of dimethylthiocarbamyl chloride in 50 ml dimethylformamide was added dropwise to the thick slurry, then the reaction mixture was heated at 85-95° for 45 minutes. The resulting brown suspension was cooled and poured into a cold solution of 5.7 g sodium hydroxide in 1200 ml water. The aqueous mixture was extracted once with benzene and twice with ether. The combined organic extracts were washed twice with water followed by brine, then dried over magnesium sulfate, filtered and the solvent evaporated giving an oil that partially crystallized. The crude reaction product was slurried in hexane, collected and dried giving 13.1 g of ethyl 4-[(dimethylamino)thioxomethyloxy]-2-methyl-5-thiazolecarboxylate, m.p. 87-90°.

B. A 12.3 g sample of the compound of Part A was heated at 170-195° under a nitrogen atmosphere for 1.5 hour. The dark reaction mixture was cooled and dissolved in methylene chloride. The solvent was evaporated to give a black oil which crystallized on scratching. Recrystallization from hexane-ethyl acetate give 9.2 g of ethyl 4-[(dimethylamino)carbonylthio]-2-methyl-5-thiazolecarboxylate, m.p. 84.5-85.5°.

C. A 21.5 g sample of the compound prepared as in Part B was added to a solution of 2.7 g of sodium in 200 ml ethanol, and the reaction mixture stirred overnight at ambient temperature. The tan suspension was concentrated, the residue was dissolved in 500 ml water and the aqueous solution acidified with cold, diluted hydrochloric acid. The aqueous mixture was extracted with methylene chloride; the organic solution was dried over magnesium sulfate, filtered and the solvent evaporated. Cold water (200 ml) was added to the oily solid; the resulting solid was collected, washed with water and dried giving 13.4 g of ethyl 4-mercapto-2-methyl-5-thiazolecarboxylate, m.p. 62-66°.

D. A solution of 14.4 g of the compound, prepared as in part B, in a mixture of 80 ml of acetic acid and 20 ml water was cooled to 0°, and 11.3 ml of liquid chlorine was added dropwise at 0-8°. The yellow-brown solution was stirred 30 minutes at approximately 5°, then poured into 500 ml water. The aqueous mixture was extracted twice with ether; the organic solution was washed twice with water, dried over magnesium sulfate, filtered and the solvent evaporated to give an oil. The crude sulfonyl chloride was dissolved in 100 ml tetrahydrofuran and the solution cooled to 5°. Ammonium hydroxide (10.4 ml) was added dropwise; the reaction mixture had a pH <7. More ammonium hydroxide (4.4 ml) was added; the reaction mixture was allowed to warm to ambient temperature and stirred 2 hours. The solvent was evaporated and 300 ml cold water was added to the residual oily solid. The resulting solid was collected, washed with water and dried giving 8.1 g of the title sulfonamide, m.p. 129-131.5°. IR: 3380 and 3225 $cm^{-1}$ ($SO_2NH_2$), 1705 $cm^{-1}$ (C=O).

Example 19

5-Ethoxycarbonyl-2-methyl-4-thiazolesulfonyl isocyanate

A mixture of 7.5 g of the sulfonamide prepared in Example 17, 3.4 ml butyl isocyanate, and a few crystals of diaza[2.2.2]bicyclooctane in 85 ml xylene was heated to 136° and 2.3 ml of phosgene was added dropside. The reaction temperature had dropped to 126° when addition of phosgene was complete. The reaction mixture was refluxed 30 minutes; the dry-ice condenser replaced with a water condenser, and the mixture refluxed an additional 30 minutes under a slow stream of nitrogen. The reaction mixture was cooled and filtered under nitrogen. Evaporation of the solvent gave the title sulfonyl isocyanate as a yellow-brown oil. IR: 2240 $cm^{-1}$ ($SO_2NCO$) and 1760 $cm^{-1}$ (C=O).

The crude isocyanate was dissolved in 25 ml of methylene for reaction with heterocyclic amines.

Example 20

Ethyl 4-[[(4-methoxy-6-ethyl-1,3,5-triazin-2-yl)aminocarbonyl]aminosulfonyl]-2-methyl-5-thiazolecarboxylate (I: Q=Q-8, $R_{26}=CH_3$, $R_{28}=CO_2C_2H_5$, Z=N, $X=OCH_3$, $Y=CH_3$)

A 7 ml portion of the methylene chloride solution of the sulfonyl isocyanate, prepared in Example 18, was added to a stirred suspension of 0.35 g of 4-methoxy-6-methyl-1,3,5-triazin-2-amine and a crystal of diaza[2.2.2]bicyclooctane in methylene chloride. The reaction mixture was stirred overnight. The clear solution was concentrated to a yellow-brown oil. On lengthy trituration with ether, a solid was obtained which was collected, washed with ether and dried to give 0.48 g of the title compound, m.p. 126-130°. IR: 1725 and 1720 $cm^{-1}$ (2 × C=O).

NMR ($CDCl_3$): $\delta$ 1.35 (t, 3H, $CH_3$), 2.6 (s, 3H, $CH_3$), 2.75 (s, 3H, $CH_3$), 4.15 (s, 3H, $OCH_3$), 4.4 (q, 2H, $OCH_2$), 8.2 (broad 2, 1H, NH) and 12.6 (broad s, 1H, NH).

Example 21

3-Chloro-4-isothiazolesulfonamide

A mixture of 20 g of 3-chloroisothiazole and 100 ml of chlorosulfonic acid was refluxed 0.5 hour, cooled and heated at 90° overnight, then refluxed for 2 days. The mixture was cooled and poured slowly onto ice. The aqueous mixture was extracted with ether; the organic solution was washed with water and brine, then dried over magnesium sulfate, filtered and the solvent evaporated. The residual oil was dissolved in ether and treated with gaseous ammonia for 1 hours at 10°. The reaction mixture was filtered and the filtrate evaporated. The residual solid was washed with hexane, collected and dried giving 8.2 g of the title sulfonamide, m.p. 110-116°. IR: 3400 and 3270 $cm^{-1}$ ($SO_2NH_2$).

Example 22

3-Chloro-4-isothiazolesulfonyl isocyanate

A mixture of 4.0 g of the sulfonamide, prepared in Example 21, 8.0 ml of butyl isocyanate, 0.1 g of diaza[2.2.2]bicyclooctane and 40 ml of xylene was heated to reflux and treated dropwise with a solution of 2.4 ml of phosgene in 10 ml of xylene. The reaction mixture was refluxed 4 hours, then cooled, and the solvent was evaporated. The residual crude sulfonyl isocyanate (IR-2250 $cm^{-1}$) was dissolved in 50 ml of methylene chloride.

Example 23

3-Chloro-N-[(4,6-dimethylpyrimidin-2-yl)aminocarbonyl]-4-isothiazolesulfonamide (I; Q = Q-10 $R_{31}$ = Cl, Z = CH, X = Y = $CH_3$)

A 10 ml portion of the methylene chloride solution of 3-chloro-4-isothiazolesulfonyl isocyanate, prepared in Example 22, was added to 0.2 g of 4,6-dimethyl-2-pyrimidinamine, and the mixture stirred 0.5 hour at ambient temperature. The solvent was evaporated and the residual solid triturated with ether, collected and dried giving 0.5 g of the title compound, m.p. 136-140°. IR: 1720 and 1700 $cm^{-1}$ (C = O).

NMR ($CDCl_3$): $\delta$ 2.5 (s, 6H, $CH_3$), 6.8 (s, 1, pyrimidine CH) and 9.5 (s, 1H, isothiazole CH).

Example 24

Methyl 4-(aminosulfonyl)-3-isothiazolecarboxylate

A. A suspension of 52.0 g of 4-amino-3-isothiazolecarboxylic acid hydrochloride [prepared by the method of K. Gewald and P. Billman, *Liebigs Ann.* 1542 (1979)] in 300 ml methanol was cooled in an ice-bath and 10 ml of thionyl chloride was added dropwise. The reaction mixture was refluxed 3 hours, cooled and the solvent evaporated. The residual solid was collected, washed in ether and dried giving 47.5 g of methyl 4-amino-3-isothiazolecarboxylate hydrochloride, m.p. 170° (d).

B. A suspension of 38 g of the amine hydrochloride, prepared in Part A, in 100 ml acetic acid was cooled to 15° and diluted with 100 ml of concentrated hydrochloric acid. The mixture was cooled to 0° and a solution of 16 g of sodium nitrite in 40 ml water was added dropwise over 30 minutes. The reaction mixture was stirred 1 hour at -5 to 0° then added portionwise to a stirred mixture of 5 g of cuprous chloride, 5 ml of concentrated hydrochloric acid, 40 ml of sulfur dioxide and 200 ml of acetic acid at 0°. After 1 hour at approximately 5°, when gas evolution had ceased, the reaction mixture was poured into 400 ml of ice-water and allowed to stand 30 minutes. The resulting solid was collected and dried giving 25.0 g of methyl 4-(chlorosulfonyl)-3-isothiazolecarboxylate, m.p. 42-46°.

C. A solution of 21.4 g of the sulfonyl chloride, prepared in Part B, in 200 ml of tetrahydrofuran was cooled to -70° and treated with 5.5 ml of liquid ammonia. The reaction mixture was allowed to warm to -20 to -10°, stirred 15 minutes, and adjusted to pH 7 with concentrated hydrochloric acid. The resulting solid was collected, washed with tetrahydrofuran and dried giving 18.8 g of the title sulfonamide, m.p. 107-113°.

NMR ($CDCl_3$/DMSO-$d_6$): $\delta$ 4.0 (s, 3H, $OCH_3$), 7.0 (broad s, 2H, $SO_2NH_2$) and 9.3 (s, 1H, isothiazole CH).

Example 25

3-Methoxycarbonyl)-4-isothiazolesulfonyl isocyanate

A mixture of 9.0 g of the sulfonamide prepared in Example 24, 6 ml of butyl isocyanate, 0.2 g of diaza-[2.2.2]bicyclooctane and 150 ml of xylene was heated to 125°, and a solution of 4 ml of phosgene in 6 ml of xylene was added dropwise. A small amount of tar had formed when addition was complete. A 0.2 g portion of diaza[2.2.2]bicyclooctane was added, and the mixture was refluxed 2 hours. The reaction mixture was cooled, decanted from the tar, and the solution evaporated giving the sulfonyl isocyanate as an orange oil. The crude sulfonyl isocyanate was dissolved in 50 ml of methylene chloride for reaction with heterocyclic amines. IR: 2240 $cm^{-1}$ ($SO_2NCO$) and 1735 $cm^{-1}$ (C = O).

Example 26

Methyl 4-[[(4,6-dimethylpyrimidin-2-yl)aminocarbonyl]aminosulfonyl]-3-isothiazolecarboxylate (I: $Q = Q_{10}$, $R_{31} = CO_2CH_3$, Z = CH, X = Y = $CH_3$)

To a solution of 0.5 g of 4,6-dimethyl-2-pyrimidinamine in 10 ml of methylene chloride was added 10 ml of the methylene chloride solution of the sulfonyl isocyanate prepared in Example 24, and the reaction mixture was stirred 15 minutes. The solvent was evaporated, and the residue was triturated with a mixture of methylene chloride and ether. The solid was collected and dried giving 1.0 g of the title compound, m.p. 167° (d). IR: 1735, 1720 and 1700 $cm^{-1}$ (C = O).

NMR ($CDCl_3$/DMSO-$d_6$): $\delta$ 2.4 (s, 6H, $CH_3$), 3.9 (s, 3H, $OCH_3$), 6.9 (s, 1H, pyrimidine CH), 9.8 (s, 1H, isothiazole CH) and 11.8 (broad s, 1H, NH).

By the methods described in Examples 1-26, or modifications thereof, the compounds of Tables I-X may be prepared.

Table Ia

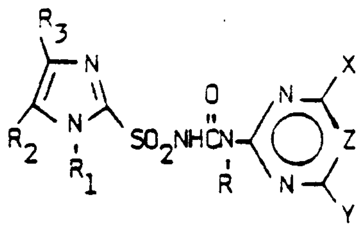

| $R_1$ | $R_2$ | $R_3$ | R | Z | X | Y | m.p.(°C) |
|---|---|---|---|---|---|---|---|
| H | H | H | H | CH | $CH_3$ | $OCH_3$ | |
| $CH_3$- | H | H | H | CH | $CH_3$ | $CH_3$ | 174-178° |
| $CH_3$- | H | H | H | CH | $CH_3$ | $OCH_3$ | 182-183° |
| $CH_3$- | H | H | H | CH | $OCH_3$ | $OCH_3$ | 158-165° |
| $CH_3$- | H | H | H | N | $OCH_3$ | $OCH_3$ | 170-172° |
| $CH_3CH_2$- | H | H | H | CH | $CH_3$ | $CH_3$ | 196-197°(d) |
| $CH_3CH_2$- | H | H | H | CH | $CH_3$ | $OCH_3$ | 178-179°(d) |
| $CH_3CH_2$- | H | H | H | CH | $OCH_3$ | $OCH_3$ | 186-187° |
| $CH_3CH_2$- | H | H | H | N | $CH_3$ | $CH_3$ | 141-143°(d) |
| $CH_3CH_2$- | H | H | H | N | $CH_3$ | $OCH_3$ | |
| $CH_3CH_2$- | H | H | H | N | $OCH_3$ | $OCH_3$ | 160-161° |
| $CH_3CH_2$- | H | H | H | CH | Cl | $OCH_3$ | |
| $CH_3CH_2CH_2$- | H | H | H | CH | $CH_3$ | $CH_3$ | 178-180° |
| $CH_3CH_2CH_2$- | H | H | H | CH | $CH_3$ | $OCH_3$ | 156-159.5° |
| $CH_3CH_2CH_2$- | H | H | H | CH | $OCH_3$ | $OCH_3$ | |
| $CH_3CH_2CH_2$- | H | H | H | CH | Cl | $OCH_3$ | |
| $CH_3CH_2CH_2$- | H | H | H | N | $CH_3$ | $CH_3$ | 122-124° |
| $CH_3CH_2CH_2$- | H | H | H | N | $CH_3$ | $OCH_3$ | |
| $CH_3CH_2CH_2$- | H | H | H | N | $OCH_3$ | $OCH_3$ | 172-174° |
| $(CH_3)_2CH$- | H | H | H | N | $CH_3$ | $CH_3$ | |
| $(CH_3)_2CH$- | H | H | H | N | $OCH_3$ | $OCH_3$ | 170-172° |
| $(CH_3)_2CH$- | H | H | H | N | $OCH_3$ | $CH_3$ | |
| $(CH_3)_2CH$- | H | H | H | CH | $CH_3$ | $CH_3$ | 185-186°(d) |
| $(CH_3)_2CH$- | H | H | H | CH | $OCH_3$ | $OCH_3$ | 166-167° |
| $(CH_3)_2CH$- | H | H | H | CH | $CH_3$ | $OCH_3$ | 157-158°(d) |

Table Ia (continued)

| $R_1$ | $R_2$ | $R_3$ | R | Z | X | Y | m.p.(°C) |
|---|---|---|---|---|---|---|---|
| $(CH_3)_2CH-$ | H | H | H | CH | Cl | $OCH_3$ | |
| $CH_3(CH_2)_3-$ | H | H | H | CH | $CH_3$ | $OCH_3$ | |
| $CH_3(CH_2)_3-$ | H | H | H | CH | $CH_3$ | $CH_3$ | |
| $CH_3(CH_2)_3-$ | H | H | H | CH | $OCH_3$ | $OCH_3$ | |
| $CH_3(CH_2)_3-$ | H | H | H | CH | Cl | $OCH_3$ | |
| $CH_3(CH_2)_3-$ | H | H | H | N | $CH_3$ | $OCH_3$ | |
| $CH_3(CH_2)_3-$ | H | H | H | N | $OCH_3$ | $OCH_3$ | |
| $CH_3-CH-CH_2CH_3$ | H | H | H | CH | $CH_3$ | $OCH_3$ | |
| $(CH_3)_3C-$ | H | H | H | CH | $CH_3$ | $OCH_3$ | |
| $CH_3-CH-CH_2-$<br>$CH_3$ | H | H | H | CH | $CH_3$ | $OCH_3$ | |
| $CH_3(CH_2)_4-$ | H | H | H | CH | $CH_3$ | $OCH_3$ | |
| $CH_3CH-CH_2CH_2-$<br>$CH_3$ | H | H | H | CH | $CH_3$ | $OCH_3$ | |
| $(CH_3)_3CCH_2-$ | H | H | H | CH | $CH_3$ | $OCH_3$ | |
| $CH_3CH_2-CH-CH_2CH_3$ | H | H | H | CH | $CH_3$ | $OCH_3$ | |
| $CH_3CH-CH_2CH_2CH_3$ | H | H | H | CH | $CH_3$ | $OCH_3$ | |
| $CH_3(CH_2)_5-$ | H | H | H | CH | $CH_3$ | $OCH_3$ | |
| $CH_3(CH_2)_3CH-CH_3$ | H | H | H | CH | $CH_3$ | $OCH_3$ | |
| $CH_3(CH_2)_2CHCH_2-CH_3$ | H | H | H | CH | $CH_3$ | $OCH_3$ | |
| $CH_3CH=CH-CH_2-$ | H | H | H | CH | $CH_3$ | $OCH_3$ | |
| $CH_3C=CH_2$ | H | H | H | CH | $CH_3$ | $OCH_3$ | |
| $CH_3CH=CH-CH_2-CH_2-$ | H | H | H | CH | $CH_3$ | $OCH_3$ | |
| $CH_2=CH-CH-CH_2-CH_2CH_2$ | H | H | H | CH | CH | OCH | |
| $CH\equiv C-CH_2-CH_2-$ | H | H | H | CH | $CH_3$ | $OCH_3$ | |
| $CH\equiv C-CH-CH_3$ | H | H | H | CH | $CH_3$ | $OCH_3$ | |
| $CH\equiv C-CH-CH_2CH_3$ | H | H | H | CH | $CH_3$ | $OCH_3$ | |
| $CH_3-C\equiv C-CH-CH_2CH_3$ | H | H | H | CH | $CH_3$ | $OCH_3$ | |

Table Ia (continued)

| $R_1$ | $R_2$ | $R_3$ | R | Z | X | Y | m.p.(°C) |
|---|---|---|---|---|---|---|---|
| $CH_3(CH_2)_7$- | H | H | H | CH | $CH_3$ | $OCH_3$ | |
| $CH_3(CH_2)_6$- | H | H | H | CH | $CH_3$ | $OCH_3$ | |
| 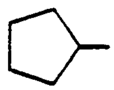 | H | H | H | CH | $CH_3$ | $OCH_3$ | |
| 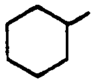 | H | H | H | CH | $CH_3$ | $OCH_3$ | |
| 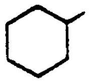 | H | H | H | CH | $CH_3$ | $CH_3$ | 182-184°(d) |
| 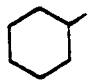 | H | H | H | CH | $OCH_3$ | $OCH_3$ | 131-134°(d) |
| 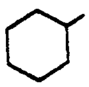 | H | H | H | N | $OCH_3$ | $OCH_3$ | 172-174°(d) |
| 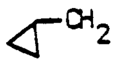 | H | H | H | CH | $CH_3$ | $OCH_3$ | |
| 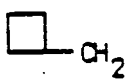 | H | H | H | CH | $CH_3$ | $OCH_3$ | |
| 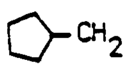 | H | H | H | CH | $CH_3$ | $OCH_3$ | |
| 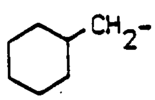 | H | H | H | CH | $CH_3$ | $OCH_3$ | |
| 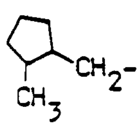 | H | H | H | CH | $CH_3$ | $OCH_3$ | |
| $CH_2=CH-CH_2$- | H | H | H | CH | $CH_3$ | $OCH_3$ | |
| $CH_2=CH-CH_2$- | H | H | H | CH | $CH_3$ | $CH_3$ | |
| $CH_2=CH-CH_2$- | H | H | H | CH | $OCH_3$ | $OCH_3$ | |
| $CH_2=CH-CH_2$- | H | H | H | CH | Cl | $OCH_3$ | |
| $CH_2=CH-CH_2$- | H | H | H | N | $CH_3$ | $OCH_3$ | |
| $CH_2=CH-CH_2$- | H | H | H | N | $OCH_3$ | $OCH_3$ | |

Table Ia (continued)

| $R_1$ | $R_2$ | $R_3$ | R | Z | X | Y | m.p.(°C) |
|---|---|---|---|---|---|---|---|
| $CH_2$=CH-$CH_2$- | H | H | H | N | $CH_3$ | $CH_3$ | |
| -$CH_2$-C≡CH | H | H | H | CH | $CH_3$ | $OCH_3$ | |
| -$CH_2$-C≡CH | H | H | H | CH | $CH_3$ | $CH_3$ | |
| -$CH_2$-C≡CH | H | H | H | CH | $OCH_3$ | $OCH_3$ | |
| -$CH_2$-C≡CH | H | H | H | CH | Cl | $OCH_3$ | |
| -$CH_2$-C≡CH | H | H | H | N | $CH_3$ | $CH_3$ | |
| -$CH_2$-C≡CH | H | H | H | N | $OCH_3$ | $OCH_3$ | |
| -$CH_2$-C≡CH | H | H | H | N | $CH_3$ | $OCH_3$ | |
| $CF_3$ | H | H | H | CH | $CH_3$ | $OCH_3$ | |
| $CH_2CF_3$ | H | H | H | CH | $CH_3$ | $OCH_3$ | |
| $CF_2CH_3$ | H | H | H | CH | $CH_3$ | $OCH_3$ | |
| $CF_3$-C-$CH_3$ | H | H | H | CH | $CH_3$ | $OCH_3$ | |
| $CFH_2$-CH-$CH_3$ | H | H | H | CH | $CH_3$ | $OCH_3$ | |
| $CF_2$H-$CH_2$-$CH_2$- | H | H | H | CH | $CH_3$ | $OCH_3$ | |
| $CH_2$Cl-CH-$CH_3$ | H | H | H | CH | $CH_3$ | $OCH_3$ | |
| $CHCl_2$-CH-$CH_3$ | H | H | H | CH | $CH_3$ | $OCH_3$ | |
| $CCl_3$-$CH_2$-$CH_2$- | H | H | H | CH | $CH_3$ | $OCH_3$ | |
| $CH_2BrCH_2$-$CH_2$- | H | H | H | CH | $CH_3$ | $OCH_3$ | |
| $CF_3CH_2CH_2CH_2$- | H | H | H | CH | $CH_3$ | $OCH_3$ | |
| $H_2CF(CH_2)_4$- | H | H | H | CH | $CH_3$ | $OCH_3$ | |
| $H_2CF(CH_2)_5$- | H | H | H | CH | $CH_3$ | $OCH_3$ | |
| $CH_3OCH_2CH_2$- | H | H | H | CH | $CH_3$ | $OCH_3$ | |
| $CH_3CH_2OCH_2$- | H | H | H | CH | $CH_3$ | $OCH_3$ | |
| $CH_3OCH_2$- | H | H | H | CH | $CH_3$ | $OCH_3$ | |
| $(CH_3)_2CH$-$OCH_2CH_2$- | H | H | H | CH | $CH_3$ | $OCH_3$ | |
| $CH_3CH_2CH_2OCH_2$- | H | H | H | CH | $CH_3$ | $OCH_3$ | |
| -$CH_2COCH_3$ | H | H | H | CH | $CH_3$ | $OCH_3$ | |
| -$CH_2COCH_2CH_3$ | H | H | H | CH | $CH_3$ | $OCH_3$ | |
| -$CH_2COCH(CH_3)_2$ | H | H | H | CH | $CH_3$ | $OCH_3$ | |

Table Ia (continued)

| $R_1$ | $R_2$ | $R_3$ | R | Z | X | Y | m.p.(°C) |
|---|---|---|---|---|---|---|---|
| $-\overset{O}{\overset{\|}{C}}-CH_3$ | H | H | H | CH | $CH_3$ | $OCH_3$ | |
| $-\overset{O}{\overset{\|}{C}}-CH_2CH_3$ | H | H | H | CH | $CH_3$ | $OCH_3$ | |
| $-\overset{O}{\overset{\|}{C}}-CH(CH_3)_2$ | H | H | H | CH | $CH_3$ | $OCH_3$ | |
| $-\underset{CH_3}{\underset{\|}{CH}}-CO-CH_3$ | H | H | H | CH | $CH_3$ | $OCH_3$ | |
| $-CH_2-\overset{O}{\overset{\|}{C}}-OCH_3$ | H | H | H | CH | $CH_3$ | $OCH_3$ | |
| $-CH_2-\overset{O}{\overset{\|}{C}}-OCH_2CH_3$ | H | H | H | CH | $CH_3$ | $OCH_3$ | |
| $-CH_2-\overset{O}{\overset{\|}{C}}-OCH_2CH_2CH_3$ | H | H | H | CH | $CH_3$ | $OCH_3$ | |
| $-CH_2-\overset{O}{\overset{\|}{C}}-OCH(CH_3)_2$ | H | H | H | CH | $CH_3$ | $OCH_3$ | |
| $-CH_2-\overset{O}{\overset{\|}{C}}-O(CH_2)_3CH_3$ | H | H | H | CH | $CH_3$ | $OCH_3$ | |
| $-CH_2-\overset{O}{\overset{\|}{C}}-OCH_2CH(CH_3)_2$ | H | H | H | CH | $CH_3$ | $OCH_3$ | |
| $-CH_2-\overset{O}{\overset{\|}{C}}-O-CH(CH_3)CH_2CH_3$ | H | H | H | CH | $CH_3$ | $OCH_3$ | |
| $-\underset{CH_3}{\underset{\|}{CH}}-\overset{O}{\overset{\|}{C}}-OCH_3$ | H | H | H | CH | $CH_3$ | $OCH_3$ | |
| $-\overset{O}{\overset{\|}{C}}-OCH_3$ | H | H | H | CH | $CH_3$ | $OCH_3$ | |
| $-\overset{O}{\overset{\|}{C}}-OCH_2CH_3$ | H | H | H | CH | $CH_3$ | $OCH_3$ | |
| $-\overset{O}{\overset{\|}{C}}-OCH_2CH_2CH_3$ | H | H | H | CH | $CH_3$ | $OCH_3$ | |

Table Ia (continued)

| $R_1$ | $R_2$ | $R_3$ | R | Z | X | Y | m.p.(°C) |
|---|---|---|---|---|---|---|---|
| $-\overset{O}{\overset{\parallel}{C}}-OCH(CH_3)_2$ | H | H | H | CH | $CH_3$ | $OCH_3$ | |
| $-\overset{O}{\overset{\parallel}{C}}-O(CH_2)_3CH_3$ | H | H | H | CH | $CH_3$ | $OCH_3$ | |
| $-\overset{O}{\overset{\parallel}{C}}OCH_2-CH(CH_3)_2$ | H | H | H | CH | $CH_3$ | $OCH_3$ | |
| $-\overset{O}{\overset{\parallel}{C}}OCH(CH_3)CH_2CH_3$ | H | H | H | CH | $CH_3$ | $OCH_3$ | |
| $-CH_2-\overset{O}{\overset{\parallel}{C}}-OCH_2CH=CH_2$ | H | H | H | CH | $CH_3$ | $OCH_3$ | |
| $-\underset{CH_3}{\underset{\vert}{CH}}-\overset{O}{\overset{\parallel}{C}}-OCH_2CH=CH_2$ | H | H | H | CH | $CH_3$ | $OCH_3$ | |
| $-\overset{O}{\overset{\parallel}{C}}-OCH_2CH=CH_2$ | H | H | H | CH | $CH_3$ | $OCH_3$ | |
| $-\overset{O}{\overset{\parallel}{C}}-O-CH_2-CH=CH-CH_3$ | H | H | H | CH | $CH_3$ | $OCH_3$ | |
| $-CH_2-\overset{O}{\overset{\parallel}{C}}-OCH_2C\equiv CH$ | H | H | H | CH | $CH_3$ | $OCH_3$ | |
| $-\underset{CH_3}{\underset{\vert}{CH}}-\overset{O}{\overset{\parallel}{C}}-OCH_2C\equiv CH$ | H | H | H | CH | $CH_3$ | $OCH_3$ | |
| $-\overset{O}{\overset{\parallel}{C}}-OCH_2C\equiv CH$ | H | H | H | CH | $CH_3$ | $OCH_3$ | |
| $-\overset{O}{\overset{\parallel}{C}}-OCH_2-C\equiv C-CH_3$ | H | H | H | CH | $CH_3$ | $OCH_3$ | |
| $-\overset{O}{\overset{\parallel}{C}}-OCH_2CH_2Cl$ | H | H | H | CH | $CH_3$ | $OCH_3$ | |
| $-\overset{O}{\overset{\parallel}{C}}-OCH_2CH_2OCH_3$ | H | H | H | CH | $CH_3$ | $OCH_3$ | |
| $-\overset{O}{\overset{\parallel}{C}}-SCH_3$ | H | H | H | CH | $CH_3$ | $OCH_3$ | |

Table Ia (continued)

| $R_1$ | $R_2$ | $R_3$ | R | Z | X | Y | m.p.(°C) |
|---|---|---|---|---|---|---|---|
| $-\overset{O}{\overset{\|}{C}}-S-(CH_2)_3CH_3$ | H | H | H | CH | $CH_3$ | $OCH_3$ | |
| $-\overset{O}{\overset{\|}{C}}-S-CH_2-CH=CH_2$ | H | H | H | CH | $CH_3$ | $OCH_3$ | |
| $-\overset{O}{\overset{\|}{C}}-S-CH_2-CH=CH-CH_3$ | H | H | H | CH | $CH_3$ | $OCH_3$ | |
| $-\overset{O}{\overset{\|}{C}}-N(CH_3)_2$ | H | H | H | CH | $CH_3$ | $OCH_3$ | |
| $-\overset{O}{\overset{\|}{C}}-N(CH_3)CH_2CH_3$ | H | H | H | CH | $CH_3$ | $OCH_3$ | |
| $-\overset{O}{\overset{\|}{C}}-N(CH_2CH_3)_2$ | H | H | H | CH | $CH_3$ | $OCH_3$ | |
| $-CH_2-\overset{O}{\overset{\|}{C}}-N(CH_3)_2$ | H | H | H | CH | $CH_3$ | $OCH_3$ | |
| $-CH_2-SO_2-N(CH_3)_2$ | H | H | H | CH | $CH_3$ | $OCH_3$ | |
| $-\underset{CH_3}{\underset{\|}{CH}}-SO_2-N(CH_2CH_3)_2$ | H | H | H | CH | $CH_3$ | $OCH_3$ | |
| $-CH_2-SO_2-N-(CH_3)CH_2CH_3$ | H | H | H | CH | $CH_3$ | $OCH_3$ | |
| $-SO_2-N(CH_3)_2$ | H | H | H | CH | $CH_3$ | $OCH_3$ | |
| $-SO_2N(CH_3)(CH_2CH_3)$ | H | H | H | CH | $CH_3$ | $OCH_3$ | |
| $-SO_2N(CH_2CH_3)_2$ | H | H | H | CH | $CH_3$ | $OCH_3$ | |
| $-CH_2-SO_2CH_3$ | H | H | H | CH | $CH_3$ | $OCH_3$ | |
| $-CH_2SO_2CF_3$ | H | H | H | CH | $CH_3$ | $OCH_3$ | |
| $-CH_2SO_2CH_2CH_3$ | H | H | H | CH | $CH_3$ | $OCH_3$ | |
| $-CH_2SO_2CH_2CH_2CH_3$ | H | H | H | CH | $CH_3$ | $OCH_3$ | |
| $-SO_2CH_3$ | H | H | H | CH | $CH_3$ | $OCH_3$ | |
| $-SO_2CF_3$ | H | H | H | CH | $CH_3$ | $OCH_3$ | |
| $-SO_2CH_2CH_3$ | H | H | H | CH | $CH_3$ | $OCH_3$ | |
| $-SO_2CH_2CH_2CH_3$ | H | H | H | CH | $CH_3$ | $OCH_3$ | |
| $-SO_2-CH(CH_3)_2$ | H | H | H | CH | $CH_3$ | $OCH_3$ | |
| $-\underset{CH_3}{\underset{\|}{CH}}-SO_2CH_3$ | H | H | H | CH | $CH_3$ | $OCH_3$ | |

Table Ia (continued)

| $R_1$ | $R_2$ | $R_3$ | R | Z | X | Y | m.p.(°C) |
|---|---|---|---|---|---|---|---|
| $-CH_2-SO_2CH(CH_3)_2$ | H | H | H | CH | $CH_3$ | $OCH_3$ | |
| $-SO_2CH_3$ | H | H | H | CH | $CH_3$ | $CH_3$ | |
| $-SO_2CH_3$ | H | H | H | CH | $OCH_3$ | $OCH_3$ | |
| $-SO_2CH_3$ | H | H. | H | CH | Cl | $OCH_3$ | |
| $-SO_2CF_3$ | H | H | H | CH | $OCH_3$ | $OCH_3$ | |
| $-SO_2CF_3$ | H | H | H | CH | Cl | $OCH_3$ | |
| $-CH_2SO_2CH_3$ | H | H | H | CH | $OCH_3$ | $OCH_3$ | |
| $-CH_2SO_2CH_3$ | H | H | H | CH | Cl | $OCH_3$ | |
| 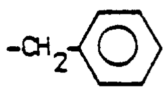 | H | H | H | CH | $CH_3$ | $OCH_3$ | |
| 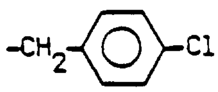 | H | H | H | CH | $CH_3$ | $OCH_3$ | |
| 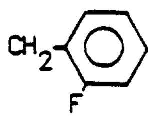 | H | H | H | CH | $CH_3$ | $OCH_3$ | |
| 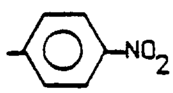 | H | H | H | CH | $CH_3$ | $OCH_3$ | |
| 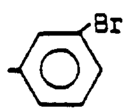 | H | H | H | CH | $CH_3$ | $OCH_3$ | |
| 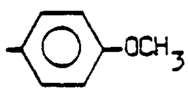 | H | H | H | CH | $CH_3$ | $OCH_3$ | |
| 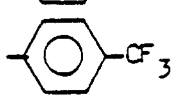 | H | H | H | CH | $CH_3$ | $OCH_3$ | |
| $-CH_2CH_3$ | H | H | $CH_3$ | CH | $CH_3$ | $OCH_3$ | |
| $-CH_2CH_3$ | H | H | $CH_3$ | CH | $OCH_3$ | $OCH_3$ | |
| $-CH_2CH_3$ | H | H | $CH_3$ | CH | $CH_3$ | $CH_3$ | |
| $-CH_2CH_3$ | H | H | $CH_3$ | N | $OCH_3$ | $OCH_3$ | |
| $-CH_2CH_3$ | H | H | $CH_3$ | N | $CH_3$ | $OCH_3$ | |
| $-CH_2CH_3$ | $CH_3$ | H | H | CH | $CH_3$ | $CH_3$ | |
| $-CH_2CH_3$ | H | $CH_3$ | H | CH | $CH_3$ | $CH_3$ | |

Table Ia (continued)

| $R_1$ | $R_2$ | $R_3$ | R | Z | X | Y | m.p.(°C) |
|---|---|---|---|---|---|---|---|
| $-CH_2CH_3$ | $CH_3$ | $CH_3$ | H | CH | $CH_3$ | $CH_3$ | |
| $-CH_2CH_3$ | H | H | H | CH | $CH_3$ | $CH_2CH_3$ | |
| $-CH_2CH_3$ | H | H | H | CH | $CH_3$ | $OCH_2CH_3$ | |
| $-CH_2CH_3$ | H | H | H | N | $CH_3$ | $CH_2OCH_3$ | |
| $-CH_2CH_3$ | H | H | H | N | $CH_3$ | $CH(OCH_3)_2$ | |
| $-CH_2CH_3$ | H | H | H | CH | $OCH_3$ | $-NH_2$ | |
| $-CH_2CH_3$ | H | H | H | N | $OCH_3$ | $CH_3NH-$ | |
| $-CH_2CH_3$ | H | H | H | N | $OCH_3$ | $(CH_3)_2N-$ | |
| $-CH_2CH_3$ | H | H | H | CH | $OCF_2H$ | $CH_3$ | |
| $-CH_2CH_3$ | H | H | H | CH | $OCF_2H$ | $OCH_3$ | |
| $-CH_2CH_3$ | H | H | H | CH | $SCF_2H$ | $CH_3$ | |
| $-CH_2CH_3$ | H | H | H | CH | $SCF_2H$ | $OCH_3$ | |
| $-CH_2CH_3$ | H | H | H | CH | $SCF_2H$ | $OCF_3$ | |
| $-CH_2CH_3$ | H | H | H | CH | $CH_3$ | $OCH_2CF_3$ | |
| $-CH_2CH_3$ | H | H | H | CH | $OCH_3$ | $OCH_2CF_3$ | |
| $-CH_2CH_3$ | H | H | H | CH | $CH_3$ | $OCF_3$ | |
| $-CH_2CH_3$ | H | H | H | CH | $OCH_3$ | $OCF_3$ | |
| $-CH_2CH_3$ | H | H | H | N | $CH_3$ | $OCF_3$ | |
| $-CH_2CH_3$ | H | H | H | CH | $CH_3$ | $OCF_2CHClF$ | |
| $-CH_2CH_3$ | H | H | H | CH | $CH_3$ | $OCF_2CF_2H$ | |
| $-CH_2CH_3$ | H | H | H | CH | $OCH_3$ | $OCF_2CF_2H$ | |
| $-CH_2CH_3$ | H | H | H | N | $CH_3$ | $OCF_2CF_2H$ | |
| $-CH_2CH_3$ | H | H | H | CH | $CH_3$ | $OCF_2CHFCF_3$ | |
| $-CH_2CH_3$ | H | H | H | CH | $OCH_3$ | $OCF_2CHFCF_3$ | |
| $-CH_2CH_3$ | H | H | H | CH | $OCF_2H$ | $CH_2CH_3$ | |
| $-CH_2CH_3$ | H | H | H | CH | $OCF_2H$ | $OCH_2CH_3$ | |
| $-CH_2CH_3$ | H | H | H | CH | $OCF_2H$ | $CH_2OCH_3$ | |
| $-CH_2CH_3$ | H | H | H | CH | $OCF_2H$ | $CH(OCH_3)_2$ | |
| $-CH_2CH_3$ | H | H | H | CH | $OCF_2H$ | $OCH_2CF_3$ | |
| $-CH_2CH_3$ | H | H | H | CH | $OCF_2H$ | $OCF_3$ | |

Table Ia (continued)

| $R_1$ | $R_2$ | $R_3$ | R | Z | X | Y | m.p.(°C) |
|---|---|---|---|---|---|---|---|
| $-CH_2CH_3$ | H | H | H | CH | $OCF_2H$ | $NH_2$ | |
| $-CH_2CH_3$ | H | H | H | CH | $OCF_2H$ | $NHCH_3$ | |
| $-CH_2CH_3$ | H | H | H | CH | $OCF_2H$ | $N(CH_3)_2$ | |
| $-CH_2CH_3$ | H | H | H | CH | $OCF_2H$ | $OCF_2H$ | |
| $-CH_2CH_3$ | H | H | H | CH | $OCF_2H$ | $OCF_2CHClF$ | |
| $-CH_2CH_3$ | H | H | H | CH | $OCF_2H$ | $OCF_2CHBrF$ | |
| $-CH_2CH_3$ | H | H | H | CH | $OCF_2H$ | $OCF_2CF_2H$ | |
| $-CH_2CH_3$ | H | H | H | CH | $OCF_2H$ | $OCF_2CHFCF_3$ | |
| $-CH_2CH_3$ | H | H | H | CH | $CH_3$ | $OCF_2CF_2H$ | |
| $-CH_2CH_3$ | H | H | H | N | $CH_3$ | $OCF_2H$ | |
| $-SO_2N(CH_3)_2$ | H | H | H | CH | $CH_3$ | $CH_3$ | 159-164° |
| $-SO_2N(CH_3)_2$ | H | H | H | CH | $OCH_3$ | $OCH_3$ | 148-155° |
| H | H | H | H | CH | $OCH_3$ | $OCH_3$ | 196-199° |

Table Ib

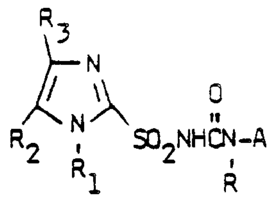

| $R_1$ | $R_2$ | $R_3$ | R | A | $Y^1$ | m.p.(°C) |
|---|---|---|---|---|---|---|
| $-CH_2CH_3$ | H | H | H | A-2 | H | |
| $-CH_2CH_3$ | H | H | H | A-2 | $CH_3$ | |
| $-CH_2CH_3$ | H | H | H | A-2 | Cl | |
| $-CH_2CH_3$ | H | H | H | A-2 | $OCH_3$ | |
| $-CH_2CH_3$ | H | H | H | A-2 | $OCF_2H$ | |
| $-CH_2CH_3$ | H | H | H | A-3 | H | |
| $-CH_2CH_3$ | H | H | H | A-3 | Cl | |
| $-CH_2CH_3$ | H | H | H | A-3 | $CH_3$ | |
| $-CH_2CH_3$ | H | H | H | A-3 | $OCH_3$ | |
| $-CH_2CH_3$ | H | H | H | A-3 | $OCF_2H$ | |
| $-CH_2CH_3$ | H | H | H | A-4 | H | |
| $-CH_2CH_3$ | H | H | H | A-4 | Cl | |
| $-CH_2CH_3$ | H | H | H | A-4 | $CH_3$ | |
| $-CH_2CH_3$ | H | H | H | A-4 | $OCH_3$ | |
| $-CH_2CH_3$ | H | H | H | A-4 | $OCF_2H$ | |

Table Ic

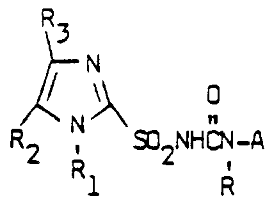

| $R_1$ | $R_2$ | $R_3$ | R | A | $X^2$ | $Y^2$ | $X^3$ | m.p.(°C) |
|---|---|---|---|---|---|---|---|---|
| $-CH_2CH_3$ | H | H | H | A-5 | $OCH_3$ | $CH_3$ | - | |
| $-CH_2CH_3$ | H | H | H | A-5 | $CH_3$ | $CH_2CH_3$ | - | |
| $-CH_2CH_3$ | H | H | H | A-5 | $CH_3$ | $CH_2CF_3$ | - | |
| $-CH_2CH_3$ | H | H | H | A-5 | $OCH_3$ | $CH_3$ | - | |
| $-CH_2CH_3$ | H | H | H | A-5 | $OCH_3$ | $CH_2CH_3$ | - | |
| $-CH_2CH_3$ | H | H | H | A-5 | $OCH_3$ | $CH_2CF_3$ | - | |
| $-CH_2CH_3$ | H | H | H | A-5 | $CH_2CH_3$ | $CH_3$ | - | |
| $-CH_2CH_3$ | H | H | H | A-5 | $CH_2CH_3$ | $CH_2CH_3$ | - | |
| $-CH_2CH_3$ | H | H | H | A-5 | $CH_2CH_3$ | $CH_2CF_3$ | - | |
| $-CH_2CH_3$ | H | H | H | A-5 | $OCH_2CH_3$ | $CH_3$ | - | |
| $-CH_2CH_3$ | H | H | H | A-5 | $OCH_2CH_3$ | $CH_2CH_3$ | - | |
| $-CH_2CH_3$ | H | H | H | A-5 | $OCH_2CH_3$ | $CH_2CF_3$ | - | |
| $-CH_2CH_3$ | H | H | H | A-5 | $SCH_3$ | $CH_3$ | - | |
| $-CH_2CH_3$ | H | H | H | A-5 | $SCH_3$ | $CH_2CH_3$ | - | |
| $-CH_2CH_3$ | H | H | H | A-5 | $SCH_3$ | $CH_2CF_3$ | - | |
| $-CH_2CH_3$ | H | H | H | A-5 | $SCH_2CH_3$ | $CH_3$ | - | |
| $-CH_2CH_3$ | H | H | H | A-5 | $SCH_2CH_3$ | $CH_2CH_3$ | - | |
| $-CH_2CH_3$ | H | H | H | A-5 | $SCH_2CH_3$ | $CH_2CF_3$ | - | |
| $-CH_2CH_3$ | H | H | H | A-6 | - | - | $OCH_3$ | |
| $-CH_2CH_3$ | H | H | H | A-6 | - | - | $CH_3$ | |

## Table IIa

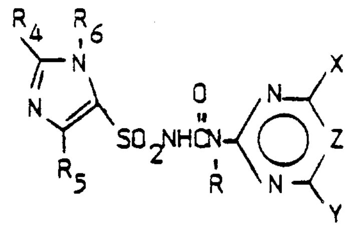

| $R_6$ | $R_5$ | $R_4$ | R | Z | X | Y | m.p.(°C) |
|---|---|---|---|---|---|---|---|
| $-CH_3$ | H | $CH_3$ | H | CH | $CH_3$ | $OCH_3$ | |
| $-CH_3$ | H | H | H | CH | $CH_3$ | $OCH_3$ | 158-163° |
| $CH_3$ | H | H | H | CH | $CH_3$ | $CH_3$ | 171-179° |
| $CH_3CH_2-$ | H | H | H | CH | $CH_3$ | $OCH_3$ | |
| $CH_3CH_2CH_2-$ | H | H | H | CH | $CH_3$ | $OCH_3$ | |
| $(CH_3)_2CH-$ | H | H | H | CH | $CH_3$ | $OCH_3$ | |
| $CH_3CH-CH_2-$<br>$CH_3$ | H | H | H | CH | $CH_3$ | $OCH_3$ | |
| $CH_3CH-CH_2CH_3$ | H | H | H | CH | $CH_3$ | $OCH_3$ | |
| $(CH_3)_3C-$ | H | H | H | CH | $CH_3$ | $OCH_3$ | |
| $CH_3CH{=}CH-$ | H | H | H | CH | $CH_3$ | $OCH_3$ | |
| $-CH_2CH{=}CH_2$ | H | H | H | CH | $CH_3$ | $OCH_3$ | |
| $CH_3-CH{=}CH-CH_2-$ | H | H | H | CH | $CH_3$ | $OCH_3$ | |
| $HC{\equiv}C-CH_2-$ | H | H | H | CH | $CH_3$ | $OCH_3$ | |
| $CH_3-C{\equiv}C-CH_2-$ | H | H | H | CH | $CH_3$ | $OCH_3$ | |
| $CF_3$ | H | H | H | CH | $CH_3$ | $OCH_3$ | |
| $CF_3CH_2-$ | H | H | H | CH | $CH_3$ | $OCH_3$ | |
| $H_2CFCH_2CH_2-$ | H | H | H | CH | $CH_3$ | $OCH_3$ | |
| $ClCH_2CH_2CH_2-$ | H | H | H | CH | $CH_3$ | $OCH_3$ | |
| $HCFClCH_2CH_2CH_2-$ | H | H | H | CH | $CH_3$ | $OCH_3$ | |
| $Br(CH_2)_4-$ | H | H | H | CH | $CH_3$ | $OCH_3$ | |
| $CH_3OCH_2-$ | H | H | H | CH | $CH_3$ | $OCH_3$ | |
| $CH_3OCH_2CH_2-$ | H | H | H | CH | $CH_3$ | $OCH_3$ | |
| $-CO_2CH_3$ | H | H | H | CH | $CH_3$ | $OCH_3$ | |
| $-CO_2CH_2CH_3$ | H | H | H | CH | $CH_3$ | $OCH_3$ | |

Table IIa (continued)

| $R_6$ | $R_5$ | $R_4$ | R | Z | X | Y | m.p.(°C) |
|---|---|---|---|---|---|---|---|
| $-CO_2CH_2CH_2CH_3$ | H | H | H | N | $CH_3$ | $OCH_3$ | |
| $-CO_2CH(CH_3)_2$ | H | H | H | N | $CH_3$ | $OCH_3$ | |
| $-CO_2CH_2CH{=}CH_2$ | H | H | H | N | $CH_3$ | $OCH_3$ | |
| $-CO_2CH_2-C{\equiv}CH$ | H | H | H | N | $CH_3$ | $OCH_3$ | |
| $-CO_2CH_2CH{=}CH-CH_3$ | H | H | H | N | $CH_3$ | $OCH_3$ | |
| $-CO_2CH_2-C{\equiv}C-CH_3$ | H | H | H | N | $CH_3$ | $OCH_3$ | |
| $-CO_2CH_2CH_2OCH_3$ | H | H | H | N | $CH_3$ | $OCH_3$ | |
| $-CO_2CH_2CH_2Cl$ | H | H | H | N | $CH_3$ | $OCH_3$ | |
| $-SO_2N(CH_3)_2$ | H | H | H | N | $CH_3$ | $OCH_3$ | |
| $-SO_2N(CH_3)(CH_2CH_3)$ | H | H | H | N | $CH_3$ | $OCH_3$ | |
| $-SO_2N(CH_2CH_3)_2$ | H | H | H | N | $CH_3$ | $OCH_3$ | |
| $-SO_2CH_3$ | H | H | H | N | $CH_3$ | $OCH_3$ | |
| $-SO_2CF_3$ | H | H | H | N | $CH_3$ | $OCH_3$ | |
| $-SO_2CH_2CH_3$ | H | H | H | N | $CH_3$ | $OCH_3$ | |
| $-SO_2CH_2CH_2CH_3$ | H | H | H | N | $CH_3$ | $OCH_3$ | |
| $-SO_2CH(CH_3)_2$ | H | H | H | N | $CH_3$ | $OCH_3$ | |
| $CH_3CH_2-$ | H | H | H | CH | $CH_3$ | $CH_3$ | |
| $CH_3CH_2-$ | H | H | H | CH | Cl | $OCH_3$ | |
| $CH_3CH_2-$ | H | H | H | CH | $OCH_3$ | $OCH_3$ | |
| $CH_3CH_2-$ | H | H | H | N | $OCH_3$ | $OCH_3$ | |
| $CH_3CH_2-$ | H | H | H | N | $CH_3$ | $OCH_3$ | |
| $CH_3CH_2-$ | H | H | H | N | $CH_3$ | $CH_3$ | |
| $CH_3CH_2-$ | H | H | $CH_3$ | CH | $OCH_3$ | $CH_3$ | |
| $CH_3CH_2-$ | H | H | $CH_3$ | CH | $CH_3$ | $CH_3$ | |
| $CH_3CH_2-$ | H | H | $CH_3$ | CH | $OCH_3$ | $OCH_3$ | |
| $CH_3CH_2-$ | H | H | $CH_3$ | N | $OCH_3$ | $OCH_3$ | |
| $CH_3CH_2-$ | H | H | $CH_3$ | N | $OCH_3$ | $CH_3$ | |
| $CH_3$ | $CH_3$ | H | H | CH | $CH_3$ | $OCH_3$ | |
| $CH_3$ | Cl | H | H | CH | $CH_3$ | $OCH_3$ | |
| $CH_3$ | $CO_2CH_3$ | H | H | CH | $CH_3$ | $OCH_3$ | |
| $CH_3$ | $NO_2$ | H | H | CH | $CH_3$ | $OCH_3$ | |

Table IIa (continued)

| $R_6$ | $R_5$ | $R_4$ | R | Z | X | Y | m.p.(°C) |
|---|---|---|---|---|---|---|---|
| $CH_3$ | $SO_2N(CH_3)_2$ | H | H | CH | $CH_3$ | $OCH_3$ | |
| $-CO_2CH_3$ | $CH_3$ | H | H | CH | $CH_3$ | $OCH_3$ | |
| $-SO_2N(CH_3)_2$ | $CH_3$ | H | H | CH | $CH_3$ | $OCH_3$ | |
| H | F | H | H | CH | $CH_3$ | $OCH_3$ | |
| H | Br | H | H | CH | $CH_3$ | $OCH_3$ | |
| $CH_3$ | $CH_3CH_2$ | H | H | CH | $CH_3$ | $OCH_3$ | |
| $CH_3$ | $CH_3CH_2CH_2$ | H | H | CH | $CH_3$ | $OCH_3$ | |
| $CH_3$ | $CH_3(CH_2)_3-$ | H | H | CH | $CH_3$ | $OCH_3$ | |
| $CH_3$ | $(CH_3)_2CHCH_2-$ | H | H | CH | $CH_3$ | $OCH_3$ | |
| $CH_3$ | $CH_3CHCH_2CH_3$ | H | H | CH | $CH_3$ | $OCH_3$ | |
| $CH_3$ | $-CO_2CH_3$ | H | H | CH | $CH_3$ | $OCH_3$ | |
| $CH_3$ | $-CO_2CH_2CH_3$ | H | H | CH | $CH_3$ | $OCH_3$ | |
| $CH_3$ | $-CO_2CH_2CH_2CH_3$ | H | H | CH | $CH_3$ | $OCH_3$ | |
| $CH_3$ | $-CO_2CH(CH_3)_2$ | H | H | CH | $CH_3$ | $OCH_3$ | |
| $CH_3$ | $SO_2N(CH_3)_2$ | H | H | CH | $OCH_3$ | $OCH_3$ | |
| $CH_3$ | $SO_2N(CH_3)(CH_2CH_3)$ | H | H | CH | $OCH_3$ | $OCH_3$ | |
| $CH_3$ | $SO_2N(CH_2CH_3)$ | H | H | CH | $OCH_3$ | $OCH_3$ | |
| $CH_3$ | $SCH_3$ | H | H | CH | $OCH_3$ | $OCH_3$ | |
| $CH_3$ | $SOCH_3$ | H | H | CH | $OCH_3$ | $OCH_3$ | |
| $CH_3$ | $SO_2CH_2CH_3$ | H | H | CH | $OCH_3$ | $OCH_3$ | |
| $CH_3$ | $SO_2CH_2CH_2CH_3$ | H | H | CH | $OCH_3$ | $OCH_3$ | |
| $CH_3$ | $SCH_2CH_3$ | H | H | CH | $OCH_3$ | $OCH_3$ | |
| $CH_3$ | $SCH_2CH_2CH_3$ | H | H | CH | $OCH_3$ | $OCH_3$ | |
| $CH_3$ | $SO_2CH(CH_3)_2$ | H | H | CH | $OCH_3$ | $OCH_3$ | |
| $CH_3CH_2-$ | H | H | H | CH | $CH_3$ | $CH_3CH_2$ | |
| H | H | H | H | CH | $CH_3$ | $CH_3$ | 240-242 |
| H | H | H | H | CH | $CH_3$ | $OCH_3$ | 207-208.5 |
| H | H | H | H | CH | $OCH_3$ | $OCH_3$ | 211-216 |
| H | H | H | H | N | $CH_3$ | $CH_3$ | 188-193 |
| H | H | H | H | N | $CH_3$ | $OCH_3$ | |
| H | H | H | H | N | $OCH_3$ | $OCH_3$ | 150-158 |

Table IIa (continued)

| $R_6$ | $R_5$ | $R_4$ | R | Z | X | Y | m.p.(°C) |
|---|---|---|---|---|---|---|---|
| $CH_3CH_2$- | H | H | H | CH | $CH_3$ | $-OCH_2CH_3$ | |
| $CH_3CH_2$- | H | H | H | CH | $CH_3$ | $-CH_2OCH_3$ | |
| $CH_3CH_2$- | H | H | H | CH | $CH_3$ | $-CH_2(OCH_3)_2$ | |
| $CH_3CH_2$- | H | H | H | CH | $OCH_3$ | $-NH_2$ | |
| $CH_3CH_2$- | H | H | H | N | $OCH_3$ | $-NHCH_3$ | |
| $CH_3CH_2$- | H | H | H | N | $OCH_3$ | $-N(CH_3)_2$ | |
| $-CH_2CH_3$ | H | H | H | CH | $OCF_2H$ | $CH_3$ | |
| $-CH_2CH_3$ | H | H | H | CH | $OCF_2H$ | $OCH_3$ | |
| $-CH_2CH_3$ | H | H | H | CH | $SCF_2H$ | $CH_3$ | |
| $-CH_2CH_3$ | H | H | H | CH | $SCF_2H$ | $OCH_3$ | |
| $-CH_2CH_3$ | H | H | H | CH | $SCF_2H$ | $OCF_3$ | |
| $-CH_2CH_3$ | H | H | H | CH | $CH_3$ | $OCH_2CF_3$ | |
| $-CH_2CH_3$ | H | H | H | CH | $OCH_3$ | $OCH_2CF_3$ | |
| $-CH_2CH_3$ | H | H | H | CH | $CH_3$ | $OCF_3$ | |
| $-CH_2CH_3$ | H | H | H | CH | $OCH_3$ | $OCF_3$ | |
| $-CH_2CH_3$ | H | H | H | N | $CH_3$ | $OCF_3$ | |
| $-CH_2CH_3$ | H | H | H | CH | $CH_3$ | $OCF_2CHClF$ | |
| $-CH_2CH_3$ | H | H | H | CH | $CH_3$ | $OCF_2CF_2H$ | |
| $-CH_2CH_3$ | H | H | H | CH | $OCH_3$ | $OCF_2CF_2H$ | |
| $-CH_2CH_3$ | H | H | H | N | $CH_3$ | $OCF_2CF_2H$ | |
| $-CH_2CH_3$ | H | H | H | CH | $CH_3$ | $OCF_2CHFCF_3$ | |
| $-CH_2CH_3$ | H | H | H | CH | $OCH_3$ | $OCF_2CHFCF_3$ | |
| $-CH_2CH_3$ | H | H | H | CH | $OCF_2H$ | $CH_2CH_3$ | |
| $-CH_2CH_3$ | H | H | H | CH | $OCF_2H$ | $OCH_2CH_3$ | |
| $-CH_2CH_3$ | H | H | H | CH | $OCF_2H$ | $CH_2OCH_3$ | |
| $-CH_2CH_3$ | H | H | H | CH | $OCF_2H$ | $CH(OCH_3)_2$ | |
| $-CH_2CH_3$ | H | H | H | CH | $OCF_2H$ | $OCH_2CF_3$ | |
| $-CH_2CH_3$ | H | H | H | CH | $OCF_2H$ | $OCF_3$ | |
| $-CH_2CH_3$ | H | H | H | CH | $OCF_2H$ | $NH_2$ | |
| $-CH_2CH_3$ | H | H | H | CH | $OCF_2H$ | $NHCH_3$ | |

Table IIa (continued)

| $R_6$ | $R_5$ | $R_4$ | R | Z | X | Y | m.p.(°C) |
|---|---|---|---|---|---|---|---|
| $-CH_2CH_3$ | H | H | H | CH | $OCF_2H$ | $N(CH_3)_2$ | |
| $-CH_2CH_3$ | H | H | H | CH | $OCF_2H$ | $OCF_2H$ | |
| $-CH_2CH_3$ | H | H | H | CH | $OCF_2H$ | $OCF_2CHClF$ | |
| $-CH_2CH_3$ | H | H | H | CH | $OCF_2H$ | $OCF_2CHBrF$ | |
| $-CH_2CH_3$ | H | H | H | CH | $OCF_2H$ | $OCF_2CF_2H$ | |
| $-CH_2CH_3$ | H | H | H | CH | $OCF_2H$ | $OCF_2CHFCF_3$ | |
| $-CH_2CH_3$ | H | H | H | CH | $CH_3$ | $OCF_2CF_2H$ | |
| $-CH_2CH_3$ | H | H | H | N | $CH_3$ | $OCF_2H$ | |
| H | Br | H | H | CH | $OCH_3$ | $OCH_3$ | 208-215° |
| H | Br | H | H | N | $CH_3$ | $OCH_3$ | 194-201° |
| $CH_3$ | $NO_2$ | H | H | CH | $CH_3$ | $CH_3$ | 215-218° |
| $CH_3$ | Cl | H | H | CH | $CH_3$ | $CH_3$ | 233-235° |
| $CH_3$ | Cl | H | H | CH | $OCH_3$ | $CH_3$ | 204-207° |
| $CH_3$ | Cl | H | H | CH | $OCH_3$ | $OCH_3$ | 170-178° |
| $CH_3$ | Cl | H | H | N | $CH_3$ | $CH_3$ | 200-203° |
| $CH_3$ | Cl | H | H | N | $OCH_3$ | $CH_3$ | 191-193° |
| $CH_3$ | Cl | H | H | N | $OCH_3$ | $OCH_3$ | 194-200° |

Table IIb

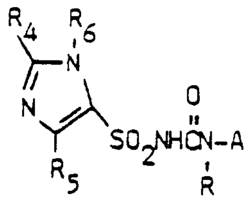

| $R_6$ | $R_5$ | $R_4$ | R | A | $Y^1$ | m.p.(°C) |
|---|---|---|---|---|---|---|
| $-CH_3CH_2$ | H | H | H | A-2 | H | |
| $-CH_3CH_2$ | H | H | H | A-2 | Cl | |
| $-CH_3CH_2$ | H | H | H | A-2 | $CH_3$ | |
| $-CH_3CH_2$ | H | H | H | A-2 | $OCH_3$ | |
| $CH_3CH_2$ | H | H | H | A-2 | $OCF_2H$ | |
| H | $-CO_2CH_3$ | H | H | A-3 | H | |
| H | $-CO_2CH_3$ | H | H | A-3 | Cl | |
| H | $-CO_2CH_3$ | H | H | A-3 | $CH_3$ | |
| H | $-CO_2CH_3$ | H | H | A-3 | $OCH_3$ | |
| $CH_3CH_2$ | H | H | H | A-2 | $OCF_2H$ | |
| H | $SO_2N(CH_3)_2$ | H | H | A-4 | H | |
| H | $SO_2N(CH_3)_2$ | H | H | A-4 | Cl | |
| H | $SO_2N(CH_3)_2$ | H | H | A-4 | $CH_3$ | |
| H | $SO_2N(CH_3)_2$ | H | H | A-4 | $OCH_3$ | |
| $CH_3CH_2$ | H | H | H | A-4 | $OCF_2H$ | |

Table IIc

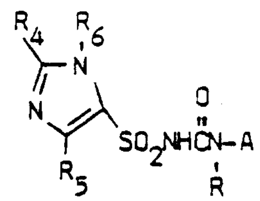

| $R_6$ | $R_5$ | $R_4$ | R | A | $X^2$ | $Y^2$ | $X^3$ | m.p.(°C) |
|---|---|---|---|---|---|---|---|---|
| H | $SO_2CH_3$ | H | H | A-5 | $OCH_3$ | $CH_3$ | - | |
| H | $SO_2CH_3$ | H | H | A-5 | $OCH_3$ | $CH_2CH_3$ | - | |
| H | $SO_2CH_3$ | H | H | A-5 | $OCH_3$ | $CH_2CF_3$ | - | |
| H | $SO_2CH_3$ | H | H | A-5 | $CH_3$ | $CH_3$ | - | |
| H | $SO_2CH_3$ | H | H | A-5 | $CH_3$ | $CH_2CH_3$ | - | |
| H | $SO_2CH_3$ | H | H | A-5 | $CH_3$ | $CH_2CF_3$ | - | |
| H | $SO_2CH_3$ | H | H | A-5 | $CH_2CH_3$ | $CH_3$ | - | |
| H | $SO_2CH_3$ | H | H | A-5 | $CH_2CH_3$ | $CH_2CH_3$ | - | |
| H | $SO_2CH_3$ | H | H | A-5 | $CH_2CH_3$ | $CH_2CF_3$ | - | |
| H | $SO_2CH_3$ | H | H | A-5 | $OCH_2CH_3$ | $CH_3$ | - | |
| H | $SO_2CH_3$ | H | H | A-5 | $OCH_2CH_3$ | $CH_2CH_3$ | - | |
| H | $SO_2CH_3$ | H | H | A-5 | $OCH_2CH_3$ | $CH_2CF_3$ | - | |
| H | $SO_2CH_3$ | H | H | A-5 | $SCH_3$ | $CH_3$ | - | |
| H | $SO_2CH_3$ | H | H | A-5 | $SCH_3$ | $CH_2CH_3$ | - | |
| H | $SO_2CH_3$ | H | H | A-5 | $SCH_3$ | $CH_2CF_3$ | - | |
| H | $SO_2CH_3$ | H | H | A-5 | $SCH_2CH_3$ | $CH_3$ | - | |
| H | $SO_2CH_3$ | H | H | A-5 | $SCH_2CH_3$ | $CH_2CH_3$ | - | |
| H | $SO_2CH_3$ | H | H | A-5 | $SCH_2CH_3$ | $CH_2CF_3$ | - | |
| H | $SO_2CH_3$ | H | H | A-6 | - | - | $OCH_3$ | |
| H | $SO_2CH_3$ | H | H | A-6 | - | - | $CH_3$ | |

Table IIIa

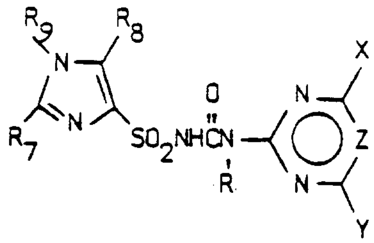

| $R_8$ | $R_9$ | $R_7$ | R | Z | X | Y | m.p.(°C) |
|---|---|---|---|---|---|---|---|
| H | $CH_3$ | $CH_3$ | H | CH | $CH_3$ | $OCH_3$ | |
| H | $CH_3$ | H | H | CH | $CH_3$ | $OCH_3$ | |
| $CH_3$- | $CH_3$ | H | H | CH | $CH_3$ | $OCH_3$ | |
| $CH_3CH_2$- | $CH_3$ | H | H | CH | $CH_3$ | $OCH_3$ | |
| $CH_3CH_2CH_2$- | $CH_3$ | H | H | CH | $CH_3$ | $OCH_3$ | |
| $CH_3(CH_2)_3$- | $CH_3$ | H | H | CH | $CH_3$ | $OCH_3$ | |
| $(CH_3)_2CHCH_2$- | $CH_3$ | H | H | CH | $CH_3$ | $OCH_3$ | |
| $(CH_3)_3C$- | $CH_3$ | H | H | CH | $CH_3$ | $OCH_3$ | |
| $CH_3CHCH_2CH_3$ | $CH_3$ | H | H | CH | $CH_3$ | $OCH_3$ | |
| $CH_2=CH-CH_2$- | $CH_3$ | H | H | CH | $CH_3$ | $OCH_3$ | |
| $CH_3CH=CH-CH_2$- | $CH_3$ | H | H | CH | $CH_3$ | $OCH_3$ | |
| $HC \equiv C-CH_2$- | $CH_3$ | H | H | CH | $CH_3$ | $OCH_3$ | |
| $CH_3C \equiv C-CH_2$- | $CH_3$ | H | H | CH | $CH_3$ | $OCH_3$ | |
| $-OCH_3$ | $CH_3$ | H | H | CH | $CH_3$ | $OCH_3$ | |
| $-OCH_2CH_3$ | $CH_3$ | H | H | CH | $CH_3$ | $OCH_3$ | |
| $-OCH(CH_3)_2$ | $CH_3$ | H | H | CH | $CH_3$ | $OCH_3$ | |
| $-OCH_2CH_2CH_3$ | $CH_3$ | H | H | CH | $CH_3$ | $OCH_3$ | |
| $-NO_2$ | $CH_3$ | H | H | CH | $CH_3$ | $OCH_3$ | |
| -F | $CH_3$ | H | H | CH | $CH_3$ | $OCH_3$ | |
| -Cl | $CH_3$ | H | H | CH | $CH_3$ | $OCH_3$ | |
| $-CO_2CH_3$ | $CH_3$ | H | H | CH | $CH_3$ | $OCH_3$ | |
| $-CO_2CH_2CH_3$ | $CH_3$ | H | H | CH | $CH_3$ | $OCH_3$ | |
| $-CO_2CH_2CH_2CH_3$ | $CH_3$ | H | H | CH | $CH_3$ | $OCH_3$ | |
| $-CO_2CH(CH_3)_2$ | $CH_3$ | H | H | CH | $CH_3$ | $OCH_3$ | |
| $-CO_2CH_2CH=CH_2$ | $CH_3$ | H | H | CH | $CH_3$ | $OCH_3$ | |

Table IIIa (continued)

| $R_8$ | $R_9$ | $R_7$ | R | Z | X | Y | m.p.(°C) |
|---|---|---|---|---|---|---|---|
| $-S-CH_3$ | $CH_3$ | H | H | CH | $CH_3$ | $OCH_3$ | |
| $-SOCH_3$ | $CH_3$ | H | H | CH | $CH_3$ | $OCH_3$ | |
| $-SCH_2CH_3$ | $CH_3$ | H | H | CH | $CH_3$ | $OCH_3$ | |
| $-SCH_2CH_2CH_3$ | $CH_3$ | H | H | CH | $CH_3$ | $OCH_3$ | |
| $-SCH(CH_3)_2$ | $CH_3$ | H | H | CH | $CH_3$ | $OCH_3$ | |
| $-SO_2CH_3$ | $CH_3$ | H | H | CH | $CH_3$ | $OCH_3$ | |
| $-SO_2CH_2CH_3$ | $CH_3$ | H | H | CH | $CH_3$ | $OCH_3$ | |
| $-SO_2CH_2CH_2CH_3$ | $CH_3$ | H | H | CH | $CH_3$ | $OCH_3$ | |
| $-SO_2CH(CH_3)_2$ | $CH_3$ | H | H | CH | $CH_3$ | $OCH_3$ | |
| $-SO_2N(CH_3)_2$ | $CH_3$ | H | H | CH | $CH_3$ | $OCH_3$ | |
| $-SO_2N(CH_3)(CH_2CH_3)$ | $CH_3$ | H | H | CH | $CH_3$ | $OCH_3$ | |
| $-CO_2CH_3$ | $CH_3CH_2-$ | H | H | CH | $CH_3$ | $OCH_3$ | |
| $-CO_2CH_3$ | $CH_3$ | H | H | CH | $CH_3$ | $CH_3$ | |
| $-CO_2CH_3$ | $CH_3$ | H | H | CH | $OCH_3$ | $OCH_3$ | |
| $-CO_2CH_3$ | $CH_3$ | H | H | CH | Cl | $OCH_3$ | |
| $-CO_2CH_3$ | $CH_3$ | H | H | N | $CH_3$ | $CH_3$ | |
| $-CO_2CH_3$ | $CH_3$ | H | H | N | $CH_3O$ | $CH_3$ | |
| $-CO_2CH_3$ | $CH_3$ | H | H | N | $CH_3O$ | $CH_3O$ | |
| $-CO_2CH_3$ | $CH_3$ | H | H | N | $CH_3O$ | $NH_2$ | |
| $-CO_2CH_3$ | $CH_3$ | H | H | N | $CH_3O$ | $NHCH_3$ | |
| $-CO_2CH_3$ | $CH_3$ | H | H | N | $CH_3O$ | $N(CH_3)_2$ | |
| $-CO_2CH_3$ | $CH_3$ | H | H | N | $CH_3$ | $OCH_2CH_3$ | |
| $-CO_2CH_3$ | $CH_3$ | H | H | N | $CH_3$ | $CH_2OCH_3$ | |
| $-CO_2CH_3$ | $CH_3$ | H | H | N | $CH_3$ | $CH(OCH_3)_2$ | |
| H | $CH_3CH_2-$ | H | H | CH | $CH_3$ | $OCH_3$ | 215-216 |
| H | $SO_2N(CH_3)_2$ | H | H | CH | $CH_3$ | $OCH_3$ | 185-188 |
| H | $SO_2N(CH_3)_2$ | H | H | N | $CH_3$ | $OCH_3$ | 118-125 |
| H | $SO_2N(CH_3)_2$ | H | H | CH | $CH_3$ | $CH_3$ | 195-202 |
| H | $CH_3$ | H | H | CH | $CH_3$ | $OCH_3$ | 222-224.5 |
| H | $CH_3$ | H | H | N | $CH_3$ | $CH_3$ | 188-193 |
| Br | $CH_3$ | H | H | CH | $CH_3$ | $CH_3$ | 241-243 |

Table IIIa (continued)

| $R_8$ | $R_9$ | $R_7$ | R | Z | X | Y | m.p.(°C) |
|---|---|---|---|---|---|---|---|
| Br | $CH_3$ | H | H | CH | $CH_3$ | $OCH_3$ | 208-210.5 |
| Br | $CH_3$ | H | H | CH | $OCH_3$ | $OCH_3$ | 205-206 |
| Br | $CH_3$ | H | H | N | $CH_3$ | $CH_3$ | 202.5-204 |
| Br | $CH_3$ | H | H | N | $CH_3$ | $OCH_3$ | 192-194 |
| Br | $CH_3$ | H | H | N | $OCH_3$ | $OCH_3$ | |
| Br | $CH_3$ | H | H | CH | $CH_3$ | $OCF_2H$ | |
| Cl | $CH_3$ | H | H | CH | $CH_3$ | $CH_3$ | 233-235 |
| Cl | $CH_3$ | H | H | CH | $CH_3$ | $OCH_3$ | 204-207 |
| Cl | $CH_3$ | H | H | CH | $OCH_3$ | $OCH_3$ | 170-178 |
| Cl | $CH_3$ | H | H | N | $CH_3$ | $CH_3$ | 200-203 |
| Cl | $CH_3$ | H | H | N | $CH_3$ | $OCH_3$ | 191-193 |
| Cl | $CH_3$ | H | H | N | $OCH_3$ | $OCH_3$ | 194-200 |
| Cl | $CH_3$ | H | H | CH | $CH_3$ | $OCF_2H$ | |
| Br | H | H | H | CH | $CH_3$ | $CH_3$ | 234.5-237 |
| Br | H | H | H | CH | $CH_3$ | $OCH_3$ | |
| Br | H | H | H | CH | $OCH_3$ | $OCH_3$ | 208-215 |
| Br | H | H | H | N | $CH_3$ | $CH_3$ | |
| Br | H | H | H | N | $CH_3$ | $OCH_3$ | |
| Br | H | H | H | N | $OCH_3$ | $OCH_3$ | |
| $-CH_2CH_3$ | $CH_3$ | H | H | CH | $OCF_2H$ | $CH_3$ | |
| $-CH_2CH_3$ | $CH_3$ | H | H | CH | $OCF_2H$ | $OCH_3$ | |
| $-CH_2CH_3$ | $CH_3$ | H | H | CH | $SCF_2H$ | $CH_3$ | |
| $-CH_2CH_3$ | $CH_3$ | H | H | CH | $SCF_2H$ | $OCH_3$ | |
| $-CH_2CH_3$ | $CH_3$ | H | H | CH | $SCF_2H$ | $OCF_3$ | |
| $-CH_2CH_3$ | $CH_3$ | H | H | CH | $CH_3$ | $OCH_2CF_3$ | |
| $-CH_2CH_3$ | $CH_3$ | H | H | CH | $OCH_3$ | $OCH_2CF_3$ | |
| $-CH_2CH_3$ | $CH_3$ | H | H | CH | $CH_3$ | $OCF_3$ | |
| $-CH_2CH_3$ | $CH_3$ | H | H | CH | $OCH_3$ | $OCF_3$ | |
| $-CH_2CH_3$ | $CH_3$ | H | H | N | $CH_3$ | $OCF_3$ | |
| $-CH_2CH_3$ | $CH_3$ | H | H | CH | $CH_3$ | $OCF_2CHClF$ | |

Table IIIa (continued)

| $R_8$ | $R_9$ | $R_7$ | R | Z | X | Y | m.p.(°C) |
|---|---|---|---|---|---|---|---|
| $-CH_2CH_3$ | $CH_3$ | H | H | CH | $CH_3$ | $OCF_2CF_2H$ | |
| $-CH_2CH_3$ | $CH_3$ | H | H | CH | $OCH_3$ | $OCF_2CF_2H$ | |
| $-CH_2CH_3$ | $CH_3$ | H | H | N | $CH_3$ | $OCF_2CF_2H$ | |
| $-CH_2CH_3$ | $CH_3$ | H | H | CH | $CH_3$ | $OCF_2CHFCF_3$ | |
| $-CH_2CH_3$ | $CH_3$ | H | H | CH | $OCH_3$ | $OCF_2CHFCF_3$ | |
| $-CH_2CH_3$ | $CH_3$ | H | H | CH | $OCF_2H$ | $CH_2CH_3$ | |
| $-CH_2CH_3$ | $CH_3$ | H | H | CH | $OCF_2H$ | $OCH_2CH_3$ | |
| $-CH_2CH_3$ | $CH_3$ | H | H | CH | $OCF_2H$ | $CH_2OCH_3$ | |
| $-CH_2CH_3$ | $CH_3$ | H | H | CH | $OCF_2H$ | $CH(OCH_3)_2$ | |
| $-CH_2CH_3$ | $CH_3$ | H | H | CH | $OCF_2H$ | $OCH_2CF_3$ | |
| $-CH_2CH_3$ | $CH_3$ | H | H | CH | $OCF_2H$ | $OCF_3$ | |
| $-CH_2CH_3$ | $CH_3$ | H | H | CH | $OCF_2H$ | $NH_2$ | |
| $-CH_2CH_3$ | $CH_3$ | H | H | CH | $OCF_2H$ | $NHCH_3$ | |
| $-CH_2CH_3$ | $CH_3$ | H | H | CH | $OCF_2H$ | $N(CH_3)_2$ | |
| $-CH_2CH_3$ | $CH_3$ | H | H | CH | $OCF_2H$ | $OCF_2H$ | |
| $-CH_2CH_3$ | $CH_3$ | H | H | CH | $OCF_2H$ | $OCF_2CHClF$ | |
| $-CH_2CH_3$ | $CH_3$ | H | H | CH | $OCF_2H$ | $OCF_2CHBrF$ | |
| $-CH_2CH_3$ | $CH_3$ | H | H | CH | $OCF_2H$ | $OCF_2CF_2H$ | |
| $-CH_2CH_3$ | $CH_3$ | H | H | CH | $OCF_2H$ | $OCF_2CHFCF_3$ | |
| $-CH_2CH_3$ | $CH_3$ | H | H | CH | $CH_3$ | $OCF_2CF_2H$ | |
| $-CH_2CH_3$ | $CH_3$ | H | H | N | $CH_3$ | $OCF_2H$ | |

Table IIIb

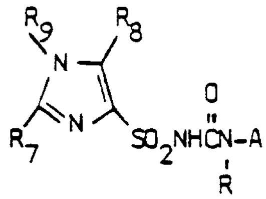

| $R_8$ | $R_9$ | $R_7$ | R | A | $Y^1$ | m.p.(°C) |
|---|---|---|---|---|---|---|
| $-SO_2N(CH_3)_2$ | $CH_3$ | H | H | A-2 | H | |
| $-SO_2N(CH_3)_2$ | $CH_3$ | H | H | A-2 | Cl | |
| $-SO_2N(CH_3)_2$ | $CH_3$ | H | H | A-2 | $CH_3$ | |
| $-SO_2N(CH_3)_2$ | $CH_3$ | H | H | A-2 | $OCH_3$ | |
| $-SO_2N(CH_3)_2$ | $CH_3$ | H | H | A-2 | $OCF_2H$ | |
| $-SO_2N(CH_3)_2$ | $CH_3$ | H | H | A-3 | H | |
| $-SO_2N(CH_3)_2$ | $CH_3$ | H | H | A-3 | Cl | |
| $-SO_2N(CH_3)_2$ | $CH_3$ | H | H | A-3 | $CH_3$ | |
| $-SO_2N(CH_3)_2$ | $CH_3$ | H | H | A-3 | $OCH_3$ | |
| $-SO_2N(CH_3)_2$ | $CH_3$ | H | H | A-3 | $OCF_2H$ | |
| $-SO_2N(CH_3)_2$ | $CH_3$ | H | H | A-4 | H | |
| $-SO_2N(CH_3)_2$ | $CH_3$ | H | H | A-4 | $CH_3$ | |
| $-SO_2N(CH_3)_2$ | $CH_3$ | H | H | A-4 | Cl | |
| $-SO_2N(CH_3)_2$ | $CH_3$ | H | H | A-4 | $OCH_3$ | |
| $-SO_2N(CH_3)_2$ | $CH_3$ | H | H | A-4 | $OCF_2H$ | |

Table IIIc

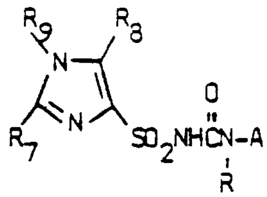

| $R_8$ | $R_9$ | $R_7$ | R | A | $X^2$ | $Y^2$ | $X^3$ | m.p.(°C) |
|---|---|---|---|---|---|---|---|---|
| $-SO_2CH_3$ | $CH_3$ | H | H | A-5 | $OCH_3$ | $CH_3$ | - | |
| $-SO_2CH_3$ | $CH_3$ | H | H | A-5 | $OCH_3$ | $CH_2CH_3$ | - | |
| $-SO_2CH_3$ | $CH_3$ | H | H | A-5 | $OCH_3$ | $CH_2CF_3$ | - | |
| $-SO_2CH_3$ | $CH_3$ | H | H | A-5 | $CH_3$ | $CH_3$ | - | |
| $-SO_2CH_3$ | $CH_3$ | H | H | A-5 | $CH_3$ | $CH_2CH_3$ | - | |
| $-SO_2CH_3$ | $CH_3$ | H | H | A-5 | $CH_3$ | $CH_2CF_3$ | - | |
| $-SO_2CH_3$ | $CH_3$ | H | H | A-5 | $CH_2CH_3$ | $CH_3$ | - | |
| $-SO_2CH_3$ | $CH_3$ | H | H | A-5 | $CH_2CH_3$ | $CH_2CH_3$ | - | |
| $-SO_2CH_3$ | $CH_3$ | H | H | A-5 | $CH_2CH_3$ | $CH_2CF_3$ | - | |
| $-SO_2CH_3$ | $CH_3$ | H | H | A-5 | $OCH_2CH_3$ | $CH_3$ | - | |
| $-SO_2CH_3$ | $CH_3$ | H | H | A-5 | $OCH_2CH_3$ | $CH_2CH_3$ | - | |
| $-SO_2CH_3$ | $CH_3$ | H | H | A-5 | $OCH_2CH_3$ | $CH_2CF_3$ | - | |
| $-SO_2CH_3$ | $CH_3$ | H | H | A-5 | $SCH_3$ | $CH_3$ | - | |
| $-SO_2CH_3$ | $CH_3$ | H | H | A-5 | $SCH_3$ | $CH_2CH_3$ | - | |
| $-SO_2CH_3$ | $CH_3$ | H | H | A-5 | $SCH_3$ | $CH_2CF_3$ | - | |
| $-SO_2CH_3$ | $CH_3$ | H | H | A-5 | $SCH_2CH_3$ | $CH_3$ | - | |
| $-SO_2CH_3$ | $CH_3$ | H | H | A-5 | $SCH_2CH_3$ | $CH_2CH_3$ | - | |
| $-SO_2CH_3$ | $CH_3$ | H | H | A-5 | $SCH_2CH_3$ | $CH_2CF_3$ | - | |
| Br | $CH_3$ | H | H | A-5 | $OCH_3$ | $CH_3$ | - | |
| Br | $CH_3$ | H | H | A-5 | $CH_3$ | $CH_2CF_3$ | - | |
| Br | $CH_3$ | H | H | A-5 | $SCH_3$ | $CH_2CH_3$ | - | |
| Br | $CH_3$ | H | H | A-5 | $SCH_2CH_3$ | $CH_2CF_3$ | - | |
| $SO_2CH_3$ | $CH_3$ | H | H | A-6 | - | - | $OCH_3$ | |
| $SO_2CH_3$ | $CH_3$ | H | H | A-6 | - | - | $CH_3$ | |
| Br | $CH_3$ | H | H | A-6 | - | - | $OCH_3$ | |
| Br | $CH_3$ | H | H | A-6 | - | - | $CH_3$ | |

Table IVa

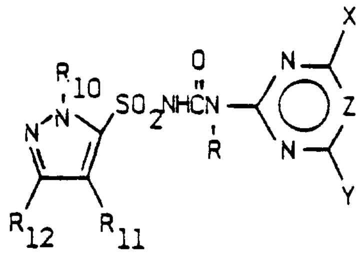

| $R_{10}$ | $R_{11}$ | $R_{12}$ | R | Z | X | Y | m.p.(°C) |
|---|---|---|---|---|---|---|---|
| H | H | H | H | CH | $CH_3$ | $OCH_3$ | |
| $CH_3$ | H | H | H | CH | $CH_3$ | $OCH_3$ | |
| $CH_3CH_2$- | H | H | H | CH | $CH_3$ | $OCH_3$ | |
| $CH_3CH_2CH_2$- | H | H | H | CH | $CH_3$ | $OCH_3$ | |
| $(CH_3)_2CH$- | H | H | H | CH | $CH_3$ | $OCH_3$ | |
| $CH_3(CH_2)_3$- | H | H | H | CH | $CH_3$ | $OCH_3$ | |
| $(CH_3)_2CHCH_2$- | H | H | H | CH | $CH_3$ | $OCH_3$ | |
| $CH_3$-CH-$CH_2CH_3$ | H | H | H | CH | $CH_3$ | $OCH_3$ | |
| $(CH_3)_3C$- | H | H | H | CH | $CH_3$ | $OCH_3$ | |
| $CH_2$=CH-$CH_2$- | H | H | H | CH | $CH_3$ | $OCH_3$ | |
| $CH_3CH$=CH-$CH_2$- | H | H | H | CH | $CH_3$ | $OCH_3$ | |
| -$CH_2$-C≡CH | H | H | H | CH | $CH_3$ | $OCH_3$ | |
| $CH_3$-C≡C-$CH_2$- | H | H | H | CH | $CH_3$ | $OCH_3$ | |
| -$CO_2CH_3$ | H | H | H | CH | $CH_3$ | $OCH_3$ | |
| -$CO_2CH_2CH_3$ | H | H | H | CH | $CH_3$ | $OCH_3$ | |
| -$CO_2CH_2CH_2CH_3$ | H | H | H | CH | $CH_3$ | $OCH_3$ | |
| -$CO_2CH(CH_3)_2$ | H | H | H | CH | $CH_3$ | $OCH_3$ | |
| -$CO_2CH_2CH$=$CH_2$ | H | H | H | CH | $CH_3$ | $OCH_3$ | |
| -$SO_2N(CH_3)_2$ | H | H | H | CH | $CH_3$ | $OCH_3$ | |
| -$SO_2N(CH_3)(CH_2CH_3)$ | H | H | H | CH | $CH_3$ | $OCH_3$ | |
| -$SO_2N(CH_3CH_3)_2$ | H | H | H | CH | $CH_3$ | $OCH_3$ | |
| -$SO_2CH_3$ | H | H | H | CH | $CH_3$ | $OCH_3$ | |
| -$SO_2CF_3$ | H | H | H | CH | $CH_3$ | $OCH_3$ | |
| -$SO_2CH_2CH_3$ | H | H | H | CH | $CH_3$ | $OCH_3$ | |
| -$SO_2CH_2CH_2CH_3$ | H | H | H | CH | $CH_3$ | $OCH_3$ | |

Table IVa (continued)

| $R_{10}$ | $R_{11}$ | $R_{12}$ | R | Z | X | Y | m.p.(°C) |
|---|---|---|---|---|---|---|---|
| $-SO_2CH(CH_3)_2$ | H | H | H | CH | $CH_3$ | $OCH_3$ | |
| $-CH_2CH_3$ | H | H | H | CH | $CH_3$ | $CH_3$ | |
| $-CH_2CH_3$ | H | H | H | CH | $OCH_3$ | $OCH_3$ | |
| $-CH_2CH_3$ | H | H | H | CH | Cl | $OCH_3$ | |
| $-CH_2CH_3$ | H | H | H | N | $CH_3$ | $CH_3$ | |
| $-CH_2CH_3$ | H | H | H | N | $CH_3$ | $OCH_3$ | |
| $-CH_2CH_3$ | H | H | H | N | $OCH_3$ | $OCH_3$ | |
| $-CH_2CH_3$ | H | H | H | N | $CH_3O$ | $-NHCH_3$ | |
| $-CH_2CH_3$ | H | H | H | N | $CH_3O$ | $-N(CH_3)_2$ | |
| $-CO_2CH_3$ | H | H | H | CH | $CH_3$ | $CH_3$ | |
| $-CO_2CH_3$ | H | H | H | CH | $OCH_3$ | $OCH_3$ | |
| $-CO_2CH_3$ | H | H | H | CH | Cl | $OCH_3$ | |
| $-CO_2CH_3$ | H | H | H | N | $CH_3$ | $CH_3$ | |
| $-CO_2CH_3$ | H | H | H | N | $CH_3$ | $OCH_3$ | |
| $-CO_2CH_3$ | H | H | H | N | $CH_3$ | $OCH_2CH_3$ | |
| $-CO_2CH_3$ | H | H | H | N | $OCH_3$ | $OCH_3$ | |
| $-CO_2CH_3$ | H | H | H | CH | Cl | $NH_2$ | |
| $-SO_2N(CH_3)_2$ | H | H | H | CH | $CH_3$ | $CH_3$ | |
| $-SO_2N(CH_3)_2$ | H | H | H | CH | $OCH_3$ | $OCH_3$ | |
| $-SO_2N(CH_3)_2$ | H | H | H | CH | Cl | $OCH_3$ | |
| $-SO_2N(CH_3)_2$ | H | H | H | N | $CH_3$ | $CH_3$ | |
| $-SO_2N(CH_3)_2$ | H | H | H | N | $CH_3$ | $OCH_3$ | |
| $-SO_2N(CH_3)_2$ | H | H | H | N | $CH_3$ | $OCH_2CH_3$ | |
| $-SO_2N(CH_3)_2$ | H | H | H | N | $OCH_3$ | $N(CH_3)_2$ | |
| $CH_3$ | $-CO_2CH_2CH_2CH_3$ | H | H | CH | $OCH_3$ | $OCH_3$ | |
| $CH_3$ | $-CO_2CH_2CH=CH_2$ | H | H | CH | $CH_3$ | $CH_3$ | |
| $CH_3$ | $-SCH_3$ | H | H | CH | Cl | $OCH_3$ | |
| $CH_3$ | $-SCH_2CH_2CH_3$ | H | H | N | $OCH_3$ | $OCH_3$ | |
| $CH_3$ | $-SCH_2CH(CH_3)_2$ | H | H | N | $CH_3$ | $OCH_3$ | |
| $CH_3$ | $-SOCH_3$ | H | H | N | $OCH_3$ | $N(CH_3)_2$ | |
| $CH_3$ | $-SOCH_2CH_3$ | H | H | CH | $CH_3$ | $OCH_3$ | |

Table IVa (continued)

| $R_{10}$ | $R_{11}$ | $R_{12}$ | R | Z | X | Y | m.p.(°C) |
|---|---|---|---|---|---|---|---|
| $CH_3$ | $-SOCH_2CH_2CH_3$ | H | H | CH | $CH_3$ | $OCH_3$ | |
| $CH_3$ | $-SO_2CH_3$ | H | H | CH | $CH_3$ | $OCH_3$ | |
| $CH_3$ | $-SO_2CH_2CH_3$ | H | H | CH | $CH_3$ | $OCH_3$ | |
| $CH_3$ | $-SO_2CH_2CH_2CH_3$ | H | H | CH | $CH_3$ | $OCH_3$ | |
| $CH_3$ | $-SO_2N(CH_3)_2$ | H | H | CH | $CH_3$ | $OCH_3$ | |
| $CH_3$ | $-SO_2N(CH_3)(CH_2CH_3)$ | H | H | CH | $CH_3$ | $OCH_3$ | |
| $CH_3$ | $-SO_2N(CH_2CH_3)_2$ | H | H | CH | $CH_3$ | $OCH_3$ | |
| H | $-CH_2CH_3$ | H | H | CH | $OCH_3$ | $OCH_3$ | |
| H | $-CH(CH_3)_2$ | H | H | CH | $CH_3$ | $CH_3$ | |
| $CH_3$ | F | H | H | CH | $CH_3$ | $OCH_3$ | |
| H | Cl | H | H | CH | Cl | $OCH_3$ | |
| $CH_3$ | Br | H | H | N | $CH_3$ | $OCH_3$ | |
| H | $NO_2$ | H | H | N | $OCH_3$ | $OCH_3$ | |
| $CH_3$ | $OCH_3$ | H | H | N | $CH_3O$ | $N(CH_3)_2$ | |
| $CH_3$ | $-OCH_2CH_3$ | H | H | CH | $CH_3O$ | $NHCH_3$ | |
| $CH_3$ | $-OCH_2CH_2CH_3$ | H | H | CH | $CH_3$ | $OCH_3$ | |
| H | $-NO_2$ | H | H | CH | $CH_3$ | $OCH_3$ | |
| H | $-CO_2CH_3$ | H | H | CH | $CH_3$ | $OCH_3$ | |
| H | $-CO_2CH_2CH_3$ | H | H | CH | $CH_3$ | $OCH_3$ | |
| $CH_3$ | $-CO_2CH(CH_3)_2$ | H | H | CH | $CH_3$ | $OCH_3$ | |
| H | Cl | H | H | CH | $OCH_3$ | $OCH_3$ | |
| $CH_3CH_2-$ | Cl | H | H | CH | $OCH_3$ | $OCH_3$ | |
| $CH_3CH_2-$ | $OCH_3$ | H | H | CH | $OCH_3$ | $OCH_3$ | |
| $CH_3CH_2-$ | $NO_2$ | H | H | CH | $OCH_3$ | $OCH_3$ | |
| $CH_3CH_2-$ | $CH_3$ | H | H | CH | $OCH_3$ | $OCH_3$ | |
| $CH_3$ | $-CO_2CH_3$ | H | H | CH | $CH_3$ | $CH_3$ | |
| $CH_3$ | $-CO_2CH_3$ | H | H | CH | $OCH_3$ | $OCH_3$ | |
| $CH_3$ | $-CO_2CH_3$ | H | H | CH | Cl | $OCH_3$ | |
| $CH_3$ | $-CO_2CH_3$ | H | H | CH | Cl | $NH_2$ | |
| $CH_3$ | $-CO_2CH_3$ | H | H | N | $OCH_3$ | $OCH_3$ | |
| $CH_3$ | $-CO_2CH_3$ | H | H | N | $CH_3$ | $OCH_3$ | |

Table IVa (continued)

| $R_{10}$ | $R_{11}$ | $R_{12}$ | R | Z | X | Y | m.p.(°C) |
|---|---|---|---|---|---|---|---|
| $CH_3$ | $-CO_2CH_3$ | H | H | N | $CH_3$ | $CH_3$ | |
| $CH_3$ | $-CO_2CH_3$ | H | H | N | $OCH_3$ | $-NHCH_3$ | |
| $CH_3$ | $-CO_2CH_3$ | H | H | N | $OCH_3$ | $-N(CH_3)_2$ | |
| $CH_3$ | $-SO_2N(CH_3)_2$ | H | H | CH | $OCH_3$ | $OCH_3$ | |
| $CH_3$ | $-SO_2N(CH_3)_2$ | H | H | CH | $CH_3$ | $CH_3$ | |
| $CH_3$ | $-SO_2N(CH_3)_2$ | H | H | CH | Cl | $OCH_3$ | |
| $CH_3$ | $-SO_2N(CH_3)_2$ | H | H | CH | Cl | $NH_2$ | |
| $CH_3$ | $-SO_2N(CH_3)_2$ | H | H | N | $OCH_3$ | $OCH_3$ | |
| $CH_3$ | $-SO_2N(CH_3)_2$ | H | H | N | $CH_3$ | $OCH_3$ | |
| $CH_3$ | $-SO_2N(CH_3)_2$ | H | H | N | $CH_3$ | $CH_3$ | |
| $CH_3$ | $-SO_2N(CH_3)_2$ | H | H | N | $CH_3O$ | $-NHCH_3$ | |
| $CH_3$ | $-SO_2N(CH_3)_2$ | H | H | N | $CH_3O$ | $-N(CH_3)_2$ | |
| $CH_3$ | $-SO_2CH_3$ | H | H | CH | $OCH_3$ | $OCH_3$ | |
| $CH_3$ | $-SO_2CH_3$ | H | H | CH | Cl | $OCH_3$ | |
| $CH_3$ | $-SO_2CH_3$ | H | H | N | $OCH_3$ | $-NHCH_3$ | |
| $CH_3$ | $-SO_2CH_3$ | H | H | N | $OCH_3$ | $-N(CH_3)_2$ | |
| $CH_3$ | $-CO_2CH_3$ | H | H | CH | $CH_3$ | $-OCH_2CH_3$ | |
| $CH_3$ | $-CO_2CH_3$ | H | H | CH | $CH_3$ | $-CH_2OCH_3$ | |
| $CH_3$ | $-CO_2CH_3$ | H | H | CH | $CH_3$ | $-CH_2(OCH_3)_2$ | |
| $CH_3CH_2-$ | $-CO_2CH_3$ | H | $CH_3$ | CH | $CH_3$ | $OCH_3$ | |
| $CH_3CH_2-$ | $-CO_2CH_3$ | H | $CH_3$ | CH | $CH_3$ | $CH_3$ | |
| $CH_3CH_2-$ | $-CO_2CH_3$ | H | $CH_3$ | CH | $OCH_3$ | $OCH_3$ | |
| $CH_3CH_2-$ | $-CO_2CH_3$ | H | $CH_3$ | N | $OCH_3$ | $OCH_3$ | |
| $CH_3CH_2-$ | $-CO_2CH_3$ | H | $CH_3$ | N | $OCH_3$ | $CH_3$ | |
| $-CH_2CH_3$ | H | H | H | CH | $OCF_2H$ | $CH_3$ | |
| $-CH_2CH_3$ | H | H | H | CH | $OCF_2H$ | $OCH_3$ | |
| $-CH_2CH_3$ | H | H | H | CH | $SCF_2H$ | $CH_3$ | |
| $-CH_2CH_3$ | H | H | H | CH | $SCF_2H$ | $OCH_3$ | |
| $-CH_2CH_3$ | H | H | H | CH | $SCF_2H$ | $OCF_3$ | |
| $-CH_2CH_3$ | H | H | H | CH | $CH_3$ | $OCH_2CF_3$ | |

Table IVa (continued)

| $R_{10}$ | $R_{11}$ | $R_{12}$ | R | Z | X | Y | m.p.(°C) |
|---|---|---|---|---|---|---|---|
| $-CH_2CH_3$ | H | H | H | CH | $OCH_3$ | $OCH_2CF_3$ | |
| $-CH_2CH_3$ | H | H | H | CH | $CH_3$ | $OCF_3$ | |
| $-CH_2CH_3$ | H | H | H | CH | $OCH_3$ | $OCF_3$ | |
| $-CH_2CH_3$ | H | H | H | N | $CH_3$ | $OCF_3$ | |
| $-CH_2CH_3$ | H | H | H | CH | $CH_3$ | $OCF_2CHClF$ | |
| $-CH_2CH_3$ | H | H | H | CH | $CH_3$ | $OCF_2CF_2H$ | |
| $-CH_2CH_3$ | H | H | H | CH | $OCH_3$ | $OCF_2CF_2H$ | |
| $-CH_2CH_3$ | H | H | H | N | $CH_3$ | $OCF_2CF_2H$ | |
| $-CH_2CH_3$ | H | H | H | CH | $CH_3$ | $OCF_2CHFCF_3$ | |
| $-CH_2CH_3$ | H | H | H | CH | $OCH_3$ | $OCF_2CHFCF_3$ | |
| $-CH_2CH_3$ | H | H | H | CH | $OCF_2H$ | $CH_2CH_3$ | |
| $-CH_2CH_3$ | H | H | H | CH | $OCF_2H$ | $OCH_2CH_3$ | |
| $-CH_2CH_3$ | H | H | H | CH | $OCF_2H$ | $CH_2OCH_3$ | |
| $-CH_2CH_3$ | H | H | H | CH | $OCF_2H$ | $CH(OCH_3)_2$ | |
| $-CH_2CH_3$ | H | H | H | CH | $OCF_2H$ | $OCH_2CF_3$ | |
| $-CH_2CH_3$ | H | H | H | CH | $OCF_2H$ | $OCF_3$ | |
| $-CH_2CH_3$ | H | H | H | CH | $OCF_2H$ | $NH_2$ | |
| $-CH_2CH_3$ | H | H | H | CH | $OCF_2H$ | $NHCH_3$ | |
| $-CH_2CH_3$ | H | H | H | CH | $OCF_2H$ | $N(CH_3)_2$ | |
| $-CH_2CH_3$ | H | H | H | CH | $OCF_2H$ | $OCF_2H$ | |
| $-CH_2CH_3$ | H | H | H | CH | $OCF_2H$ | $OCF_2CHClF$ | |
| $-CH_2CH_3$ | H | H | H | CH | $OCF_2H$ | $OCF_2CHBrF$ | |
| $-CH_2CH_3$ | H | H | H | CH | $OCF_2H$ | $OCF_2CF_2H$ | |
| $-CH_2CH_3$ | H | H | H | CH | $OCF_2H$ | $OCF_2CHFCF_3$ | |
| $-CH_2CH_3$ | H | H | H | CH | $CH_3$ | $OCF_2CF_2H$ | |
| $-CH_2CH_3$ | H | H | H | N | $CH_3$ | $OCF_2H$ | |

Table IVb

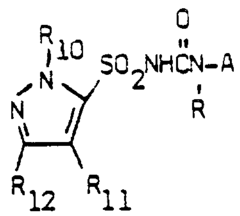

| $R_{10}$ | $R_{11}$ | $R_{12}$ | R | A | Y' | m.p.(°C) |
|---|---|---|---|---|---|---|
| $CH_2CH_3$ | H | H | H | A-2 | H | |
| $CH_2CH_3$ | H | H | H | A-2 | Cl | |
| $CH_2CH_3$ | H | H | H | A-2 | $CH_3$ | |
| $CH_2CH_3$ | H | H | H | A-2 | $OCH_3$ | |
| $CH_2CH_3$ | H | H | H | A-2 | $OCF_2H$ | |
| $CH_2CH_3$ | H | H | H | A-3 | H | |
| $CH_2CH_3$ | H | H | H | A-3 | Cl | |
| $CH_2CH_3$ | H | H | H | A-3 | $CH_3$ | |
| $CH_2CH_3$ | H | H | H | A-3 | $OCH_3$ | |
| $CH_2CH_3$ | H | H | H | A-3 | $OCF_2H$ | |
| $CH_2CH_3$ | H | H | H | A-4 | H | |
| $CH_2CH_3$ | H | H | H | A-4 | Cl | |
| $CH_2CH_3$ | H | H | H | A-4 | $CH_3$ | |
| $CH_2CH_3$ | H | H | H | A-4 | $OCH_3$ | |
| $CH_2CH_3$ | H | H | H | A-4 | $OCF_2H$ | |

Table IVc

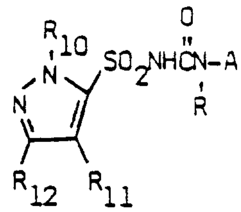

| $R_{10}$ | $R_{11}$ | $R_{12}$ | R | A | $X^2$ | $Y^2$ | $X^3$ | m.p.(°C) |
|---|---|---|---|---|---|---|---|---|
| $-CH_2CH_3$ | H | H | H | A-5 | $OCH_3$ | $CH_3$ | - | |
| $-CH_2CH_3$ | H | H | H | A-5 | $OCH_3$ | $CH_2CH_3$ | - | |
| $-CH_2CH_3$ | H | H | H | A-5 | $OCH_3$ | $CH_2CF_3$ | - | |
| $-CH_2CH_3$ | H | H | H | A-5 | $CH_3$ | $CH_3$ | - | |
| $-CH_2CH_3$ | H | H | H | A-5 | $CH_3$ | $CH_2CH_3$ | - | |
| $-CH_2CH_3$ | H | H | H | A-5 | $CH_3$ | $CH_2CF_3$ | - | |
| $-CH_2CH_3$ | H | H | H | A-5 | $CH_2CH_3$ | $CH_3$ | - | |
| $-CH_2CH_3$ | H | H | H | A-5 | $CH_2CH_3$ | $CH_2CH_3$ | - | |
| $-CH_2CH_3$ | H | H | H | A-5 | $CH_2CH_3$ | $CH_2CF_3$ | - | |
| $-CH_2CH_3$ | H | H | H | A-5 | $OCH_2CH_3$ | $CH_3$ | - | |
| $-CH_2CH_3$ | H | H | H | A-5 | $OCH_2CH_3$ | $CH_2CH_3$ | - | |
| $-CH_2CH_3$ | H | H | H | A-5 | $OCH_2CH_3$ | $CH_2CF_3$ | - | |
| $-CH_2CH_3$ | H | H | H | A-5 | $SCH_3$ | $CH_3$ | - | |
| $-CH_2CH_3$ | H | H | H | A-5 | $SCH_3$ | $CH_2CH_3$ | - | |
| $-CH_2CH_3$ | H | H | H | A-5 | $SCH_3$ | $CH_2CF_3$ | - | |
| $-CH_2CH_3$ | H | H | H | A-5 | $SCH_2CH_3$ | $CH_3$ | - | |
| $-CH_2CH_3$ | H | H | H | A-5 | $SCH_2CH_3$ | $CH_2CH_3$ | - | |
| $-CH_2CH_3$ | H | H | H | A-5 | $SCH_2CH_3$ | $CH_2CF_3$ | - | |
| $-CH_2CH_3$ | H | H | H | A-6 | - | - | $OCH_3$ | |
| $-CH_2CH_3$ | H | H | H | A-6 | - | - | $CH_3$ | |

Table Va

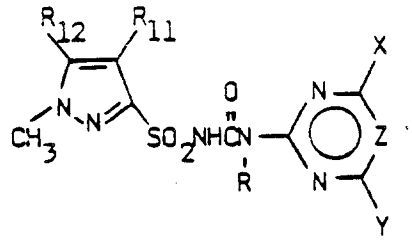

| $R_{11}$ | $R_{12}$ | R | Z | X | Y | m.p.(°C) |
|---|---|---|---|---|---|---|
| H | H | H | CH | $CH_3$ | $OCH_3$ | |
| $CH_3$ | H | H | CH | $OCH_3$ | $OCH_3$ | |
| $CH_3CH_2$- | H | H | CH | $CH_3$ | $CH_3$ | |
| $CH_3CH_2CH_2$- | H | H | CH | Cl | $OCH_3$ | |
| $(CH_3)_2CH$- | H | H | N | $CH_3$ | $OCH_3$ | |
| F | H | H | N | $CH_3$ | $OCH_3$ | |
| Cl | H | H | N | $OCH_3$ | $OCH_3$ | |
| Br | H | H | N | $OCH_3$ | $-N(CH_3)_2$ | |
| $NO_2$ | H | H | N | $OCH_3$ | $-N(CH_3)_2$ | |
| $OCH_3$ | H | H | CH | $CH_3$ | $OCH_3$ | |
| $-OCH_2CH_3$ | H | H | N | $CH_3$ | $OCH_3$ | |
| $-OCH_2CH_2CH_3$ | H | H | N | $OCH_3$ | $-N(CH_3)_2$ | |
| $-OCH(CH_3)_2$ | H | H | CH | Cl | $OCH_3$ | |
| $-CO_2CH_3$ | H | H | CH | $OCH_3$ | $OCH_3$ | |
| $-CO_2CH_2CH_3$ | H | H | CH | $CH_3$ | $OCH_3$ | |
| $-CO_2CH_2CH_2CH_3$ | H | H | CH | $CH_3$ | $CH_3$ | |
| $-CO_2CH(CH_3)_2$ | H | H | CH | $OCH_3$ | $OCH_3$ | |
| $-CO_2CH_2CH=CH_2$ | H | H | CH | $CH_3$ | $OCH_3$ | |
| $-SCH_3$ | H | H | N | $OCH_3$ | $OCH_3$ | |
| $-SCH_2CH_3$ | H | H | N | $OCH_3$ | $OCH_3$ | |
| $-SCH_2CH_2CH_3$ | H | H | N | $OCH_3$ | $OCH_3$ | |
| $-SOCH_3$ | H | H | N | $OCH_3$ | $OCH_3$ | |
| $-CO_2CH_3$ | H | H | CH | $CH_3$ | $OCH_3$ | |
| $-CO_2CH_3$ | H | $CH_3$ | CH | $CH_3$ | $OCH_3$ | |
| $-CO_2CH_3$ | H | $CH_3$ | CH | $CH_3$ | $CH_3$ | |

Table Va (continued)

| $R_{11}$ | $R_{12}$ | R | Z | X | Y | m.p.(°C) |
|---|---|---|---|---|---|---|
| $-CO_2CH_3$ | H | $CH_3$ | CH | $OCH_3$ | $OCH_3$ | |
| $-CO_2CH_3$ | H | $CH_3$ | N | $OCH_3$ | $OCH_3$ | |
| $-CO_2CH_3$ | H | $CH_3$ | N | $CH_3$ | $OCH_3$ | |
| $-SO_2CH_3$ | H | H | CH | $CH_3$ | $-OCH_2CH_3$ | |
| $-SO_2CH_3$ | H | H | N | $OCH_3$ | $NH_2$ | |
| $-SO_2CH_3$ | H | H | N | $OCH_3$ | $-NHCH_3$ | |
| $-SO_2CH_3$ | H | H | N | $OCH_3$ | $-N(CH_3)_2$ | |
| $-SO_2CH_3$ | H | H | CH | $CH_3$ | $-CH_2OCH_3$ | |
| $-SO_2CH_3$ | H | H | CH | $CH_3$ | $-CH(OCH_3)_2$ | |
| $-SO_2CH_3$ | H | H | CH | $OCH_3$ | $OCH_3$ | |
| $-SO_2CH_2CH_3$ | H | H | CH | $OCH_3$ | $CH_3$ | |
| $-SO_2CH_2CH_2CH_3$ | H | H | N | $OCH_3$ | $OCH_3$ | |
| $-SO_2N(CH_3)_2$ | H | H | N | $CH_3$ | $OCH_3$ | |
| $-SO_2N(CH_3)(CH_2CH_3)$ | H | H | N | $OCH_3$ | $-N(CH_3)_2$ | |
| $-SO_2N(CH_2CH_3)_2$ | H | H | N | $OCH_3$ | $CH_3$ | |
| $-CO_2CH_3$ | $CH_3$ | H | CH | $CH_3$ | $OCH_3$ | |
| $-CO_2CH_3$ | H | H | CH | $CH_3$ | $CH_3$ | |
| $-CO_2CH_3$ | H | H | CH | $OCH_3$ | $OCH_3$ | |
| $-CO_2CH_3$ | H | H | CH | $CH_3$ | $OCH_3$ | |
| $-CO_2CH_3$ | H | H | N | $CH_3$ | $OCH_3$ | |
| $-CO_2CH_3$ | H | H | N | $OCH_3$ | $OCH_3$ | |
| $-CO_2CH_3$ | H | H | CH | $OCF_2H$ | $CH_3$ | |
| $-CO_2CH_3$ | H | H | CH | $OCF_2H$ | $OCH_3$ | |
| $-CO_2CH_3$ | H | H | CH | $SCF_2H$ | $CH_3$ | |
| $-CO_2CH_3$ | H | H | CH | $SCF_2H$ | $OCH_3$ | |
| $-CO_2CH_3$ | H | H | CH | $SCF_2H$ | $OCF_3$ | |
| $-CO_2CH_3$ | H | H | CH | $CH_3$ | $OCH_2CF_3$ | |
| $-CO_2CH_3$ | H | H | CH | $OCH_3$ | $OCH_2CF_3$ | |
| $-CO_2CH_3$ | H | H | CH | $CH_3$ | $OCF_3$ | |
| $-CO_2CH_3$ | H | H | CH | $OCH_3$ | $OCF_3$ | |

Table Va (continued)

| $R_{11}$ | $R_{12}$ | R | Z | X | Y | m.p.(°C) |
|---|---|---|---|---|---|---|
| $-CO_2CH_3$ | H | H | N | $CH_3$ | $OCF_3$ | |
| $-CO_2CH_3$ | H | H | CH | $CH_3$ | $OCF_2CHClF$ | |
| $-CO_2CH_3$ | H | H | CH | $CH_3$ | $OCF_2CF_2H$ | |
| $-CO_2CH_3$ | H | H | CH | $OCH_3$ | $OCF_2CF_2H$ | |
| $-CO_2CH_3$ | H | H | N | $CH_3$ | $OCF_2CF_2H$ | |
| $-CO_2CH_3$ | H | H | CH | $CH_3$ | $OCF_2CHFCF_3$ | |
| $-CO_2CH_3$ | H | H | CH | $OCH_3$ | $OCF_2CHFCF_3$ | |
| $-CO_2CH_3$ | H | H | CH | $OCF_2H$ | $CH_2CH_3$ | |
| $-CO_2CH_3$ | H | H | CH | $OCF_2H$ | $OCH_2CH_3$ | |
| $-CO_2CH_3$ | H | H | CH | $OCF_2H$ | $CH_2OCH_3$ | |
| $-CO_2CH_3$ | H | H | CH | $OCF_2H$ | $CH(OCH_3)_2$ | |
| $-CO_2CH_3$ | H | H | CH | $OCF_2H$ | $OCH_2CF_3$ | |
| $-CO_2CH_3$ | H | H | CH | $OCF_2H$ | $OCF_3$ | |
| $-CO_2CH_3$ | H | H | CH | $OCF_2H$ | $NH_2$ | |
| $-CO_2CH_3$ | H | H | CH | $OCF_2H$ | $NHCH_3$ | |
| $-CO_2CH_3$ | H | H | CH | $OCF_2H$ | $N(CH_3)_2$ | |
| $-CO_2CH_3$ | H | H | CH | $OCF_2H$ | $OCF_2H$ | |
| $-CO_2CH_3$ | H | H | CH | $OCF_2H$ | $OCF_2CHClF$ | |
| $-CO_2CH_3$ | H | H | CH | $OCF_2H$ | $OCF_2CHBrF$ | |
| $-CO_2CH_3$ | H | H | CH | $OCF_2H$ | $OCF_2CF_2H$ | |
| $-CO_2CH_3$ | H | H | CH | $OCF_2H$ | $OCF_2CHFCF_3$ | |
| $-CO_2CH_3$ | H | H | CH | $CH_3$ | $OCF_2CF_2H$ | |
| $-CO_2CH_3$ | H | H | N | $CH_3$ | $OCF_2H$ | |

Table Vb

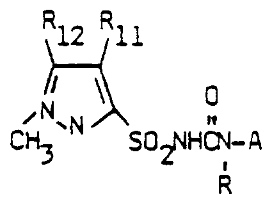

| $R_{11}$ | $R_{12}$ | R | A | Y' | m.p.(°C) |
|---|---|---|---|---|---|
| $CO_2CH_3$ | H | H | A-2 | H | |
| $CO_2CH_3$ | H | H | A-2 | Cl | |
| $CO_2CH_3$ | H | H | A-2 | $CH_3$ | |
| $CO_2CH_3$ | H | H | A-2 | $OCH_3$ | |
| $CO_2CH_3$ | H | H | A-2 | $OCF_2H$ | |
| $CO_2CH_3$ | H | H | A-3 | H | |
| $CO_2CH_3$ | H | H | A-3 | Cl | |
| $CO_2CH_3$ | H | H | A-3 | $CH_3$ | |
| $CO_2CH_3$ | H | H | A-3 | $OCH_3$ | |
| $CO_2CH_3$ | H | H | A-3 | $OCF_2H$ | |
| $CO_2CH_3$ | H | H | A-4 | H | |
| $CO_2CH_3$ | H | H | A-4 | Cl | |
| $CO_2CH_3$ | H | H | A-4 | $CH_3$ | |
| $CO_2CH_3$ | H | H | A-4 | $OCH_3$ | |
| $CO_2CH_3$ | H | H | A-4 | $OCF_2H$ | |

Table Vc

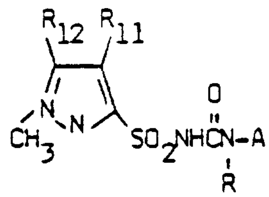

| $R_{11}$ | $R_{12}$ | R | A | $X^2$ | $Y^2$ | $X^3$ | m.p.(°C) |
|---|---|---|---|---|---|---|---|
| $-CO_2CH_3$ | H | H | A-5 | $OCH_3$ | $CH_3$ | - | |
| $-CO_2CH_3$ | H | H | A-5 | $OCH_3$ | $CH_2CH_3$ | - | |
| $-CO_2CH_3$ | H | H | A-5 | $OCH_3$ | $CH_2CF_3$ | - | |
| $-CO_2CH_3$ | H | H | A-5 | $CH_3$ | $CH_3$ | - | |
| $-CO_2CH_3$ | H | H | A-5 | $CH_3$ | $CH_2CH_3$ | - | |
| $-CO_2CH_3$ | H | H | A-5 | $CH_3$ | $CH_2CF_3$ | - | |
| $-CO_2CH_3$ | H | H | A-5 | $CH_2CH_3$ | $CH_3$ | - | |
| $-CO_2CH_3$ | H | H | A-5 | $CH_2CH_3$ | $CH_2CH_3$ | - | |
| $-CO_2CH_3$ | H | H | A-5 | $CH_2CH_3$ | $CH_2CF_3$ | - | |
| $-CO_2CH_3$ | H | H | A-5 | $OCH_2CH_3$ | $CH_3$ | - | |
| $-CO_2CH_3$ | H | H | A-5 | $OCH_2CH_3$ | $CH_2CH_3$ | - | |
| $-CO_2CH_3$ | H | H | A-5 | $OCH_2CH_3$ | $CH_2CF_3$ | - | |
| $-CO_2CH_3$ | H | H | A-5 | $SCH_3$ | $CH_3$ | - | |
| $-CO_2CH_3$ | H | H | A-5 | $SCH_3$ | $CH_2CH_3$ | - | |
| $-CO_2CH_3$ | H | H | A-5 | $SCH_3$ | $CH_2CF_3$ | - | |
| $-CO_2CH_3$ | H | H | A-5 | $SCH_2CH_3$ | $CH_3$ | - | |
| $-CO_2CH_3$ | H | H | A-5 | $SCH_2CH_3$ | $CH_2CH_3$ | - | |
| $-CO_2CH_3$ | H | H | A-5 | $SCH_2CH_3$ | $CH_2CF_3$ | - | |
| $-CO_2CH_3$ | H | H | A-6 | - | - | $OCH_3$ | |
| $-CO_2CH_3$ | H | H | A-6 | - | - | $CH_3$ | |

Table VIa

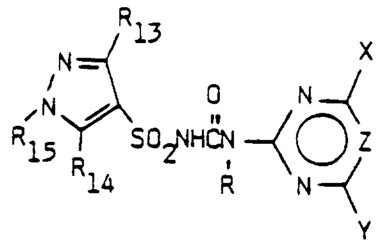

| $R_{13}$ | $R_{14}$ | $R_{15}$ | R | Z | X | Y | m.p.(°C) |
|---|---|---|---|---|---|---|---|
| H | H | H | H | CH | $CH_3$ | $OCH_3$ | |
| $CH_3$- | H | H | H | CH | $CH_3$ | $OCH_3$ | |
| $CH_3CH_2$- | $CH_3$ | H | H | CH | $CH_3$ | $OCH_3$ | |
| $CH_3CH_2CH_2$- | H | H | H | CH | $CH_3$ | $OCH_3$ | |
| $(CH_3)_2CH$- | $CH_3$ | H | H | CH | $CH_3$ | $OCH_3$ | |
| $CH_3O$- | H | H | H | CH | $CH_3$ | $OCH_3$ | |
| $CH_3CH_2O$- | $CH_3$ | H | H | CH | $CH_3$ | $OCH_3$ | |
| $CH_3CH_2CH_2O$- | H | H | H | CH | $CH_3$ | $OCH_3$ | |
| F | H | H | H | CH | $CH_3$ | $OCH_3$ | |
| Cl | H | H | H | CH | $CH_3$ | $OCH_3$ | |
| Br | H | H | H | CH | $CH_3$ | $OCH_3$ | |
| $NO_2$ | H | H | H | CH | $CH_3$ | $OCH_3$ | |
| $-CO_2CH_3$ | H | H | H | CH | $CH_3$ | $OCH_3$ | |
| $-CO_2CH_2CH_3$ | H | H | H | CH | $CH_3$ | $OCH_3$ | |
| $-CO_2CH_2CH_2CH_3$ | H | H | H | CH | $CH_3$ | $OCH_3$ | |
| $-CO_2CH_2CH{=}CH_2$ | H | H | H | CH | $CH_3$ | $OCH_3$ | |
| $-SCH_3$ | H | H | H | CH | $CH_3$ | $OCH_3$ | |
| $-SCH_2CH_3$ | H | H | H | CH | $CH_3$ | $OCH_3$ | |
| $-SCH_2CH_2CH_3$ | H | H | H | CH | $CH_3$ | $OCH_3$ | |
| $-SOCH_3$ | H | H | H | CH | $CH_3$ | $OCH_3$ | |
| $-SO_2CH_3$ | H | H | H | CH | $CH_3$ | $OCH_3$ | |
| $-SO_2CH_2CH_3$ | H | H | H | CH | $CH_3$ | $OCH_3$ | |
| $-SO_2CH_2CH_2CH_3$ | H | H | H | CH | $CH_3$ | $OCH_3$ | |
| $-SO_2N(CH_3)_2$ | H | H | H | CH | $CH_3$ | $OCH_3$ | |
| $-SO_2N(CH_3)(CH_2CH_3)$ | H | H | H | CH | $CH_3$ | $OCH_3$ | |

Table VIa (continued)

| $R_{13}$ | $R_{14}$ | $R_{15}$ | R | Z | X | Y | m.p.(°C) |
|---|---|---|---|---|---|---|---|
| $-SO_2N(CH_2CH_3)_2$ | H | H | H | CH | $CH_3$ | $OCH_3$ | |
| $-CO_2CH_3$ | H | H | H | CH | $OCH_3$ | $OCH_3$ | |
| $-CO_2CH_3$ | H | H | H | CH | $CH_3$ | $CH_3$ | |
| $-CO_2CH_3$ | H | H | H | CH | Cl | $OCH_3$ | |
| $-CO_2CH_3$ | H | H | H | CH | Cl | $NH_2$ | |
| $-CO_2CH_3$ | H | H | H | N | $OCH_3$ | $OCH_3$ | |
| $-CO_2CH_3$ | H | H | H | N | $OCH_3$ | $CH_3$ | |
| $-CO_2CH_3$ | H | H | H | N | $OCH_3$ | $-NHCH_3$ | |
| $-CO_2CH_3$ | H | H | H | N | $OCH_3$ | $-N(CH_3)_2$ | |
| $-SO_2CH_3$ | H | H | H | CH | $OCH_3$ | $OCH_3$ | |
| $-SO_2CH_3$ | H | H | H | CH | $CH_3$ | $CH_3$ | |
| $-SO_2CH_3$ | H | H | H | CH | Cl | $OCH_3$ | |
| $-SO_2CH_3$ | H | H | H | CH | Cl | $NH_2$ | |
| $-SO_2CH_3$ | H | H | H | N | $CH_3$ | $CH_3$ | |
| $-SO_2CH_3$ | H | H | H | N | $OCH_3$ | $OCH_3$ | |
| $-SO_2CH_3$ | H | H | H | N | $CH_3$ | $OCH_3$ | |
| $-SO_2CH_3$ | H | H | H | N | $OCH_3$ | $NH_2$ | |
| $-SO_2N(CH_3)_2$ | H | H | H | CH | $OCH_3$ | $OCH_3$ | |
| $-SO_2N(CH_3)_2$ | H | H | H | CH | $CH_3$ | $CH_3$ | |
| $-SO_2N(CH_3)_2$ | H | H | H | CH | Cl | $OCH_3$ | |
| $-SO_2N(CH_3)_2$ | H | H | H | CH | Cl | $NH_2$ | |
| $-SO_2N(CH_3)_2$ | H | H | H | N | $CH_3O$ | $-NHCH_3$ | |
| $-SO_2N(CH_3)_2$ | H | H | H | N | $CH_3O$ | $-N(CH_3)_2$ | |
| $-SO_2N(CH_3)_2$ | H | H | H | N | $CH_3O$ | $CH_3O$ | |
| $-SO_2N(CH_3)_2$ | H | H | H | N | $CH_3$ | $OCH_3$ | |
| $CH_3$ | H | $CH_3$ | H | CH | $CH_3$ | $OCH_3$ | 207-210° |
| $CH_3CH_2-$ | H | $CH_3$ | H | N | $CH_3$ | $OCH_3$ | |
| $CH_3$ | $CH_3$ | H | H | N | $CH_3$ | $OCH_3$ | |
| $CH_3$ | Cl | H | H | N | $CH_3$ | $OCH_3$ | |
| Cl | Cl | H | H | N | $CH_3$ | $OCH_3$ | |
| $NO_2$ | Cl | H | H | N | $CH_3$ | $OCH_3$ | |

Table VIa (continued)

| $R_{13}$ | $R_{14}$ | $R_{15}$ | R | Z | X | Y | m.p.(°C) |
|---|---|---|---|---|---|---|---|
| H | $CH_3$ | $CH_3$ | H | CH | $CH_3$ | $OCH_3$ | 179-181° |
| H | $CH_3CH_2$- | $CH_3$ | H | CH | $CH_3$ | $OCH_3$ | |
| H | $CH_3CH_2CH_2$- | $CH_3$ | H | CH | $CH_3$ | $OCH_3$ | |
| H | $(CH_3)_2CH$- | $CH_3$ | H | CH | $CH_3$ | $OCH_3$ | |
| H | $-OCH_3$ | $CH_3$ | H | CH | $CH_3$ | $OCH_3$ | |
| H | $-OCH_2CH_3$ | $CH_3$ | H | CH | $CH_3$ | $OCH_3$ | |
| H | $-OCH_2CH_2CH_3$ | $CH_3$ | H | CH | $OCH_3$ | $OCH_3$ | |
| H | F | $CH_3$ | H | CH | $CH_3$ | $CH_3$ | |
| H | Cl | $CH_3$ | H | CH | $CH_3$ | $OCH_3$ | |
| H | Br | $CH_3$ | H | CH | $OCH_3$ | $OCH_3$ | |
| H | $NO_2$ | $CH_3$ | H | CH | $CH_3$ | $CH_3$ | |
| H | $-CO_2CH_3$ | $CH_3$ | H | CH | $OCH_3$ | $OCH_3$ | |
| H | $-CO_2CH_2CH_3$ | $CH_3$ | H | CH | $OCH_3$ | $CH_3$ | |
| H | $-SO_2CH_3$ | $CH_3$ | H | N | $OCH_3$ | $OCH_3$ | |
| H | $-SO_2CH_2CH_3$ | $CH_3$ | H | N | $CH_3$ | $OCH_3$ | |
| H | $-SO_2CH_2CH_2CH_3$ | $CH_3$ | H | N | $CH_3$ | $OCH_3$ | |
| H | $-SO_2N(CH_3)_2$ | $CH_3$ | H | N | $CH_3$ | $OCH_3$ | |
| H | $-SO_2(CH_3)$ | $CH_3$ | H | N | $CH_3$ | $OCH_3$ | |
| $-CO_2CH_3$ | H | H | $CH_3$ | CH | $CH_3$ | $CH_3$ | |
| $-CO_2CH_3$ | H | H | $CH_3$ | CH | $CH_3$ | $OCH_3$ | |
| $-CO_2CH_3$ | H | H | $CH_3$ | CH | $OCH_3$ | $OCH_3$ | |
| $-CO_2CH_3$ | H | H | $CH_3$ | N | $OCH_3$ | $OCH_3$ | |
| $-CO_2CH_3$ | H | H | $CH_3$ | N | $OCH_3$ | $-N(CH_3)_2$ | |
| $-CO_2CH_3$ | H | H | $CH_3$ | N | $CH_3$ | $OCH_3$ | |
| $-CO_2CH_3$ | H | H | $CH_3$ | N | $CH_3$ | $CH_3$ | |
| $-SO_2CH_3$ | H | H | H | CH | Cl | $-NH_2$ | |
| $-SO_2CH_3$ | H | H | H | CH | Cl | $-NHCH_3$ | |
| $-SO_2CH_3$ | H | H | H | N | $CH_3$ | $-OCH_2CH_3$ | |
| $-SO_2CH_3$ | H | H | H | N | $CH_3$ | $-CH_2OCH_3$ | |
| $-SO_2CH_3$ | H | H | H | N | $CH_3$ | $-CH(OCH_3)_2$ | |
| $-CO_2CH_3$ | H | H | H | CH | $OCF_2H$ | $CH_3$ | |

Table VIa (continued)

| $R_{13}$ | $R_{14}$ | $R_{15}$ | R | Z | X | Y | m.p.(°C) |
|---|---|---|---|---|---|---|---|
| $-CO_2CH_3$ | H | H | H | CH | $OCF_2H$ | $OCH_3$ | |
| $-CO_2CH_3$ | H | H | H | N | $OCF_2H$ | $CH_3$ | |
| $-CO_2CH_3$ | H | H | H | CH | $SCF_2H$ | $CH_3$ | |
| $-CO_2CH_3$ | H | H | H | CH | $SCF_2H$ | $OCH_3$ | |
| $-CO_2CH_3$ | H | H | H | CH | $SCF_2H$ | $OCF_3$ | |
| $-CO_2CH_3$ | H | H | H | CH | $CH_3$ | $OCH_2CF_3$ | |
| $-CO_2CH_3$ | H | H | H | CH | $OCH_3$ | $OCH_2CF_3$ | |
| $-CO_2CH_3$ | H | H | H | CH | $CH_3$ | $OCF_3$ | |
| $-CO_2CH_3$ | H | H | H | CH | $OCH_3$ | $OCF_3$ | |
| $-CO_2CH_3$ | H | H | H | N | $CH_3$ | $OCF_3$ | |
| $-CO_2CH_3$ | H | H | H | CH | $CH_3$ | $OCF_2CHClF$ | |
| $-CO_2CH_3$ | H | H | H | CH | $CH_3$ | $OCF_2CF_2H$ | |
| $-CO_2CH_3$ | H | H | H | CH | $OCH_3$ | $OCF_2CF_2H$ | |
| $-CO_2CH_3$ | H | H | H | N | $CH_3$ | $OCF_2CF_2H$ | |
| $-CO_2CH_3$ | H | H | H | CH | $CH_3$ | $OCF_2CHFCF_3$ | |
| $-CO_2CH_3$ | H | H | H | CH | $OCH_3$ | $OCF_2CHFCF_3$ | |
| $-CO_2CH_3$ | H | H | H | CH | $OCF_2H$ | $CH_2CH_3$ | |
| $-CO_2CH_3$ | H | H | H | CH | $OCF_2H$ | $OCH_2CH_3$ | |
| $-CO_2CH_3$ | H | H | H | CH | $OCF_2H$ | $CH_2OCH_3$ | |
| $-CO_2CH_3$ | H | H | H | CH | $OCF_2H$ | $CH(OCH_3)_2$ | |
| $-CO_2CH_3$ | H | H | H | CH | $OCF_2H$ | $OCH_2CF_3$ | |
| $-CO_2CH_3$ | H | H | H | CH | $OCF_2H$ | $OCF_3$ | |
| $-CO_2CH_3$ | H | H | H | CH | $OCF_2H$ | $NH_2$ | |
| $-CO_2CH_3$ | H | H | H | CH | $OCF_2H$ | $NHCH_3$ | |
| $-CO_2CH_3$ | H | H | H | CH | $OCF_2H$ | $N(CH_3)_2$ | |
| $-CO_2CH_3$ | H | H | H | CH | $OCF_2H$ | $OCF_2H$ | |
| $-CO_2CH_3$ | H | H | H | CH | $OCF_2H$ | $OCF_2CHClF$ | |
| $-CO_2CH_3$ | H | H | H | CH | $OCF_2H$ | $OCF_2CHBrF$ | |
| $-CO_2CH_3$ | H | H | H | CH | $OCF_2H$ | $OCF_2CF_2H$ | |
| $-CO_2CH_3$ | H | H | H | CH | $OCF_2H$ | $OCF_2CHFCF_3$ | |
| $-CO_2CH_3$ | H | H | H | CH | $CH_3$ | $OCF_2CF_2H$ | |

Table VIa (continued)

| $R_{13}$ | $R_{14}$ | $R_{15}$ | R | Z | X | Y | m.p.(°C) |
|---|---|---|---|---|---|---|---|
| $-CO_2CH_3$ | H | H | H | N | $CH_3$ | $OCF_2H$ | |
| -H | $CH_3$ | $CH_3$ | H | CH | $OCH_3$ | $OCH_3$ | 207-210 |
| -H | $CH_3$ | $CH_3$ | H | CH | $CH_3$ | $CH_3$ | 204-208 |
| -H | $CH_3$ | $CH_3$ | H | N | $CH_3$ | $OCH_3$ | 197-201 |
| -H | $CH_3$ | $CH_3$ | H | N | $OCH_3$ | $OCH_3$ | 174-179 |
| -H | $CH_3$ | $CH_3$ | H | N | $CH_3$ | $CH_3$ | 211-215 |
| $-CH_3$ | H | $CH_3$ | H | CH | $OCH_3$ | $OCH_3$ | 189-191 |
| $-CH_3$ | H | $CH_3$ | H | CH | $CH_3$ | $CH_3$ | 210-215 |
| $-CH_3$ | H | $CH_3$ | H | N | $CH_3$ | $OCH_3$ | 183-186 |
| $-CH_3$ | H | $CH_3$ | H | N | $OCH_3$ | $OCH_3$ | 187-189 |
| $-CH_3$ | H | $CH_3$ | H | N | $CH_3$ | $CH_3$ | 225-226 |
| H | H | $CH_3$ | H | CH | $OCH_3$ | $CH_3$ | 212-214 |
| H | H | $CH_3$ | H | N | $OCH_3$ | $CH_3$ | 194-196 |
| H | H | $CH_3$ | H | N | $OCH_3$ | $OCH_3$ | 188-192 |
| H | H | $CH_3$ | H | N | $CH_3$ | $CH_3$ | 219-223 |
| H | H | $CH_3$ | H | CH | $OCH_3$ | $OCH_3$ | 203-206 |
| H | H | $CH_3$ | H | CH | $CH_3$ | $CH_3$ | 219-222 |

Table VIb

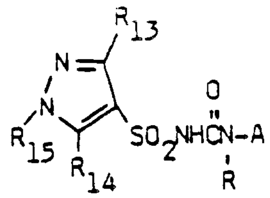

| $R_{13}$ | $R_{14}$ | $R_{15}$ | R | A | Y' | m.p.(°C) |
|---|---|---|---|---|---|---|
| $CO_2CH_3$ | H | H | H | A-2 | H | |
| $CO_2CH_3$ | H | H | H | A-2 | Cl | |
| $CO_2CH_3$ | H | H | H | A-2 | $CH_3$ | |
| $CO_2CH_3$ | H | H | H | A-2 | $OCH_3$ | |
| $CO_2CH_3$ | H | H | H | A-2 | $OCF_2H$ | |
| $CO_2CH_3$ | H | H | H | A-3 | H | |
| $CO_2CH_3$ | H | H | H | A-3 | Cl | |
| $CO_2CH_3$ | H | H | H | A-3 | $CH_3$ | |
| $CO_2CH_3$ | H | H | H | A-3 | $OCH_3$ | |
| $CO_2CH_3$ | H | H | H | A-3 | $OCF_2H$ | |
| $CO_2CH_3$ | H | H | H | A-4 | H | |
| $CO_2CH_3$ | H | H | H | A-4 | Cl | |
| $CO_2CH_3$ | H | H | H | A-4 | $CH_3$ | |
| $CO_2CH_3$ | H | H | H | A-4 | $OCH_3$ | |
| $CO_2CH_3$ | H | H | H | A-4 | $OCF_2H$ | |

Table VIc

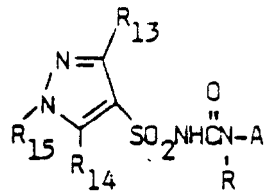

| $R_{13}$ | $R_{14}$ | $R_{15}$ | R | A | $X^2$ | $Y^2$ | $X^3$ | m.p.(°C) |
|---|---|---|---|---|---|---|---|---|
| $-CO_2CH_3$ | H | H | H | A-5 | $OCH_3$ | $CH_3$ | - | |
| $-CO_2CH_3$ | H | H | H | A-5 | $OCH_3$ | $CH_2CH_3$ | - | |
| $-CO_2CH_3$ | H | H | H | A-5 | $OCH_3$ | $CH_2CF_3$ | - | |
| $-CO_2CH_3$ | H | H | H | A-5 | $CH_3$ | $CH_3$ | - | |
| $-CO_2CH_3$ | H | H | H | A-5 | $CH_3$ | $CH_2CH_3$ | - | |
| $-CO_2CH_3$ | H | H | H | A-5 | $CH_3$ | $CH_2CF_3$ | - | |
| $-CO_2CH_3$ | H | H | H | A-5 | $CH_2CH_3$ | $CH_3$ | - | |
| $-CO_2CH_3$ | H | H | H | A-5 | $CH_2CH_3$ | $CH_2CH_3$ | - | |
| $-CO_2CH_3$ | H | H | H | A-5 | $CH_2CH_3$ | $CH_2CF_3$ | - | |
| $-CO_2CH_3$ | H | H | H | A-5 | $OCH_2CH_3$ | $CH_3$ | - | |
| $-CO_2CH_3$ | H | H | H | A-5 | $OCH_2CH_3$ | $CH_2CH_3$ | - | |
| $-CO_2CH_3$ | H | H | H | A-5 | $OCH_2CH_3$ | $CH_2CF_3$ | - | |
| $-CO_2CH_3$ | H | H | H | A-5 | $SCH_3$ | $CH_3$ | - | |
| $-CO_2CH_3$ | H | H | H | A-5 | $SCH_3$ | $CH_2CH_3$ | - | |
| $-CO_2CH_3$ | H | H | H | A-5 | $SCH_3$ | $CH_2CF_3$ | - | |
| $-CO_2CH_3$ | H | H | H | A-5 | $SCH_2CH_3$ | $CH_3$ | - | |
| $-CO_2CH_3$ | H | H | H | A-5 | $SCH_2CH_3$ | $CH_2CH_3$ | - | |
| $-CO_2CH_3$ | H | H | H | A-5 | $SCH_2CH_3$ | $CH_2CF_3$ | - | |
| $-CO_2CH_3$ | H | H | H | A-6 | - | - | $OCH_3$ | |
| $-CO_2CH_3$ | H | H | H | A-6 | - | - | $CH_3$ | |

Table VII

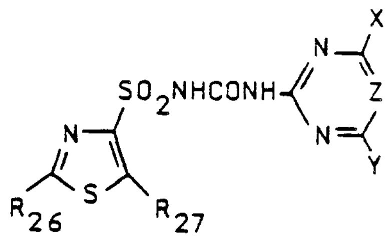

| $R_{26}$ | $R_{27}$ | Y | X | Z | m.p.(°C) |
|---|---|---|---|---|---|
| H | H | $OCH_3$ | $OCH_3$ | CH | 201-203° |
| H | H | $CH_3$ | $CH_3$ | CH | |
| H | H | $CH_3$ | $OCH_3$ | N | |
| H | H | $OCH_3$ | $OCH_3$ | N | |
| H | H | $CH_3$ | $CH_3$ | N | |
| H | H | $OC_2H_5$ | $CH_3$ | CH | |
| H | H | $CH_2OCH_3$ | $OCH_3$ | CH | |
| $CH_3$ | $OC_2H_5$ | $CH_3$ | $OCH_3$ | CH | 157-160° |
| $CH_3$ | $OC_2H_5$ | $CH_3$ | $CH_3$ | CH | 175-178° |
| $CH_3$ | $OC_2H_5$ | $OCH_3$ | $OCH_3$ | CH | 140-143° |
| $CH_3$ | $OC_2H_5$ | $CH_3$ | $OCH_3$ | N | |
| $CH_3$ | $OC_2H_5$ | $CH_3$ | $CH_3$ | N | |
| $CH_3$ | $OC_2H_5$ | $OCH_3$ | $OCH_3$ | N | |
| H | $OCH_3$ | $OCH_3$ | $OCH_3$ | CH | |
| H | $OCH_3$ | $CH_3$ | $CH_3$ | CH | |
| H | $OCH_3$ | $OC_2H_5$ | $OCH_3$ | CH | |
| H | $OCH_3$ | $OCH_3$ | $OCH_3$ | CH | |
| H | $OCH_3$ | $OCH_3$ | $CH_3$ | N | |
| H | $OCH_3$ | $OCH_3$ | $CH_3$ | N | |
| H | $OCH_3$ | $CH_2OCH_3$ | $OCH_3$ | N | |
| H | $OC_3H_7$ | $OCH_3$ | $OCH_3$ | CH | |
| H | $CH_3$ | $CH_3$ | $OCH_3$ | N | |
| H | $C_2H_5$ | $CH_3$ | $CH_3$ | CH | |
| H | $C_3H_7$ | $OCH_3$ | $OCH_3$ | CH | |
| $CH_3$ | $SCH_3$ | $CH_3$ | $OCH_3$ | CH | |

Table VII (continued)

| $R_{26}$ | $R_{27}$ | Y | X | Z | m.p.(°C) |
|---|---|---|---|---|---|
| $CH_3$ | $OC_2H_5$ | $OCF_2H$ | $CH_3$ | CH | |
| $CH_3$ | $OC_2H_5$ | $OCF_2H$ | $CH_3O$ | CH | |
| $CH_3$ | $OC_2H_5$ | $OCF_2H$ | $OCF_2H$ | CH | |
| $CH_3$ | $SC_2H_5$ | $OCH_3$ | $OCH_3$ | N | |
| $CH_3$ | $SC_3H_7$ | $CH_3$ | $OCH_3$ | N | |
| $CH_3$ | $CO_2C_2H_5$ | $CH_3$ | $CH_3$ | CH | 150-153° |
| $CH_3$ | $CO_2C_2H_5$ | $CH_3$ | $OCH_3$ | CH | 87-97° |
| $CH_3$ | $CO_2C_2H_5$ | $OCH_3$ | $OCH_3$ | CH | 121-123° |
| $CH_3$ | $CO_2C_2H_5$ | $CH_3$ | $OCH_3$ | N | 126-130° |
| $CH_3$ | $CO_2C_2H_5$ | $OCH_3$ | $OCH_3$ | N | 140-145° |
| $CH_3$ | $CO_2C_2H_5$ | $CH_2OCH_3$ | $OCH_3$ | N | |
| $CH_3$ | $CO_2C_2H_5$ | $OC_2H_5$ | $CH_3$ | N | |
| H | $CO_2CH_3$ | $CH_3$ | $CH_3$ | CH | |
| H | $CO_2CH_3$ | $CH_3$ | $OCH_3$ | CH | |
| H | $CO_2CH_3$ | $OCH_3$ | $OCH_3$ | CH | |
| H | $CO_2CH_3$ | $OCH_3$ | $CH_3$ | N | |
| H | $CO_2CH_3$ | $OCH_3$ | $OCH_3$ | N | |
| H | $CO_2C_3H_7$ | $OCH_3$ | $OCH_3$ | CH | |
| $C_2H_5$ | H | $CH_3$ | $OCH_3$ | N | |
| $C_3H_7$ | $SCH_3$ | $CH_3$ | $OCH_3$ | CH | |
| $CH_3$ | $OCH_3$ | $OCF_2H$ | $CH_3$ | CH | |

Table VIII

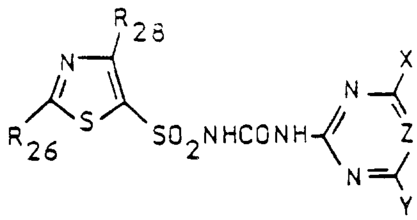

| $R_{26}$ | $R_{28}$ | Y | X | Z | m.p.(°C) |
|---|---|---|---|---|---|
| $CH_3CONH$ | $CH_3$ | $CH_3$ | $CH_3$ | CH | |
| $CH_3CONH$ | $CH_3$ | $CH_3$ | $OCH_3$ | CH | |
| $CH_3CONH$ | $CH_3$ | $OCH_3$ | $OCH_3$ | CH | 191-195°(d) |
| $CH_3CONH$ | $CH_3$ | $CH_2OCH_3$ | $CH_3$ | CH | |
| $CH_3CONH$ | $CH_3$ | $CH_3$ | $CH_3$ | N | |
| $CH_3CONH$ | $CH_3$ | $OCH_3$ | $OCH_3$ | N | |
| $CH_3CONH$ | $CH_3$ | $OC_2H_5$ | $OCH_3$ | N | |
| $CH_3CONH$ | $CH_3$ | $CH_3$ | $OCH_3$ | N | |
| H | $C_2H_5$ | $OCH_3$ | $OCH_3$ | CH | |
| H | $C_3H_7$ | $CH_3$ | $OCH_3$ | N | |
| $CH_3$ | $OCH_3$ | $OCH_3$ | $OCH_3$ | CH | |
| $CH_3$ | $OC_2H_5$ | $CH_3$ | $OCH_3$ | CH | |
| $CH_3$ | $OC_3H_7$ | $CH_3$ | $OCH_3$ | CH | |
| $C_2H_5$ | $SCH_3$ | $OCH_3$ | $OCH_3$ | N | |
| $C_3H_7$ | H | $CH_3$ | $OCH_3$ | CH | |
| H | $CO_2CH_3$ | $CH_3$ | $CH_3$ | CH | 174-175°(d) |
| H | $CO_2CH_3$ | $OCH_3$ | $CH_3$ | CH | 179-180°(d) |
| H | $CO_2CH_3$ | $OCH_3$ | $OCH_3$ | CH | 172-174°(d) |
| H | $CO_2CH_3$ | $CH_3$ | $OCH_3$ | N | 166-169°(d) |
| H | $CO_2CH_3$ | $OCH_3$ | $OCH_3$ | N | 177-178°(d) |
| H | $CO_2C_2H_5$ | $OCH_3$ | $CH_3$ | CH | |
| H | $CO_2C_3H_7$ | $CH_3$ | $OCH_3$ | CH | |
| $CH_3CONH$ | $CH_3$ | $OCF_2H$ | $CH_3$ | CH | |
| H | H | $CH_3$ | $OCH_3$ | N | |
| H | H | $OCH_3$ | $OCH_3$ | N | |

Table VIII (continued)

| $R_{26}$ | $R_{28}$ | Y | X | Z | m.p.(°C) |
|---|---|---|---|---|---|
| H | H | $CH_3$ | $OCH_3$ | CH | |
| H | H | $OCH_3$ | $OCH_3$ | CH | |
| $CH_3CONH$ | $CF_3$ | $CH_3$ | $OCH_3$ | CH | |
| $CH_3CONH$ | $NO_2$ | $OCH_3$ | $OCH_3$ | CH | |
| $CH_3CONH$ | Br | $OCH_3$ | $OCH_3$ | CH | |
| $CH_3CONH$ | Cl | $OCH_3$ | $OCH_3$ | CH | |

Table IX

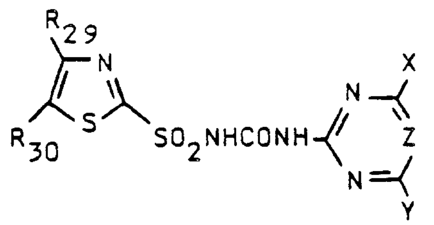

| $R_{29}$ | $R_{30}$ | Y | X | Z | m.p.(°C) |
|---|---|---|---|---|---|
| Cl | H | $CH_3$ | $OCH_3$ | CH | 182-183°(d) |
| Cl | H | $OCH_3$ | $OCH_3$ | CH | 80-84°(d) |
| Cl | H | $CH_3$ | $CH_3$ | CH | |
| Cl | H | $CH_3$ | $OCH_3$ | N | |
| Cl | H | $CH_3$ | $CH_3$ | N | |
| Cl | H | $OCH_3$ | $OCH_3$ | N | |
| H | H | $OCH_3$ | $CH_3$ | CH | |
| H | $CH_3$ | $CH_3$ | $OCH_3$ | N | |
| $CH_3$ | $CH_3$ | $OCH_3$ | $OCH_3$ | CH | |
| $CH_3$ | H | $CH_3$ | $OCH_3$ | CH | |
| H | Cl | $OCH_3$ | $OCH_3$ | CH | |
| Cl | H | $OCF_2H$ | $CH_3$ | CH | |

Table X

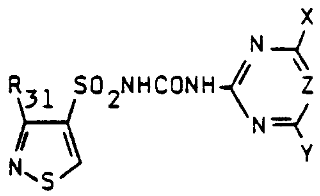

| $R_{31}$ | Y | X | Z | m.p.(°C) |
|---|---|---|---|---|
| Cl | $CH_3$ | $CH_3$ | CH | 136-140°(d) |
| Cl | $OCH_3$ | $CH_3$ | CH | 153-155°(d) |
| Cl | $OCH_3$ | $OCH_3$ | CH | 145-150° |
| Cl | $CH_2OCH_3$ | $CH_3$ | CH | |
| Cl | $CH_3$ | $OCH_3$ | N | 156-160°(d) |
| Cl | $OCH_3$ | $OCH_3$ | N | 163-167°(d) |
| Cl | $CH_3$ | $CH_3$ | N | |
| Cl | $CH_2OCH_3$ | $OCH_3$ | N | |
| $CH_3O_2C$ | $CH_3$ | $CH_3$ | CH | 167°(d) |
| $CH_3O_2C$ | $OCH_3$ | $CH_3$ | CH | 120°(d) |
| $CH_3O_2C$ | $OCH_3$ | $OCH_3$ | CH | 161°(d) |
| $CH_3O_2C$ | $OCH_3$ | $CH_3$ | N | 181°(d) |
| $CH_3O_2C$ | $OCH_3$ | $OCH_3$ | N | 180°(d) |
| $CH_3O_2C$ | $CH_3$ | $CH_3$ | N | |
| $C_2H_5O_2C$ | $CH_3$ | $OCH_3$ | CH | |
| $C_2H_5O_2C$ | $OCH_3$ | $CH_3$ | CH | |
| $C_2H_5O_2C$ | $OCH_3$ | $OCH_3$ | CH | |
| $C_3H_7O_2C$ | $OCH_3$ | $OCH_3$ | CH | |
| $CH_3$ | $CH_3$ | $CH_3$ | CH | |
| $CH_3$ | $CH_3$ | $OCH_3$ | CH | |
| $CH_3$ | $OCH_3$ | $OCH_3$ | CH | |
| $CH_3$ | $CH_3$ | $OCH_3$ | N | |
| $CH_3$ | $OCH_3$ | $OCH_3$ | N | |
| $CH_3$ | $CH_3$ | $CH_3$ | N | |
| $C_2H_5$ | $CH_3$ | $OCH_3$ | N | |
| Cl | $OCF_2H$ | $CH_3$ | CH | |
| $C_2H_5$ | $OCH_3$ | $OCH_3$ | N | |
| $C_2H_5$ | $OCH_3$ | $OCH_3$ | CH | |
| $C_3H_7$ | $CH_3$ | $OCH_3$ | CH | |
| $C_3H_7$ | $CH_3$ | $CH_3$ | CH | |
| $C_3H_7$ | $OCH_3$ | $OCH_3$ | CH | |

## Formulations

Useful formulations of the compounds of Formula I can be prepared in conventional ways. They include dusts, granules, pellets, solutions, suspensions, emulsions, wettable powders, emulsifiable concentrates and

the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99% solid or liquid diluent(s). More specifically, they will contain these ingredients in the following approximate proportions:

Table XI

| | Active Ingredient | Weight Percent* | |
|---|---|---|---|
| | | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al, "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example. Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Son, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 27

*Wettable Powder*

| | |
|---|---|
| N-[(4,6-dimethoxypyrimidin-2-yl)aminocarbonyl]-1-(1-methylethyl)-1H-imidazole-2-sulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 28

*Wettable Powder*

| | |
|---|---|
| N-[(4-methoxy-6-methylpyrimidin-2-yl)aminocarbonyl]-1-(1-methylethyl)-1H-imidazole-2-sulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 29

*Granule*

| | |
|---|---|
| Wettable Powder of Example 28 | 5% |
| attapulgite granules (U.S.S. 20-40 mesh; 0.84-0.42 mm) | 95% |

A slurry of wettable powder containing ≈25% solids is sprayed on the surface of attapulgite granules in a double-cone blender. The granules are dried and packaged.

Example 30

*Extruded Pellet*

| | |
|---|---|
| N-[(4,6-dimethoxypyrimidin-2-yl)aminocarbonyl]-1-(1-methylethyl)-1H-imidazole-2-sulfonamide | 25% |
| anhydrous sodium sulfate | 10% |
| crude calcium ligninsufonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 31

*Oil Suspension*

| | |
|---|---|
| N-[(4,6-dimethoxypyrimidin-2-yl)aminocarbonyl]-1-ethyl-1H-imidazole-2-sulfonamide | 25% |
| polyoxythylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 32

*Wettable Powder*

| | |
|---|---|
| N-[(4-methoxy-6-methylpyrimidin-2-yl)aminocarbonyl]-1-ethyl-1H-imidazole-2-sulfonamide | 20% |
| sodium alkylnaphthalenesulfonate | 4% |
| sodium ligninsulfonate | 4% |
| low voscosity methyl cellulose | 3% |
| attapulgite | 69% |

The ingredients are thoroughly blended. After grinding in a hammer-mill to produce particles essentially all below 100 microns, the material is re-blended and sifted through a U.S.S. No. 50 sieve (0.3 mm opening) and packaged.

Example 33

*Low Strength Granule*

| | |
|---|---|
| N-[(4,6-dimethoxypyrimidin-2-yl)aminocarbonyl]-1-ethyl-1H-imidazole-2-sulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 34

*Aqueous Suspension*

| | |
|---|---|
| N-[(4,6-dimethylpyrimidin-2-yl)aminocarbonyl]-1-(1-methylethyl)-1H-imidazole-2-sulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 35

*Solution*

| | |
|---|---|
| N-[(4-methoxy-6-methylpyrimidin-2-yl)aminocarbonyl]-1-(1-methylethyl)-1H-imidazole-2-sulfonamide, sodium salt | 5% |
| water | 95% |

The salt is added directly to the water with stirring to produce the solution, which may then be packaged for use.

Example 36

*Low Strength Granule*

| | |
|---|---|
| N-[(4,6-dimethoxypyrimidin-2-yl)aminocarbonyl]-1-(1-methylethyl)-1H-imidazole-2-sulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 37

*Granule*

| | |
|---|---|
| N-[(4,6-dimethoxypyrimidin-2-yl)aminocarbonyl]-1-ethyl-1H-imidazole-2-sulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 38

*High Strength Concentrate*

| | |
|---|---|
| N-[(4-methoxy-6-methylpyrimidin-2-yl)aminocarbonyl]-1-ethyl-1H-imidazole-2-sulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening), the concentrate may be formulated further if necessary.

Example 39

*Wettable Powder*

| | |
|---|---|
| N-[(4,6-dimethoxypyrimidin-2-yl)aminocarbonyl]-1-ethyl-1H-imidazole-2-sulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 40

*Wettable Powder*

| | |
|---|---|
| N-[(4,6-dimethylpyrimidin-2-yl)aminocarbonyl]-1-(1-methylethyl)-1H-imidazole-2-sulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material in reblended and then packaged.

Example 41

*Oil Suspension*

| | |
|---|---|
| N-[(4-methoxy-6-methylpyrimidin-2-yl)aminocarbonyl]-1-(1-methylethyl-1H-imidazole-2-sulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

*Utility*

Test results indicate that the compounds of the present invention are powerful herbicides. Many of them have utility for broad-spectrum pre- and/or post-emergence weed control in areas where complete control of all vegetation is desired, such as around fuel storage tanks, ammunition depots, industrial storage areas, parking lots, drive-in theatres, around billboards, highway and railroad structures. Alternatively, the subject compounds should be useful to modify plant growth.

The rate of application for the compounds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of around 0.02 to 10 kg/ha, the lower rates being suggested for use on lighter soils and/or those having a low organic matter content, for plant growth modification or for situations where only short-term persistence is required.

The compounds of the invention may be used in combination with any other commercial herbicide, examples of which are those of the triazine, triazole, uracil, urea, amide, diphenylether, carbamate and bipyridylium types.

The herbicidal properties of the subject compounds were discovered in a number of greenhouse tests. The test procedures and results follow.

Although some of the compounds do not exhibit a high degree of activity at the rate tested, it is expected that these compounds will exhibit herbicidal effects at higher rates.

*Test A*

Seeds of crabgrass *(Digitaria* sp.), barnyardgrass *(Echinochloa crusgalli),* wild oats *(Avena fatua),* sicklepod *(Cassia obtusifolia),* morningglory *(Ipomoea* sp.), cocklebur *(Xanthium* sp.), sorghum, corn, soybean, rice, wheat and nutsedge tubers *(Cyperus rotundus)* were planted in a growth medium and treated pre-emergence with the chemicals dissolved in a non-phytotoxic solvent. At the same time, cotton having five leaves (including cotyledonary ones), bush beans with the third trifoliolate leaf expanding, crabgrass, barnyardgrass and wild oats with two leaves, sicklepod with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three to five leaves were sprayed. Treated plants and controls were maintained in a greenhouse for sixteen days, whereupon all species were compared to controls and visually rated for response to treatment. The ratings are based on a numerical scale extending from 0 = no injury, to 10 = complete kill. The accompanying descriptive symbols have the following meanings:

C = chlorosis/necrosis;
D = defoliation;
E = emergence inhibition;
G = growth retardation;
H = formative effects;
U = unusual pigmentation;
X = axillary stimulation; and
6Y = abscised buds or flowers.

The ratings for the compounds tested by this procedure are presented in Table A. It will be seen that, at the low rates of application selected for this test, the compounds tested have utility for plant growth modification, and also are highly active herbicides.

Compounds

Compound 1

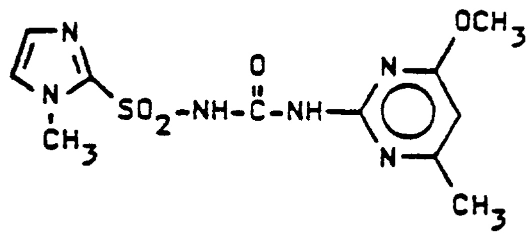

Compound 2

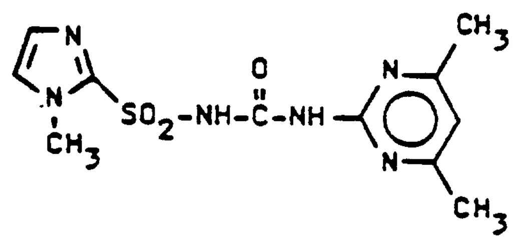

Compound 3

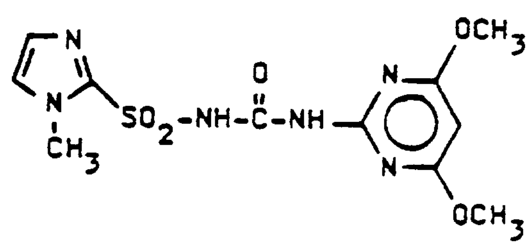

Compound 4

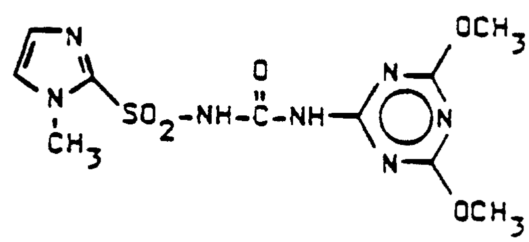

Compounds (continued)

Compound 5

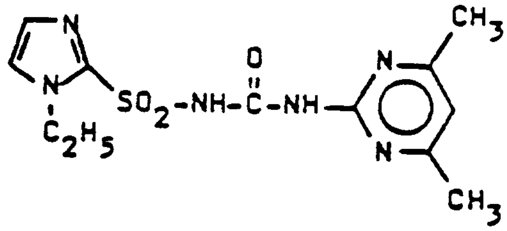

Compound 6

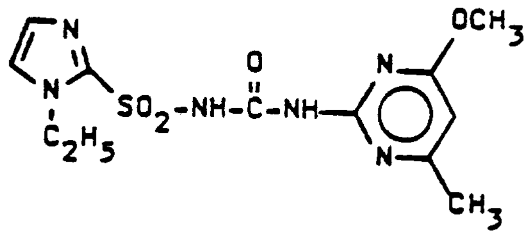

Compound 7

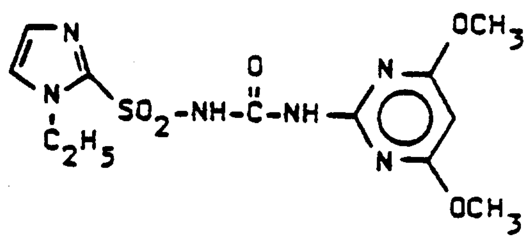

Compound 8

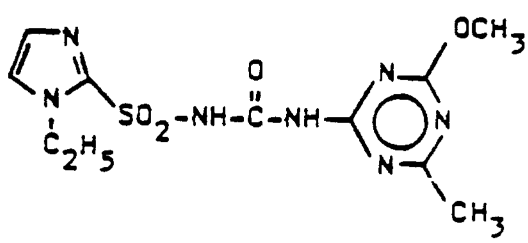

Compounds (continued)

Compound 9

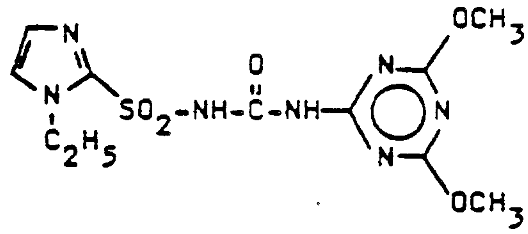

Compound 10

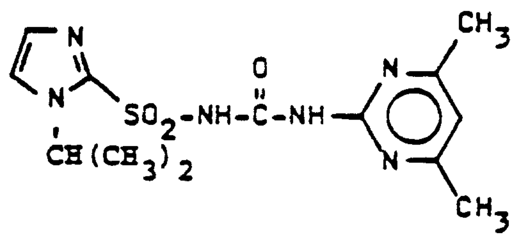

Compound 11

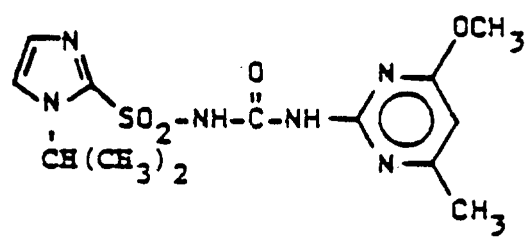

Compound 12

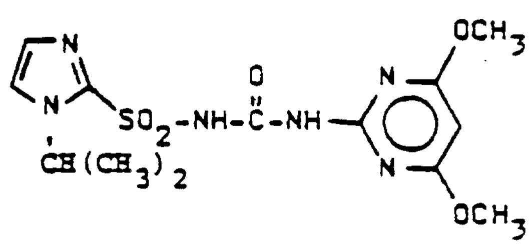

Compounds (continued)

Compound 13

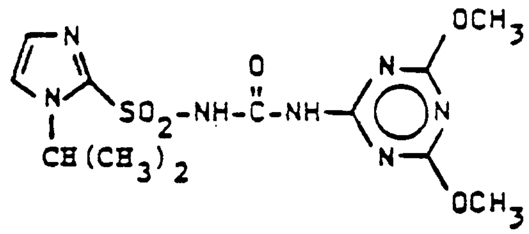

Compound 14

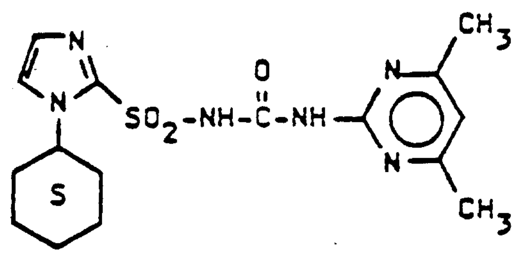

Compound 15

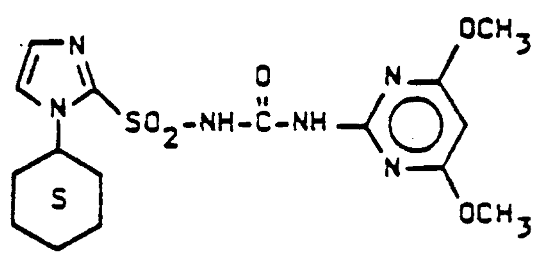

Compounds (continued)

Compound 16

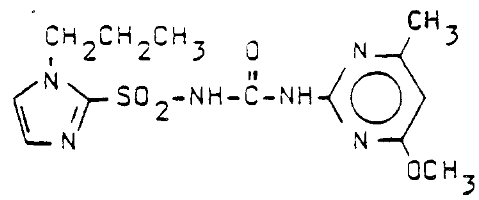

Compound 17

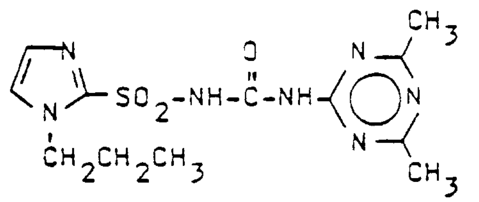

Compound 18

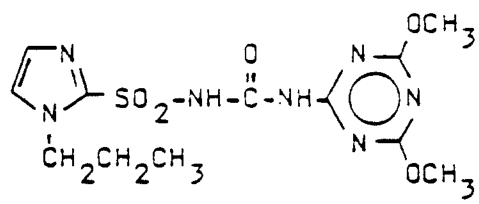

Compound 19

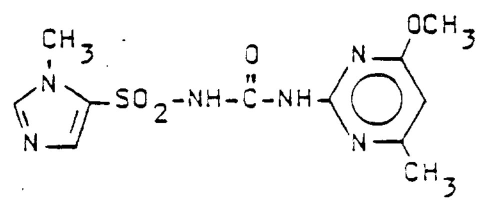

Compound 20

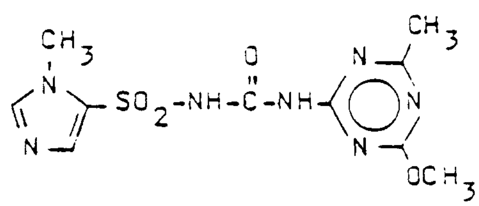

Compounds (continued)

Compound 21

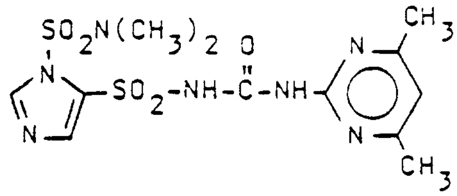

Compound 22

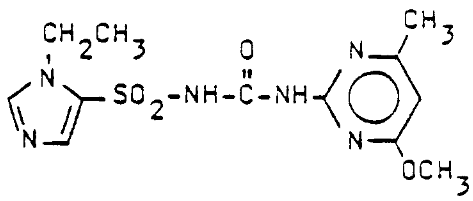

Compound 23

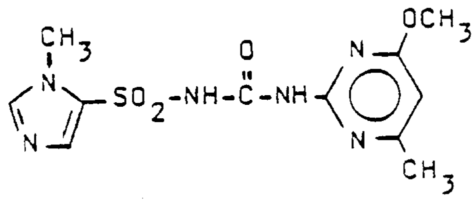

Compound 24

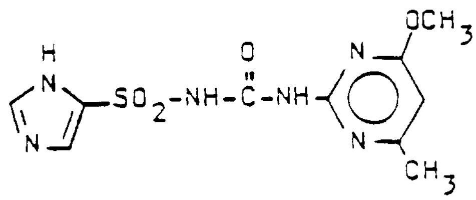

Compound 25

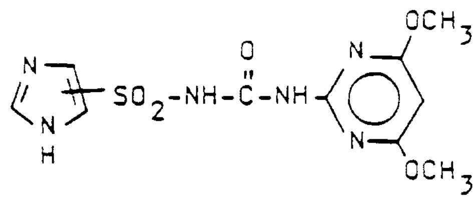

Compounds (continued)

Compound 26

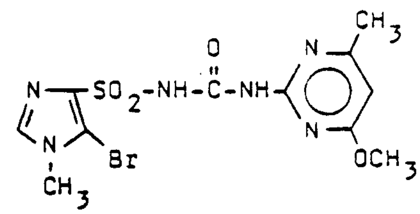

Compound 27

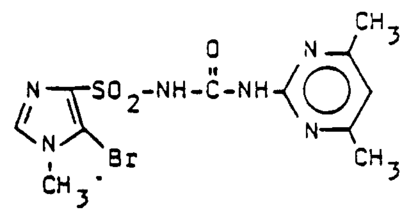

Compound 28

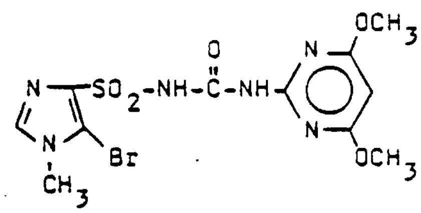

Compound 29

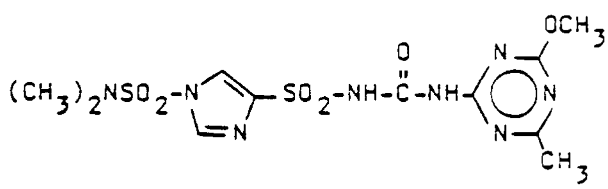

Compound 30

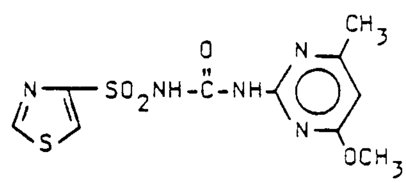

Compound 31

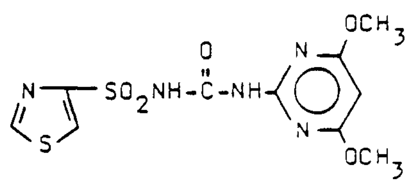

Compounds (continued)

Compound 32

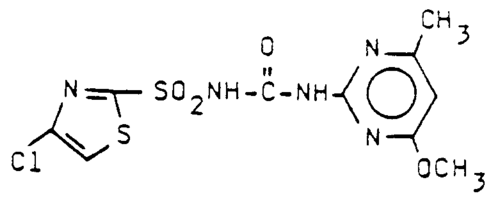

Compound 33

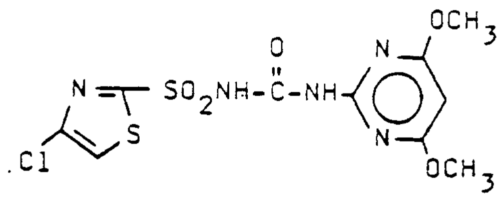

Compound 34

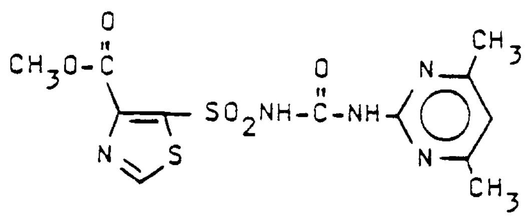

Compound 35

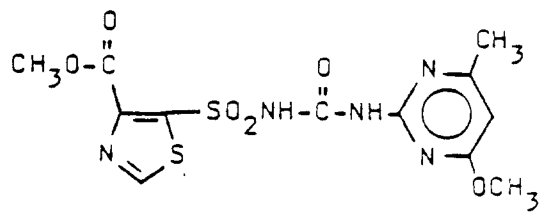

Compound 36

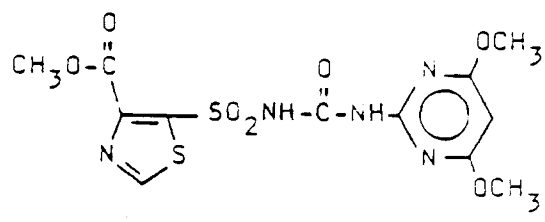

Compounds (continued)

Compound 37

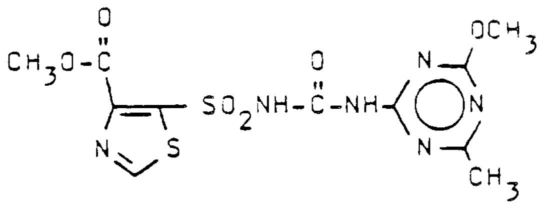

Compound 38

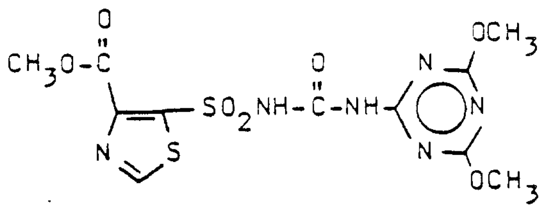

Compound 39

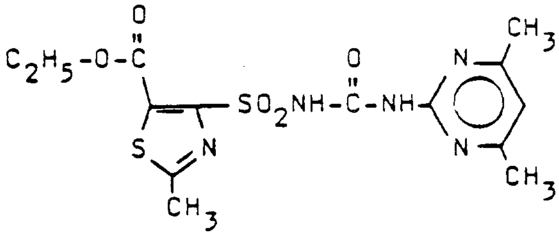

Compound 40

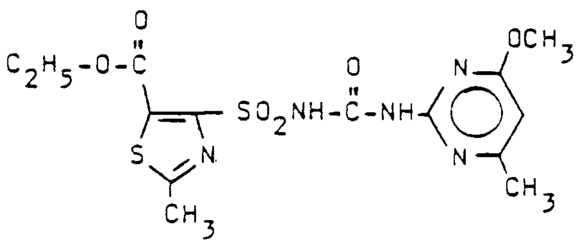

Compounds (continued)

Compound 41

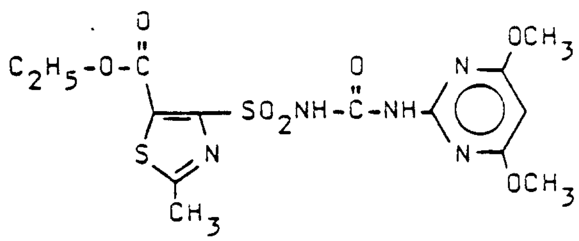

Compound 42

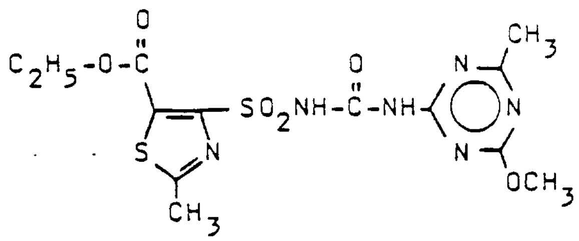

Compound 43

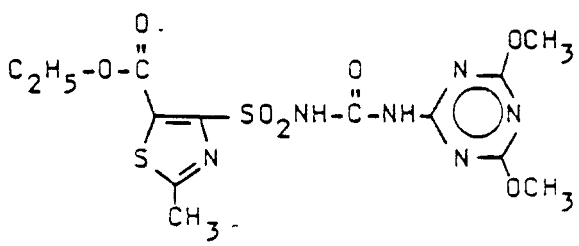

Compound 44

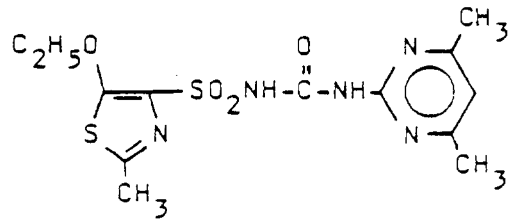

Compounds (continued)

Compound 45

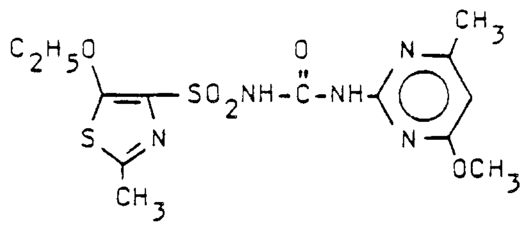

Compound 46

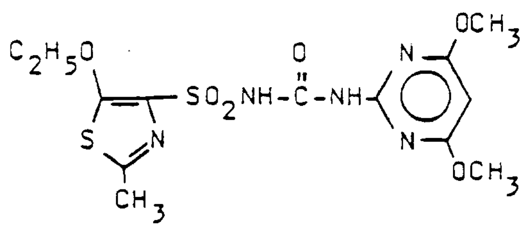

Compound 47

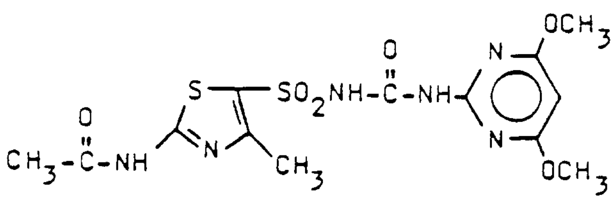

Compound 48

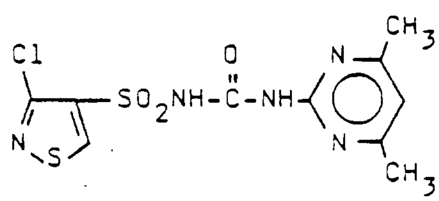

Compound 49

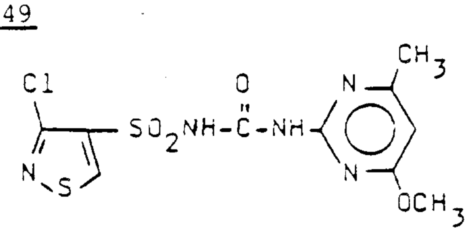

Compounds (continued)

Compound 50

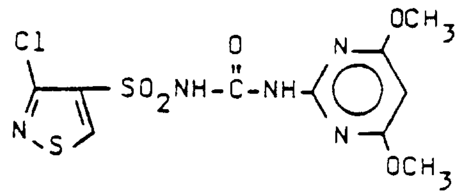

Compound 51

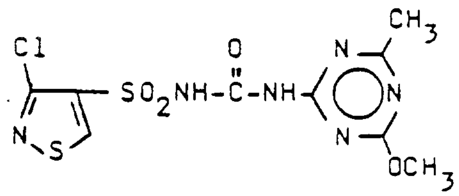

Compound 52

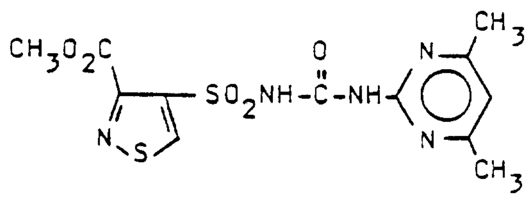

Compound 53

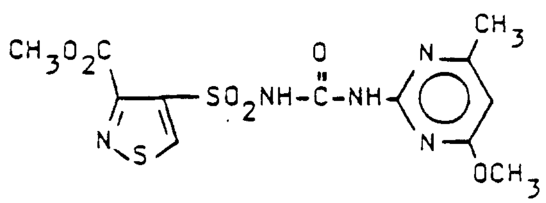

Compounds (continued)

Compound 54

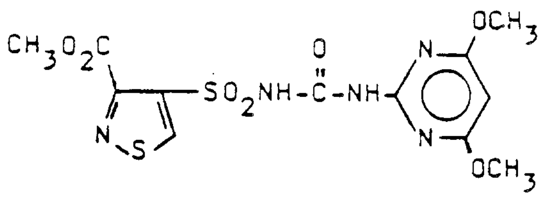

Compound 55

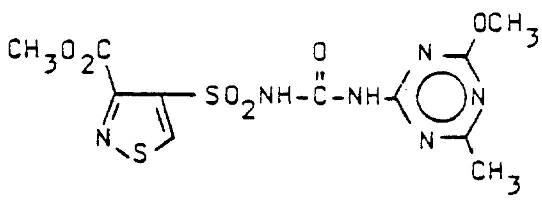

Compound 56

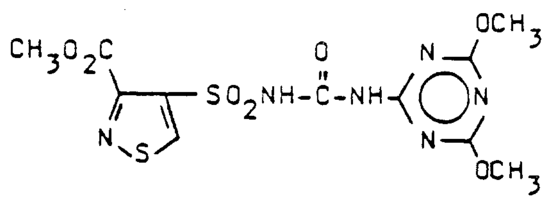

Compound 57

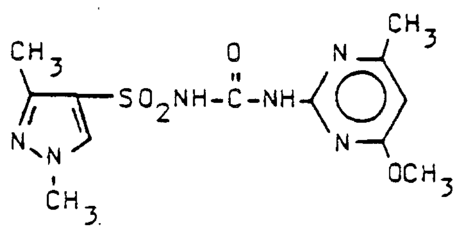

Compound 58

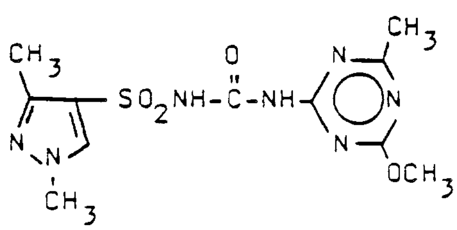

Compounds (continued)

Compound 59

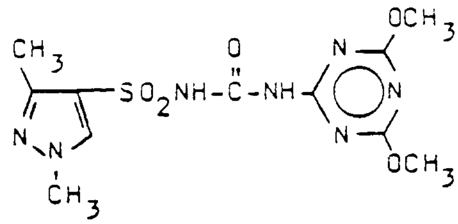

Compound 60

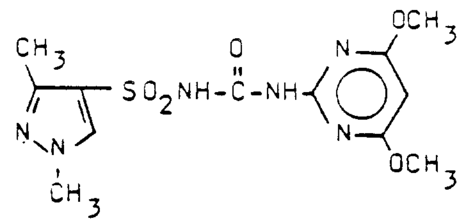

Compound 61

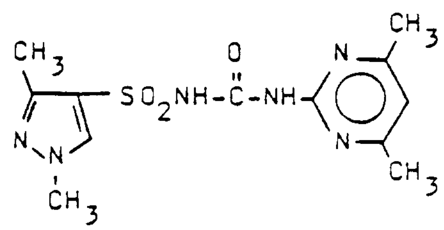

Compounds (continued)

Compound 62

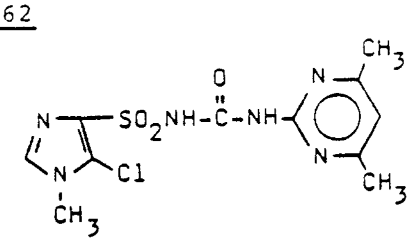

Compound 63

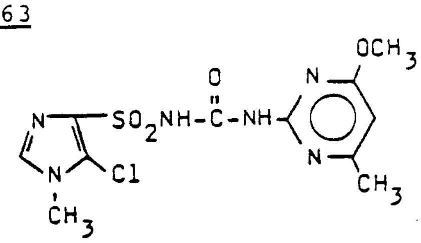

Compound 64

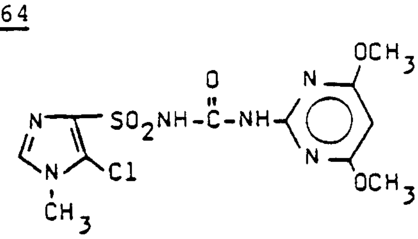

Compound 65

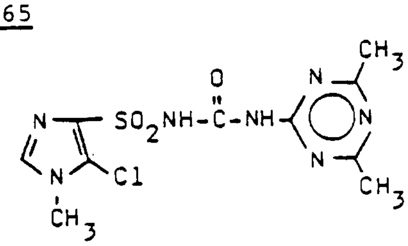

Compound 66

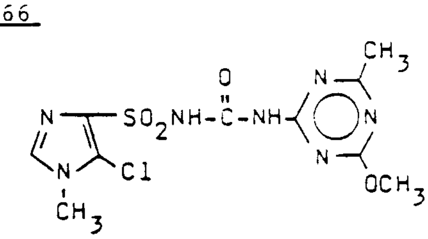

Compound 67

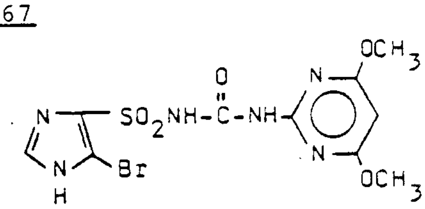

Table A

| | Cmpd. 1 | Cmpd. 2 | Cmpd. 3 | Cmpd. 4 |
|---|---|---|---|---|
| Rate kg/ha | 0.4 | 0.4 | 0.4 | 0.4 |
| POST-EMERGENCE | | | | |
| Bush bean | 9C | 5C,6G,6Y | 9C | 0 |
| Cotton | 2U,4C,9G | 5C,8G | 5C,9G | 0 |
| Morningglory | 6C,9G | 4C,9H | 9C | 0 |
| Cocklebur | 9C | 2C,8G | 10C | 0 |
| Sicklepod | 5C,9G | 2C | 5C,8G | 0 |
| Nutsedge | 2C,9G | 2C,8G | 4C,9G | 0 |
| Crabgrass | 2C,8G | 2C,7G | 2C,8G | 0 |
| Barnyardgrass | 9C | 3C,9H | 9C | 2G |
| Wild Oats | 3C,9G | 2C,9H | 9G,5X | 3G |
| Wheat | 2C,9G | 2C,9G | 6G | 2G |
| Corn | 4U,9G | 1C,5G | 7U,9G | 0 |
| Soybean | 6C,9G | 1C,4G | 6C,9G | 0 |
| Rice | 6C,9G | 5C,9G | 6C,9G | 2C,6G |
| Sorghum | 4U,9G | 3U,9G | 2U,9G | 0 |
| PRE-EMERGENCE | | | | |
| Morningglory | 9C | 9G | 10C | 0 |
| Cocklebur | 9H | 9H | 9H | 0 |
| Sicklepod | 2C,9G | 2C,4G | 9G | 0 |
| Nutsedge | 10E | 2C,7G | 10E | 0 |
| Crabgrass | 9G,5C | 2C,7G | 2C,7G | 0 |
| Barnyardgrass | 5C,9H | 5C,9H | 5C,9H | 0 |
| Wild Oats | 5C,9H | 3C,8G | 5C,9H | 0 |
| Wheat | 3C,9H | 2C,9H | 2C,9H | 2G |
| Corn | 5C,9G | 2C,8H | 9G | 3G |
| Soybean | 9H | 2C | 9H | 0 |
| Rice | 10E | 9H | 10E | 9H |
| Sorghum | 5C,9H | 2C,9H | 10H | 0 |

Table A (continued)

| | Cmpd. 5 | Cmpd. 6 | Cmpd. 7 | Cmpd. 8 |
|---|---|---|---|---|
| Rate kg/ha | 0.05 | 0.05 | 0.05 | 0.05 |
| POST-EMERGENCE | | | | |
| Bush bean | 9C | 9C | 9C | 1C |
| Cotton | 4C,9G | 5C,9G | 5C,9G | 0 |
| Morningglory | 5C,9G | 9C | 9C | 0 |
| Cocklebur | 5C,9G | 9C | 9C | 0 |
| Sicklepod | 5C,9G | 9C | 9C | 0 |
| Nutsedge | 4C,9G | 6C,9G | 9C | 0 |
| Crabgrass | 2C,9G | 6C,9G | 9C | 0 |
| Barnyardgrass | 10C | 9C | 9C | 0 |
| Wild Oats | 5C,9G | 5C,9G | 2C,9G | 0 |
| Wheat | 3U,9G | 2U,9G | 3C,9G | 0 |
| Corn | 3U,9G | 5U,9C | 4U,9G | 0 |
| Soybean | 9C | 9C | 9C | 0 |
| Rice | 2U,9G | 4C,9G | 5C,9G | 0 |
| Sorghum | 9C | 5U,9C | 3U,9C | 0 |
| PRE-EMERGENCE | | | | |
| Morningglory | 9G | 9C | 9C | 5G |
| Cocklebur | 9H | 9H | 9H | 0 |
| Sicklepod | 2C,9G | 5C,9G | 5C,9G | 2G |
| Nutsedge | 10E | 10E | 10E | 0 |
| Crabgrass | 3C,8G | 3C,8G | 3C,8G | 0 |
| Barnyardgrass | 4C,9H | 6C,9H | 5C,9H | 2C |
| Wild Oats | 5C,9H | 6C,9H | 5C,9H | 0 |
| Wheat | 9H | 10E | 9H | 0 |
| Corn | 9G | 10E | 10E | 2C |
| Soybean | 9H | 9H | 9H | 0 |
| Rice | 10E | 10E | 10E | 3C,4G |
| Sorghum | 5C,9H | 7C,9H | 5C,9H | 3G |

Table A (continued)

| | Cmpd. 9 | Cmpd. 10 | Cmpd. 11 | Cmpd. 12 |
|---|---|---|---|---|
| Rate kg/ha | 0.05 | 0.05 | 0.05 | 0.05 |
| POST-EMERGENCE | | | | |
| Bush bean | 3C,9G,6Y | 9C | 9C | 9C |
| Cotton | 1C | 6C,9G | 5C,9G | 5C,9G |
| Morningglory | 1C,3G | 4C,9G | 9C | 5C,9G |
| Cocklebur | 1C,5H | 5C,9G | 10C | 10C |
| Sicklepod | 1C | 2C,6G | 7G | 2C,8G |
| Nutsedge | 0 | 2C,7G | 2C,7G | 2C,8G |
| Crabgrass | 0 | 1C,5G | 2C,4H | 2C,5G |
| Barnyardgrass | 2C,3H | 6C,9H | 5C,9H | 5C,9H |
| Wild Oats | 2C,5G | 5C,9G | 5C,9H | 2C,9G |
| Wheat | 0 | 8G | 7G | 9G |
| Corn | 2C,8H | 2U,9G | 2U,9H | 5U,9C |
| Soybean | 0 | 5C,9G | 4C,9H | 9C |
| Rice | 4C,9G | 6C,9G | 5C,9G | 6C,9G |
| Sorghum | 4C,9H | 6C,9G | 4U,9G | 10C |
| PRE-EMERGENCE | | | | |
| Morningglory | 2C | 9G | 9C | 9G |
| Cocklebur | - | 10E | 9H | 9H |
| Sicklepod | 5G | 4C,9G | 4C,9G | 3C,9G |
| Nutsedge | 0 | 10E | 9G | 10E |
| Crabgrass | 0 | 4G,2C | 2C,5G | 3C,9G |
| Barnyardgrass | 2C,5H | 5C,9H | 5C,9H | 9H |
| Wild Oats | 2C,8G | 4C,8H | 5C,9H | 5C,9H |
| Wheat | 2C,8G | 8H | 3C,9H | 3C,9H |
| Corn | 2C,9H | 4C,7G | 9H | 9H |
| Soybean | 2G | 4C,7H | 8H | 9H |
| Rice | 10E | 10E | 10E | 10E |
| Sorghum | 2C,9H | 4C,9G | 5C,9H | 2C,9H |

Table A (continued)

| | Cmpd. 13 | Cmpd. 14 | Cmpd. 15 |
|---|---|---|---|
| Rate kg/ha | 0.05 | 0.05 | 0.05 |
| POST-EMERGENCE | | | |
| Bush bean | 5C,9G,6Y | 2C,2H | 9C |
| Cotton | 2C,8G | 2C,5G | 2C,5G |
| Morningglory | 2C | 2C | 2C,8G |
| Cocklebur | 1H | 1C | 3C,9H |
| Sicklepod | 1C | 0 | 2C,5G |
| Nutsedge | 0 | 0 | 2G |
| Crabgrass | 0 | 0 | 0 |
| Barnyardgrass | 1H | 0 | 2C,7H |
| Wild Oats | 0 | 0 | 2G |
| Wheat | 0 | 0 | 0 |
| Corn | 0 | 0 | 2C,5G |
| Soybean | 1C,5H | 0 | 3C,9H |
| Rice | 4C,9G | 3G | 7G |
| Sorghum | 3C,9H | 2G | 3C,9H |
| PRE-EMERGENCE | | | |
| Morningglory | 0 | 0 | 1C,9G |
| Cocklebur | 0 | 0 | - |
| Sicklepod | 0 | 0 | 2C,7G |
| Nutsedge | 0 | 0 | 2C,7G |
| Crabgrass | 1C | 0 | 1C |
| Barnyardgrass | 0 | 0 | 3C,8G |
| Wild Oats | 0 | 0 | 1C,7G |
| Wheat | 2G | 0 | 1C,7G |
| Corn | 2C | 0 | 3C,9H |
| Soybean | 2C | 0 | 7H |
| Rice | 3C | 3G | 3C,7H |
| Sorghum | 3C | 0 | 3C,8H |

Table A (continued)

| | Compound 16 | | Cmpd. 17 | Cmpd. 18 |
|---|---|---|---|---|
| Rate g/ha | 50 | 50 | 50 | 50 |
| POST-EMERGENCE | | | | |
| Bush bean | 3C,9G,6Y | 9C | 3C,6H,6Y | 5C,9G,6Y |
| Cotton | 0 | 5C,9G | 0 | 1C |
| Morningglory | 3C,8G | 9C | 0 | 2C,4G |
| Cocklebur | 3C,8G | 9C | 2G | 2C,9G |
| Sicklepod | 1C | 9C | 0 | 2C |
| Nutsedge | 1C | 2C,8G | 0 | 0 |
| Crabgrass | 0 | 2C,8G | 0 | 0 |
| Barnyardgrass | 1C,2H | 9C | 1H | 0 |
| Wild Oats | 0 | 2C,9G | 5G | 2C,8G |
| Wheat | 0 | 3C,9G | 2C,5G | 6G |
| Corn | 2C,7H | 2U,9G | 2C,9G | 0 |
| Soybean | 5G | 5C,9G | 0 | 2C,9G |
| Rice | 6G | 5C,9G | 5C,9G | 5C,9G |
| Sorghum | 4G | 4C,9G | 2C,8H | 2C,9G |
| Sugar beet | 2G | 5C,9G | 0 | 4C,9G |
| PRE-EMERGENCE | | | | |
| Morningglory | 5G | 9C | 0 | 5G |
| Cocklebur | 9H | 9H | 0 | 0 |
| Sicklepod | 5G | 9G | 0 | 2C,9G |
| Nutsedge | 0 | 10E | 0 | 0 |
| Crabgrass | 0 | 2C,8G | 0 | 2C |
| Barnyardgrass | 2C,2H | 2C,9H | 0 | 2C,4G |
| Wild Oats | 3G | 5C,9H | 0 | 2C,9G |
| Wheat | 0 | 5C,9H | 0 | 2C,9G |
| Corn | 2G | 10H | 0 | 2C,9G |
| Soybean | 2C | 9H | 0 | 3C,3H |
| Rice | 3C,5G | 10E | 0 | 10E |
| Sorghum | 0 | 4C,9H | 0 | 3C,9H |
| Sugar beet | 9G | 5C,9G | 0 | 5C,9G |

Table A (continued)

| | Cmpd. 19 | Cmpd. 20 | Cmpd. 21 | Cmpd. 22 |
|---|---|---|---|---|
| Rate g/ha | 50 | 50 | 400 | 50 |
| POST-EMERGENCE | | | | |
| Bush bean | 4C,5G,6Y | 4C,7G,6Y | 4C,5H,6Y | 2C,4C,6Y |
| Cotton | 1C | 2C,2H | 1C | 2C |
| Morningglory | 2C,4G | 2C,2H | 2C | 2C,4G |
| Cocklebur | 2C,6G | 3C,8G | 1C | 2C,2H |
| Sicklepod | 2C | 2G | 0 | 0 |
| Nutsedge | 2G | 0 | 0 | 0 |
| Crabgrass | 2G | 2C,2G | 2G | 2C,9G |
| Barnyardgrass | 2C,7H | 2C,4H | 1H | 5C,9H |
| Wild Oats | 5C,9G | 0 | 0 | 5C,7H |
| Wheat | 9C | 0 | 0 | 9C |
| Corn | 4C,9H | 3C,9H | 2C,4G | 5G |
| Soybean | 3C,9G,5X | 4C,6G | 0 | 3C,8G,8X |
| Rice | 5C,9G | 2C,7G | 2C,5G | 2C,9G |
| Sorghum | 4C,9G | 4C,9H | 2C,5G | 2C,9G |
| Sugar beet | 3C,7G | 4C,6G | 2C | 0 |
| PRE-EMERGENCE | | | | |
| Morningglory | 0 | 2G | 2C | 0 |
| Cocklebur | 0 | 0 | 5G | 0 |
| Sicklepod | 0 | 2G | 2G | 0 |
| Nutsedge | 0 | 0 | 0 | 0 |
| Crabgrass | 0 | 2G | 2G | 2C,5G |
| Barnyardgrass | 0 | 1C | 1C | 2H |
| Wild Oats | 0 | 2G | 2G | 2C,7G |
| Wheat | 0 | 0 | 2G | 5C,9G |
| Corn | 1C | 2C,5G | 2C | 1C |
| Soybean | 1C | 0 | 0 | 0 |
| Rice | 0 | 1C | 2C,9H | 2G |
| Sorghum | 2G | 2C,7G | 3C,7G | 2C |
| Sugar beet | 3H | 6G | 3G | 3H |

Table A (continued)

| | Cmpd. 23 | Cmpd. 24 |
|---|---|---|
| Rate g/ha | 50 | 50 |
| POST-EMERGENCE | | |
| Bush bean | 1C | 5C,8G,6Y |
| Cotton | 0 | 1C |
| Morningglory | 1C,2H | 3C,8G |
| Cocklebur | 2C,8G | 2C,9H |
| Sicklepod | 1C | 3C |
| Nutsedge | 0 | 8G |
| Crabgrass | 0 | 2C,9G |
| Barnyardgrass | 0 | 9C |
| Wild Oats | 0 | 5C,9G |
| Wheat | 0 | 6C,9G |
| Corn | 0 | 2C,9H |
| Soybean | 2H | 4C,8G |
| Rice | 0 | 5C,9G |
| Sorghum | 0 | 5C,9G |
| Sugar beet | 2C,7G | 3C,6G |
| PRE-EMERGENCE | | |
| Morningglory | 2C | 5G |
| Cocklebur | - | 5H |
| Sicklepod | 2G | 2G |
| Nutsedge | 0 | 0 |
| Crabgrass | 2G | 2C,4G |
| Barnyardgrass | 2C | 4C,9H |
| Wild Oats | 0 | 2C,9G |
| Wheat | 0 | 6C,9H |
| Corn | 0 | 2C,9G |
| Soybean | 0 | 1C,1H |
| Rice | 2C | 9H |
| Sorghum | 2G | 5C,9H |
| Sugar beet | 6G | 8G |
| Cotton | - | - |

Table A (continued)

| | Cmpd. 25 |
|---|---|
| Rate g/ha | 50 |
| POST-EMERGENCE | |
| Bush bean | - |
| Cotton | 4C,6H |
| Morningglory | 3C,8G |
| Cocklebur | 4C,9H |
| Sicklepod | 4C,9G |
| Nutsedge | 2C,9G |
| Crabgrass | 3C,9G |
| Barnyardgrass | 3C,9H |
| Wild Oats | 4C,9G |
| Wheat | 4C,9G |
| Corn | 4U,9G |
| Soybean | 2C,8G |
| Rice | 5C,9G |
| Sorghum | 4C,9G |
| Sugar beet | 5C,9H |
| PRE-EMERGENCE | |
| Morningglory | 8G |
| Cocklebur | 2C,8H |
| Sicklepod | 5G |
| Nutsedge | 9G |
| Crabgrass | 2C,6G |
| Barnyardgrass | 5C,9H |
| Wild Oats | 3C,8H |
| Wheat | 4C,9H |
| Corn | 9G |
| Soybean | 1C |
| Rice | 10E |
| Sorghum | 4C,9H |
| Sugar beet | 2C,8G |
| Cotton | 6G |

Table A (continued)

| | Cmpd. 26 | Cmpd. 27 | Cmpd. 28 |
|---|---|---|---|
| Rate g/ha | 50 | 50 | 50 |
| POST-EMERGENCE | | | |
| Bush bean | - | - | - |
| Cotton | 5C,9G | 3C,4H | 4C,9G |
| Morningglory | 9C | 1C,3G | 9C |
| Cocklebur | 2C,9G | 0 | 5C,9G |
| Sicklepod | 5C,9G | 0 | 5C,9G |
| Nutsedge | 4C,9G | 6G | 9G |
| Crabgrass | 3C,8G | 2C,5H | 2C,9G |
| Barnyardgrass | 3C,8H | 2C,8G | 3C,9H |
| Wild Oats | 4C,9G | 10C | 3C,9G |
| Wheat | 5C,9G | 2U,9G | 9C |
| Corn | 4C,9G | 3C,8G | 4U,9G |
| Soybean | 3C,9G | 5C,9G | 9C |
| Rice | 5C,9G | 3C,9G | 5C,9G |
| Sorghum | 5C,9G | 2C,8G | 4C,9G |
| Sugar beet | 9C | 2C,6H | 5C,9G |
| PRE-EMERGENCE | | | |
| Morningglory | 9G | 0 | 9G |
| Cocklebur | 8G | 0 | 9H |
| Sicklepod | 4C,9G | 0 | 9G |
| Nutsedge | 2C | 0 | 9G |
| Crabgrass | 1C | 0 | 7G |
| Barnyardgrass | 2C,3G | 0 | 2C,7H |
| Wild Oats | 5C,8H | 1C,3G | 3C,8G |
| Wheat | 6C,9H | 5C,9H | 2C,8H |
| Corn | 5C,9H | 8G | 2C,9G |
| Soybean | 4C,9H | 0 | 8H |
| Rice | 10E | 9H | 10E |
| Sorghum | 5C,9G | 2C,5G | 5C,9H |
| Sugar beet | 5C,9G | 3H | 4C,9G |
| Cotton | 7G | 0 | 9G |

Table A (continued)

| | Cmpd. 29 |
|---|---|
| Rate g/ha | 50 |
| POST-EMERGENCE | |
| Bush bean | 0 |
| Cotton | 0 |
| Morningglory | 0 |
| Cocklebur | 0 |
| Sicklepod | 0 |
| Nutsedge | 5G |
| Crabgrass | 0 |
| Barnyardgrass | 0 |
| Wild Oats | 0 |
| Wheat | 0 |
| Corn | 0 |
| Soybean | 0 |
| Rice | 0 |
| Sorghum | 0 |
| Sugar beet | 0 |
| PRE-EMERGENCE | |
| Morningglory | 0 |
| Cocklebur | 0 |
| Sicklepod | 0 |
| Nutsedge | 5G |
| Crabgrass | 0 |
| Barnyardgrass | 0 |
| Wild Oats | 0 |
| Wheat | 0 |
| Corn | 0 |
| Soybean | 0 |
| Rice | 0 |
| Sorghum | 0 |
| Sugar beet | 0 |
| Cotton | 0 |

Table A (continued)

| | Cmpd. 30 | Cmpd. 31 | Cmpd. 32 | Cmpd. 33 |
|---|---|---|---|---|
| Rate kg/ha | 0.05 | 0.05 | 0.05 | 0.05 |
| POST-EMERGENCE | | | | |
| Bush bean | 4C,9G,6Y | 2C,5G,6Y | 5S,9G,6Y | 4C,5G,6Y |
| Cotton | 4C,9G | 3C,9G | 4C,5H,9G | 5C,9G |
| Morningglory | 4C,9G | 3C,7G | 0 | 1C,3G |
| Cocklebur | 5C,9G | 9C | 10C | 10C |
| Sicklepod | 3C,9G | 4C,9G | 1C | 2H |
| Nutsedge | 9G | 9G | 0 | 0 |
| Crabgrass | 2C,6G | 2C,5G | 0 | 0 |
| Barnyardgrass | 2C,9H | 4C,8H | 0 | 0 |
| Wild Oats | 2C,8G | 2C,9G | 0 | 0 |
| Wheat | 1C,8G | 2C,9G | 0 | 0 |
| Corn | 4U,9G | 2U,9G | 3H | 1H |
| Soybean | 9C | 9C | 3H | 5C,9G |
| Rice | 5C,9G | 4C,9G | 0 | 0 |
| Sorghum | 9G | 2C,9G | 2G | 0 |
| Sugar beet | - | - | - | - |
| PRE-EMERGENCE | | | | |
| Morningglory | 9G | 8G | 6G | 5G |
| Cocklebur | 8H | 8H | 8H | 8H |
| Sicklepod | 9G | 9G | 5G | 7G |
| Nutsedge | 3G | 10E | 0 | 5G |
| Crabgrass | 3C,7G | 5G | 1C | 0 |
| Barnyardgrass | 3C,7G | 3C,6G | 2C | 2C |
| Wild Oats | 3C,8H | 6G | 0 | 0 |
| Wheat | 9H | 2C,8G | 0 | 0 |
| Corn | 3C,9G | 2C,8G | 2C,6H | 2C,6G |
| Soybean | 8H | 1H | 1C | 2C |
| Rice | 9H | 9H | 2G | 2C |
| Sorghum | 3C,9H | 2C,9G | 0 | 2G |
| Sugar beet | - | - | - | - |
| Cotton | - | - | - | - |

Table A (continued)

Compound 34

| Rate kg/ha | 0.4 | 2 |
|---|---|---|
| POST-EMERGENCE | | |
| Bush bean | 2G | - |
| Cotton | - | 4C,9G |
| Morningglory | 2C,2H | 3C,6H |
| Cocklebur | 3C,7H | 5C,9H |
| Sicklepod | 1C | 3C,3H |
| Nutsedge | 0 | 5G |
| Crabgrass | 0 | 5G |
| Barnyardgrass | 3H | 5C,9H |
| Wild Oats | 0 | 0 |
| Wheat | 0 | 2G |
| Corn | 2C,2H | 2C,6H |
| Soybean | 1H | 2C,8G |
| Rice | 0 | 4C,9G |
| Sorghum | 2G | 3C,8H |
| Sugar beet | - | 5C,9G |
| PRE-EMERGENCE | | |
| Morningglory | 0 | 9G |
| Cocklebur | 0 | 9H |
| Sicklepod | 0 | 7G |
| Nutsedge | 0 | 0 |
| Crabgrass | 0 | 0 |
| Barnyardgrass | 0 | 3G |
| Wild Oats | 0 | 0 |
| Wheat | 0 | 2G |
| Corn | 0 | 2C,5G |
| Soybean | 0 | 2C |
| Rice | 2C,5G | 9H |
| Sorghum | 0 | 4C,8H |
| Sugar beet | - | 4C,9G |
| Cotton | - | 6G |

Table A (continued)

| | Compound 35 | | Compound 36 | |
|---|---|---|---|---|
| Rate kg/ha | 0.05 | 0.4 | 0.05 | 0.4 |
| POST-EMERGENCE | | | | |
| Bush bean | 6C,9G,6Y | 9C | 3C,5G,6Y | 9C |
| Cotton | - | - | - | - |
| Morningglory | 4C,8H | 7C | 2C | 2C,9H |
| Cocklebur | 3C,8G | 9C | 3C,9H | 10C |
| Sicklepod | 1C | 2C | 2C | 1C,2G |
| Nutsedge | 0 | 2C | 2C,3G | 3C,8G |
| Crabgrass | 2C | 2C,7G | 2C | 2C,5G |
| Barnyardgrass | 3C,8H | 5C,9H | 2C,3H | 2C,9H |
| Wild Oats | 0 | 0 | 0 | 0 |
| Wheat | 0 | 2G | 0 | 0 |
| Corn | 2C,5H | 2C,9H | 2C,5H | 2C,9G |
| Soybean | 2C,3H | 3C,8G | 2C,5H | 5C,9G |
| Rice | 3G | 2C,8G | 3G | 2C,8G |
| Sorghum | 1C,3G | 3C,7G | 2C,4G | 3C,9H |
| Sugar beet | - | - | - | - |
| PRE-EMERGENCE | | | | |
| Morningglory | 8G | 8H | 3G | 9H |
| Cocklebur | - | 9H | 8H | 9H |
| Sicklepod | 2G | 1C | 2C | 1C |
| Nutsedge | 0 | 0 | 0 | 2C,8G |
| Crabgrass | 0 | 2C,5G | 2G | 2C,5G |
| Barnyardgrass | 3H | 5C,9H | 2G | 4C,9H |
| Wild Oats | 0 | 1C | 0 | 0 |
| Wheat | 0 | 7G | 0 | 0 |
| Corn | 1C,3G | 3C,9H | 2C | 2C,7G |
| Soybean | 1C,1H | 3C,5H | 1C | 3C,6H |
| Rice | 2C,7G | 10E | 2C,6G | 4C,9H |
| Sorghum | 2G | 3C,9H | 2G | 3C,7H |
| Sugar beet | - | - | - | - |
| Cotton | - | - | - | - |

Table A (continued)

| | Compound 37 | Compound 38 |
|---|---|---|
| Rate kg/ha | 2 | 2 |
| POST-EMERGENCE | | |
| Bush bean | - | - |
| Cotton | 0 | 0 |
| Morningglory | 1C | 3G |
| Cocklebur | 2C,5H | 0 |
| Sicklepod | 1C | 0 |
| Nutsedge | 0 | 0 |
| Crabgrass | 0 | 1C,4G |
| Barnyardgrass | 5C,9H | 3C,9H |
| Wild Oats | 0 | 0 |
| Wheat | 2C,6G | 2G |
| Corn | 3U,9G | 8G |
| Soybean | 1C | 0 |
| Rice | 5C,9G | 3C,8G |
| Sorghum | 3C,8H | 1C,4H |
| Sugar beet | 1C,2H | 0 |
| PRE-EMERGENCE | | |
| Morningglory | 0 | 0 |
| Cocklebur | 2G | 0 |
| Sicklepod | - | - |
| Nutsedge | 0 | 0 |
| Crabgrass | 0 | 0 |
| Barnyardgrass | 2G | 2C |
| Wild Oats | 0 | 0 |
| Wheat | 0 | 0 |
| Corn | 2C,3G | 2C,4G |
| Soybean | 0 | 0 |
| Rice | 4C,9H | 3C,9H |
| Sorghum | 3C,6G | 3C,7G |
| Sugar beet | 0 | 0 |
| Cotton | 0 | - |

Table A (continued)

| | Compound 39 | | Cmpd. 40 | Cmpd. 41 |
|---|---|---|---|---|
| Rate kg/ha | 0.05 | 0.4 | 0.05 | 0.05 |
| POST-EMERGENCE | | | | |
| Bush bean | 4C,9G,6Y | - | 5C,9G,6Y | 9C |
| Cotton | 4C,5G | 3C,9G | 4C,8G | 4C,9H |
| Morningglory | 3C | 3C,6G | 4C,8G | 4C,9H |
| Cocklebur | 3C,8H | 5C,9G | 9C | 10C |
| Sicklepod | 2C | 3C,8G | 3C,6H | 5C,9H |
| Nutsedge | 0 | 3C,9G | 2C,7G | 3C,8G |
| Crabgrass | 0 | 1C,3G | 1C | 4G |
| Barnyardgrass | 1C | 3C,8H | 2C,7H | 9C |
| Wild Oats | 0 | 1C,9G | 1C,4G | 5G |
| Wheat | 0 | 3C,9G | 8G,5X | 7G |
| Corn | 2C | 3C,9G | 2C,9H | 2U,9G |
| Soybean | 1C,2G | 6C,9G | 5C,9G | 9C |
| Rice | 2C,6G | 3C,8G | 4C,9G | 2C,9G |
| Sorghum | 3C,9H | 3C,9G | 4C,9H | 3C,9G |
| Sugar beet | 3C,8H | 5C,9G | 3C,8H | 10C |
| PRE-EMERGENCE | | | | |
| Morningglory | 1C | 3C,9G | 9G | 9C |
| Cocklebur | 6H | 9H | 9H | 9H |
| Sicklepod | 1C,5G | 2C,5G | 9G,2C | 9G |
| Nutsedge | 0 | 2C,8G | 7G | 8G |
| Crabgrass | 0 | 3G | 0 | 1C |
| Barnyardgrass | 1C | 2C | 2C,6G | 3C,8H |
| Wild Oats | 0 | 3C,8H | 3G | 2C,7G |
| Wheat | 0 | 1C,7G | 2C,9H | 2C,9G |
| Corn | 2C | 3C,9H | 2C,9H | 2C,9H |
| Soybean | 0 | 3G | 2C,5H | 2C,6H |
| Rice | 2C | 4C,9H | 3C,8H | 3C,9H |
| Sorghum | 2C,7G | 4C,9H | 3C,9H | 3C,9H |
| Sugar beet | 1C,2G | 9C | 10E | 10E |
| Cotton | - | 9C | - | - |

Table A (continued)

| | Cmpd. 42 | Compound 43 | |
|---|---|---|---|
| Rate kg/ha | 0.05 | 0.05 | 0.4 |
| POST-EMERGENCE | | | |
| Bush bean | 4C,9G,6Y | 5C,8G,6Y | - |
| Cotton | 2C,4G | 2C | 3C,5G |
| Morningglory | 2C,4H | 2C | 3C,5G |
| Cocklebur | 2C | 2C | 3C,8H |
| Sicklepod | 2C | 2C | 3C,5H |
| Nutsedge | 4G | 0 | 1C,4G |
| Crabgrass | 0 | 0 | 1C,3G |
| Barnyardgrass | 1C | 1C,2H | 2C,6H |
| Wild Oats | 0 | 0 | 4G |
| Wheat | 3G | 0 | 3G |
| Corn | 2C,8H | 1C | 1U,8G |
| Soybean | 2C,4G | 1C,2H | 6C,9G |
| Rice | 5C,9G | 5C,9G | 5C,9G |
| Sorghum | 2C,9G | 1C,5G | 3C,9H |
| Sugar beet | 3C,7H | 2C,5G | 3C,8H |
| PRE-EMERGENCE | | | |
| Morningglory | 1C,2H | 2C | 9G |
| Cocklebur | 5G | 0 | 8G |
| Sicklepod | 1C | 1C | 8G |
| Nutsedge | 0 | 0 | 0 |
| Crabgrass | 0 | 0 | 0 |
| Barnyardgrass | 1C | 0 | 2C |
| Wild Oats | 2C | 0 | 2C,8G |
| Wheat | 3C,8G | 0 | 2C,8G |
| Corn | 2C,6G | 1C | 3C,8H |
| Soybean | 1C | 1H | 2C,2H |
| Rice | 2C,9H | 6H | 10E |
| Sorghum | 4C,9H | 2C,6G | 4C,9H |
| Sugar beet | 1C,1H | 0 | 4C,8G |
| Cotton | - | - | 6G |

Table A (continued)

| | Cmpd. 44 | Cmpd. 45 | Cmpd. 46 |
|---|---|---|---|
| Rate kg/ha | 0.05 | 0.05 | 0.05 |
| POST-EMERGENCE | | | |
| Bush bean | 3S,8G,6Y | 9C | 5C,9G,6Y |
| Cotton | 2C,3G | 3C,9G | 4C,8G |
| Morningglory | 2C | 5C,9G | 5C,8G |
| Cocklebur | 2C | 4C,9G | 5C,9G |
| Sicklepod | 2C | 6C,9G | 6C,9G |
| Nutsedge | 1C | 2C,8G | 9G |
| Crabgrass | 1C,4G | 4C,9H | 4C,8G |
| Barnyardgrass | 3C,6G | 4C,9H | 5C,9G |
| Wild Oats | 0 | 3C,9G | 2C,7G |
| Wheat | 0 | 3C,9G | 5C,9G |
| Corn | 1C,3G | 2C,9G | 5U,9C |
| Soybean | 2C,8G | 5C,9G | 9C |
| Rice | 4C,8G | 5C,9G | 4C,9G |
| Sorghum | 4C,9G | 5C,9G | 3C,9G |
| Sugar beet | - | - | - |
| PRE-EMERGENCE | | | |
| Morningglory | 2G | 9G | 9G |
| Cocklebur | 0 | 8H | 8H |
| Sicklepod | 1H | 9G | 9G |
| Nutsedge | 0 | 6G | 10E |
| Crabgrass | 0 | 2C,8G | 3C,6G |
| Barnyardgrass | 1C | 6C,9H | 3C,9H |
| Wild Oats | 1H | 4C,7G | 2C,6H |
| Wheat | 0 | 2C,9G | 9G |
| Corn | 2C,3G | 3C,9G | 4C,9G |
| Soybean | 1C,1H | 3C,9H | 5H |
| Rice | 0 | 3C,9H | 9H |
| Sorghum | 3C,4G | 9H | 5C,9H |
| Sugar beet | - | - | - |
| Cotton | - | - | - |

Table A (continued)

| | Cmpd. 47 | Cmpd. 48 |
|---|---|---|
| Rate kg/ha | 0.05 | 0.05 |
| POST-EMERGENCE | | |
| Bush bean | 3C,9G,6Y | 0 |
| Cotton | 4C,8G | 0 |
| Morningglory | 3C,7H | 0 |
| Cocklebur | 2C,8G | 0 |
| Sicklepod | 3C,5H | 0 |
| Nutsedge | 9G | 0 |
| Crabgrass | 3C,3G | 0 |
| Barnyardgrass | 1C | 0 |
| Wild Oats | 0 | 0 |
| Wheat | 2G | 0 |
| Corn | 9G,5I | 0 |
| Soybean | 3C,9G | 0 |
| Rice | 5C,9G | 0 |
| Sorghum | 9G,5I | 0 |
| Sugar beet | - | - |
| PRE-EMERGENCE | | |
| Morningglory | 2C,7H | 0 |
| Cocklebur | 2G | 6H |
| Sicklepod | 4G | 5H |
| Nutsedge | 0 | 0 |
| Crabgrass | 3G | 0 |
| Barnyardgrass | 2C | 0 |
| Wild Oats | 4G | 0 |
| Wheat | 1C,6G | 0 |
| Corn | 2C,7G | 0 |
| Soybean | 2C,2H | 0 |
| Rice | 9H | 0 |
| Sorghum | 2C,9G | 0 |
| Sugar beet | - | - |
| Cotton | - | - |

Table A (continued)

| | Cmpd. 49 | Cmpd. 50 | Cmpd. 51 |
|---|---|---|---|
| Rate kg/ha | 0.05 | 0.05 | 0.05 |
| POST-EMERGENCE | | | |
| Bush bean | 2C,5G,6Y | 1C | 1C |
| Cotton | 4C,8G | 2C,2H | 1C,2H |
| Morningglory | 3C | 0 | 0 |
| Cocklebur | 3C,9H | 3H | 1C,4G |
| Sicklepod | 1C,3G | 0 | 0 |
| Nutsedge | 2C,5G | 0 | 0 |
| Crabgrass | 3H | 0 | 0 |
| Barnyardgrass | 3C,6H | 0 | 0 |
| Wild Oats | 0 | 0 | 0 |
| Wheat | 1C,2G | 0 | 0 |
| Corn | 2C.8H | 0 | 0 |
| Soybean | 1C,4H | 0 | 1H |
| Rice | 3C,8G | 0 | 0 |
| Sorghum | 9H | 0 | 0 |
| Sugar beet | - | - | - |
| PRE-EMERGENCE | | | |
| Morningglory | 3G | 0 | 0 |
| Cocklebur | 9H | 8H | 5H |
| Sicklepod | 6H | 4G | 0 |
| Nutsedge | 0 | 0 | 0 |
| Crabgrass | 2C | 0 | 0 |
| Barnyardgrass | 3C,7H | 0 | 0 |
| Wild Oats | 2C | 0 | 0 |
| Wheat | 1C | 0 | 0 |
| Corn | 3C,7G | 0 | 0 |
| Soybean | 1C | 0 | 0 |
| Rice | 2C,5H | 0 | 0 |
| Sorghum | 2C,8H | 0 | 0 |
| Sugar beet | - | - | - |
| Cotton | - | - | - |

Table A (continued)

| | Cmpd. 52 | Cmpd. 53 | Cmpd. 54 | Cmpd. 55 |
|---|---|---|---|---|
| Rate kg/ha | 0.05 | 0.05 | 0.05 | 0.05 |
| POST-EMERGENCE | | | | |
| Bush bean | 1C | 3C,3H,6Y | 2C,2G | 4C,8G,6Y |
| Cotton | 2C | 4C | 4C,8G | 4C |
| Morningglory | 2C,4H | 4C,6H | 3C,6G | 3C |
| Cocklebur | 4G | 2C,6G | 5C,9G | 4C,8G |
| Sicklepod | 0 | 0 | 0 | 2C |
| Nutsedge | 2G | 0 | 4G | 0 |
| Crabgrass | 0 | 0 | 2G | 0 |
| Barnyardgrass | 3H | 3C,8H | 3C,8H | 1C,1H |
| Wild Oats | 0 | 0 | 0 | 0 |
| Wheat | 0 | 0 | 0 | 0 |
| Corn | 2H | 2C,3H | 2H | 3H |
| Soybean | 0 | 3G | 1C,6G | 2G |
| Rice | 0 | 2C,7G | 1C,7G | 0 |
| Sorghum | 2C,8H | 2C,9G | 4C,9H | 0 |
| Sugar beet | 4C,9G | 4C,9G | 5C,9G | 5C,8G |
| PRE-EMERGENCE | | | | |
| Morningglory | 5C,7H | 9G | 9G | 3C,6H |
| Cocklebur | 6H | - | 9H | 5H |
| Sicklepod | 1C | 7H | 8G | 2C |
| Nutsedge | 0 | 0 | 3G | 0 |
| Crabgrass | 0 | 2G | 2G | 1C |
| Barnyardgrass | 0 | 4C,8H | 3C,8H | 1C |
| Wild Oats | 0 | 2C,6G | 3C,4G | 0 |
| Wheat | 0 | 8G | 2C,6G | 0 |
| Corn | 2C,5G | 2C,6G | 2C,6G | 2G |
| Soybean | 0 | 0 | 0 | 1C |
| Rice | 2C,6G | 9H | 10E | 2C,3G |
| Sorghum | 2C,8G | 9H | 3C,8H | 1C,4G |
| Sugar beet | 5C,9G | 5C,9G | 5C,9G | 3C,8G |
| Cotton | - | - | - | - |

Table A (continued)

| | Cmpd. 56 | Cmpd. 57 | Cmpd. 58 | Cmpd. 59 |
|---|---|---|---|---|
| Rate kg/ha | 0.05 | 0.05 | 0.05 | 0.05 |
| POST-EMERGENCE | | | | |
| Bush bean | 2C,2H | - | - | - |
| Cotton | 2C | 9G,5C | 9G,5C | 4C,8G |
| Morningglory | 3C | 9C | 2C,8G | 3C,6H |
| Cocklebur | 0 | 5C,9G | 4C,9H | 3G |
| Sicklepod | 0 | 6C,9G | 4C,9G | 3C,3G |
| Nutsedge | 0 | 2C,9G | 0 | 0 |
| Crabgrass | 0 | 3C,9G | 2C,6G | 2H |
| Barnyardgrass | 0 | 5C,9G | 5C,9H | 3C,8H |
| Wild Oats | 0 | 9C | 5C,9G | 5G |
| Wheat | 0 | 9C | 9C | 8G |
| Corn | 0 | 5C,9G | 10C | 2U,9G |
| Soybean | 0 | 5C,9G | 3C,9H | 4C,9G |
| Rice | 0 | 5C,9G | 4C,9G | 5C,9G |
| Sorghum | 5H | 5C,9G | 4C,9G | 4C,9H |
| Sugar beet | 4C,8G | 5C,9G | 4C,8G | 2C,5G |
| PRE-EMERGENCE | | | | |
| Morningglory | 2C,5H | 9G | 4G | 4G |
| Cocklebur | 0 | 9H | 1H | 0 |
| Sicklepod | 0 | 3C,9G | 5G | 0 |
| Nutsedge | 0 | 8G | 0 | 0 |
| Crabgrass | 0 | 3C,8G | 0 | 0 |
| Barnyardgrass | 2C | 4C,9H | 3C,7G | 0 |
| Wild Oats | 2C | 9C | 3C,8G | 2C |
| Wheat | 2C | 10C | 4C,9H | 7G |
| Corn | 0 | 5C,9H | 2U,8G | 2C,6G |
| Soybean | 0 | 8H | 3C,3G | 2C |
| Rice | 1C | 10E | 10E | 9H |
| Sorghum | 5G | 5C,9H | 4C,9H | 2C,7H |
| Sugar beet | 3C,7G | 5C,9G | 5G | 0 |
| Cotton | - | 2C,9G | 5G | 0 |

Table A (continued)

| | Cmpd. 60 | Cmpd. 61 |
|---|---|---|
| Rate kg/ha | 0.05 | 0.05 |
| POST-EMERGENCE | | |
| Bush bean | - | - |
| Cotton | 9H | 9H |
| Morningglory | 3C,7H | 2C,2G |
| Cocklebur | 9C | 2C,9H |
| Sicklepod | 5C,9G | 4C,6H |
| Nutsedge | 9G | 4C,8G |
| Crabgrass | 2C,9G | 3C,7H |
| Barnyardgrass | 2C,9H | 3C,7H |
| Wild Oats | 2C,9G | 2C,9G |
| Wheat | 9C | 10C |
| Corn | 5C,9G | 2C,9G |
| Soybean | 6C,9G | 3C,9G |
| Rice | 6C,9G | 5C,9G |
| Sorghum | 9G | 3C,9H |
| Sugar beet | 6C,9G | 3C,8H |
| PRE-EMERGENCE | | |
| Morningglory | 9G | 2C |
| Cocklebur | 9H | 2C,2H |
| Sicklepod | 9G | 5G |
| Nutsedge | 5C,9G | 0 |
| Crabgrass | 6G | 1C |
| Barnyardgrass | 3C,7G | 1C |
| Wild Oats | 5C,7G | 3C,7G |
| Wheat | 5C,9H | 2C,8G |
| Corn | 9G | 3C,8G |
| Soybean | 4C,5G | 2C |
| Rice | 10E | 2C,8G |
| Sorghum | 2C,9H | 4C,7G |
| Sugar beet | 4C,9G | 4H |
| Cotton | 8G | 1C |

Table A (continued)

| | Compound 62 | | Cmpd. 63 | Cmpd. 64 |
|---|---|---|---|---|
| Rate kg/ha | 2 | 0.05 | 0.05 | 0.05 |
| POST-EMERGENCE | | | | |
| Bush bean | - | - | - | - |
| Cotton | 3C,8G | 0 | 4C,7H | 4C,9G |
| Morningglory | 3C,8G | 0 | 2C,7G | 3C,8G |
| Cocklebur | 2C,9G | 0 | 3C,5G | 3C,9G |
| Sicklebod | 2C | 0 | 2C,5G | 3C,9G |
| Nutsedge | 9G | 0 | 1C,3G | 3C,9G |
| Crabgrass | 5C,9G | 0 | 2C,4H | 2C,5G |
| Barnyardgrass | 4C,9H | 1H | 2C,6G | 2C,6H |
| Wild Oats | 4C,9G | 3G | 2C,8G | 2C,9G |
| Wheat | 9C | 0 | 2C,8G | 2C,5G |
| Corn | 2C,9G | 3H | 2C,9G | 2U,9G |
| Soybean | 3C,7G | 1C,2H | 4C,9G | 5C,9G |
| Rice | 4C,9G | 3C,9G | 5C,9G | 5C,9G |
| Sorghum | 4C,9H | 2C,5H | 5C,9G | 2C,8G |
| Sugar beet | 9C | 0 | 5C,9G | 5C,9G |
| PRE-EMERGENCE | | | | |
| Morningglory | 9G | 1C | 8G | 9G |
| Cocklebur | 9H | 6H | 9H | 8H |
| Sicklepod | 1C,9G | 2C | 3G | 8G |
| Nutsedge | 10E | 0 | 0 | 2C,8G |
| Crabgrass | 9C | 0 | 0 | 3G |
| Barnyardgrass | 5C,9H | 0 | 0 | 2C,3H |
| Wild Oats | 5C,9G | 0 | 3C | 2C,9G |
| Wheat | 4C,9G | 0 | 2C,6G | 2C,9G |
| Corn | 5C,9H | 1C | 3C,5G | 8G |
| Soybean | 3C,8H | 1H | 2C,8G | 2C,6G |
| Rice | 10E | 0 | 3C,3H | 10E |
| Sorghum | 10H | 0 | 3C,6G | 3C,9G |
| Sugar beet | 2H | 10E | 4C,7H | 5C,9G |
| Cotton | 9G | 0 | 4C,7G | 2C,8G |

Table A (continued)

| | Compound 65 | | Compound 66 | |
|---|---|---|---|---|
| Rate kg/ha | 0.05 | 0.4 | 0.05 | 0.4 |
| POST-EMERGENCE | | | | |
| Bush bean | - | - | - | - |
| Cotton | 0 | 0 | 0 | 0 |
| Morningglory | 2C | 3G | 0 | 0 |
| Cocklebur | 2G | 0 | 0 | 0 |
| Sicklepod | 0 | 0 | 0 | 0 |
| Nutsedge | 0 | 0 | 0 | 0 |
| Crabgrass | 0 | 0 | 0 | 0 |
| Barnyardgrass | 0 | 0 | 3C | 9H |
| Wild Oats | 0 | 0 | 2C | 9G |
| Wheat | 0 | 0 | 0 | 9C |
| Corn | 0 | 0 | 0 | 0 |
| Soybean | 1C | 1H | 1C | 0 |
| Rice | 0 | 3G | 3C | 9H |
| Sorghum | 0 | 0 | 2C | 9H |
| Sugar beet | 0 | 0 | 0 | 0 |

Table A (continued)

| | Cmpd. 67 |
|---|---|
| Rate kg/ha | 0.05 |
| POST-EMERGENCE | |
| Bush bean | - |
| Cotton | 9H |
| Morningglory | 3C,8G |
| Cocklebur | 1C |
| Sicklepod | 3C,7H |
| Nutsedge | 9G |
| Crabgrass | 5G |
| Barnyardgrass | 0 |
| Wild Oats | 2G |
| Wheat | 2G |
| Corn | 3C,9G |
| Soybean | 3C,9G |
| Rice | 5C,9G |
| Sorghum | 3C,9G |
| Sugar beet | 4C,9G |
| PRE-EMERGENCE | |
| Morningglory | 6G |
| Cocklebur | 5G |
| Sicklepod | 3C,8G |
| Nutsedge | 9G |
| Crabgrass | 2G |
| Barnyardgrass | 3C,6G |
| Wild Oats | 3C,5G |
| Wheat | 4G |
| Corn | 9G |
| Soybean | 3C,3H |
| Rice | 10E |
| Sorghum | 5C,9H |
| Sugar beet | 5C,9G |
| Cotton | 2C,8G |

## Claims

### Claims for the following Contracting States : CH, DE, FR, GB, IT, LI, NL

**1.** A compound of the formula:

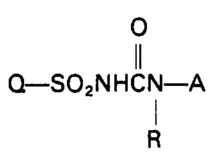

where

R is H or $CH_3$;

Q is

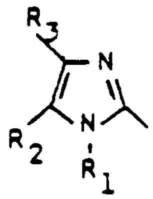,

Q-1

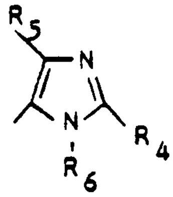,

Q-2

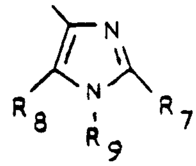,

Q-3

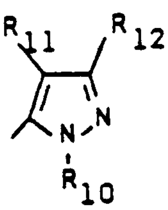,

Q-4

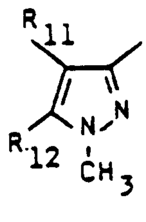

Q-5

or

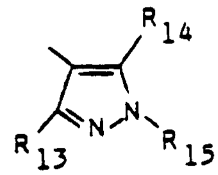;

Q-6

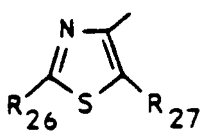,

Q-7

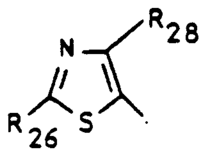,

Q-8

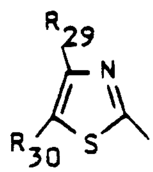

Q-9

or

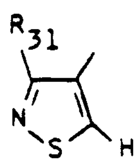;

Q-10

$R_1$ is H, $C_1$-$C_8$ alkyl, $C_3$-$C_6$ alkenyl, $C_5$-$C_6$ cycloalkyl, $C_5$-$C_6$ cycloalkenyl, $C_3$-$C_6$ alkynyl, $C_4$-$C_7$ cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

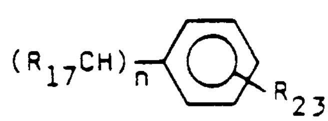;

or $C_1$-$C_6$ alkyl
substituted either with
a) 1-3 atoms of F, Br or Cl; or
b) $OR_{16}$;
$R_2$, $R_3$ and $R_4$ are independently H or $CH_3$;
$R_5$ is H, $C_1$-$C_4$ alkyl, $-OR_6$, $NO_2$, F, Cl, Br, $CO_2R_{24}$, $S(O)_mR_{25}$ or $SO_2NR_{20}R_{21}$;
$R_6$ is H, $C_1$-$C_4$ alkyl, $C_3$-$C_4$ alkenyl, $C_3$-$C_4$ alkynyl, $CO_2R_{18}$, $SO_2NR_{20}R_{21}$, $SO_2R_{22}$ or $C_1$-$C_4$ alkyl substituted with a) 1-3 atoms of F, Cl or Br; or b) $OCH_3$;
$R_7$ is H or $CH_3$;
$R_8$ is H, $C_1$-$C_4$ alkyl, $-OR_{16}$, $NO_2$, F, Cl, Br, $CO_2R_{24}$, $S(O)_mR_{25}$ or $SO_2NR_{20}R_{21}$;
$R_9$ is $CH_3$ or $C_2H_5$;

$R_{10}$ is H, $C_1$-$C_4$ alkyl, $C_3$-$C_4$ alkenyl, $C_3$-$C_4$ alkynyl, $CO_2R_{24}$, $SO_2NR_{20}R_{21}$ or $SO_2R_{22}$;
$R_{11}$ is H, $C_1$-$C_3$ alkyl, F, Cl, Br, $NO_2$, $-OR_{16}$, $CO_2R_{24}$, $S(O)_mR_{25}$ or $SO_2NR_{20}R_{21}$;
$R_{12}$ is H or $CH_3$;
$R_{13}$ and $R_{14}$ are independently H, $C_1$-$C_3$ alkyl $-OR_{16}$, F, Cl, Br, $NO_2$, $CO_2R_{24}$, $S(O)_mR_{25}$ or $SO_2NR_{20}R_{21}$;
$R_{15}$ is H or $CH_3$;
$R_{16}$ is $C_1$-$C_3$ alkyl;
$R_{17}$ is H or $CH_3$;
$R_{18}$ is $C_1$-$C_4$ alkyl, $C_3$-$C_4$ alkenyl, $C_3$-$C_4$ alkynyl, $CH_2CH_2Cl$ or $CH_2CH_2OCH_3$;
$R_{19}$ is $C_1$-$C_4$ alkyl, $C_3$-$C_4$ alkenyl, $C_3$-$C_4$ alkynyl or $CH_2CH_2OCH_3$;
$R_{20}$ and $R_{21}$ are independently $CH_3$ or $C_2H_5$;
$R_{22}$ is $C_1$-$C_3$ alkyl or $CF_3$;
$R_{23}$ is H, Cl, Br, $CH_3$, F, $CF_3$, $OCH_3$ or $NO_2$;
$R_{24}$ is $C_1$-$C_3$ alkyl or allyl;
$R_{25}$ is $C_1$-$C_3$ alkyl;
m is 0, 1 or 2;
n is 0 or 1;
$R_{26}$ is H, $C_1$-$C_3$ alkyl or $CH_3C(O)NH$;
$R_{27}$ a H, $C_1$-$C_3$ alkyl, $C_1$-$C_3$ alkoxy, $C_1$-$C_3$ alkylthio or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
$R_{31}$ is Cl, $C_1$-$C_3$ alkoxycarbonyl or $C_1$-$C_3$ alkyl;
A is

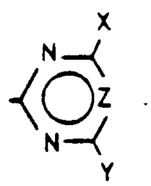 ,
A-1

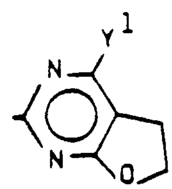 ,
A-2

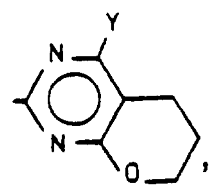 ,
A-3

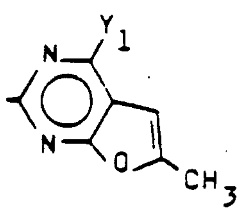 ,
A-4

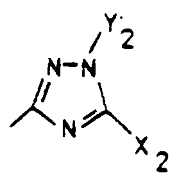 or
A-5

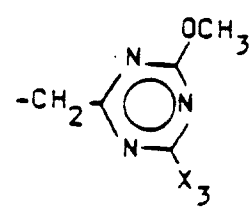 ;
A-6

X is $CH_3$, $OCH_3$, Cl, F, $OCF_2H$ or $SCF_2H$;
Y is $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ or $GCF_2T$ where G is O or S and T is H, CHClF, CHBrF, $CF_2H$ or $CHFCF_3$;
Z is CH or N;
$Y_1$ is H, Cl, $CH_3$, $OCH_3$ or $OCF_2H$;
$X_2$ is $OCH_3$, $CH_3$, $CH_2CH_3$, $OCH_2CH_3$, $SCH_3$ or $SCH_2CH_3$;
$Y_2$ is $CH_3$, $CH_2CH_3$ or $CH_2CF_3$;
$X_3$ is $OCH_3$ or $CH_3$;

provided that,
a) when Q is Q-7, Q-8, Q-9 or Q-10, then R is H;
b) the total number of carbon atoms in $R_1$ is less than or equal to 8;
c) if $R_1$ is other than $C_1$-$C_3$ alkyl, then $R_3$ must be H;

d) when $R_5$ is other than H, $CH_3$, $OCH_3$ or $NO_2$, then $R_6$ is H or $CH_3$;
e) when $R_6$ is $CO_2R_{18}$, $SO_2NR_{20}R_{21}$ or $SO_2R_{22}$,then $R_5$ is H, $CH_3$, $OCH_3$ or $NO_2$;
f) when $R_{10}$ is other than $C_1$-$C_3$ alkyl, then $R_{11}$ is H, Cl, $OCH_3$, $NO_2$ or $CH_3$;
g) when either of $R_{13}$ or $R_{14}$ is $CO_2R_{24}$, $S(O)_mR_{25}$ or $SO_2NR_{20}R_{21}$, then the other is H, Cl, $CH_3$, $OCH_3$ or $NO_2$;
h) $R_{29}$ and $R_{30}$ are not simultaneously Cl;
i) when X is Cl or F, then Z is CH and Y is $OCH_3$, $NH_2$, $NHCH_3$ or $N(CH_3)_2$;
j) when Q is Q-7, Q-8, Q-9 or Q-10, then A is A-1; X is $CH_3$, $OCH_3$ or $OCF_2H$, Y is $CH_3$, $OCH_3$, $OC_2H_5$, $OCF_2H$ or $CH_2OCH_3$; and,
k) when either X or Y is $OCF_2H$, then Z is CH;
  $k_1$) when Q is Q-2, R is H, Z is CH and when one of X and Y is $OCH_3$ then the other is not $CH_3$ when $R_4$, $R_5$ and $R_6$ are H;
l) when Q is Q-6, R is H, Z is CH and Y is $CH_3$ or $OCH_3$, then X is other than $CH_3$ or $OCH_3$ when
  (1) $R_{13}$ and $R_{14}$ are $CH_3$ and $R_{15}$ is H or $CH_3$;
  (2) $R_{13}$ and $R_{15}$ are $CH_3$ and $R_{14}$ is Br;
  (3) $R_{13}$ is H, $R_{14}$ is Cl and $R_{15}$ is $CH_3$;
  (4) $R_{13}$ is $CH_2CH_2CH_3$ or $CH(CH_3)_2$, $R_{14}$ is Cl and $R_{15}$ is $CH_3$;
  (5) $R_{13}$ is $CH_2CH_3$ or $CH(CH_3)_2$, $R_{14}$ is $OCH_3$ and $R_{15}$ is $CH_3$;
  (6) $R_{13}$ and $R_{14}$ are H and $R_{15}$ is H or $CH_3$;
m) when Q is Q-6, R is H, Z is CH or N and Y is $CH_3$ or $OCH_3$, then X is other than $CH_3$ or $OCH_3$ when
  (1) $R_{13}$ and $R_{15}$ are $CH_3$ and $R_{14}$ is H, Cl or $OCH_3$;
  (2) $R_{13}$ is H and $R_{14}$ and $R_{15}$ are $CH_3$;
n) when Q is Q-6, R is H, Z is N and Y is $CH_3$ or $OCH_3$, then X is other than $OCH_3$ when
  (1) $R_{13}$ and $R_{14}$ are $CH_3$ and $R_{15}$ is H or $CH_3$;
  (2) $R_{13}$ and $R_{15}$ are $CH_3$ and $R_{14}$ is Br;
  (3) $R_{13}$ is H, $R_{14}$ is Cl and $R_{15}$ is $CH_3$;
  (4) $R_{13}$ is $CH_2CH_3$ or $CH(CH_3)_2$, $R_{14}$ is $OCH_3$ and $R_{15}$ is $CH_3$;
  (5) $R_{13}$ and $R_{14}$ are H and $R_{15}$ is H or $CH_3$;
o) when Q is Q-6, R is H, Z is CH and Y is $CH_3$ or $OCH_3$ then X is other than $CH_3$ when
  (1) $R_{13}$ is $CH_2CH_3$ $R_{14}$ is H or Cl and $R_{15}$ is $CH_3$;
  (2) $R_{13}$ is $CH_3$, $R_{14}$ is Cl and $R_{15}$ is H;
  (3) $R_{13}$ and $R_{15}$ are $CH_3$ and $R_{14}$ is $OCH_2CH_3$;
  (4) $R_{13}$ is $CH(CH_3)_2$, $R_{14}$ is H and $R_{15}$ is $CH_3$;
  (5) $R_{13}$ and $R_{14}$ are $CH_2CH_3$ and $R_{15}$ is H;
  (6) $R_{13}$ is H, $R_{14}$ is $CH_2CH_3$ or $CH(CH_3)_2$ and $R_{15}$ is $CH_3$;
  (7) $R_{13}$ is $CH_2CH_3$ or $CH(CH_3)_2$, $R_{14}$ is $OCH_3$ and $R_{15}$ is H;
  (8) $R_{13}$ is $CH_2CH_2CH_3$, $R_{14}$ is $OCH_3$ and $R_{15}$ is $CH_3$;
p) when Q is Q-6, R is H, Z is CH and Y is $CH_2CH_3$, then X is other than $OCH_3$ when $R_{13}$ and $R_{15}$ are $CH_3$ and $R_{14}$ is Cl;
q) Q is Q-6, R is H, Z is N and Y is $CH_3$, then X is other than $OCH_3$ when
  (1) $R_{13}$ is $CH_2CH_3$, $R_{14}$ is H or Cl and $R_{15}$ is $CH_3$;
  (2) $R_{13}$ is $CH_3$, $R_{14}$ is Cl and $R_{15}$ is H;
  (3) $R_{13}$ is $CH(CH_3)_2$, $R_{14}$ is H and $R_{15}$ is $CH_3$;
  (4) $R_{13}$ and $R_{14}$ are $CH_2CH_3$ and $R_{15}$ is H;
  (5) $R_{13}$ and $R_{15}$ are $CH_3$ and $R_{14}$ is $OCH_2CH_3$;
  (6) $R_{13}$ is H, $R_{14}$ is $CH_2CH_3$ or $CH(CH_3)_2$ and $R_{15}$ is $CH_3$;
  (7) $R_{13}$ is $CH_2CH_3$ of $CH(CH_3)_2$, $R_{14}$ is $OCH_3$ and $R_{15}$ is H;
  (8) $R_{13}$ is $CH_2CH_2CH_3$, $R_{14}$ is $OCH_3$ and $R_{15}$ is $CH_3$;
r) when Q is Q-6, R is H, Z is N and Y is $OCH_2CF_3$ then X is other than $OCH_3$ when $R_{13}$ and $R_{15}$ are $CH_3$ and $R_{14}$ is $OCH_3$;
s) when Q is Q-6, R is H, Z is CH and Y is $CF_3$ or $OCH_2CF_3$, then X is other than $CH_3$ when $R_{13}$ and $R_{15}$ are $CH_3$ and $R_{14}$ is $CO_2CH_3$;
t) when Q is Q-6, R is H, Z is N and Y is $OCH_2CF_3$, then X is other than $CH_3$ when $R_{13}$ and $R_{15}$ are $CH_3$ and $R_{14}$ is $SO_2CH_2CH_2CH_3$;
u) when Q is Q-6, R is H and A is A-2, then $Y_1$ is other than $CH_3$ when $R_{13}$ and $R_{14}$ are H and $R_{15}$ is $CH_3$;

v) when Q is Q-6, R is H, Z is N and Y is $CH_3$, then X is other than $CH_3$ when $R_{13}$ and $R_{14}$ are H and $R_{15}$ is $CH_2$;
w) when Q is Q-3, R is H, Z is N and Y is $OCH_3$ then X is other than $CH_3$ when $R_7$ and $R_8$ are H and $R_9$ is $CH_3$;
x) when Q is Q-10, R is H, Z is N and Y is $OCH_3$ then X is other than $OCH_3$ when $R_{31}$ is $CH_3$;
y) when Q is Q-9, R is H, Z is CH and Y is $OCH_3$ then X is other than $CH_3$ when $R_{29}$ is $CH_3$ and $R_{30}$ is H;
z) when Q is Q-1, R is H, Z is N and Y is $OCH_3$ then X is other than $CH_3$ when $R_1$ is $CH_3$ and $R_2$ and $R_3$ are H;
$a_1$) when Q is Q-1, R is H, Z is CH and Y is $CH_3$ or $OCH_3$ then X is other than $CH_3$ or $OCH_3$ when $R_1$ is $CH_3$ and $R_2$ and $R_3$ are H;
$b_1$) when Q is Q-1 R is H, Z is CH and Y is $OCHF_2$, then X is other than $CH_3$ when $R_1$ is $CH_3$ and $R_2$ and $R_3$ are H;
$c_1$) when Q is Q-4, R is H, Z is CH and Y is $CH_2CH_3$, then X is other than $CH_3$ when $R_{10}$, $R_{11}$ and $R_{12}$ are H;
$d_1$) when Q is Q-1, R is $CH_3$, Z is N and Y is $OCH_3$ then X is other than $CH_3$ when $R_1$, $R_2$ and $R_3$ are H;
$e_1$) when Q is Q-9, R is H, Z is N and Y is $CH_3$, then X is other than $CH_3$ when $R_{29}$ and $R_{30}$ are H;
$f_1$) when Q is Q-6, A is A-2, $Y^1$ is $CH_3$, R is H, $R_{15}$ and $R_{13}$ are $CH_3$, then $R_{14}$ is other than Cl, $CO_2CH_3$, $CO_2CH_2CH_3$, $SO_2C_3H_7$-n, $SO_2C_3H_7$-i or $SO_2N(CH_3)_2$;
$g_1$) when Q is Q-6, A is A-2, $Y^1$ is $CH_3$, R is H, $R_{15}$ is $CH_3$ and $R_{13}$ is H, then $R_{14}$ is other than H or $SO_2C_3H_7$-n;
$h_1$) when Q is Q-6, A is A-2, $Y^1$ is $CH_3$, R is H, $R_{15}$ is $CH_3$ and $R_{13}$ is $CO_2CH_3$, the $R_{14}$ is other than $CH_3$;
$i_1$) when Q is Q-4, A is A-2, $Y^1$ is $CH_3$, R is H and $R_{10}$ and $R_{12}$ are $CH_3$, then $R_{11}$ is other than Cl, $NO_2$ or $SO_2N(CH_3)_2$; and
$j_1$) when Q is Q-5, R, $R_{11}$ and $R_{12}$ are H, and A is A-2, then $Y^1$ is other than $CH_3$; and agriculturally suitable salts thereof.

**2.** A compound of Claim 1 where
Q is Q-1, Q-2, Q-3, Q-4, Q-5, or Q-6;
A is

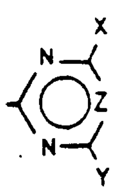 , 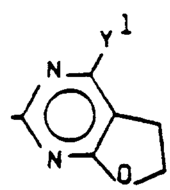 , 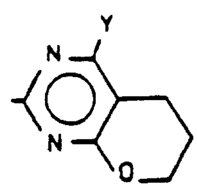 or 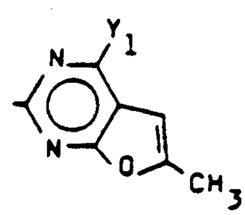 ;

X is $CH_3$, $OCH_3$ or Cl;
Y is $CH_3$, $C_2H_5$, $OCH_3$, $OC_2H_5$, $CH_2OCH_3$, $CH(OCH_3)_2$, $NH_2$, $NHCH_3$ or $N(CH_3)_2$;
Z is CH or N;
$Y_1$ is H, Cl, $CH_3$ or $OCH_3$;
$X_2$ is $OCH_3$ or $CH_3$; and
$Y_2$ is $CH_3$.

**3.** A compound of Claim 1 where
Q is Q-7, Q-8 or Q-9;
$R_{26}$ is H, $C_1$-$C_3$ alkyl or $CH_3C(O)NH$; $R_{27}$ is H, $C_1$-$C_3$ alkyl, $C_1$-$C_3$ alkoxy, $C_1$-$C_3$ alkylthio, or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkylsulfonyl, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
A is

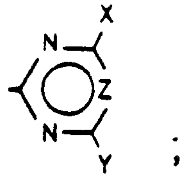

Y is $CH_3$, $OCH_3$, $OCF_2H$, $OC_2H_5$ or $CH_2OCH_3$;
X is $CH_3$, $OCH_3$ or $OCF_2H$; and
Z is CH or N.

**4.** A compound of Claim 1 where
Q is Q-10;
$R_{31}$ is Cl, $C_1$-$C_3$ alkoxycarbonyl or $C_1$-$C_3$ alkyl;
A is

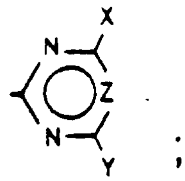

X is $CH_3$, $OCH_3$ or $OCF_2H$;
Y is $CH_3$, $OCH_3$, $OC_2H_5$ or $CH_2OCH_3$; and
Z is CH or N.

**5.** A compound of Claim 1 where R is H.

**6.** A compound of Claim 1 or 5 where A is A-1 and Z is CH.

**7.** A compound of Claim 1, 5 or 6 where Q is Q-1 or Q-6.

**8.** A compound of Claim 1 wherein Q is Q-4, R is H, $R_{10}$ is $CH_3$, $R_{12}$ is H, $R_{11}$ is $CO_2R_{24}$, $R_{24}$ is $C_1$-$C_3$ alkyl, A is A-1, X and Y are $OCH_3$ and Z is CH.

**9.** A compound of Claim 1 which is
N-[(4,6-dimethylpyrimidin-2-yl)aminocarbonyl]-1-(1-methylethyl)-1H-imidazole-2-sulfonamide;
N-[(4,6-dimethylpyrimidin-2-yl)aminocarbonyl]-1-ethyl)-1H-imidazole-2-sulfonamide; or an agriculturally suitable salt of either or these.

**10.** A compound of Claim 1 which is selected from N-[(4-methoxy-6-methylpyrimidin-2-yl)aminocarbonyl-1-(1-methylethyl)-1H-imidazole-2-sulfonamide;
N-[(4-methoxy-6-methylpyrimidin-2-yl)aminocarbonyl]-1-ethyl-1H-imidazole-2-sulfonamide;
N-[(4-methoxy-6-methylpyrimidin-2-yl)aminocarbonyl]-4-thiazolesulfonamide;
methyl 4-[(methoxy-6-methylpyrimidin-2-yl)aminocarbonyl]aminosulfonyl]-3-isothiazolecarboxylate;
or an agriculturally suitable salt of any of these.

**11.** A compound of Claim 1 which is selected from:
N-[(4,6-dimethoxypyrimidin-2-yl)aminocarbonyl]-1-(1-methylethyl)-1H-imidazole-2-sulfonamide;
N-[(4,6-dimethoxypyrimidin-2-yl)aminocarbonyl]-1-ethyl)-1H-imidazole-2-sulfonamide;
N-[(4,6-dimethoxypyrimidin-2-yl)aminocarbonyl]-5-bromo-1-methyl-1H-imidazole-4-sulfonamide;
N-[(4,6-dimethoxypyrimidin-2-yl)aminocarbonyl]-4-thiazolesulfonamide;
methyl 4-[(4,6-dimethoxypyrimidin-2-yl)aminocarbonyl]aminosulfonyl)]-3-isothiazolecarboxylate; or an agriculturally suitable salt of any of these

**12.** An agricultural composition comprising an herbically effective amount of one or more compounds of any of Claims 1-11 and at least one of the following: (a) a surficant and (b) a solid or liquid diluent.

**13.** A method for controlling the growth of undesired vegetation comprising applying to the locus of such vegetation an agriculturally effective amount of one or more compounds of any of Claims 1-11.

**14.** A method for regulating the growth of plants which comprises applying to the locus of such plants an effective but substantially non-phytotoxic amount of a plant growth regulant selected from compounds of any of Claims 1 to 11.

**15.** A process for preparing a compound of Claim 1 comprising either

(a) contacting an aminoheterocycle of the formula

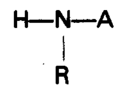

with

i) a sulfonyl isocyanate of the formula $QSO_2NCO$, or with

ii) an N-phenylsulfonylcarbamate of the formula

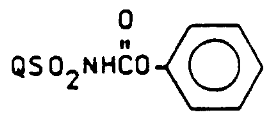 ;

or

(b) contacting a sulfonamide of the formula $QSO_2NH_2$ with

i) a methyl carbamate of the formula

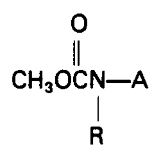

in the presence of an equimolar amount of trimethylaluminum, or with

ii) a heterocyclic isocyanate of the formula

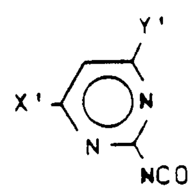

where X' is Cl and Y' is Cl or Br, optionally followed by reaction with a nucleophile such as methoxide or ethoxide ion; or with

iii) an N-heterocyclic carbamate of the formula

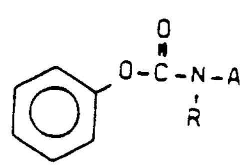

in the presence of a base.

**Claims for the following Contracting State : BE**

**1.** A compound of the formula:

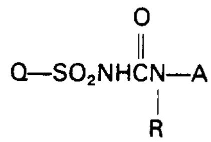

where

R is H or $CH_3$;

Q is

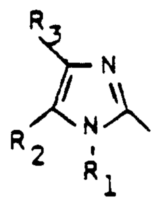
,
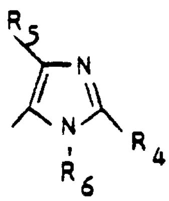
,
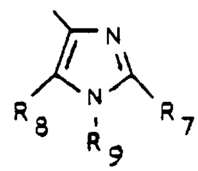
,

Q-1 Q-2 Q-3

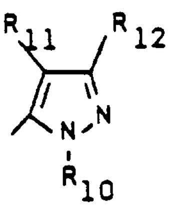
,
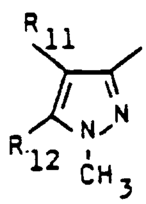
or
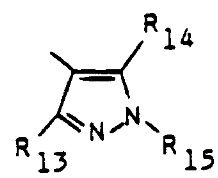
;

Q-4 Q-5 Q-6

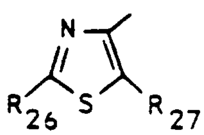
,
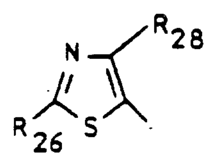
,

Q-7 Q-8

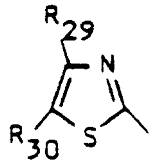
or
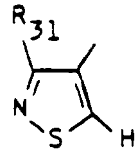
;

Q-9 Q-10

$R_1$ is H, $C_1$-$C_8$ alkyl, $C_3$-$C_6$ alkenyl, $C_5$-$C_6$ cycloalkyl, $C_5$-$C_6$ cyclolkenyl, $C_3$-$C_6$ alkynyl, $C_4$-$C_7$ cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

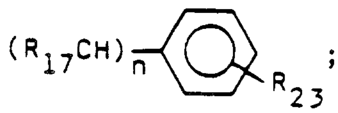

or $C_1$-$C_6$ alkyl
substituted either with
a) 1-3 atoms of F, Br or Cl; or
b) $OR_{16}$;
$R_2$, $R_3$ and $R_4$ are independently H or $CH_3$;
$R_5$ is H, $C_1$-$C_4$ alkyl, $-OR_6$, $NO_2$, F, Cl, Br, $CO_2R_{24}$, $S(O)_mR_{25}$ or $SO_2NR_{20}R_{21}$;
$R_6$ is H, $C_1$-$C_4$ alkyl, $C_3$-$C_4$ alkenyl, $C_3$-$C_4$ alkynyl, $CO_2R_{18}$, $SO_2NR_{20}R_{21}$, $SO_2R_{22}$ or $C_1$-$C_4$ alkyl substituted with a) 1-3 atoms of F, Cl or Br; or b) $OCH_3$;
$R_7$ is H or $CH_3$;
$R_8$ is H, $C_1$-$C_4$ alkyl, $-OR_{16}$, $NO_2$, F, Cl, Br, $CO_2R_{24}$, $S(O)_mR_{25}$ or $SO_2NR_{20}R_{21}$;
$R_9$ is $CH_3$ or $C_2H_5$;
$R_{10}$ is H, $C_1$-$C_4$ alkyl, $C_3$-$C_4$ alkenyl, $C_3$-$C_4$ alkynyl, $CO_2R_{24}$, $SO_2NR_{20}R_{21}$ or $SO_2R_{22}$;
$R_{11}$is H, $C_1$-$C_3$ alkyl, F, Cl, Br, $NO_2$, $-OR_{16}$, $CO_2R_{24}$, $S(O)_mR_{25}$ or $SO_2NR_{20}R_{21}$;
$R_{12}$ is H or $CH_3$;
$R_{13}$ and $R_{14}$ are independently H, $C_1$-$C_3$ alkyl $-OR_{16}$, F, Cl, Br, $NO_2$, $CO_2R_{24}$, $S(O)_mR_{25}$ or $SO_2NR_{20}R_{21}$;
$R_{15}$ is H or $CH_3$;
$R_{16}$ is $C_1$-$C_3$ alkyl;
$R_{17}$ is H or $CH_3$;
$R_{18}$ is $C_1$-$C_4$ alkyl, $C_3$-$C_4$ alkenyl, $C_3$-$C_4$ alkynyl, $CH_2CH_2Cl$ or $CH_2CH_2OCH_3$;
$R_{19}$ is $C_1$-$C_4$ alkyl, $C_3$-$C_4$ alkenyl, $C_3$-$C_4$ alkynyl or $CH_2CH_2OCH_3$;
$R_{20}$ and $R_{21}$ are independently $CH_3$ or $C_2H_5$;
$R_{22}$ is $C_1$-$C_3$ alkyl or $CF_3$;
$R_{23}$ is H, Cl, Br, $CH_3$, F, $CF_3$, $OCH_3$ or $NO_2$;
$R_{24}$ is $C_1$-$C_3$ alkyl or allyl;
$R_{25}$ is $C_1$-$C_3$ alkyl;
m is 0, 1 or 2;
n is 0 or 1;
$R_{26}$ is H, $C_1$-$C_3$ alkyl or $CH_3C(O)NH$;
$R_{27}$ ia H, $C_1$-$C_3$ alkyl, $C_1$-$C_3$ alkoxy, $C_1$-$C_3$ alkylthio or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
$R_{31}$ is Cl, $C_1$-$C_3$ alxycarbonyl or $C_1$-$C_3$ alkyl;
A is

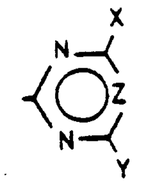 , 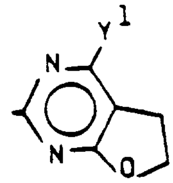 , 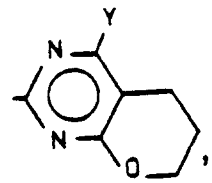 ,

A-1 A-2 A-3

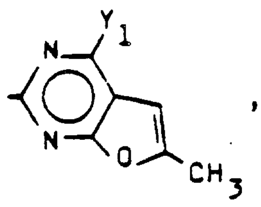 , 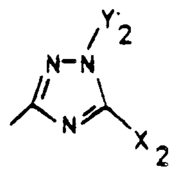 or 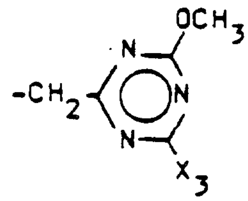 ;

A-4 A-5 A-6

X is $CH_3$, $OCH_3$, Cl, F, $OCF_2H$ or $SCF_2H$;

Y is $CH_3$, $C_2H_5$, $OCH_3$, $OC_2H_5$, $CH_2OCH_3$,$CH(OCH_3)_2$, $OCH_2CF_3$, $OCF_3$, $NH_2$, $NHCH_3$, N-$(CH_3)_2$ or $GCF_2T$ where G is O or S and T is H, CHClF, CHBrF, $CF_2H$ or $CHFCF_3$;

Z is CH or N;

$Y_1$ is H, Cl, $CH_3$, $OCH_3$ or $OCF_2H$;

$X_2$ is $OCH_3$, $CH_3$, $CH_2CH_3$, $OCH_2CH_3$, $SCH_3$ or $SCH_2CH_3$;

$Y_2$ is $CH_3$, $CH_2CH_3$ or $CH_2CF_3$;

$X_3$ is $OCH_3$ or $CH_3$;

provided that,

a) when Q is Q-7, Q-8, Q-9 or Q-10, then R is H;

b) the total number of carbon atoms in $R_1$ is less than or equal to 8;

c) if $R_1$ is other than $C_1$-$C_3$ alkyl, then $R_3$ must be H;

d) when $R_5$ is other than H, $CH_3$, $OCH_3$ or $NO_2$, then $R_6$ is H or $CH_3$;

e) when $R_6$ is $CO_2R_{18}$, $SO_2NR_{20}R_{21}$ or $SO_2R_{22}$,then $R_5$ is H, $CH_3$, $OCH_3$ or $NO_2$;

f) when $R_{10}$ is other than $C_1$-$C_3$ alkyl, then $R_{11}$ is H, Cl, $OCH_3$, $NO_2$ or $CH_3$;

g) when either of $R_{13}$ or $R_{14}$ is $CO_2R_{24}$, $S(O)_mR_{25}$ or $SO_2NR_{20}R_{21}$, then the other is H, Cl, $CH_3$, $OCH_3$ or $NO_2$;

h) $R_{29}$ and $R_{30}$ are not simultaneously Cl;

i) when X is Cl or F, then Z is CH and Y is $OCH_3$, $NH_2$, $NHCH_3$ or $N(CH_3)_2$;

j) when Q is Q-7, Q-8, Q-9 or Q-10, then A is A-1; X is $CH_3$, $OCH_3$ or $OCF_2H$, Y is $CH_3$, $OCH_3$, $OC_2H_5$, $OCF_2H$ or $CH_2OCH_3$; and,

k) when either X or Y is $OCF_2H$, then Z is CH;

$k_1$) when Q is Q-2, R is H, Z is CH and when one of X and Y is $OCH_3$ then the other is not $CH_3$ when $R_4$, $R_5$ and $R_6$ are H;

l) when Q is Q-6, R is H, Z is CH and Y is $CH_3$ or $OCH_3$, then X is other than $CH_3$ or $OCH_3$ when

(1) $R_{13}$ and $R_{14}$ are $CH_3$ and $R_{15}$ is H or $CH_3$;

(2) $R_{13}$ and $R_{15}$ are $CH_3$ and $R_{14}$ is Br;

(3) $R_{13}$ is H, $R_{14}$ is Cl and $R_{15}$ is $CH_3$;

(4) $R_{13}$ is $CH_2CH_2CH_3$ or $CH(CH_3)_2$, $R_{14}$ is Cl and $R_{15}$ is $CH_3$;

(5) $R_{13}$ is $CH_2CH_3$ or $CH(CH_3)_2$, $R_{14}$ is $OCH_3$ and $R_{15}$ is $CH_3$;

(6) $R_{13}$ and $R_{14}$ are H and $R_{15}$ is H or $CH_3$;

m) when Q is Q-6, R is H, Z is CH or N and Y is $CH_3$ or $OCH_3$, then X is other than $CH_3$ or $OCH_3$ when

(1) $R_{13}$ and $R_{15}$ ae $CH_3$ and $R_{14}$ is H, Cl or $OCH_3$;

(2) $R_{13}$ is H and $R_{14}$ and $R_{15}$ are $CH_3$;

n) when Q is Q-6, R is H, Z is N and Y is $CH_3$ or $OCH_3$, then X is other than $OCH_3$ when

(1) $R_{13}$ and $R_{14}$ are $CH_3$ and $R_{15}$ is H or $CH_3$;

(2) $R_{13}$ and $R_{15}$ are $CH_3$ and $R_{14}$ is Br;
(3) $R_{13}$ is H, $R_{14}$ is Cl and $R_{15}$ is $CH_3$;
(4) $R_{13}$ is $CH_2CH_3$ or $CH(CH_3)_2$, $R_{14}$ is $OCH_3$ and $R_{15}$ is $CH_3$;
(5) $R_{13}$ and $R_{14}$ are H and $R_{15}$ is H or $CH_3$;

o) when Q is Q-6, R is H, Z is CH and Y is $CH_3$ or $OCH_3$ then X is other than $CH_3$ when
(1) $R_{13}$ is $CH_2CH_3$ $R_{14}$ is H or Cl and $R_{15}$ is $CH_3$;
(2) $R_{13}$ is $CH_3$, $R_{14}$ is Cl and $R_{15}$ is H;
(3) $R_{13}$ and $R_{15}$ are $CH_3$ and $R_{14}$ is $OCH_2CH_3$;
(4) $R_{13}$ is $CH(CH_3)_2$, $R_{14}$ is H and $R_{15}$ is $CH_3$;
(5) $R_{13}$ and $R_{14}$ are $CH_2CH_3$ and $R_{15}$ is H;
(6) $R_{13}$ is H, $R_{14}$ is $CH_2CH_3$ or $CH(CH_3)_2$ and $R_{15}$ is $CH_3$;
(7) $R_{13}$ is $CH_2CH_3$ or $CH(CH_3)_2$, $R_{14}$ is $OCH_3$ and $R_{15}$ is H;
(8) $R_{13}$ is $CH_2CH_2CH_3$, $R_{14}$ is $OCH_3$ and $R_{15}$ is $CH_3$;

p) when Q is Q-6, R is H, Z is CH and Y is $CH_2CH_3$, then X is other than $OCH_3$ when $R_{13}$ and $R_{15}$ are $CH_3$ and $R_{14}$ is Cl;

q) Q is Q-6, R is H, Z is N and Y is $CH_3$, then X is other than $OCH_3$ when
(1) $R_{13}$ is $CH_2CH_3$, $R_{14}$ is H or Cl and $R_{15}$ is $CH_3$;
(2) $R_{13}$ is $CH_3$, $R_{14}$ is Cl and $R_{15}$ is H;
(3) $R_{13}$ is $CH(CH_3)_2$, $R_{14}$ is H and $R_{15}$ is $CH_3$;
(4) $R_{13}$ and $R_{14}$ are $CH_2CH_3$ and $R_{15}$ is H;
(5) $R_{13}$ and $R_{15}$ are $CH_3$ and $R_{14}$ is $OCH_2CH_3$;
(6) $R_{13}$ is H, $R_{14}$ is $CH_2CH_3$ or $CH(CH_3)_2$ and $R_{15}$ is $CH_3$;
(7) $R_{13}$ is $CH_2CH_3$ or $CH(CH_3)_2$, $R_{14}$ is $OCH_3$ and $R_{15}$ is H;
(8) $R_{13}$ is $CH_2CH_2CH_3$, $R_{14}$ is $OCH_3$ and $R_{15}$ is $CH_3$;

r; when Q is Q-6, R is H, Z is N and Y is $OCH_2CF_3$ then X is other than $OCH_3$ when $R_{13}$ and $R_{15}$ are $CH_3$ and $R_{14}$ is $OCH_3$;

s) when Q is Q-6, R is H, Z is CH and Y is $CF_3$ or $OCH_2CF_3$, then X is other than $CH_3$ when $R_{13}$ and $R_{15}$ are $CH_3$ and $R_{14}$ is $CO_2CH_3$;

t) when Q is Q36, R is H, Z is N and Y is $OCH_2CF_3$, then X is other than $CH_3$ when $R_{13}$ and $R_{15}$ are $CH_3$ and $R_{14}$ is $SO_2CH_3CH_2CH_3$;

u) when Q is Q-6, R is H and A is A-2, then $Y_1$ is other than $CH_3$ when $R_{13}$ and $R_{14}$ are H and $R_{15}$ is $CH_3$;

v) when Q is Q-6, R 0s H, Z is N and Y is $CH_3$, then X is other than $CH_3$ when $R_{13}$ and $R_{14}$ are H and $R_{15}$ is $CH_3$;

w) when Q is Q-6, A is A-2, $Y^1$ is $CH_3$, R is H and $R_{15}$ is $CH_3$, then
(i) when $R_{13}$ is $CH_3$, $R_{14}$ is other than Cl, $CO_2CH_3$, $CO_2CH_2CH_3$, $SO_2C_3H_7$-n, $SO_2C_3H_7$-i or $SO_2N(CH_3)_2$; and
(ii) when $R_{13}$ is H, $R_{14}$ is other than H or $SO_2C_3H_7$-n;

x) when Q is Q-6, A is A-2, $Y^1$ is CH3, R is H, $R_{15}$ is $CH_3$ and $R_{13}$ is $CO_2CH_3$, then $R_{14}$ is other than $CH_3$;

y) when Q is Q-4, A is A-2, $Y^1$ is $CH_3$, R is H and $R_{10}$ and $R_{12}$ are $CH_3$, then $R_{11}$ is other than Cl, $NO_2$ or $SO_2N(CH_3)_2$; and

z) when Q is Q-5, R, $R_{11}$ and $R_{12}$ are H, and A is A-2, then $Y^1$ is other than $CH_3$;

and agriculturally suitable salts thereof.

**2.** A compound of Claim 1 where
Q is Q-1, Q-2, Q-3, Q-4, Q-5, or Q-6;
A is

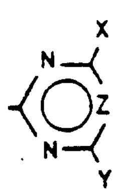 , 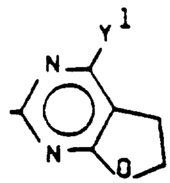 , 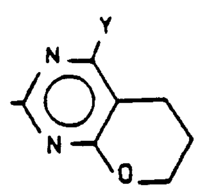 or 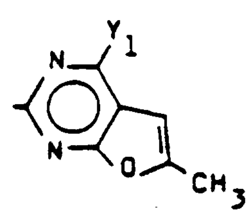 ;

X is $CH_3$, $OCH_3$ or Cl;
Y is $CH_3$, $C_2H_5$, $OCH_3$, $OC_2H_5$, $CH_2OCH_3$, $CH(OCH_3)_2$, $NH_2$, $NHCH_3$ or $N(CH_3)_2$;
Z is CH or N;
$Y_1$ is H, Cl, $CH_3$ or $OCH_3$;
$X_2$ is $OCH_3$ or $CH_3$; and
$Y_2$ is $CH_3$.

**3.** A compound of Claim 1 where
Q is Q-7, Q-8 or Q-9;
$R_{26}$ is H, $C_1$-$C_3$ alkyl or $CH_3C(O)NH$;
$R_{27}$ is H, $C_1$-$C_3$ alkyl, $C_1$-$C_3$ alkoxy, $C_1$-$C_3$ alkylthio, or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkylsulfonyl, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
A is

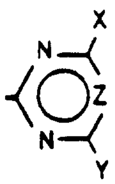

;

Y is $CH_3$, $OCH_3$, $OCF_2H$, $OC_2H_5$ or $CH_2OCH_3$;
X is $CH_3$, $OCH_3$ or $OCF_2H$; and
Z is CH or N.

**4.** A compound of Claim 1 where
Q is Q-10;
$R_{31}$ is Cl, $C_1$-$C_3$ alkoxycarbonyl or $C_1$-$C_3$ alkyl;
A is

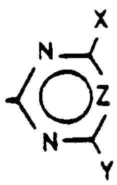

;

X is $CH_3$, $OCH_3$ or $OCF_2H$;
Y is $CH_3$, $OCH_3$, $OC_2H_5$ or $CH_2OCH_3$; and
Z is CH or N.

**5.** A compound of Claim 1 where R is H.

**6.** A compound of Claim 1 or 5 where A is A-1 and Z is CH.

**7.** A compound of Claim 1, 5 or 6 where Q is Q-1 or Q-6.

**8.** A compound of Claim 1 wherein Q is Q-4, R is H, $R_{10}$ is $CH_3$, $R_{12}$ is H, $R_{11}$ is $CO_2R_{24}$, $R_{24}$ is $C_1$-$C_3$ alkyl, A is A-1, X and Y are $OCH_3$ and Z is CH.

**9.** A compound of Claim 1 which is:
N-[(4,6-dimethylpyrimidin-2-yl)aminocarbonyl]-1-(1-methylethyl)-1H-imidazole-2-sulfonamide;
N-[(4,6-dimethylpyrimidin-2-yl)aminocarbonyl]-1-ethyl-1H-imidazole-2-sulfonamide; or an agriculturally suitable salt of either or these.

**10.** A compound of Claim 1 which is selected from N-[(4-methoxylpyrimidin-2-yl)aminocarbonyl-1-(methylethyl)-1H-imidazole-2-sulfonamide;
N-[(4-methoxy-6-methylpyrimidin-2-yl)aminocarbonyl]-1-ethyl-1H-imidazole-2-sulfonamide;
N-[(4-methoxy-6-methylpyrimidin-2-yl)aminocarbonyl]-4-thiazolesulfonamide;
methyl 4-[(4-methoxy-6-methylpyrimidin-2-yl)aminocarbonyl]aminosulfonyl]-3-isothiazolecarboxylate; or an agriculturally suitable salt of any of these.

**11.** A compound of Claim 1 which is selected from:
N-[(4,6-dimethoxypyrimidin-2-yl)aminocarbonyl]-1-(1-methylethyl)-1H-imidazole-2-sulfonamide;
N-[(4,6-dimethoxypyrimidin-2-yl)aminocarbonyl]-1-ethyl-1H-imidazole-2-sulfonamide;
N-[(4,6-dimethoxypyrimidin-2-yl)aminocarbonyl]-5-bromo-1-methyl-1H-imidazole-4-sulfonamide;
N-[(4,6-dimethoxypyrimidin-2-yl)aminocarbonyl]-4-thiazolesulfonamide;
methyl 4-[(4,6-dimethoxypyrimidin-2-yl)aminocarbonyl]aminosulfonyl)]-3-isothiazolecarboxylate; or an agriculturally suitable salt of any of these

**12.** An agricultural composition comprising an herbicidally effective amount of one or more compounds of any of Claims 1-11 and at least one of the following: (a) a surfactant and (b) a solid or liquid diluent.

**13.** A method for controlling the growth of undesired vegetation comprising applying to the locus of such vegetation an agriculturally effective amount of one or more compounds of any of Claims 1-11.

**14.** A method for regulating the growth of plants which comprises applying to the locus of such plants an effective but substantially non-phytotoxic amount of a plant growth regulant selected from compounds of any of Claims 1 to 11.

**15.** A process for preparing a compound of Claim 1 comprising either
(a) contacting an aminoheterocycle of the formula

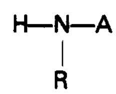

with
i) a sulfonyl isocyanate of the formula $QSO_2NCO$, or with
ii) an N-phenylsulfonylcarbamate of the formula

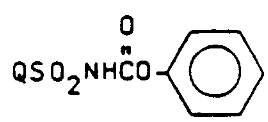 ;

or
(b) contacting a sulfonamide of the formula $QSO_2NH_2$ with
i) a methyl carbamate of the formula

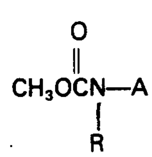

in the presence of an equimolar amount of trimethylaluminum, or with

ii) a heterocyclic isocyanate of the formula

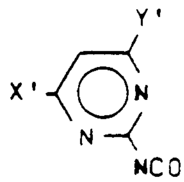

where X' is Cl and Y' is Cl or Br, optionally followed by reaction with a nucleophile such as methoxide or ethoxide ion; or with
iii) an N-heterocyclic carbamate of the formula

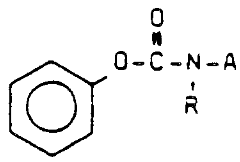

in the presence of a base.

**Claims for the following Contracting States : LU, SE**

**1.** A compound of the formula:

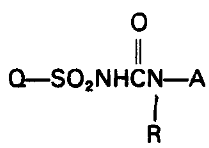

where
R is H or $CH_3$;
Q is

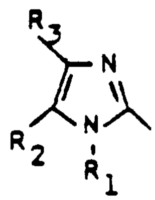 ,

Q-1

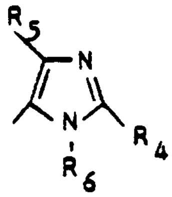 ,

Q-2

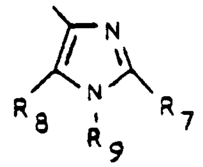 ,

Q-3

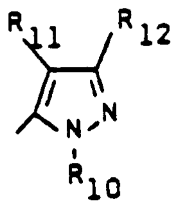 ,

Q-4

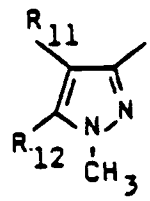 or

Q-5

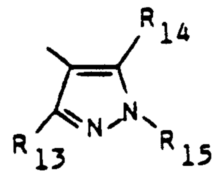 ;

Q-6

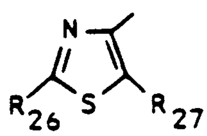 ,

Q-7

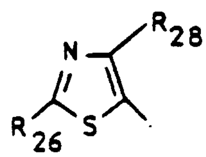 ,

Q-8

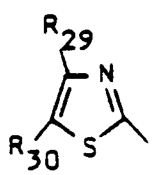 or

Q-9

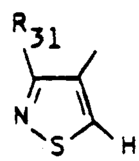 ;

Q-10

$R_1$ is H, $C_1$-$C_8$ alkyl, $C_3$-$C_6$ alkenyl, $C_5$-$C_6$ cycloalkyl, $C_5$-$C_6$ cyclolkenyl, $C_3$-$C_6$ alkynyl, $C_4$-$C_7$ cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

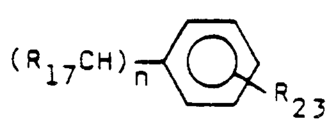 ;

or $C_1$-$C_6$ alkyl
substituted either with

a) 1-3 atoms of F, Br or Cl; or

b) $OR_{16}$;

$R_2$, $R_3$ and $R_4$ are independently H or $CH_3$;

$R_5$ is H, $C_1$-$C_4$ alkyl, $-OR_6$, $NO_2$, F, Cl, Br, $CO_2R_{24}$, $S(O)_mR_{25}$ or $SO_2NR_{20}R_{21}$;

$R_6$ is H, $C_1$-$C_4$ alkyl, $C_3$-$C_4$ alkenyl, $C_3$-$C_4$ alkynyl, $CO_2R_{18}$, $SO_2NR_{20}R_{21}$, $SO_2R_{22}$ or $C_1$-$C_4$ alkyl substituted with a) 1-3 atoms of F, Cl or Br; or b) $OCH_3$;

$R_7$ is H or $CH_3$;

$R_8$ is H, $C_1$-$C_4$ alkyl, $-OR_{16}$, $NO_2$, F, Cl, Br, $CO_2R_{24}$, $S(O)_mR_{25}$ or $SO_2NR_{20}R_{21}$;

$R_9$ is $CH_3$ or $C_2H_5$;
$R_{10}$ is H, $C_1$-$C_4$ alkyl, $C_3$-$C_4$ alkenyl, $C_3$-$C_4$ alkynyl, $CO_2R_{24}$, $SO_2NR_{20}R_{21}$ or $SO_2R_{22}$;
$R_{11}$ is H, $C_1$-$C_3$ alkyl, F, Cl, Br, $NO_2$, $-OR_{16}$, $CO_2R_{24}$, $S(O)_mR_{25}$ or $SO_2NR_{20}R_{21}$;
$R_{12}$ is H or $CH_3$;
$R_{13}$ and $R_{14}$ are independently H, $C_1$-$C_3$ alkyl $-OR_{16}$, F, Cl, Br, $NO_2$, $CO_2R_{24}$, $S(O)_mR_{25}$ or $SO_2NR_{20}R_{21}$;
$R_{15}$ is H or $CH_3$;
$R_{16}$ is $C_1$-$C_3$ alkyl;
$R_{17}$ is H or $CH_3$;
$R_{18}$ is $C_1$-$C_4$ alkyl, $C_3$-$C_4$ alkenyl, $C_3$-$C_4$ alkynyl, $CH_2CH_2Cl$ or $CH_2CH_2OCH_3$;
$R_{19}$ is $C_1$-$C_4$ alkyl, $C_3$-$C_4$ alkenyl, $C_3$-$C_4$ alkynyl or $CH_2CH_2OCH_3$;
$R_{20}$ and $R_{21}$ are independently $CH_3$ or $C_2H_5$;
$R_{22}$ is $C_1$-$C_3$ alkyl or $CF_3$;
$R_{23}$ is H, Cl, Br, $CH_3$, F, $CF_3$, $OCH_3$ or $NO_2$;
$R_{24}$ is $C_1$-$C_3$ alkyl or allyl;
$R_{25}$ is $C_1$-$C_3$ alkyl;
m is 0, 1 or 2;
n is 0 or 1;
$R_{26}$ is H, $C_1$-$C_3$ alkyl or $CH_3C(O)NH$;
$R_{27}$ is H, $C_1$-$C_3$ alkyl, $C_1$-$C_3$ alkoxy, $C_1$-$C_3$ alkylthio or $C_1$-$C_3$ alkoxycarbonyl;
$R_{28}$ is H, $C_2$-$C_3$ alkyl, $C_1$-$C_3$ alkoxy, $C_1$-$C_3$ alkylthio, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
$R_{31}$ is Cl, $C_1$-$C_3$ alkoxycarbonyl or $C_1$-$C_3$ alkyl;
A is

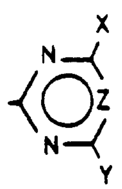 , 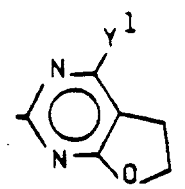 , 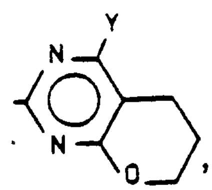 ,

A-1 A-2 A-3

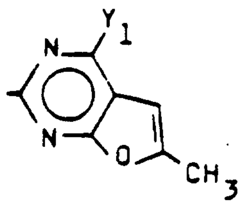 , 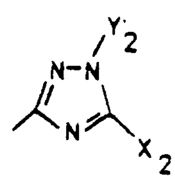 or 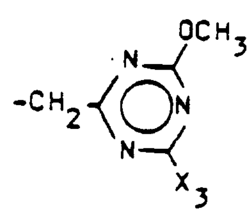 ;

A-4 A-5 A-6

X is $CH_3$, $OCH_3$, Cl, F, $OCF_2H$ or $SCF_2H$;
Y is $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ or $GCF_2T$ where G is O or S and T is H, CHClF, CHBrF, $CF_2H$ or $CHFCF_3$;
Z is CH or N;
$Y_1$ is H, Cl, $CH_3$ $OCH_3$ or $OCF_2H$;
$X_2$ is $OCH_3$, $CH_3$, $CH_2CH_3$, $OCH_2CH_3$, $SCH_3$ or $SCH_2CH_3$;
$Y_2$ is $CH_3$, $CH_2CH_3$ or $CH_2CF_3$;
$X_3$ is $OCH_3$ or $CH_3$;
provided that,
a) when Q is Q-7, Q-8, Q-9 or Q-10, then R is H;
b) the total number of carbon atoms in $R_1$ is less than or equal to 8;

c) if $R_1$ is other than $C_1$-$C_3$ alkyl, then $R_3$ must be H;
d) when $R_5$ is other than H, $CH_3$, $OCH_3$ or $NO_2$, then $R_6$ is H or $CH_3$;
e) when $R_6$ is $CO_2R_{18}$, $SO_2NR_{20}R_{21}$ or $SO_2R_{22}$, then $R_5$ is H, $CH_3$, $OCH_3$ and $NO_2$;
f) when $R_{10}$ is other than $C_1$-$C_3$ alkyl, then $R_{11}$ is H, Cl, $OCH_3$, $NO_2$ or $CH_3$;
g) when either of $R_{13}$ or $R_{14}$ is $CO_2R_{24}$, $S(O)_mR_{25}$ or $SO_2NR_{20}R_{21}$, then the other is H, Cl, $CH_3$, $OCH_3$ or $NO_2$;
h) $R_{29}$ and $R_{30}$ are not simultaneously Cl;
i) when X is Cl or F, then Z is CH and Y is $OCH_3$, $NH_2$, $NHCH_3$ or $N(CH_3)_2$;
j) when Q is Q-7, Q-8, Q-9 or Q-10, then A is A-1; X is $CH_3$, $OCH_3$ or $OCF_2H$, Y is $CH_3$, $OCH_3$, $OC_2H_5$, $OCF_2H$ or $CH_2OCH_3$; and
k) when either X or Y is $OCF_2H$ then Z is CH;
and agriculturally suitable salts thereof.

**2.** A compound of Claim 1 where
Q is Q-1, Q-2, Q-3, Q-4, Q-5, or Q-6;
A is

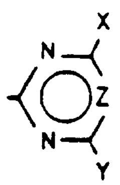
,
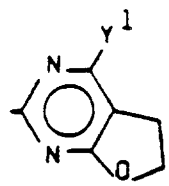
,
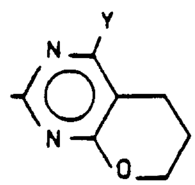
or
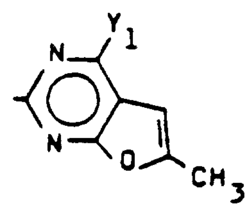
;

X is $CH_3$, $OCH_3$ or Cl;
Y is $CH_3$, $C_2H_5$, $OCH_3$, $OC_2H_5$, $CH_3OCH_3$, $CH(OCH_3)_2$, $NH_2$, $NHCH_3$ or $N(CH_3)_2$;
Z is CH or N;
$Y_1$ is H, Cl, $CH_3$ or $OCH_3$;
$X_2$ is $OCH_3$ or $CH_3$; and
$Y_2$ is $CH_3$.

**3.** A compound of Claim 1 where
Q is Q-7, Q-8 or Q-9;
$R_{26}$ is H, $C_1$-$C_3$ alkyl or $CH_3C(O)NH$;
$R_{27}$ is H, $C_1$-$C_3$ alkyl, $C_1$-$C_3$ alkoxy, $C_1$-$C_3$ alkylthio, or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkylsulfonyl, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
A is

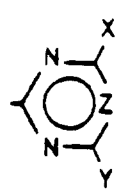
;

Y is $CH_3$, $OCH_3$, $OCF_2H$, $OC_2H_5$ or $CH_2OCH_3$;
X is $CH_3$, $OCH_3$ or $OCF_2H$; and
Z is CH or N.

**4.** A compound of Claim 1 where
Q is Q-10;
$R_{31}$ is Cl, $C_1$-$C_3$ alkoxycarbonyl or $C_1$-$C_3$ alkyl;
A is

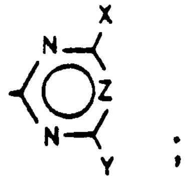

X is $CH_3$, $OCH_3$ or $OCF_2H$;
Y is $CH_3$, $OCH_3$, $OC_2H_5$ or $CH_2OCH_3$; and
Z is CH or N.

**5.** A compound of Claim 1 where R is H.

**6.** A compound of Claim 1 or 5 where A is A-1 and Z is CH.

**7.** A compound of Claim 1, 5 or 6 where Q is Q-1 or Q-6.

**8.** A compound of Claim 1 wherein Q is Q-4, R is H, $R_{10}$ is $CH_3$, $R_{12}$ is H, $R_{11}$ is $CO_2R_{24}$, $R_{24}$ is $C_1$-$C_3$ alkyl, A is A-1, X and Y are $OCH_3$ aand Z is CH.

**9.** A compound of Claim 1 which is
N-[(4,6-dimethylpyrimidin-2-yl)aminocarbonyl]-1-(methylethyl)-1H-imidazole-2-sulfonamide;
N-[(4,6-dimethylpyrimidin-2-yl)aminocarbonyl]-1-ethyl)-1H-imidazole-2-sulfonamide; or an agriculturally suitable salt of either or these.

**10.** A compound of Claim 1 which is selected from N-[(4-methylpyrimidin-2-yl)aminocarbonyl-1-(1-methylethyl)-1H-imidazole-2-sulfonamide;
N-[(4-methoxy-6-methylpyrimidin-2-yl)aminocarbonyl]-1-ethyl-1H-imidazole-2-sulfonamide;
N-[(4-methoxy-6-methylpyrimidin-2-yl)aminocarbonyl]-4-thiazolesulfonamide;
methyl 4-[(4-methoxy-6-methylpyrimidin-2-yl)aminocarbonyl]aminosulfonyl]-3-isothiazolecarboxylate; or an agriculturally suitable salt of any of these.

**11.** A compound of Claim 1 which is selected from:
N-[(4,6-dimethoxypyrimidin-2-yl)aminocarbonyl]-1-(methylethyl)-1H-imidazole-2-sulfonamide;
N-[(4,6-dimethoxypyrimidin-2-yl)aminocarbonyl]-1-ethyl-1H-imidazole-2-sulfonamide;
N-[(4,6-dimethoxypyrimidin-2-yl)aminocarbonyl]-5-bromo-1-methyl-1H-imidazole-4-sulfonamide;
N-[(4,6-dimethoxypyrimidin-2-yl)aminocarbonyl]-4-thiazolesulfonamide;
methyl 4-[(4,6-dimethoxypyrimidin-2-yl)aminocarbonyl]aminosulfonyl)]-3-isothiazolecarboxylate; or an agriculturally suitable salt of any of these.

**12.** An agricultural composition comprising an herbically effective amount of one or more compounds of any of Claims 1-11 and at least one of the following: (a) a surfactant and (b) a solid or liquid diluent.

**13.** A method for controlling the growth of undesired vegetation comprising applying to the locus of such vegetation an agriculturally effective amount of one or more compounds of any of Claims 1-11.

**14.** A method for regulating the growth of plants which comprises applying to the locus of such plants an effective but substantially non-phytotoxic amount of a plant growth regulant selected from compounds of any of Claims 1 to 11.

**15.** A process for preparing a compound of Claim 1 comprising either
(a) contacting an aminoheterocycle of the formula

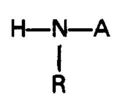

with

i) sulfonyl isocyanate of the formula $QSO_2NCO$, or with

ii) an N-phenylsulfonylcarbamate of the formula

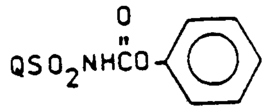 ;

or

(b) contacting a sulfonamide of the formula $QSO_2NH_2$ with

i) a methyl carbamate of the formula

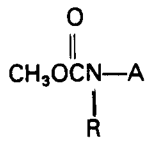

in the presence of an equimolar amount of trimethylaluminum, or with

ii) a heterocyclic isocyanate of the formula

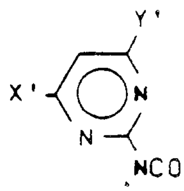

where X' is Cl and Y' is Cl or Br, optionally followed by reaction with a nucleophile such as methoxide or ethoxide ion; or with

iii) an N-heterocyclic carbamate of the formula

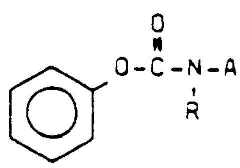

in the presence of a base.

**Claims for the following Contracting State : AT**

**1.** A process for the preparation of a compound of the formula:

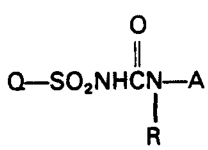

where

R is H or $CH_3$;

Q is

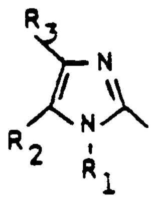 , 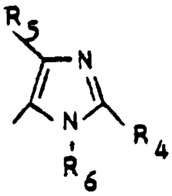 , 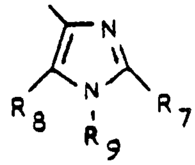 ,

Q-1 Q-2 Q-3

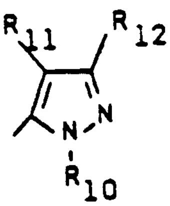 , 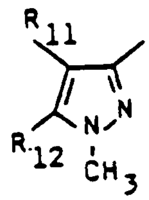 or 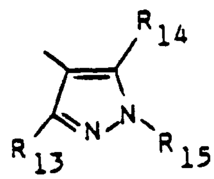 ;

Q-4 Q-5 Q-6

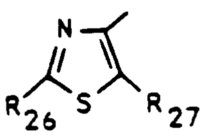 , 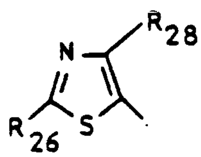 , 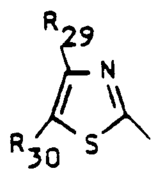 or 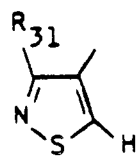 ;

Q-7 Q-8 Q-9 Q-10

$R_1$ is H, $C_1$-$C_8$ alkyl, $C_3$-$C_6$ alkenyl, $C_5$-$C_6$ cycloalkyl, $C_5$-$C_6$ cyclolkenyl, $C_3$-$C_6$ alkynyl, $C_4$-$C_7$ cycloalkylalkyl, $(R_{17}CH)_nC(O)R_{16}$, $(R_{17}CH)_n)CO_2R_{18}$, $(R_{17}CH)_nCOSR_{19}$, $(R_{17}CH)_nCONR_{20}R_{21}$, $(R_{17}CH)$-$_nSO_2NR_{20}R_{21}$, $(R_{17}CH)_nSO_2R_{22}$,

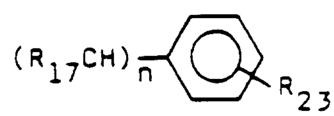 ;

or $C_1$-$C_6$ alkyl
substituted either with

a) 1-3 atoms of F, Br or Cl; or

b) $OR_{16}$;

$R_2$, $R_3$ and $R_4$ are independently H or $CH_3$;

$R_5$ is H, $C_1$-$C_4$ alkyl, $-OR_6$, $NO_2$, F, Cl, Br, $CO_2R_{24}$, $S(O)_mR_{25}$ or $SO_2NR_{20}R_{21}$;

$R_6$ is H, $C_1$-$C_4$ alkyl, $C_3$-$C_4$ alkenyl, $C_3$-$C_4$ alkynyl, $CO_2R_{18}$, $SO_2NR_{20}R_{21}$, $SO_2R_{22}$ or $C_1$-$C_4$ alkyl substituted with a) 1-3 atoms of F, Cl or Br; or b) $OCH_3$;

$R_7$ is H or $CH_3$;

$R_8$ is H, $C_1$-$C_4$ alkyl, $-OR_{16}$, $NO_2$, F, Cl, Br, $CO_2R_{24}$, $S(O)_mR_{25}$ or $SO_2NR_{20}R_{21}$;

$R_9$ is $CH_3$ or $C_2H_5$;

$R_{10}$ is H, $C_1$-$C_4$ alkyl, $C_3$-$C_4$ alkenyl, $C_3$-$C_4$ alkynyl, $CO_2R_{24}$, $SO_2NR_{20}R_{21}$ or $SO_2R_{22}$;

$R_{11}$ is H, $C_1$-$C_3$ alkyl, F, Cl, Br, $NO_2$, $-OR_{16}$, $CO_2R_{24}$, $S(O)_mR_{25}$ or $SO_2NR_{20}R_{21}$;

$R_{12}$ is H or $CH_3$;

$R_{13}$ and $R_{14}$ are independently H, $C_1$-$C_3$ alkyl $-OR_{16}$, F, Cl, Br, $NO_2$, $CO_2R_{24}$, $S(O)_mR_{25}$ or $SO_2NR_{20}R_{21}$;

$R_{15}$ is H or $CH_3$;

$R_{16}$ is $C_1$-$C_3$ alkyl;

$R_{17}$ is H or $CH_3$;

$R_{18}$ is $C_1$-$C_4$ alkyl, $C_3$-$C_4$ alkenyl, $C_3$-$C_4$ alkynyl, $CH_2CH_2Cl$ or $CH_2CH_2OCH_3$;
$R_{19}$ is $C_1$-$C_4$ alkyl, $C_3$-$C_4$ alkenyl, $C_3$-$C_4$ alkynyl or $CH_2CH_2OCH_3$;
$R_{20}$ and $R_{21}$ are independently $CH_3$ or $C_2H_5$;
$R_{22}$ is $C_1$-$C_3$ alkyl or $CF_3$;
$R_{23}$ is H, Cl, Br, $CH_3$, F, $CF_3$, $OCH_3$ or $NO_2$;
$R_{24}$ is $C_1$-$C_3$ alkyl or allyl;
$R_{25}$ is $C_1$-$C_3$ alkyl;
m is 0, 1 or 2;
n is 0 or 1;
$R_{26}$ is H, $C_1$-$C_3$ alkyl or $CH_3C(O)NH$;
$R_{27}$ is H, $C_1$-$C_3$ alkyl, $C_1$-$C_3$ alkoxy, $C_1$-$C_3$ alkylthio or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
$R_{31}$ is Cl, $C_1$-$C_3$ alkoxycarbonyl or $C_1$-$C_3$ alkyl;
A is

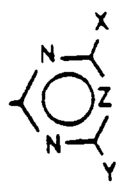
,
A-1

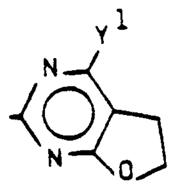
,
A-2

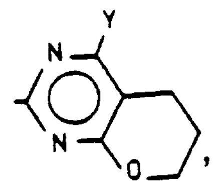
,
A-3

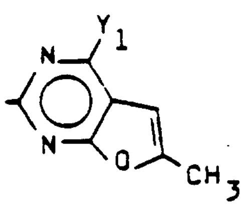
,
A-4

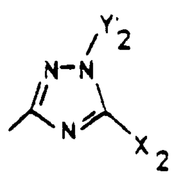
or
A-5

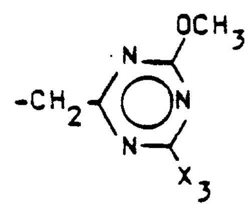
;
A-6

X is $CH_3$, $OCH_3$, Cl, F, $OCF_2H$ or $SCF_2H$;
Y is $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ or $GCF_2T$ where G is O or S and T is H, CHClF, CHBrF, $CF_2H$ or $CHFCF_3$;
Z is CH or N;
$Y_1$ is H, Cl, $CH_3$, $OCH_3$ or $OCF_2H$;
$X_2$ is $OCH_3$, $CH_3$, $CH_2CH_3$, $OCH_2CH_3$, $SCH_3$ or $SCH_2CH_3$;
$Y_2$ is $CH_3$, $CH_2CH_3$ or $CH_2CF_3$;
$X_3$ is $OCH_3$ or $CH_3$;

provided that,

a) when Q is Q-7, Q-8, Q-9 or Q-10, then R is H;
b) the total number of carbon atoms in $R_1$ is less than or equal to 8;
c) if $R_1$ is other than $C_1$-$C_3$ alkyl, then $R_3$ must be H;
d) when $R_5$ is other than H, $CH_3$, $OCH_3$ or $NO_2$, then $R_6$ is H or $CH_3$;
e) when $R_6$ is $CO_2R_{18}$, $SO_2NR_{20}R_{21}$ or $SO_2R_{22}$, then $R_5$ is H, $CH_3$, $OCH_3$ or $NO_2$;
f) when $R_{10}$ is other than $C_1$-$C_3$ alkyl, then $R_{11}$ is H, Cl, $OCH_3$, $NO_2$ or $CH_3$;
g) when either of $R_{13}$ or $R_{14}$ is $CO_2R_{24}$, $S(O)_mR_{25}$ or $SO_2NR_{20}R_{21}$, then the other is H, Cl, $CH_3$, $OCH_3$ or $NO_2$;
h) $R_{29}$ and $R_{30}$ are not simultaneously Cl;
i) when X is Cl or F, then Z is CH and Y is $OCH_3$, $NH_2$, $NHCH_3$ or $N(CH_3)_2$;

j) when Q is Q-7, Q-8, Q-9 or Q-10, then A is A-1; X is $CH_3$, $OCH_3$ or $OCF_2H$, Y is $CH_3$, $OCH_3$, $OC_2H_5$, $OCF_2H$ or $CH_2OCH_3$; and,

k) when either X or Y is $OCF_2H$, then Z is CH;

$k_1$) when Q is Q-2, R is H, Z is CH and when one of X and Y is $OCH_3$ then the other is not $CH_3$ when $R_4$, $R_5$ and $R_6$ are H;

l) when Q is Q-6, R is H, Z is CH and Y is $CH_3$ or $OCH_3$, then X is other than $CH_3$ or $OCH_3$ when

(1) $R_{13}$ and $R_{14}$ are $CH_3$ and $R_{15}$ is H or $CH_3$;
(2) $R_{13}$ and $R_{15}$ are $CH_3$ and $R_{14}$ is Br;
(3) $R_{13}$ is H, $R_{14}$ is Cl and $R_{15}$ is $CH_3$;
(4) $R_{13}$ is $CH_2CH_2CH_3$ or $CH(CH_3)_2$, $R_{14}$ is Cl and $R_{15}$ is $CH_3$;
(5) $R_{13}$ is $CH_2CH_3$ or $CH(CH_3)_2$, $R_{14}$ is $OCH_3$ and $R_{15}$ is $CH_3$;
(6) $R_{13}$ and $R_{14}$ are H and $R_{15}$ is H or $CH_3$;

m) when Q is Q-6, R is H, Z is CH or N and Y is $CH_3$ of $OCH_3$, then X is other than $CH_3$ or $OCH_3$ when

(1) $R_{13}$ and $R_{15}$ are $CH_3$ and $R_{14}$ is H, Cl or $OCH_3$;
(2) $R_{13}$ is H and $R_{14}$ and $R_{15}$ are $CH_3$;

n) when Q is Q-6, R is H, Z is N and Y is $CH_3$ or $OCH_3$, then X is other than $OCH_3$ when

(1) $R_{13}$ and $R_{14}$ are $CH_3$ and $R_{15}$ is H or $CH_3$;
(2) $R_{13}$ and $R_{15}$ are $CH_3$ and $R_{16}$ is Br;
(3) $R_{13}$ is H, $R_{14}$ is Cl and $R_{15}$ is $CH_3$;
(4) $R_{13}$ is $CH_2CH_3$ or $CH(CH_3)_2$, $R_{14}$ is $OCH_3$ and $R_{15}$ is $CH_3$;
(5) $R_{13}$ and $R_{14}$ are H and $R_{15}$ is H or $CH_3$;

o) when Q is Q-6, R is H, Z is CH and Y is $CH_3$ or $OCH_3$ then X is other than $CH_3$ when

(1) $R_{13}$ is $CH_2CH_3$ $R_{14}$ is H or Cl and $R_{15}$ is $CH_3$;
(2) $R_{13}$ is $CH_3$, $R_{14}$ is Cl and $R_{15}$ is H;
(3) $R_{13}$ and $R_{15}$ are $CH_3$ and $R_{14}$ is $OCH_2CH_3$;
(4) $R_{13}$ is $CH(CH_3)_2$, $R_{14}$ is H and $R_{15}$ is $CH_3$;
(5) $R_{13}$ and $R_{14}$ are $CH_2CH_3$ and $R_{15}$ is H;
(6) $R_{13}$ is H, $R_{14}$ is $CH_2CH_3$ or $CH(CH_3)_2$ and $R_{15}$ is $CH_3$;
(7) $R_{13}$ is $CH_2CH_3$ or $CH(CH_3)_2$, $R_{14}$ is $OCH_3$ and $R_{15}$ is H;
(8) $R_{13}$ is $CH_2CH_2CH_3$, $R_{14}$ is $OCH_3$ and $R_{15}$ is $CH_3$;

p) when Q is Q-6, R is H, Z is CH and Y is $CH_2CH_3$, then X is other than $OCH_3$ when $R_{13}$ and $R_{15}$ are $CH_3$ and $R_{14}$ is Cl;

q) when Q is Q-6, R is H, Z is N and Y is $CH_3$, then X is other than $OCH_3$ when

(1) $R_{13}$ is $CH_2CH_3$, $R_{14}$ is H or Cl and $R_{15}$ is $CH_3$;
(2) $R_{13}$ is $CH_3$, $R_{14}$ is Cl and $R_{15}$ is H;
(3) $R_{13}$ is $CH(CH_3)_2$, $R_{14}$ is H and $R_{15}$ is $CH_3$;
(4) $R_{13}$ and $R_{14}$ are $CH_2CH_3$ and $R_{15}$ is H;
(5) $R_{13}$ and $R_{15}$ are $CH_3$ and $R_{14}$ is $OCH_2CH_3$;
(6) $R_{13}$ is H, $R_{14}$ is $CH_2CH_3$ or $CH(CH_3)_2$ and $R_{15}$ is $CH_3$;
(7) $R_{13}$ is $CH_2CH_3$ of $CH(CH_3)2$, $R_{14}$ is $OCH_3$ and $R_{15}$ is H;
(8) $R_{13}$ is $CH_2CH_2CH_3$, $R_{14}$ is $OCH_3$ and $R_{15}$ is $CH_3$;

r) when Q is Q-6, R is H, Z is N and Y is $OCH_2CF_3$ then X is other than $OCH_3$ when $R_{13}$ and $R_{15}$ are $CH_3$ and $R_{14}$ is $OCH_3$;

s) when Q is Q-6, R is H, Z is CH and Y is $CF_3$ or $OCH_2CF_3$, then X is other than $CH_3$ when $R_{13}$ and $R_{15}$ are $CH_3$ and $R_{14}$ is $CO_2CH_3$;

t) when Q is Q-6, R is H, Z is N and Y is $OCH_2CF_3$, then X is other than $CH_3$ when $R_{13}$ and $R_{15}$ are $CH_3$ and $R_{14}$ is $SO_2CH_2CH_2CH_3$;

u) when Q is Q-6, R is H and A is A-2, then $Y_1$ is other than $CH_3$ when $R_{13}$ and $R_{14}$ are H and $R_{15}$ is $CH_3$;

v) when Q is Q-6, R is H, Z is N and Y is $CH_3$, then X is other than $CH_3$ when $R_{13}$ and $R_{14}$ are H and $R_{15}$ is $CH_2$;

w) when Q is Q-6, A is A-2, $Y^1$ is $CH_3$, R is H and $R_{15}$ is $CH_3$, then

(i) when $R_{13}$ is $CH_3$, $R_{14}$ is other than Cl, $CO_2CH_3$, $CO_2CH_2CH_3$, $SO_2C_3H_7$-n, $SO_2C_3H_7$-i or $SO_2N(CH_3)_2$; and
(ii) when $R_{13}$ is H, $R_{14}$ is other than H or $SO_2C_3H_7$-n;

x) when Q is Q-6, A is A-2, $Y^1$ is $CH_3$, R is H, $R_{15}$ is $CH_3$ and $R_{13}$ is $CO_2CH_3$, then $R_{14}$ is other than $CH_3$;

y) when Q is Q-4, A is A-2, $Y^1$ is $CH_3$, R is H and $R_{10}$ and $R_{12}$ are $CH_3$, then $R_{11}$ is other than Cl, $NO_2$ or $SO_2N(CH_3)_2$; and

z) when Q is Q-5, R, $R_{11}$ and $R_{12}$ are H, and A is A-2, then $Y^1$ is other than $CH_3$;

or an agriculturally suitable salts thereof; which comprises either

(a) contacting an aminoheterocycle of the formula

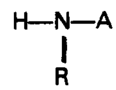

with

i) sulfonyl isocyanate of the formula $QSO_2NCO$, or with

ii) an N-phenylsulfonylcarbamate of the formula

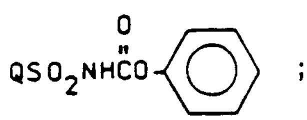

or

(b) contacting a sulfonamide of the formula $QSO_2NH_2$ with

i) a methyl carbamate of the formula

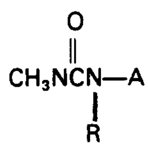

in the presence of an equimolar amount of trimethylaluminum, or with

ii) a heterocyclic isocyanate of the formula

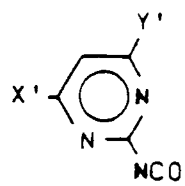

where X' is Cl and Y' is Cl or Br, optionally followed by reaction with a nucleophile such as methoxide or ethoxide ion; or with

iii) an N-heterocyclic carbamate of the formula

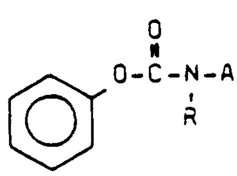

in the presence of a base.

**2.** A process of Claim 1 where

Q is Q-1, Q-2, Q-3, Q-4, Q-5, or Q-6;

A is

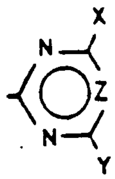 , 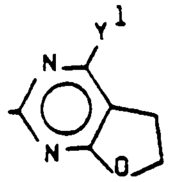 , 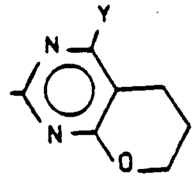 or 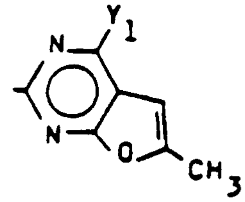 ;

X is $CH_3$, $OCH_3$ or Cl;
Y is $CH_3$, $C_2H_5$, $OCH_3$, $OC_2H_5$, $CH_2OCH_3$, $CH(OCH_3)_2$, $NH_2$, $NHCH_3$ or $N(CH_3)_2$;
Z is CH or N;
$Y_1$ is H, Cl, $CH_3$ or $OCH_3$;
$X_2$ is $OCH_3$ or $CH_3$; and
$Y_2$ is $CH_3$.

**3.** A process of Claim 1 where
Q is Q-7, Q-8 or Q-9;
$R_{26}$ is H, $C_1$-$C_3$ alkyl or $CH_3C(O)NH$; $R_{27}$ is H, $C_1$-$C_3$ alkyl, $C_1$-$C_3$ alkoxy, $C_1$-$C_3$ alkylthio, or $C_1$-$C_3$ alkoxycarbonyl;
$R_{28}$ is H, $C_1$-$C_3$ alkyl, $C_1$-$C_3$ alkoxy, $C_1$-$C_3$ alkylthio, $C_1$-$C_3$ alkylsulfonyl, $C_1$-$C_3$ alkoxycarbonyl, $NO_2$, Cl, Br or $CF_3$;
$R_{29}$ is H, Cl or $CH_3$;
$R_{30}$ is H, Cl or $CH_3$;
A is

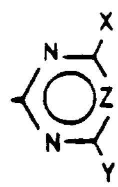 ;

Y is $CH_3$, $OCH_3$, $OCF_2H$, $OC_2H_5$ or $CH_2OCH_3$;
X is $CH_3$, $OCH_3$ or $OCF_2H$; and
Z is CH or N.

**4.** A process of Claim 1 where
Q is Q-10;
$R_{31}$ is Cl, $C_1$-$C_3$ alkoxycarbonyl or $C_1$-$C_3$ alkyl;
A is

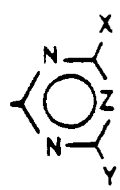 ;

X is $CH_3$, $OCH_3$ or $OCF_2H$;
Y is $CH_3$, $OCH_3$, $OC_2H_5$, $OCF_2H$ or $CH_2OCH_3$; and
Z is CH or N.

**5.** A process of Claim 1 where R is H.

**6.** A process of Claim 1 or 5 where A is A-1 and Z is CH.

**7.** A process of Claim 1, 5 or 6 where Q is Q-1 or Q-6.

**8.** A process of Claim 1 wherein Q is Q-4, R is H, $R_{10}$ is $CH_3$, $R_{12}$ is H, $R_{11}$ is $CO_2R_{24}$, $R_{24}$ is $C_1$-$C_3$ alkyl, A is A-1, X and Y are $OCH_3$ aand Z is CH.

**9.** A process of Claim 1 wherein the product is
N-[(4,6-dimethylpyrimidin-2-yl)aminocarbonyl]-1-(1-methylethyl)-1H-imidazole-2-sulfonamide;
N-[(4,6-dimethylpyrimidin-2-yl)aminocarbonyl]-1-ethyl-1H-imidazole-2-sulfonamide; or an agriculturally suitable salt of either or these.

**10.** A process of Claim 1 wherein the product is a compound selected from:
N-[(4-methoxy-6-methylpyrimidin-2-yl)aminocarbonyl-1-(1-methylethyl)-1H-imidazole-2-sulfonamide;
N-[(4-methoxy-6-methylpyrimidin-2-yl)aminocarbonyl]-1-ethyl-1H-imidazole-2-sulfonamide;
N-[(4-methoxy-6-methylpyrimidin-2-yl)aminocarbonyl]-4-thiazolesulfonamide;
methyl 4-[(4-methoxy-6-methylpyrimidin-2-yl)aminocarbonyl]aminosulfonyl]-3-isothiazolecarboxylate; or an agriculturally suitable salt of any of these.

**11.** A process of Claim 1 wherein the product is a compound selected from:
N-[(4,6-dimethoxypyrimdin-2-yl)aminocarbonyl]-1-(1-methylethyl)-1H-imidazole-2-sulfonamide;
N-[(4,6-dimethoxypyrimidin-2-yl)aminocarbonyl]-1-ethyl)-1H-imidazole-2-sulfonamide;
N-[(4,6-dimethoxypyrimidin-2-yl)aminocarbonyl]-5-bromo-1-methyl-1H-imidazole-4-sulfonamide;
N-[(4,6-dimethoxypyrimidin-2-yl)aminocarbonyl]-4-thiazolesulfonamide;
methyl 4-[(4,6-dimethoxypyrimidino-2-yl)aminocarbonyl]aminosulfonyl]-3-isothiazolecarboxylate; or an agriculturally suitable salt of any of these

**12.** A method for controlling the growth of undesired vegetation comprising applying to the locus of such vegetation an agriculturally effective amount of one or more compounds of formula (I) as defined in any of Claims 1-11 or an agriculturally suitable salt thereof.

**13.** A method of claim 14 wherein a compound as defined in claims 9, 10 or 11 is employed.

**Patentansprüche**
**Patentansprüche für folgende Vertragsstaaten : CH, DE, FR, GB, IT, LI, NL**

**1.** Verbindung der Formel

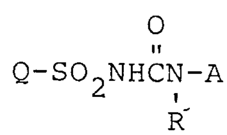

worin R H oder $CH_3$ ist;
Q

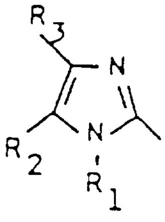,

Q-1

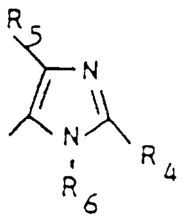,

Q-2

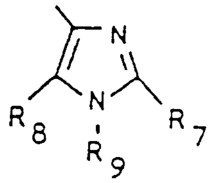,

Q-3

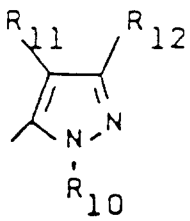,

Q-4

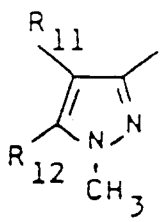,

Q-5

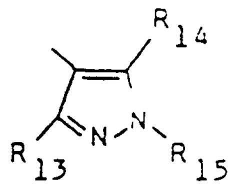;

Q-6

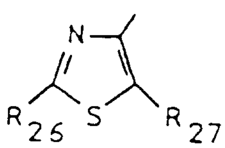,

Q-7

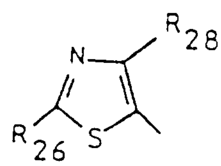,

Q-8

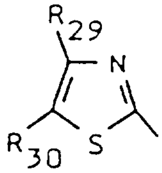 oder 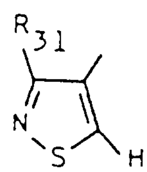

Q-9 Q-10

ist;

$R_1$ H, $C_1$-$C_8$-Alkyl, $C_3$-$C_6$-Alkenyl, $C_5$-$C_6$-Cycloalkyl, $C_5$-$C_6$-Cycloalkenyl, $C_3$-$C_6$-Alkinyl, $C_4$-$C_7$-Cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$,$(R_{17}CH)_nSO_2NR_{20}R_{21}$, $(R_{17}CH)_nSO_2R_{22}$,

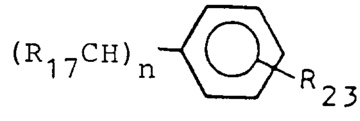

oder $C_1$-$C_6$-Alkyl, substituiert entweder mit

a) 1 bis 3 Atomen aus F, Br oder Cl; oder

b) $OR_{16}$; ist,

$R_2$, $R_3$ und $R_4$ unabhängig H oder $CH_3$ sind;

$R_5$ H, $C_1$-$C_4$-Alkyl, -$OR_6$, $NO_2$, F, Cl, Br, $CO_2R_{24}$,$S(O)_mR_{25}$ oder $SO_2NR_{20}R_{21}$ ist;

$R_6$ H, $C_1$-$C_4$-Alkyl, $C_3$-$C_4$-Alkenyl, $C_3$-$C_4$-Alkinyl, $CO_2R_{18}$, $SO_2NR_{20}R_{21}$, $SO_2R_{22}$ oder $C_1$-$C_4$-Alkyl, substituiert mit

a) 1 bis 3 Atomen aus F, Cl oder Br; oder
b) $OCH_3$; ist;

$R_7$ H oder $CH_3$ ist;
$R_8$ H, $C_1$-$C_4$-Alkyl, -$OR_{16}$, $NO_2$, F, Cl, Br, $CO_2R_{24}$, $S(O)_mR_{25}$ oder $SO_2NR_{20}R_{21}$ ist;
$R_9$ $CH_3$ oder $C_2H_5$ ist;
$R_{10}$ H, $C_1$-$C_4$-Alkyl, $C_3$-$C_4$-Alkenyl, $C_3$-$C_4$-Alkinyl, $CO_2R_{24}$, $SO_2NR_{20}R_{21}$ oder $SO_2R_{22}$ ist;
$R_{11}$ H, $C_1$-$C_3$-Alkyl, F, Cl, Br, $NO_2$, -$OR_{16}$, $CO_2R_{24}$, $S(O)_mR_{25}$ oder $SO_2NR_{20}R_{21}$ ist;
$R_{12}$ H oder $CH_3$ ist;
$R_{13}$ und $R_{14}$ unabhängig H, $C_1$-$C_3$-Alkyl, -$OR_{16}$, F, Cl, Br, $NO_2$, $CO_2R_{24}$, $S(O)_mR_{25}$ oder $SO_2NR_{20}R_{21}$ sind;
$R_{15}$ H oder $CH_3$ ist;
$R_{16}$ $C_1$-$C_3$-Alkyl ist;
$R_{17}$ H oder $CH_3$ ist;
$R_{18}$ $C_1$-$C_4$-Alkyl, $C_3$-$C_4$-Alkenyl, $C_3$-$C_4$-Alkinyl, $CH_2CH_2Cl$ oder $CH_2CH_2OCH_3$ ist;
$R_{19}$ $C_1$-$C_4$-Alkyl, $C_3$-$C_4$-Alkenyl, $C_3$-$C_4$-Alkinyl oder $CH_2CH_2OCH_3$ ist;
$R_{20}$ und $R_{21}$ unabhängig $CH_3$ oder $C_2H_5$ sind;
$R_{22}$ $C_1$-$C_3$-Alkyl oder $CF_3$ ist;
$R_{23}$ H, Cl, Br, $CH_3$, F, $CF_3$, $OCH_3$ oder $NO_2$ ist;
$R_{24}$ $C_1$-$C_3$-Alkyl oder Allyl ist;
$R_{25}$ $C_1$-$C_3$-Alkyl ist;
m 0, 1 oder 2 ist;
n 0 oder 1 ist;
$R_{26}$ H, $C_1$-$C_3$-Alkyl oder $CH_3C(O)NH$ ist;
$R_{27}$ H, $C_1$-$C_3$-Alkyl, $C_1$-$C_3$-Alkoxy, $C_1$-$C_3$-Alkylthio oder $C_1$-$C_3$-Alkoxycarbonyl ist;
$R_{28}$ H, $C_1$-$C_3$-Alkyl, $C_1$-$C_3$-Alkoxy, $C_1$-$C_3$-Alkylthio, $C_1$-$C_3$-Alkoxycarbonyl, $NO_2$, Cl, Br oder $CF_3$ ist;
$R_{29}$ H, Cl oder $CH_3$ ist;
$R_{30}$ H, Cl oder $CH_3$ ist;
$R_{31}$ Cl, $C_1$-$C_3$-Alkoxycarbonyl oder $C_1$-$C_3$-Alkyl ist;
A

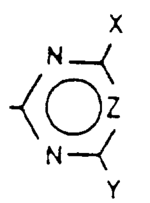 , 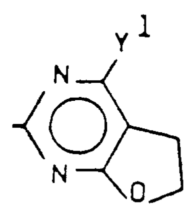 , 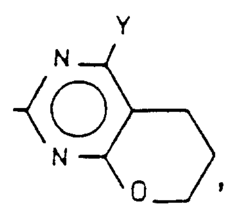 ,

A-1 A-2 A-3

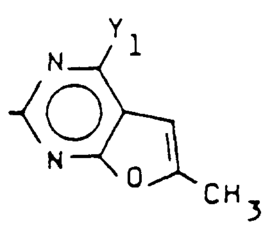 , 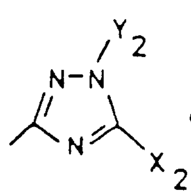 oder 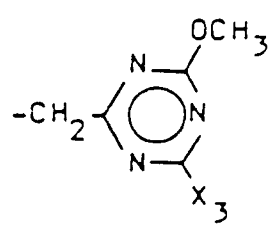

A-4 A-5 A-6

ist;
X $CH_3$, $OCH_3$, Cl, F, $OCF_2H$ oder $SCF_2H$ ist;
Y $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ oder $GCF_2T$ ist, worin G O oder S ist und T H, CHClF, CHBrF,

$CF_2H$ oder $CHFCF_3$ ist;
Z CH oder N ist;
$Y_1$ H, Cl, $CH_3$, $OCH_3$ oder $OCF_2H$ ist;
$X_2$ $OCH_3$, $CH_3$, $CH_2CH_3$, $OCH_2CH_3$, $SCH_3$ oder $SCH_2CH_3$ ist;
$Y_2$ $CH_3$, $CH_2CH_3$ oder $CH_2CF_3$ ist;
$X_3$ $OCH_3$ oder $CH_3$ ist;

mit der Maßgabe, daß

a) wenn Q Q-7, Q-8, Q-9 oder Q-10 ist, dann R H ist;
b) die Gesamtzahl der Kohlenstoffatome in $R_1$ weniger als oder gleich 8 ist;
c) falls $R_1$ von $C_1$-$C_3$-Alkyl verschieden ist, dann $R_3$ H ist;
d) wenn $R_5$ von H, $CH_3$, $OCH_3$ oder $NO_2$ verschieden ist, dann $R_6$ H oder $CH_3$ ist;
e) wenn $R_6$ $CO_2R_{18}$, $SO_2NR_{20}R_{21}$ oder $SO_2R_{22}$ ist, dann $R_5$ H, $CH_3$, $OCH_3$ oder $NO_2$ ist;
f) wenn $R_{10}$ von $C_1$-$C_3$-Alkyl verschieden ist, dann $R_{11}$ H, Cl, $OCH_3$, $NO_2$ oder $CH_3$ ist;
g) wenn entweder $R_{13}$ oder $R_{14}$ $CO_2R_{24}$, $S(O)_mR_{25}$ oder $SO_2NR_{20}R_{21}$ ist, dann das andere H, Cl, $CH_3$, $OCH_3$ oder $NO_2$ ist;
h) $R_{29}$ und $R_{30}$ nicht gleichzeitig Cl sind;
i) wenn X Cl oder F ist, dann Z CH ist und Y $OCH_3$, $NH_2$, $NHCH_3$ oder $N(CH_3)_2$ ist;
j) wenn Q Q-7, Q-8, Q-9 oder Q-10 ist, dann A A-1 ist; X $CH_3$, $OCH_3$ oder $OCF_2H$ ist, Y $CH_3$, $OCH_3$, $OC_2H_5$, $OCF_2H$ oder $CH_2OCH_3$ ist; und
k) wenn entweder X oder Y $OCF_2H$ ist, dann Z CH ist;
  $k_1$) wenn Q Q-2 ist, R H ist, Z CH ist und wenn entweder X oder Y $OCH_3$ ist, dann das andere nicht $CH_3$ ist, wenn $R_4$, $R_5$, $R_6$ H sind;
l) wenn Q Q-6 ist, R H ist, Z CH ist und Y $CH_3$ oder $OCH_3$ ist, dann X von $CH_3$ oder $OCH_3$ verschieden ist, wenn
  (1) $R_{13}$ und $R_{14}$ $CH_3$ sind und $R_{15}$ H oder $CH_3$ ist;
  (2) $R_{13}$ und $R_{15}$ $CH_3$ sind und $R_{14}$ Br ist;
  (3) $R_{13}$ H ist, $R_{14}$ Cl ist und $R_{15}$ $CH_3$ ist;
  (4) $R_{13}$ $CH_2CH_2CH_3$ oder $CH(CH_3)_2$ ist, $R_{14}$ Cl ist und $R_{15}$ $CH_3$ ist;
  (5) $R_{13}$ $CH_2CH_3$ oder $CH(CH_3)_2$ ist, $R_{14}$ $OCH_3$ ist und $R_{15}$ $CH_3$ ist;
  (6) $R_{13}$ und $R_{14}$ H sind und $R_{15}$ H oder $CH_3$ ist;
m) wenn Q Q-6 ist, R H ist, Z CH oder N ist und Y $CH_3$ oder $OCH_3$ ist, dann X von $CH_3$ oder $OCH_3$ verschieden ist, wenn
l)
  (1) $R_{13}$ und $R_{15}$ $CH_3$ sind und $R_{14}$ H, Cl oder $OCH_3$ ist;
  (2) $R_{13}$ H ist und $R_{14}$ und $R_{15}$ $CH_3$ sind;
n) wenn Q Q-6 ist, R H ist, Z N ist und Y $CH_3$ oder $OCH_3$ ist, dann X von $OCH_3$ verschieden ist, wenn
  (1) $R_{13}$ und $R_{14}$ $CH_3$ sind und $R_{15}$ H oder $CH_3$ ist;
  (2) $R_{13}$ und $R_{15}$ $CH_3$ sind und $R_{14}$ Br ist;
  (3) $R_{13}$ H ist, $R_{14}$ Cl ist und $R_{15}$ $CH_3$ ist;
  (4) $R_{13}$ $CH_2CH_3$ oder $CH(CH_3)_2$ ist, $R_{14}$ $OCH_3$ ist und $R_{15}$ $CH_3$ ist;
  (5) $R_{13}$ und $R_{14}$ H sind und $R_{15}$ H oder $CH_3$ ist;
o) wenn Q Q-6 ist, R H ist, Z CH ist und Y $CH_3$ oder $OCH_3$ ist, dann X von $CH_3$ verschieden ist, wenn
  (1) $R_{13}$ $CH_2CH_3$ ist, $R_{14}$ H oder Cl ist und $R_{15}$ $CH_3$ ist;
  (2) $R_{13}$ $CH_3$ ist, $R_{14}$ Cl ist und $R_{15}$ H ist;
  (3) $R_{13}$ und $R_{15}$ $CH_3$ sind und $R_{14}$ $OCH_2CH_3$ ist;
  (4) $R_{13}$ $CH(CH_3)_2$ ist, $R_{14}$ H ist und $R_{15}$ $CH_3$ ist;
  (5) $R_{13}$ und $R_{14}$ $CH_2CH_3$ sind und $R_{15}$ H ist;
  (6) $R_{13}$ H ist, $R_{14}$ $CH_2CH_3$ oder $CH(CH_3)_2$ ist und $R_{15}$ $CH_3$ ist;
  (7) $R_{13}$ $CH_2CH_3$ oder $CH(CH_3)_2$ ist, $R_{14}$ $OCH_3$ ist und $R_{15}$ H ist;
  (8) $R_{13}$ $CH_2CH_2CH_3$, $R_{14}$ $OCH_3$ und $R_{15}$ $CH_3$ ist;
p) wenn Q Q-6 ist, R H ist, Z CH ist und Y $CH_2CH_3$ ist, dann X von $OCH_3$ verschieden ist, wenn $R_{13}$ und $R_{15}$ $CH_3$ sind und $R_{14}$ Cl ist;
q) wenn Q Q-6 ist, R H ist, Z N ist und Y $CH_3$ ist, dann X von $OCH_3$ verschieden ist, wenn
  (1) $R_{13}$ $CH_2CH_3$ ist, $R_{14}$ H oder Cl ist und $R_{15}$ $CH_3$ ist;
  (2) $R_{13}$ $CH_3$ ist, $R_{14}$ Cl ist und $R_{15}$ H ist;
  (3) $R_{13}$ $CH(CH_3)_2$ ist, $R_{14}$ H ist und $R_{15}$ $CH_3$ ist;

(4) $R_{13}$ und $R_{14}$ $CH_2CH_3$ sind und $R_{15}$ H ist;
(5) $R_{13}$ und $R_{15}$ $CH_3$ sind und $R_{14}$ $OCH_2CH_3$ ist;
(6) $R_{13}$ H ist, $R_{14}$ $CH_2CH_3$ oder $CH(CH_3)_2$ ist und $R_{15}$ $CH_3$ ist;
(7) $R_{13}$ $CH_2CH_3$ oder $CH(CH_3)_2$ ist, $R_{14}$ $OCH_3$ ist und $R_{15}$ H ist;
(8) $R_{13}$ $CH_2CH_2CH_3$ ist, $R_{14}$ $OCH_3$ ist und $R_{15}$ $CH_3$ ist;

r) wenn Q Q-6 ist, R H ist, Z N ist und Y $OCH_2CF_3$ ist, dann X von $OCH_3$ verschieden ist, wenn $R_{13}$ und $R_{15}$ $CH_3$ sind und $R_{14}$ $OCH_3$ ist;
s) wenn Q Q-6 ist, R H ist, Z CH ist und Y $CF_3$ oder $OCH_2CF_3$ ist, dann X von $CH_3$ verschieden ist, wenn $R_{13}$ und $R_{15}$ $CH_3$ sind und $R_{14}$ $CO_2CH_3$ ist;
t) wenn Q Q-6 ist, R H ist, Z N ist und Y $OCH_2CF_3$ ist, dann X von $CH_3$ verschieden ist, wenn $R_{13}$ und $R_{15}$ $CH_3$ sind und $R_{14}$ $SO_2CH_2CH_2CH_3$ ist;
u) wenn Q Q-6 ist, R H ist und A A-2 ist, dann $Y_1$ von $CH_3$ verschieden ist, wenn $R_{13}$ und $R_{14}$ H sind und $R_{15}$ $CH_3$ ist;
v) wenn Q Q-6 ist, R H ist, Z N ist und Y $CH_3$ ist, dann X von $CH_3$ verschieden ist, wenn $R_{13}$ und $R_{14}$ H sind und $R_{15}$ $CH_3$ ist;
w) wenn Q Q-3 ist, R H ist, Z N ist und Y $OCH_3$ ist, dann X von $CH_3$ verschieden ist, wenn $R_7$ und $R_8$ H sind und $R_9$ $CH_3$ ist;
x) wenn Q Q-10 ist, R H ist, Z N ist und Y $OCH_3$ ist, dann X von $OCH_3$ verschieden ist, wenn $R_{31}$ $CH_3$ ist;
y) wenn Q Q-9 ist, R H ist, Z CH ist und Y $OCH_3$ ist, dann X von $CH_3$ verschieden ist, wenn $R_{29}$ $CH_3$ ist und $R_{30}$ H ist;
z) wenn Q Q-1 ist, R H ist, Z N ist und Y $OCH_3$ ist, dann X von $CH_3$ verschieden ist, wenn $R_1$ $CH_3$ ist und $R_2$ und $R_3$ H sind;

$a_1$) wenn Q Q-1 ist, R H ist, Z CH ist und Y $CH_3$ oder $OCH_3$ ist, dann X von $CH_3$ oder $OCH_3$ verschiedenist, wenn $R_1$ $CH_3$ ist und $R_2$ und $R_3$ H sind;
$b_1$) wenn Q Q-1 ist, R H ist, Z CH ist und Y $OCHF_2$ ist, dann X von $CH_3$ verschieden ist, wenn $R_1$ $CH_3$ ist und $R_2$ und $R_3$ H sind;
$c_1$) wenn Q Q-4 ist, R H ist, Z CH ist und Y $CH_2CH_3$ ist, dann X von $CH_3$ verschieden ist, wenn $R_{10}$, $R_{11}$ und $R_{12}$ H sind;
$d_1$) wenn Q Q-1 ist, R $CH_3$ ist, Z N ist und Y $OCH_3$ ist, dann X von $CH_3$ verschieden ist, wenn $R_1$, $R_2$ und $R_3$ H sind;
$e_1$) wenn Q Q-9 ist, R H ist, Z N ist und Y $CH_3$ ist, dann X von $CH_3$ verschieden ist, wenn $R_{29}$ und $R_{30}$ sind;
$f_1$) wenn Q Q-6 ist, A A-2 ist, $Y^1$ $CH_3$ ist, R H ist, $R_{15}$ und $R_{13}$ $CH_3$ sind, dann $R_{14}$ von Cl, $CO_2CH_3$, $CO_2CH_2CH_3$, $SO_2C_3H_7$-n, $SO_2C_3H_7$-i oder $SO_2N(CH_3)_2$ verschieden ist;
$g_1$) wenn Q Q-6 ist, A A-2 ist, $Y^1$ $CH_3$ ist, R H ist, $R_{15}$ $CH_3$ ist und $R_{13}$ H ist, dann $R_{14}$ von H oder $SO_2C_3H_7$-n verschieden ist;
$h_1$) wenn Q Q-6 ist, A A-2 ist, $Y^1$ $CH_3$ ist, R H ist, $R_{15}$ $CH_3$ ist und $R_{13}$ $CO_2CH_3$ ist, dann $R_{14}$ von $CH_3$ verschieden ist;
$i_1$) wenn Q Q-4 ist, A A-2 ist, $Y^1$ $CH_3$ ist, R H ist und $R_{10}$ und $R_{12}$ $CH_3$ sind, dann $R_{11}$ von Cl, $NO_2$ oder $SO_2N(CH_3)_2$ verschieden ist; und
$j_1$) wenn Q Q-5 ist, R, $R_{11}$ und $R_{12}$ H sind und A A-2 ist, dann $Y^1$ von $CH_3$ verschieden ist;

und landwirtschaftlich geeignete Salze davon.

**2.** Verbindung nach Anspruch 1,
worin Q Q-1, Q-2, Q-3, Q-4, Q-5, Q-6 ist;
A

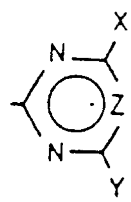 , 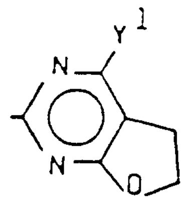 , 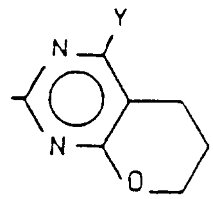

oder 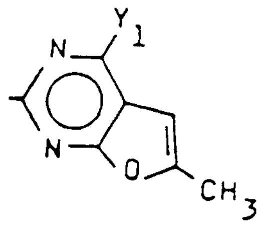

ist;

X $CH_3$, $OCH_3$ oder Cl ist;

Y $CH_3$, $C_2H_5$, $OCH_3$, $OC_2H_5$, $CH_2OCH_3$, $CH(OCH_3)_2$, $NH_2$, $NHCH_3$ oder $N(CH_3)_2$ ist;

Z CH oder N ist;

$Y_1$ H, Cl, $CH_3$ oder $OCH_3$ ist;

$X_2$ $OCH_3$ oder $CH_3$ ist; und

$Y_2$ $CH_3$ ist.

**3.** Verbindung nach Anspruch 1, worin Q Q-7, Q-8 oder Q-9 ist;

$R_{26}$ H, $C_1$-$C_3$-Alkyl oder $CH_3C(O)NH$ ist;

$R_{27}$ H, $C_1$-$C_3$-Alkyl, $C_1$-$C_3$-Alkoxy, $C_1$-$C_3$-Alkylthio oder $C_1$-$C_3$-Alkoxycarbonyl ist;

$R_{28}$ H, $C_1$-$C_3$-Alkyl, $C_1$-$C_3$-Alkoxy, $C_1$-$C_3$-Alkylthio, $C_1$-$C_3$-Alkylsulfonyl, $C_1$-$C_3$-Alkoxycarbonyl, $NO_2$, Cl, Br oder $CF_3$ ist;

$R_{29}$ H, Cl oder $CH_3$ ist;

$R_{30}$ H, Cl oder $CH_3$ ist;

A

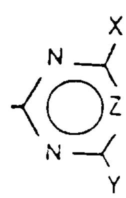

ist;

Y $CH_3$, $OCH_3$, $OCF_2H$, $OC_2H_5$ oder $CH_2OCH_3$ ist;

X $CH_3$, $OCH_3$ oder $OCF_2H$ ist; und

Z CH oder N ist.

**4.** Verbindung nach Anspruch 1, worin Q Q-10 ist;

$R_{31}$ Cl, $C_1$-$C_3$-Alkoxycarbonyl oder $C_1$-$C_3$-Alkyl ist;

A

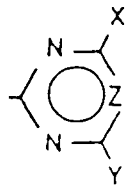

ist;

X $CH_3$, $OCH_3$ oder $OCF_2H$ ist;

Y $CH_3$, $OCH_3$, $OC_2H_5$, $OCF_2H$ oder $CH_2OCH_3$ ist; und

Z CH oder N ist.

**5.** Verbindung nach Anspruch 1, worin R H ist.

**6.** Verbindung nach Anspruch 1 oder 5, worin A A-1 ist und Z CH ist.

**7.** Verbindung nach Anspruch 1, 5 oder 6, worin Q Q-1 oder Q-6 ist.

**8.** Verbindung nach Anspruch 1, worin Q Q-4 ist, R H ist, $R_{10}$ $CH_3$ ist, $R_{12}$ H ist, $R_{11}$ $CO_2R_{24}$ ist, $R_{24}$ $C_1$-$C_3$ Alkyl ist, A A-1 ist, X und Y $OCH_3$ sind und Z CH ist.

**9.** Verbindung nach Anspruch 1, welche N-[(4,6-Dimethylpyrimidin-2-yl)aminocarbonyl]-1-(1-methylethyl)-1H-imidazol-2-sulfonamid;
N-[(4,6-Dimethylpyrimidin-2-yl)aminocarbonyl]-1-ethyl-1H-imidazol-2-sulfonamid;
oder ein landwirtschaftlich geeignetes Salz von beiden ist.

**10.** Verbindung nach Anspruch 1, ausgewählt aus N-[(4-Methoxy-6-methylpyrimidin-2-yl)aminocarbonyl]-1-(1-methylethyl)-1H-imidazol-2-sulfonamid;
N-[(4-Methoxy-6-methylpyrimidin-2-yl)aminocarbonyl]-1-ethyl-1H-imidazol-2-sulfonamid;
N-[(4-Methoxy-6-methylpyrimidin-2-yl)aminocarbonyl]-4-thiazolsulfonamid;
Methyl-4-[(4-methoxy-6-methylpyrimidin-2-yl)aminocarbonyl]aminosulfonyl)-3-isothiazolcarboxylat
oder ein landwirtschaftlich geeignetes Salz von einer davon.

**11.** Verbindung nach Anspruch 1, ausgewählt aus:
N-[(4,6-Dimethoxypyrimidin-2-yl)aminocarbonyl]-1-(1-methylethyl)-1H-imidazol-2-sulfonamid;
N-[(4,6-Dimethoxypyrimidin-2-yl)aminocarbonyl]-1-ethyl-1H-imidazol-2-sulfonamid;
N-[(4,6-Dimethoxypyrimidin-2-yl)aminocarbonyl]-5-brom-1-methyl-1H-imidazol-4-sulfonamid;
N-[(4,6-Dimethoxypyrimidin-2-yl)aminocarbonyl]-4-thiazolsulfonamid;
Methyl-4-[(4,6-Dimethoxypyrimidin-2-yl)aminocarbonyl]aminosulfonyl-3-isothiazolcarboxylat;
oder ein landwirtschaftlich geeignetes Salz von einer davon.

**12.** Landwirtschaftliche Zusammensetzung, umfassend eine herbizid wirksame Menge einer oder mehrerer Verbindungen nach einem der Ansprüche 1 bis 11 und wenigstens eines der folgenden: (a) eines oberflächenaktiven Mittels und (b) eines festen oder flüssigen Verdünnungsmittels.

**13.** Verfahren zur Bekämpfung des Wachstums unerwünschter Vegetation, welches die Anwendung einer landwirtschaftlich wirksamen Menge einer oder mehrerer Verbindungen nach einem der Ansprüche 1 bis 11 auf den Ort solcher Vegetation umfaßt.

**14.** Verfahren zur Wachstumssteuerung von Pflanzen, welches die Anwendung einer wirksamen, jedoch im wesentlichen nicht phytotoxischen Menge eines Pflanzenwachstumsregulans, ausgewählt aus den Verbindungen nach einem der Ansprüche 1 bis 11, auf den Ort solcher Pflanzen umfaßt.

**15.** Verfahren zur Herstellung einer Verbindung nach Anspruch 1 durch entweder

(a) Inberührungbringen eines Aminoheterocyclus der Formel

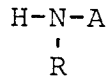

mit

i) einem Sulfonylisocyanat der Formel $QSO_2NCO$ oder mit

ii) einem N-Phenylsulfonylcarbamat der Formel

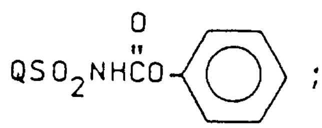

(b) Inberührungbringen eines Sulfonamids der Formel $QSO_2NH_2$ mit

i) einem Methylcarbamat der Formel

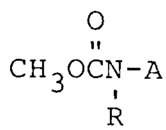

in Gegenwart einer äquimolaren Menge Trimethylaluminium oder mit

ii) einem heterocyclischen Isocyanat der Formel

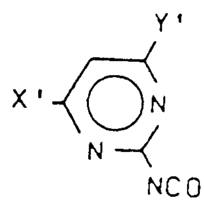

worin X' Cl ist Y' Cl oder Br ist, gegebenenfalls gefolgt von der Reaktion mit einem Nukleophil, wie Methoxid- oder Ethoxidionen; oder mit

iii) einem N-heterocyclischen Carbamat der Formel

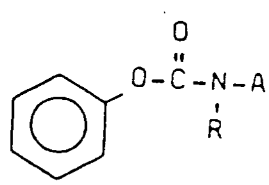

in Gegenwart einer Base.

**Patentansprüche für folgenden Vertragsstaat : BE**

**1.** Verbindung der Formel

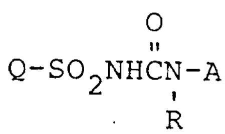

worin R H oder $CH_3$ ist;

Q

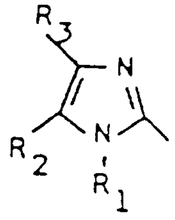,
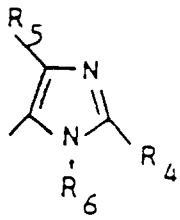,
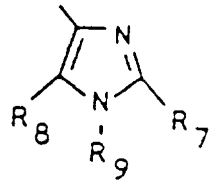,

Q-1 Q-2 Q-3

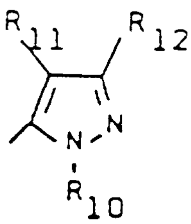,
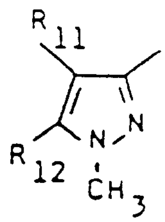,
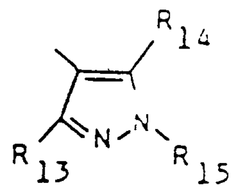;

Q-4 Q-5 Q-6

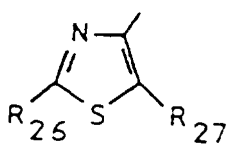,
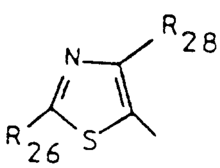,

Q-7 Q-8

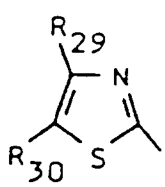 oder 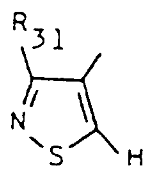

Q-9 Q-10

ist;

$R_1$ H, $C_1$-$C_8$-Alkyl, $C_3$-$C_6$-Alkenyl, $C_5$-$C_6$-Cycloalkyl, $C_5$-$C_6$-Cycloalkenyl, $C_3$-$C_6$-Alkinyl, $C_4$-$C_7$-Cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

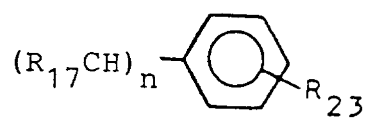

oder $C_1$-$C_6$-Alkyl, substituiert entweder mit

a) 1 bis 3 Atomen aus F, Br oder Cl; oder

b) $OR_{16}$; ist,

$R_2$, $R_3$ und $R_4$ unabhängig H oder $CH_3$ sind;

| | |
|---|---|
| $R_5$ | H, $C_1$-$C_4$-Alkyl, -$OR_6$, $NO_2$, F, Cl, Br, $CO_2R_{24}$,$S(O)_mR_{25}$ oder $SO_2NR_{20}R_{21}$ ist; |
| $R_6$ | H, $C_1$-$C_4$-Alkyl, $C_3$-$C_4$-Alkenyl, $C_3$-$C_4$-Alkinyl, $CO_2R_{18}$, $SO_2NR_{20}R_{21}$, $SO_2R_{22}$ oder $C_1$-$C_4$-Alkyl, substituiert mit<br>a) 1 bis 3 Atomen aus F, Cl oder Br; oder<br>b) $OCH_3$; ist; |
| $R_7$ | H oder $CH_3$ ist; |
| $R_8$ | H, $C_1$-$C_4$-Alkyl, -$OR_{16}$, $NO_2$, F, Cl, Br, $CO_2R_{24}$,$S(O)_mR_{25}$ oder $SO_2NR_{20}R_{21}$ ist; |
| $R_9$ | $CH_3$ oder $C_2H_5$ ist; |
| $R_{10}$ | H, $C_1$-$C_4$-Alkyl, $C_3$-$C_4$-Alkenyl, $C_3$-$C_4$-Alkinyl, $CO_2R_{24}$, $SO_2NR_{20}R_{21}$ oder $SO_2R_{22}$ ist; |
| $R_{11}$ | H, $C_1$-$C_3$-Alkyl, F, Cl, Br, $NO_2$, -$OR_{16}$, $CO_2R_{24}$,$S(O)_mR_{25}$ oder $SO_2NR_{20}R_{21}$ ist; |
| $R_{12}$ | H oder $CH_3$ ist; |
| $R_{13}$ und $R_{14}$ | unabhängig H, $C_1$-$C_3$-Alkyl, -$OR_{16}$, F, Cl, Br, $NO_2$, $CO_2R_{24}$, $S(O)_mR_{25}$ oder $SO_2NR_{20}R_{21}$ sind; |
| $R_{15}$ | H oder $CH_3$ ist; |
| $R_{16}$ | $C_1$-$C_3$-Alkyl ist; |
| $R_{17}$ | H oder $CH_3$ ist; |
| $R_{18}$ | $C_1$-$C_4$-Alkyl, $C_3$-$C_4$-Alkenyl, $C_3$-$C_4$-Alkinyl, $CH_2CH_2Cl$ oder $CH_2CH_2OCH_3$ ist; |
| $R_{19}$ | $C_1$-$C_4$-Alkyl, $C_3$-$C_4$-Alkenyl, $C_3$-$C_4$-Alkinyl oder $CH_2CH_2OCH_3$ ist; |
| $R_{20}$ und $R_{21}$ | unabhängig $CH_3$ oder $C_2H_5$ sind; |
| $R_{22}$ | $C_1$-$C_3$-Alkyl oder $CF_3$ ist; |
| $R_{23}$ | H, Cl, Br, $CH_3$, F, $CF_3$, $OCH_3$ oder $NO_2$ ist; |
| $R_{24}$ | $C_1$-$C_3$-Alkyl oder Allyl ist; |
| $R_{25}$ | $C_1$-$C_3$-Alkyl ist; |
| m | 0, 1 oder 2 ist; |
| n | 0 oder 1 ist; |
| $R_{26}$ | H, $C_1$-$C_3$-Alkyl oder $CH_3C(O)NH$ ist; |
| $R_{27}$ | H, $C_1$-$C_3$-Alkyl, $C_1$-$C_3$-Alkoxy, $C_1$-$C_3$-Alkylthio oder $C_1$-$C_3$-Alkoxycarbonyl ist; |
| $R_{28}$ | H, $C_1$-$C_3$-Alkyl, $C_1$-$C_3$-Alkoxy, $C_1$-$C_3$-Alkylthio, $C_1$-$C_3$-Alkoxycarbonyl, $NO_2$, Cl, Br oder $CF_3$ ist; |
| $R_{29}$ | H, Cl oder $CH_3$ ist; |
| $R_{30}$ | H, Cl oder $CH_3$ ist; |
| $R_{31}$ | Cl, $C_1$-$C_3$-Alkoxycarbonyl oder $C_1$-$C_3$-Alkyl ist; |
| A | |

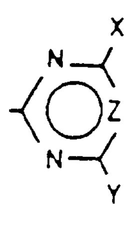
,
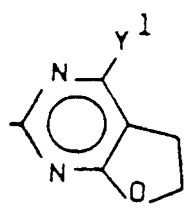
,
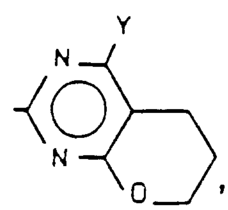
,

A-1 A-2 A-3

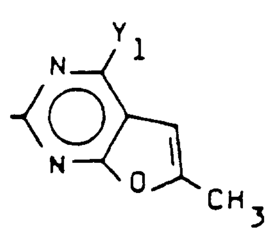
,
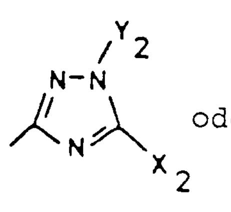
oder
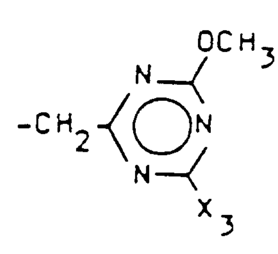

A-4 A-5 A-6

ist;

X $CH_3$, $OCH_3$, Cl, F, $OCF_2H$ oder $SCF_2H$ ist;

Y $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ oder $GCF_2T$ ist, worin G O oder S ist und T H, CHClF, CHBrF, $CF_2H$ oder $CHFCF_3$ ist;

Z CH oder N ist;

$Y_1$ H, Cl, $CH_3$, $OCH_3$ oder $OCF_2H$ ist;

$X_2$ $OCH_3$, $CH_3$, $CH_2CH_3$, $OCH_2CH_3$, $SCH_3$ oder $SCH_2CH_3$ ist;

$Y_2$ $CH_3$, $CH_2CH_3$ oder $CH_2CF_3$ ist;

$X_3$ $OCH_3$ oder $CH_3$ ist;

mit der Maßgabe, daß

a) wenn Q Q-7, Q-8, Q-9 oder Q-10 ist, dann R H ist;

b) die Gesamtzahl der Kohlenstoffatome in $R_1$ weniger als oder gleich 8 ist;

c) falls $R_1$ von $C_1$-$C_3$-Alkyl verschieden ist, dann $R_3$ H ist;

d) wenn $R_5$ von H, $CH_3$, $OCH_3$ oder $NO_2$ verschieden ist, dann $R_6$ H oder $CH_3$ ist;

e) wenn $R_6$ $CO_2R_{18}$, $SO_2NR_{20}R_{21}$ oder $SO_2R_{22}$ ist, dann $R_5$ H, $CH_3$, $OCH_3$ oder $NO_2$ ist;

f) wenn $R_{10}$ von $C_1$-$C_3$-Alkyl verschieden ist, dann $R_{11}$ H, Cl, $OCH_3$, $NO_2$ oder $CH_3$ ist;

g) wenn entweder $R_{13}$ oder $R_{14}$ $CO_2R_{24}$, $S(O)_mR_{25}$ oder $SO_2NR_{20}R_{21}$ ist, dann das andere H, Cl, $CH_3$, $OCH_3$ oder $NO_2$ ist;

h) $R_{29}$ und $R_{30}$ nicht gleichzeitig Cl sind;

i) wenn X Cl oder F ist, dann Z CH ist und Y $OCH_3$, $NH_2$, $NHCH_3$ oder $N(CH_3)_2$ ist;

j) wenn Q Q-7, Q-8, Q-9 oder Q-10 ist, dann A A-1 ist; X $CH_3$, $OCH_3$ oder $OCF_2H$ ist, Y $CH_3$, $OCH_3$, $OC_2H_5$, $OCF_2H$ oder $CH_2OCH_3$ ist; und

k) wenn entweder X oder Y $OCF_2H$ ist, dann Z CH ist;

$k_1$) wenn Q Q-2 ist, R H ist, Z CH ist und wenn entweder X oder Y $OCH_3$ ist, dann das andere nicht $CH_3$ ist, wenn $R_4$, $R_5$, $R_6$ H sind;

l) wenn Q Q-6 ist, R H ist, Z CH ist und Y $CH_3$ oder $OCH_3$ ist, dann X von $CH_3$ oder $OCH_3$ verschieden ist, wenn

(1) $R_{13}$ und $R_{14}$ $CH_3$ sind und $R_{15}$ H oder $CH_3$ ist;

(2) $R_{13}$ und $R_{15}$ $CH_3$ sind und $R_{14}$ Br ist;

(3) $R_{13}$ H ist, $R_{14}$ Cl ist und $R_{15}$ $CH_3$ ist;

(4) $R_{13}$ $CH_2CH_2CH_3$ oder $CH(CH_3)_2$ ist, $R_{14}$ Cl ist und $R_{15}$ $CH_3$ ist;

(5) $R_{13}$ $CH_2CH_3$ oder $CH(CH_3)_2$ ist, $R_{14}$ $OCH_3$ ist und $R_{15}$ $CH_3$ ist;

(6) $R_{13}$ und $R_{14}$ H sind und $R_{15}$ H oder $CH_3$ ist;

m) wenn Q Q-6 ist, R H ist, Z CH oder N ist und Y $CH_3$ oder $OCH_3$ ist, dann X von $CH_3$ oder $OCH_3$ verschieden ist, wenn

l)

(1) $R_{13}$ und $R_{15}$ $CH_3$ sind und $R_{14}$ H, Cl oder $OCH_3$ ist;

(2) $R_{13}$ H ist und $R_{14}$ und $R_{15}$ $CH_3$ sind;

n) wenn Q Q-6 ist, R H ist, Z N ist und Y $CH_3$ oder $OCH_3$ ist, dann X von $OCH_3$ verschieden ist, wenn

(1) $R_{13}$ und $R_{14}$ $CH_3$ sind und $R_{15}$ H oder $CH_3$ ist;

(2) $R_{13}$ und $R_{15}$ $CH_3$ sind und $R_{14}$ Br ist;

(3) $R_{13}$ H ist, $R_{14}$ Cl ist und $R_{15}$ $CH_3$ ist;

(4) $R_{13}$ $CH_2CH_3$ oder $CH(CH_3)_2$ ist, $R_{14}$ $OCH_3$ ist und $R_{15}$ $CH_3$ ist;

(5) $R_{13}$ und $R_{14}$ H sind und $R_{15}$ H oder $CH_3$ ist;

o) wenn Q Q-6 ist, R H ist, Z CH ist und Y $CH_3$ oder $OCH_3$ ist, dann X von $CH_3$ verschieden ist, wenn

(1) $R_{13}$ $CH_2CH_3$ ist, $R_{14}$ H oder Cl ist und $R_{15}$ $CH_3$ ist;

(2) $R_{13}$ $CH_3$ ist, $R_{14}$ Cl ist und $R_{15}$ H ist;

(3) $R_{13}$ und $R_{15}$ $CH_3$ sind und $R_{14}$ $OCH_2CH_3$ ist;

(4) $R_{13}$ $CH(CH_3)_2$ ist, $R_{14}$ H ist und $R_{15}$ $CH_3$ ist;

(5) $R_{13}$ und $R_{14}$ $CH_2CH_3$ sind und $R_{15}$ H ist;

(6) $R_{13}$ H ist, $R_{14}$ $CH_2CH_3$ oder $CH(CH_3)_2$ ist und $R_{15}$ $CH_3$ ist;

(7) $R_{13}$ $CH_2CH_3$ oder $CH(CH_3)_2$ ist, $R_{14}$ $OCH_3$ ist und $R_{15}$ H ist;

(8) $R_{13}$ $CH_2CH_2CH_3$, $R_{14}$ $OCH_3$ und $R_{15}$ $CH_3$ ist;

p) wenn Q Q-6 ist, R H ist, Z CH ist und Y $CH_2CH_3$ ist, dann X von $OCH_3$ verschieden ist, wenn $R_{13}$ und $R_{15}$ $CH_3$ sind und $R_{14}$ Cl ist;

q) wenn Q Q-6 ist, R H ist, Z N ist und Y $CH_3$ ist, dann X von $OCH_3$ verschieden ist, wenn

(1) $R_{13}$ $CH_2CH_3$ ist, $R_{14}$ H oder Cl ist und $R_{15}$ $CH_3$ ist;
(2) $R_{13}$ $CH_3$ ist, $R_{14}$ Cl ist und $R_{15}$ H ist;
(3) $R_{13}$ $CH(CH_3)_2$ ist, $R_{14}$ H ist und $R_{15}$ $CH_3$ ist;
(4) $R_{13}$ und $R_{14}$ $CH_2CH_3$ sind und $R_{15}$ H ist;
(5) $R_{13}$ und $R_{15}$ $CH_3$ sind und $R_{14}$ $OCH_2CH_3$ ist;
(6) $R_{13}$ H ist, $R_{14}$ $CH_2CH_3$ oder $CH(CH_3)_2$ ist und $R_{15}$ $CH_3$ ist;
(7) $R_{13}$ $CH_2CH_3$ oder $CH(CH_3)_2$ ist, $R_{14}$ $OCH_3$ ist und $R_{15}$ H ist;
(8) $R_{13}$ $CH_2CH_2CH_3$ ist, $R_{14}$ $OCH_3$ ist und $R_{15}$ $CH_3$ ist;

r) wenn Q Q-6 ist, R H ist, Z N ist und Y $OCH_2CF_3$ ist, dann X von $OCH_3$ verschieden ist, wenn $R_{13}$ und $R_{15}$ $CH_3$ sind und $R_{14}$ $OCH_3$ ist;
s) wenn Q Q-6 ist, R H ist, Z CH ist und Y $CF_3$ oder $OCH_2CF_3$ ist, dann X von $CH_3$ verschieden ist, wenn $R_{13}$ und $R_{15}$ $CH_3$ sind und $R_{14}$ $CO_2CH_3$ ist;
t) wenn Q Q-6 ist, R H ist, Z N ist und Y $OCH_2CF_3$ ist, dann X von $CH_3$ verschieden ist, wenn $R_{13}$ und $R_{15}$ $CH_3$ sind und $R_{14}$ $SO_2CH_2CH_2CH_3$ ist;
u) wenn Q Q-6 ist, R H ist und A A-2 ist, dann $Y_1$ von $CH_3$ verschieden ist, wenn $R_{13}$ und $R_{14}$ H sind und $R_{15}$ $CH_3$ ist;
v) wenn Q Q-6 ist, R H ist, Z N ist und Y $CH_3$ ist, dann X von $CH_3$ verschieden ist, wenn $R_{13}$ und $R_{14}$ H sind und $R_{15}$ $CH_3$ ist;
w) wenn Q Q-6 ist, A A-2 ist, Y $CH_3$, R H ist und $R_{15}$ $CH_3$ ist, dann
(i) wenn $R_{13}$ $CH_3$ ist, $R_{14}$ von Cl, $CO_2CH_3$, $CO_2CH_2CH_3$, $SO_2C_3H_7$-n, $SO_2C_3H_7$-i oder $SO_2N(CH_3)_2$ verschieden ist; und
(ii) wenn $R_{13}$ H ist, $R_{14}$ von H oder $SO_2C_3H_7$-n verschieden ist;
x) wenn Q Q-6 ist, A A-2 ist, $Y^1$ $CH_3$ ist, R H ist, $R_{15}$ $CH_3$ ist und $R_{13}$ $CO_2CH_3$ ist, dann $R_{14}$ von $CH_3$ verschieden ist;
y) wenn Q Q-4 ist, A A-2 ist, $Y^1$ $CH_3$ ist, R H ist, und $R_{10}$ und $R_{12}$ $CH_3$ sind, dann $R_{11}$ von Cl, $NO_2$ oder $SO_2N(CH_3)_2$ verschieden ist; und
z) wenn Q Q-5 ist, R, $R_{11}$ und $R_{12}$ H sind, und A A-2 ist, dann $Y^1$ von $CH_3$ verschieden ist;

und landwirtschaftlich geeignete Salze davon.

**2.** Verbindung nach Anspruch 1,
worin Q Q-1, Q-2, Q-3, Q-4, Q-5, Q-6 ist;
A

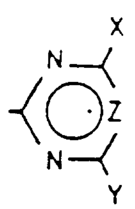
,
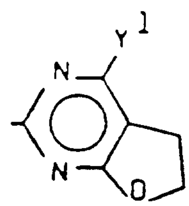
,
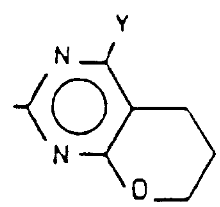

oder
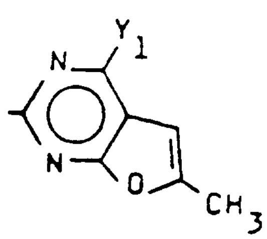

ist;

X $CH_3$, $OCH_3$ oder Cl ist;
Y $CH_3$, $C_2H_5$, $OCH_3$, $OC_2H_5$, $CH_2OCH_3$, $CH(OCH_3)_2$, $NH_2$, $NHCH_3$ oder $N(CH_3)_2$ ist;
Z CH oder N ist;
$Y_1$ H, Cl, $CH_3$ oder $OCH_3$ ist;
$X_2$ $OCH_3$ oder $CH_3$ ist; und
$Y_2$ $CH_3$ ist.

**3.** Verbindung nach Anspruch 1,
worin Q Q-7, Q-8 oder Q-9 ist;

$R_{26}$ H, $C_1$-$C_3$-Alkyl oder $CH_3C(O)NH$ ist;
$R_{27}$ H, $C_1$-$C_3$-Alkyl, $C_1$-$C_3$-Alkoxy, $C_1$-$C_3$-Alkylthio oder $C_1$-$C_3$-Alkoxycarbonyl ist;
$R_{28}$ H, $C_1$-$C_3$-Alkyl, $C_1$-$C_3$-Alkoxy, $C_1$-$C_3$-Alkylthio, $C_1$-$C_3$-Alkylsulfonyl, $C_1$-$C_3$-Alkoxycarbonyl, $NO_2$, Cl, Br oder $CF_3$ ist;
$R_{29}$ H, Cl oder $CH_3$ ist;
$R_{30}$ H, Cl oder $CH_3$ ist;
A

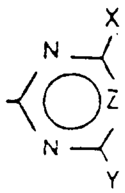

ist;
Y $CH_3$, $OCH_3$, $OCF_2H$, $OC_2H_5$ oder $CH_2OCH_3$ ist;
X $CH_3$, $OCH_3$ oder $OCF_2H$ ist; und
Z CH oder N ist.

**4.** Verbindung nach Anspruch 1,
worin Q Q-10 ist;

$R_{31}$ Cl, $C_1$-$C_3$-Alkoxycarbonyl oder $C_1$-$C_3$-Alkyl ist;
A

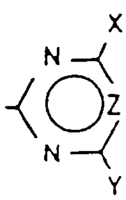

ist;
X $CH_3$, $OCH_3$ oder $OCF_2H$ ist;
Y $CH_3$, $OCH_3$, $OC_2H_5$, $OCF_2H$ oder $CH_2OCH_3$ ist; und
Z CH oder N ist.

**5.** Verbindung nach Anspruch 1, worin R H ist.

**6.** Verbindung nach Anspruch 1 oder 5, worin A A-1 ist und Z CH ist.

**7.** Verbindung nach Anspruch 1, 5 oder 6, worin Q Q-1 oder Q-6 ist.

**8.** Verbindung nach Anspruch 1, worin Q Q-4 ist, R H ist, $R_{10}$ $CH_3$ ist, $R_{12}$ H ist, $R_{11}$ $CO_2R_{24}$ ist, $R_{24}$ $C_1$-$C_3$ Alkyl ist, A A-1 ist, X und Y $OCH_3$ sind und Z CH ist.

**9.** Verbindung nach Anspruch 1, welche N-[(4,6-Dimethylpyrimidin-2-yl)aminocarbonyl]-1-(1-methylethyl)-1H-imidazol-2-sulfonamid;
N-[(4,6-Dimethylpyrimidin-2-yl)aminocarbonyl]-1-ethyl-1H-imidazol-2-sulfonamid;
oder ein landwirtschaftlich geeignetes Salz von beiden ist.

**10.** Verbindung nach Anspruch 1, ausgewählt aus N-[(4-Methoxy-6-methylpyrimidin-2-yl)aminocarbonyl]-1-(1-methylethyl)-1H-imidazol-2-sulfonamid;
N-[(4-Methoxy-6-methylpyrimidin-2-yl)aminocarbonyl]-1-ethyl-1H-imidazol-2-sulfonamid;

N-[(4-Methoxy-6-methylpyrimidin-2-yl)aminocarbonyl]-4-thiazolsulfonamid;
Methyl-4-[(4-methoxy-6-methylpyrimidin-2-yl)aminocarbonyl]aminosulfonyl)-3-isothiazolcarboxylat
oder ein landwirtschaftlich geeignetes Salz von einer davon.

**11.** Verbindung nach Anspruch 1, ausgewählt aus:
N-[(4,6-Dimethoxypyrimidin-2-yl)aminocarbonyl]-1-(1-methylethyl)-1H-imidazol-2-sulfonamid;
N-[(4,6-Dimethoxypyrimidin-2-yl)aminocarbonyl]-1-ethyl-1H-imidazol-2-sulfonamid;
N-[(4,6-Dimethoxypyrimidin-2-yl)aminocarbonyl]-5-brom-1-methyl-1H-imidazol-4-sulfonamid;
N-[(4,6-Dimethoxypyrimidin-2-yl)aminocarbonyl]-4-thiazolsulfonamid;
Methyl-4-[(4,6-Dimethoxypyrimidin-2-yl)aminocarbonyl]aminosulfonyl-3-isothiazolcarboxylat;
oder ein landwirtschaftlich geeignetes Salz von einer davon.

**12.** Landwirtschaftliche Zusammensetzung, umfassend eine herbizid wirksame Menge einer oder mehrerer Verbindungen nach einem der Ansprüche 1 bis 11 und wenigstens eines der folgenden: (a) eines oberflächenaktiven Mittels und (b) eines festen oder flüssigen Verdünnungsmittels.

**13.** Verfahren zur Bekämpfung des Wachstums unerwünschter Vegetation, welches die Anwendung einer landwirtschaftlich wirksamen Menge einer oder mehrerer Verbindungen nach einem der Ansprüche 1 bis 11 auf den Ort solcher Vegetation umfaßt.

**14.** Verfahren zur Wachstumssteuerung von Pflanzen, welches die Anwendung einer wirksamen, jedoch im wesentlichen nicht phytotoxischen Menge eines Pflanzenwachstumsregulans, ausgewählt aus den Verbindungen nach einem der Ansprüche 1 bis 11, auf den Ort solcher Pflanzen umfaßt.

**15.** Verfahren zur Herstellung einer Verbindung nach Anspruch 1 durch entweder
(a) Inberührungbringen eines Aminoheterocyclus der Formel

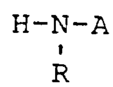

mit
i) einem Sulfonylisocyanat der Formel $QSO_2NCO$ oder mit
ii) einem N-Phenylsulfonylcarbamat der Formel

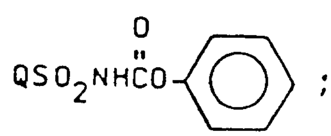

(b) Inberührungbringen eines Sulfonamids der Formel $QSO_2NH_2$ mit
i) einem Methylcarbamat der Formel

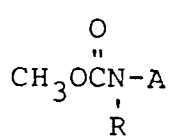

in Gegenwart einer äquimolaren Menge Trimethylaluminium oder mit

ii) einem heterocyclischen Isocyanat der Formel

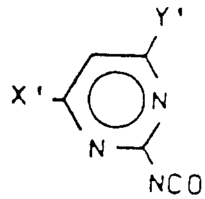

worin X' Cl ist Y' Cl oder Br ist, gegebenenfalls gefolgt von der Reaktion mit einem Nukleophil, wie Methoxid- oder Ethoxidionen; oder mit

iii) einem N-heterocyclischen Carbamat der Formel

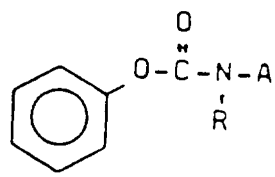

in Gegenwart einer Base.

**Patentansprüche für folgende Vertragsstaaten : LU, SE**

**1.** Verbindung der Formel

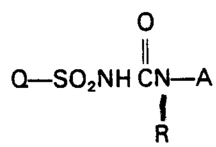

worin R H oder $CH_3$ ist;

Q

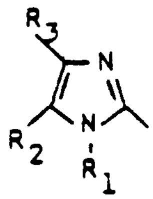 , 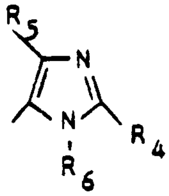 , 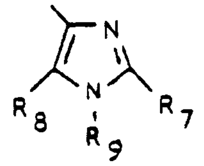 ,

Q-1 Q-2 Q-3

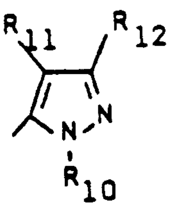 , 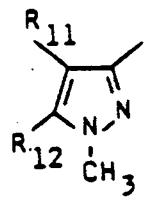 , 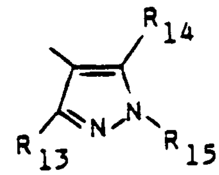 ;

Q-4 Q-5 Q-6

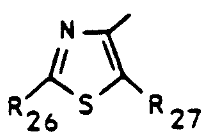 , 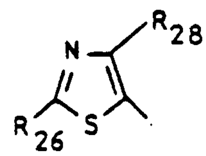 ,

Q-7 Q-8

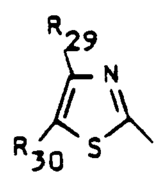 oder 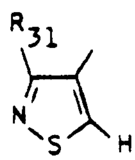

Q-9 Q-10

ist;

$R_1$ H, $C_1$-$C_8$-Alkyl, $C_3$-$C_6$-Alkenyl, $C_5$-$C_6$-Cycloalkyl, $C_5$-$C_6$-Cycloalkenyl, $C_3$-$C_6$-Alkinyl, $C_4$-$C_7$-Cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

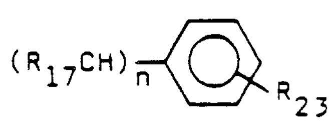

oder $C_1$-$C_6$-Alkyl, substituiert entweder mit

a) 1 bis 3 Atomen aus F, Br oder Cl; oder

b) $OR_{16}$; ist

$R_2$, $R_3$ und $R_4$ unabhängig H oder $CH_3$ sind;

$R_5$ H, $C_1$-$C_4$-Alkyl, $-OR_6$, $NO_2$, F, Cl, Br, $CO_2R_{24}$, $S(O)_mR_{25}$ oder $SO_2NR_{20}R_{21}$ ist;

$R_6$ H, $C_1$-$C_4$-Alkyl, $C_3$-$C_4$-Alkenyl, $C_3$-$C_4$-Alkinyl, $CO_2R_{18}$, $SO_2NR_{20}R_{21}$, $SO_2R_{22}$ oder $C_1$-$C_4$-Alkyl, substituiert mit

a) 1 bis 3 Atomen aus F, Cl oder Br; oder

b) $OCH_3$; ist;

$R_7$ H oder $CH_3$ ist;

$R_8$ H, $C_1$-$C_4$-Alkyl, -$OR_{16}$, $NO_2$, F, Cl, Br, $CO_2R_{24}$, $S(O)_mR_{25}$ oder $SO_2NR_{20}R_{21}$ ist;
$R_9$ $CH_3$ oder $C_2H_5$ ist;
$R_{10}$ H, $C_1$-$C_4$-Alkyl, $C_3$-$C_4$-Alkenyl, $C_3$-$C_4$-Alkinyl, $CO_2R_{24}$, $SO_2NR_{20}R_{21}$ oder $SO_2R_{22}$ ist;
$R_{11}$ H, $C_1$-$C_3$-Alkyl, F, Cl, Br, $NO_2$, -$OR_{16}$, $CO_2R_{24}$, $S(O)_mR_{25}$ oder $SO_2NR_{20}R_{21}$ ist;
$R_{12}$ H oder $CH_3$ ist;
$R_{13}$ und $R_{14}$ unabhängig H, $C_1$-$C_3$-Alkyl, -$OR_{16}$, F, Cl, Br, $NO_2$, $CO_2R_{24}$, $S(O)_mR_{25}$ oder $SO_2NR_{20}R_{21}$ sind;
$R_{15}$ H oder $CH_3$ ist;
$R_{16}$ $C_1$-$C_3$-Alkyl ist;
$R_{17}$ H oder $CH_3$ ist;
$R_{18}$ $C_1$-$C_4$-Alkyl, $C_3$-$C_4$-Alkenyl, $C_3$-$C_4$-Alkinyl, $CH_2CH_2Cl$ oder $CH_2CH_2OCH_3$ ist;
$R_{19}$ $C_1$-$C_4$-Alkyl, $C_3$-$C_4$-Alkenyl, $C_3$-$C_4$-Alkinyl oder $CH_2CH_2OCH_3$ ist;
$R_{20}$ und $R_{21}$ unabhängig $CH_3$ oder $C_2H_5$ sind;
$R_{22}$ $C_1$-$C_3$-Alkyl oder $CF_3$ ist;
$R_{23}$ H, Cl, Br, $CH_3$, F, $CF_3$, $OCH_3$ oder $NO_2$ ist;
$R_{24}$ $C_1$-$C_3$-Alkyl oder Alkyl ist;
$R_{25}$ $C_1$-$C_3$-Alkyl ist;
m 0, 1 oder 2 ist;
n 0 oder 1 ist;
$R_{26}$ H, $C_1$-$C_3$-Alkyl oder $CH_3C(O)NH$ ist;
$R_{27}$ H, $C_1$-$C_3$-Alkyl, $C_1$-$C_3$-Alkoxy, $C_1$-$C_3$-Alkylthio oder $C_1$-$C_3$-Alkoxycarbonyl ist;
$R_{28}$ H, $C_1$-$C_3$-Alkyl, $C_1$-$C_3$-Alkoxy, $C_1$-$C_3$-Alkylthio, $C_1$-$C_3$-Alkoxycarbonyl, $NO_2$, Cl, Br oder $CF_3$ ist;
$R_{29}$ H, Cl oder $CH_3$ ist;
$R_{30}$ H, Cl oder $CH_3$ ist;
$R_{31}$ Cl, $C_1$-$C_3$-Alkoxycarbonyl oder $C_1$-$C_3$-Alkyl ist;
A

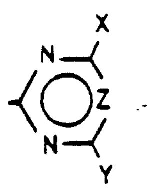
,
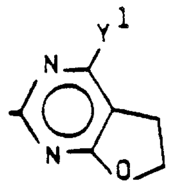
,
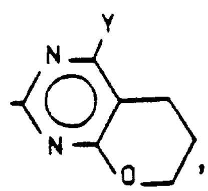
,

A-1 A-2 A-3

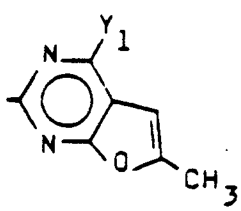
,
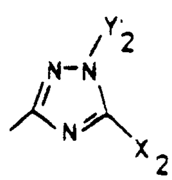
oder
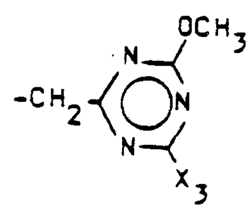

A-4 A-5 A-6

ist;
X $CH_3$, $OCH_3$, Cl, F, $OCF_2H$ oder $SCF_2H$ ist;
Y $CH_3$, $C_2H_5$, $OCH_3$, $OC_2H_5$, $CH_2OCH_5$, $CH(OCH_3)_2$, $OCH_2CF_3$, $OCF_3$, $NH_2$, $NHCH_3$, $N(CH_3)_2$ oder $GCF_2T$ ist, worin G O oder S ist und T H, CHClF, CHBrF, $CF_2H$ oder $CHFCF_3$ ist;
Z CH oder N ist;
$Y_1$ H, Cl, $CH_3$, $OCH_3$ oder $OCF_2H$ ist;
$X_2$ $OCH_3$, $CH_3$, $CH_2CH_3$, $OCH_2CH_3$, $SCH_3$ oder $SCH_2CH_3$ ist;
$Y_2$ $CH_3$, $CH_2CH_3$ oder $CH_2CF_3$ ist;
$X_3$ $OCH_3$ oder $CH_3$ ist;

mit der Maßgabe, daß

a) wenn Q Q-7, Q-8, Q-9 oder Q-10 ist, dann R H ist;
b) die Gesamtzahl der Kohlenstoffatome in $R_1$ weniger als oder gleich 8 ist;
c) falls $R_1$ von $C_1$-$C_3$-Alkyl verschieden ist, dann $R_3$ H ist;
d) wenn $R_5$ von H, $CH_3$, $OCH_3$ oder $NO_2$ verschieden ist, dann $R_6$ H oder $CH_3$ ist;
e) wenn $R_6$ $CO_2R_{18}$, $SO_2NR_{20}R_{21}$ oder $SO_2R_{22}$ ist, dann $R_5$ H, $CH_3$, $OCH_3$ oder $NO_2$ ist;
f) wenn $R_{10}$ von $C_1$-$C_3$-Alkyl verschieden ist, dann $R_{11}$ H, Cl, $OCH_3$, $NO_2$ oder $CH_3$ ist;
g) wenn entweder $R_{13}$ oder $R_{14}$ $CO_2R_{24}$, $S(O)_mR_{25}$ oder $SO_2NR_{20}R_{21}$ ist, dann das andere H, Cl, $CH_3$, $OCH_3$ oder $NO_2$ ist;
h) $R_{29}$ und $R_{30}$ nicht gleichzeitig Cl sind;
i) wenn X Cl oder F ist, dann Z CH ist und Y $OCH_3$, $NH_2$, $NHCH_3$ oder $N(CH_3)_2$ ist;
j) wenn Q Q-7, Q-8, Q-9 oder Q-10 ist, dann A A-1, ist; X $CH_3$, $OCH_3$ oder $OCF_2H$ ist, Y $CH_3$, $OCH_3$, $OC_2H_5$, $OCF_2H$ oder $CH_2OCH_3$ ist; und
k) wenn entweder X oder Y $OCF_2H$ ist, dann Z CH ist;

und landwirtschaftlich geeignete Salze davon.

**2.** Verbindung nach Anspruch 1, worin Q Q-1, Q-2, Q-3, Q-4, Q-5, Q-6 ist;
A

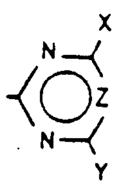,
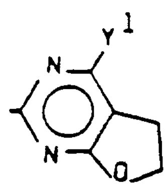,
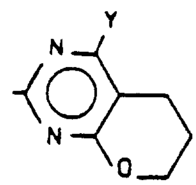
oder
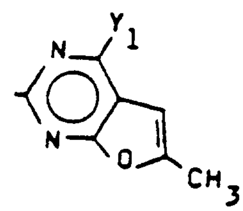

ist;
X $CH_3$, $OCH_3$ oder Cl ist;
Y $CH_3$, $C_2H_5$, $OCH_3$, $OC_2H_5$, $CH_2OCH_3$, $CH(OCH_3)_2$, $NH_2$, $NHCH_3$ oder $N(CH_3)_2$ ist;
Z CH oder N ist;
$Y_1$ H, Cl, $CH_3$ oder $OCH_3$ ist;
$X_2$ $OCH_3$ oder $CH_3$ ist; und
$Y_2$ $CH_3$ ist.

**3.** Verbindung nach Anspruch 1 worin
Q Q-7, Q-8 oder Q-9 ist;
$R_{26}$ H, $C_1$-$C_3$-Alkyl oder $CH_3C(O)NH$ ist;
$R_{27}$ H, $C_1$-$C_3$-Alkyl, $C_1$-$C_3$-Alkoxy, $C_1$-$C_3$-Alkylthio oder $C_1$-$C_3$-Alkoxycarbonyl ist;
$R_{28}$ H, $C_1$-$C_3$-Alkyl, $C_1$-$C_3$-Alkoxy, $C_1$-$C_3$-Alkylthio, $C_1$-$C_3$-Alkylsulfonyl, $C_1$-$C_3$-Alkoxycarbonyl, $NO_2$, Cl, Br oder $CF_3$ ist;
$R_{29}$ H, Cl oder $CH_3$ ist,
$R_{30}$ H, Cl oder $CH_3$ ist
A

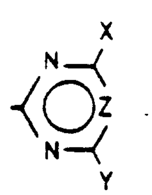

ist;
Y $CH_3$, $OCH_3$, $OCF_2H$, $OC_2H_5$ oder $CH_2OCH_3$ ist;
X $CH_3$, $OCH_3$ oder $OCF_2H$ ist; und
Z CH oder N ist.

**4.** Verbindung nach Anspruch 1, worin Q Q-10 ist;
$R_{31}$ Cl, $C_1$-$C_3$-Alkoxycarbonyl oder $C_1$-$C_3$-Alkyl ist;
A

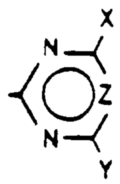

ist;
X $CH_3$, $OCH_3$ oder $OCF_2H$ ist;
Y $CH_3$, $OCH_3$, $OC_2H_5$, $OCF_2H$ oder $CH_2OCH_3$ ist; und
Z CH oder N ist.

**5.** Verbindung nach Anspruch 1, worin R H ist.

**6.** Verbindung nach Anspruch 1 oder 5, worin A A-1 ist und Z CH ist.

**7.** Verbindung nach Anspurch 1, 5 oder 6, worin Q Q-1 oder Q-6 ist.

**8.** Verbindung nach Anspruch 1, worin Q Q-4 ist, R H ist, $R_{10}$ $CH_3$ ist, $R_{12}$ H ist, $R_{11}$ $CO_2R_{24}$ ist, $R_{24}$ $C_1$-$C_3$ Alkyl ist, A A-1 ist, X und Y $OCH_3$ sind und Z CH ist.

**9.** Verbindung nach Anspruch 1, welche
N-[(4,6-Dimethylpyrimidin-2-yl)aminocarbonyl]-1-(1-methylethyl)-1H-imidazol-2-sulfonamid;
N-[(4,6-Dimethylpyrimidin-2-yl)aminocarbonyl]-1-ethyl-1H-imidazol-2-sulfonamid; oder ein landwirtschaftlich geeignetes Salz von beiden ist.

**10.** Verbindung nach Anspruch 1, ausgewählt aus
N-[(4-Methoxy-6-methylpyrimidin-2-yl)aminocarbonyl]-1-(1-methylethyl)-1H-imidazole-2-sulfonamid;
N-[(4-Methoxy-6-methylpyrimidin-2-yl)aminocarbonyl]-1-ethyl-1H-imidazol-4-sulfonamid;
N-[(4-Methoxy-6-methylpyrimidin-2-yl)aminocarbonyl]-2-thiazolsulfonamid;
Methyl-4-[(4-methoxy-6-methylpyrimidin-2-yl)aminocarbonyl]aminosulfonyl)-3-isothiazolcarboxylat
oder ein landwirtschaftlich geeignetes Salz von einer davon.

**11.** Verbindung nach Anspruch 1, ausgewählt aus:
N-[(4,6-Dimethoxypyrimidin-2-yl)aminocarbonyl]-1-(1-methylethyl)-1H-imidazole-2-sulfonamid;
N-[(4,6-Dimethoxypyrimidin-2-yl)aminocarbonyl]-1-ethyl-1H-imidazol-2-sulfonamid;
N-[(4,6-Dimethoxypyrimidin-2-yl)aminocarbonyl]-5-brom-1-methyl-1H-imidazol-4-sulfonamid;
N-[(4,6-Dimethoxypyrimidin-2-yl)aminocarbonyl]-4-thiazolsulfonamid;
Methyl-4-[(4,6-Dimethoxypyrimidin-2-yl)aminocarbonyl]aminosulfonyl-3-isothiazolcarboxylat; oder ein landwirtschaftlich geeignetes Salz von einer davon.

**12.** Landwirtschaftliche Zusammensetzung, umfassend eine herbizid wirksame Menge einer oder mehrerer Verbindungen nach einem der Ansprüche 1 bis 11 und wenigstens eines der folgenden: (a) eines oberflächenaktiven Mittels und (b) eines festen oder flüssigen Verdünnungsmittels.

**13.** Verfahren zur Bekämpfung des Wachstums unerwünschter Vegetation, welches die Anwendung einer landwirtschaftlich wirksamen Menge einer oder mehrerer Verbindungen nach einem der Ansprüche 1 bis 11 auf den Ort solcher Vegetation umfaßt.

**14.** Verfahren zur Wachstumssteuerung von Pflanzen, welches die Anwendung einer wirksamen, jedoch im wesentlichen nicht phytotoxischen Menge eines Pflanzenwachstumsregulans, ausgewählt aus den Verbindungen nach einem der Ansprüche 1 bis 11, auf den Ort solcher Pflanzen umfaßt.

**15.** Verfahren zur Herstellung einer Verbindung nach Anspruch 1 durch entweder

(a) Inberührungbringen eines Aminoheterocyclus der Formel

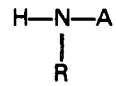

mit

i) einem Sulfonylisocyanat der Formel $QSO_2NCO$ oder mit

ii) einem N-Phenylsulfonylcarbamat der Formel

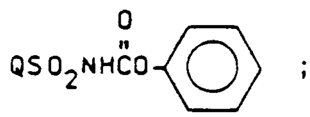

(b) Inberührungbringen eines Sulfonamids der Formel $QSO_2NH_2$ mit

i) einem Methylcarbamat der Formel

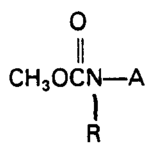

in Gegenwart einer äquimolaren Menge Trimethylaluminium oder mit

ii) einem heterocyclischen Isocyanat der Formel

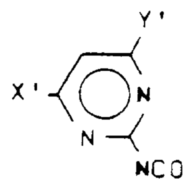

worin X' Cl ist Y' Cl oder Br ist, gegebenenfalls gefolgt von der Reaktion mit einem Nukleophil, wie Methoxid- oder Ethoxidionen; oder mit

iii) einem N-hetereocyclishen Carbamat der Formel

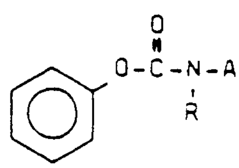

in Gegenwart einer Base.

**Patentansprüche für folgenden Vertragsstaat : AT**

**1.** Verfahren zur Herstellung einer Verbindung der Formel

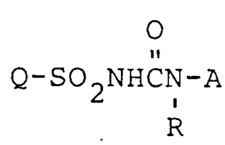

worin R H oder $CH_3$ ist;

Q

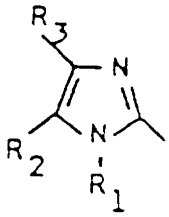 , 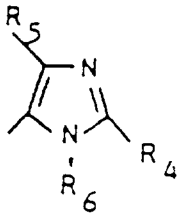 , 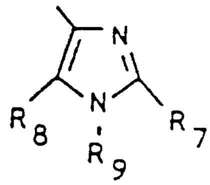 ,

Q-1 Q-2 Q-3

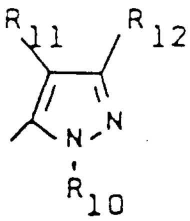 , 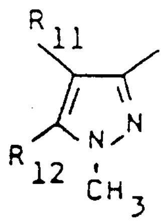 , 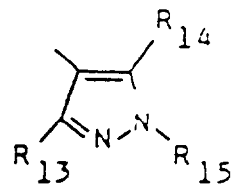 ;

Q-4 Q-5 Q-6

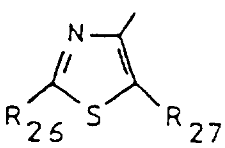 , 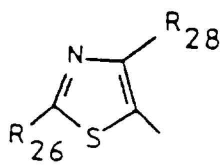 ,

Q-7 Q-8

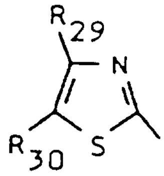 oder 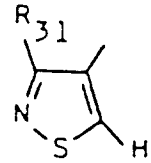

Q-9 Q-10

ist;

$R_1$ H, $C_1$-$C_8$-Alkyl, $C_3$-$C_6$-Alkenyl, $C_5$-$C_6$-Cycloalkyl, $C_5$-$C_6$-Cycloalkenyl, $C_3$-$C_6$-Alkinyl, $C_4$-$C_7$-Cycloalkylalkyl, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

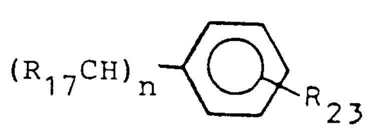

oder $C_1$-$C_6$-Alkyl, substituiert entweder mit

a) 1 bis 3 Atomen aus F, Br oder Cl; oder

b) $OR_{16}$; ist,

$R_2$, $R_3$ und $R_4$ unabhängig H oder $CH_3$ sind;

| | |
|---|---|
| $R_5$ | H, $C_1$-$C_4$-Alkyl, -$OR_6$, $NO_2$, F, Cl, Br, $CO_2R_{24}$,$S(O)_mR_{25}$ oder $SO_2NR_{20}R_{21}$ ist; |
| $R_6$ | H, $C_1$-$C_4$-Alkyl, $C_3$-$C_4$-Alkenyl, $C_3$-$C_4$-Alkinyl, $CO_2R_{18}$, $SO_2NR_{20}R_{21}$, $SO_2R_{22}$ oder $C_1$-$C_4$-Alkyl, substituiert mit<br>a) 1 bis 3 Atomen aus F, Cl oder Br; oder<br>b) $OCH_3$; ist; |
| $R_7$ | H oder $CH_3$ ist; |
| $R_8$ | H, $C_1$-$C_4$-Alkyl, -$OR_{16}$, $NO_2$, F, Cl, Br, $CO_2R_{24}$,$S(O)_mR_{25}$ oder $SO_2NR_{20}R_{21}$ ist; |
| $R_9$ | $CH_3$ oder $C_2H_5$ ist; |
| $R_{10}$ | H, $C_1$-$C_4$-Alkyl, $C_3$-$C_4$-Alkenyl, $C_3$-$C_4$-Alkinyl, $CO_2R_{24}$, $SO_2NR_{20}R_{21}$ oder $SO_2R_{22}$ ist; |
| $R_{11}$ | H, $C_1$-$C_3$-Alkyl, F, Cl, Br, $NO_2$, -$OR_{16}$, $CO_2R_{24}$,$S(O)_mR_{25}$ oder $SO_2NR_{20}R_{21}$ ist; |
| $R_{12}$ | H oder $CH_3$ ist; |
| $R_{13}$ und $R_{14}$ | unabhängig H, $C_1$-$C_3$-Alkyl, -$OR_{16}$, F, Cl, Br, $NO_2$, $CO_2R_{24}$, $S(O)_mR_{25}$ oder $SO_2NR_{20}R_{21}$ sind; |
| $R_{15}$ | H oder $CH_3$ ist; |
| $R_{16}$ | $C_1$-$C_3$-Alkyl ist; |
| $R_{17}$ | H oder $CH_3$ ist; |
| $R_{18}$ | $C_1$-$C_4$-Alkyl, $C_3$-$C_4$-Alkenyl, $C_3$-$C_4$-Alkinyl, $CH_2CH_2Cl$ oder $CH_2CH_2OCH_3$ ist; |
| $R_{19}$ | $C_1$-$C_4$-Alkyl, $C_3$-$C_4$-Alkenyl, $C_3$-$C_4$-Alkinyl oder $CH_2CH_2OCH_3$ ist; |
| $R_{20}$ und $R_{21}$ | unabhängig $CH_3$ oder $C_2H_5$ sind; |
| $R_{22}$ | $C_1$-$C_3$-Alkyl oder $CF_3$ ist; |
| $R_{23}$ | H, Cl, Br, $CH_3$, F, $CF_3$, $OCH_3$ oder $NO_2$ ist; |
| $R_{24}$ | $C_1$-$C_3$-Alkyl oder Allyl ist; |
| $R_{25}$ | $C_1$-$C_3$-Alkyl ist; |
| m | 0, 1 oder 2 ist; |
| n | 0 oder 1 ist; |
| $R_{26}$ | H, $C_1$-$C_3$-Alkyl oder $CH_3C(O)NH$ ist; |
| $R_{27}$ | H, $C_1$-$C_3$-Alkyl, $C_1$-$C_3$-Alkoxy, $C_1$-$C_3$-Alkylthio oder $C_1$-$C_3$-Alkoxycarbonyl ist; |
| $R_{28}$ | H, $C_1$-$C_3$-Alkyl, $C_1$-$C_3$-Alkoxy, $C_1$-$C_3$-Alkylthio, $C_1$-$C_3$-Alkoxycarbonyl, $NO_2$, Cl, Br oder $CF_3$ ist; |
| $R_{29}$ | H, Cl oder $CH_3$ ist; |
| $R_{30}$ | H, Cl oder $CH_3$ ist; |
| $R_{31}$ | Cl, $C_1$-$C_3$-Alkoxycarbonyl oder $C_1$-$C_3$-Alkyl ist; |
| A | |

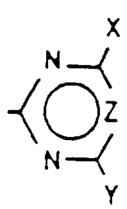

,

A-1

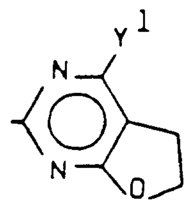

,

A-2

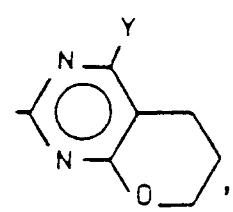

,

A-3

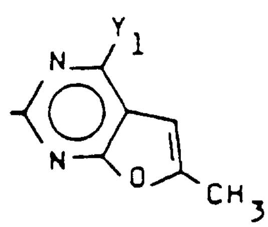

,

A-4

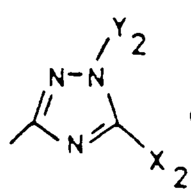

oder

A-5

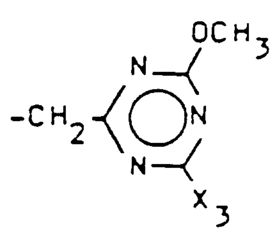

A-6

ist;

X $CH_3$, $OCH_3$, Cl, F, $OCF_2H$ oder $SCF_2H$ ist;
Y $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ oder $GCF_2T$ ist, worin G O oder S ist und T H, CHClF, CHBrF, $CF_2H$ oder $CHFCF_3$ ist;
Z CH oder N ist;
$Y_1$ H, Cl, $CH_3$, $OCH_3$ oder $OCF_2H$ ist;
$X_2$ $OCH_3$, $CH_3$, $CH_2CH_3$, $OCH_2CH_3$, $SCH_3$ oder $SCH_2CH_3$ ist;
$Y_2$ $CH_3$, $CH_2CH_3$ oder $CH_2CF_3$ ist;
$X_3$ $OCH_3$ oder $CH_3$ ist;

mit der Maßgabe, daß

a) wenn Q Q-7, Q-8, Q-9 oder Q-10 ist, dann R H ist;
b) die Gesamtzahl der Kohlenstoffatome in $R_1$ weniger als oder gleich 8 ist;
c) falls $R_1$ von $C_1$-$C_3$-Alkyl verschieden ist, dann $R_3$ H ist;
d) wenn $R_5$ von H, $CH_3$, $OCH_3$ oder $NO_2$ verschieden ist, dann $R_6$ H oder $CH_3$ ist;
e) wenn $R_6$ $CO_2R_{18}$, $SO_2NR_{20}R_{21}$ oder $SO_2R_{22}$ ist, dann $R_5$ H, $CH_3$, $OCH_3$ oder $NO_2$ ist;
f) wenn $R_{10}$ von $C_1$-$C_3$-Alkyl verschieden ist, dann $R_{11}$ H, Cl, $OCH_3$, $NO_2$ oder $CH_3$ ist;
g) wenn entweder $R_{13}$ oder $R_{14}$ $CO_2R_{24}$, $S(O)_mR_{25}$ oder $SO_2NR_{20}R_{21}$ ist, dann das andere H, Cl, $CH_3$, $OCH_3$ oder $NO_2$ ist;
h) $R_{29}$ und $R_{30}$ nicht gleichzeitig Cl sind;
i) wenn X Cl oder F ist, dann Z CH ist und Y $OCH_3$, $NH_2$, $NHCH_3$ oder $N(CH_3)_2$ ist;
j) wenn Q Q-7, Q-8, Q-9 oder Q-10 ist, dann A A-1 ist; X $CH_3$, $OCH_3$ oder $OCF_2H$ ist, Y $CH_3$, $OCH_3$, $OC_2H_5$, $OCF_2H$ oder $CH_2OCH_3$ ist; und
k) wenn entweder X oder Y $OCF_2H$ ist, dann Z CH ist;
  $k_1$) wenn Q Q-2 ist, R H ist, Z CH ist und wenn entweder X oder Y $OCH_3$ ist, dann das andere nicht $CH_3$ ist, wenn $R_4$, $R_5$, $R_6$ H sind;
l) wenn Q Q-6 ist, R H ist, Z CH ist und Y $CH_3$ oder $OCH_3$ ist, dann X von $CH_3$ oder $OCH_3$ verschieden ist, wenn
  (1) $R_{13}$ und $R_{14}$ $CH_3$ sind und $R_{15}$ H oder $CH_3$ ist;
  (2) $R_{13}$ und $R_{15}$ $CH_3$ sind und $R_{14}$ Br ist;
  (3) $R_{13}$ H ist, $R_{14}$ Cl ist und $R_{15}$ $CH_3$ ist;
  (4) $R_{13}$ $CH_2CH_2CH_3$ oder $CH(CH_3)_2$ ist, $R_{14}$ Cl ist und $R_{15}$ $CH_3$ ist;
  (5) $R_{13}$ $CH_2CH_3$ oder $CH(CH_3)_2$ ist, $R_{14}$ $OCH_3$ ist und $R_{15}$ $CH_3$ ist;
  (6) $R_{13}$ und $R_{14}$ H sind und $R_{15}$ H oder $CH_3$ ist;
m) wenn Q Q-6 ist, R H ist, Z CH oder N ist und Y $CH_3$ oder $OCH_3$ ist, dann X von $CH_3$ oder $OCH_3$ verschieden ist, wenn
l)
  (1) $R_{13}$ und $R_{15}$ $CH_3$ sind und $R_{14}$ H, Cl oder $OCH_3$ ist;
  (2) $R_{13}$ H ist und $R_{14}$ und $R_{15}$ $CH_3$ sind;
n) wenn Q Q-6 ist, R H ist, Z N ist und Y $CH_3$ oder $OCH_3$ ist, dann X von $OCH_3$ verschieden ist, wenn
  (1) $R_{13}$ und $R_{14}$ $CH_3$ sind und $R_{15}$ H oder $CH_3$ ist;
  (2) $R_{13}$ und $R_{15}$ $CH_3$ sind und $R_{14}$ Br ist;
  (3) $R_{13}$ H ist, $R_{14}$ Cl ist und $R_{15}$ $CH_3$ ist;
  (4) $R_{13}$ $CH_2CH_3$ oder $CH(CH_3)_2$ ist, $R_{14}$ $OCH_3$ ist und $R_{15}$ $CH_3$ ist;
  (5) $R_{13}$ und $R_{14}$ H sind und $R_{15}$ H oder $CH_3$ ist;
o) wenn Q Q-6 ist, R H ist, Z CH ist und Y $CH_3$ oder $OCH_3$ ist, dann X von $CH_3$ verschieden ist, wenn
  (1) $R_{13}$ $CH_2CH_3$ ist, $R_{14}$ H oder Cl ist und $R_{15}$ $CH_3$ ist;
  (2) $R_{13}$ $CH_3$ ist, $R_{14}$ Cl ist und $R_{15}$ H ist;
  (3) $R_{13}$ und $R_{15}$ $CH_3$ sind und $R_{14}$ $OCH_2CH_3$ ist;
  (4) $R_{13}$ $CH(CH_3)_2$ ist, $R_{14}$ H ist und $R_{15}$ $CH_3$ ist;
  (5) $R_{13}$ und $R_{14}$ $CH_2CH_3$ sind und $R_{15}$ H ist;
  (6) $R_{13}$ H ist, $R_{14}$ $CH_2CH_3$ oder $CH(CH_3)_2$ ist und $R_{15}$ $CH_3$ ist;
  (7) $R_{13}$ $CH_2CH_3$ oder $CH(CH_3)_2$ ist, $R_{14}$ $OCH_3$ ist und $R_{15}$ H ist;
  (8) $R_{13}$ $CH_2CH_2CH_3$, $R_{14}$ $OCH_3$ und $R_{15}$ $CH_3$ ist;
p) wenn Q Q-6 ist, R H ist, Z CH ist und Y $CH_2CH_3$ ist, dann X von $OCH_3$ verschieden ist, wenn $R_{13}$ und $R_{15}$ $CH_3$ sind und $R_{14}$ Cl ist;
q) wenn Q Q-6 ist, R H ist, Z N ist und Y $CH_3$ ist, dann X von $OCH_3$ verschieden ist, wenn

(1) $R_{13}$ $CH_2CH_3$ ist, $R_{14}$ H oder Cl ist und $R_{15}$ $CH_3$ ist;
(2) $R_{13}$ $CH_3$ ist, $R_{14}$ Cl ist und $R_{15}$ H ist;
(3) $R_{13}$ $CH(CH_3)_2$ ist, $R_{14}$ H ist und $R_{15}$ $CH_3$ ist;
(4) $R_{13}$ und $R_{14}$ $CH_2CH_3$ sind und $R_{15}$ H ist;
(5) $R_{13}$ und $R_{15}$ $CH_3$ sind und $R_{14}$ $OCH_2CH_3$ ist;
(6) $R_{13}$ H ist, $R_{14}$ $CH_2CH_3$ oder $CH(CH_3)_2$ ist und $R_{15}$ $CH_3$ ist;
(7) $R_{13}$ $CH_2CH_3$ oder $CH(CH_3)_2$ ist, $R_{14}$ $OCH_3$ ist und $R_{15}$ H ist;
(8) $R_{13}$ $CH_2CH_2CH_3$ ist, $R_{14}$ $OCH_3$ ist und $R_{15}$ $CH_3$ ist;

r) wenn Q Q-6 ist, R H ist, Z N ist und Y $OCH_2CF_3$ ist, dann X von $OCH_3$ verschieden ist, wenn $R_{13}$ und $R_{15}$ $CH_3$ sind und $R_{14}$ $OCH_3$ ist;

s) wenn Q Q-6 ist, R H ist, Z CH ist und Y $CF_3$ oder $OCH_2CF_3$ ist, dann X von $CH_3$ verschieden ist, wenn $R_{13}$ und $R_{15}$ $CH_3$ sind und $R_{14}$ $CO_2CH_3$ ist;

t) wenn Q Q-6 ist, R H ist, Z N ist und Y $OCH_2CF_3$ ist, dann X von $CH_3$ verschieden ist, wenn $R_{13}$ und $R_{15}$ $CH_3$ sind und $R_{14}$ $SO_2CH_2CH_2CH_3$ ist;

u) wenn Q Q-6 ist, R H ist und A A-2 ist, dann $Y_1$ von $CH_3$ verschieden ist, wenn $R_{13}$ und $R_{14}$ H sind und $R_{15}$ $CH_3$ ist;

v) wenn Q Q-6 ist, R H ist, Z N ist und Y $CH_3$ ist, dann X von $CH_3$ verschieden ist, wenn $R_{13}$ und $R_{14}$ H sind und $R_{15}$ $CH_3$ ist;

w) wenn Q Q-6 ist, A A-2 ist, Y $CH_3$, R H ist und $R_{15}$ $CH_3$ ist, dann
(i) wenn $R_{13}$ $CH_3$ ist, $R_{14}$ von Cl, $CO_2CH_3$, $CO_2CH_2CH_3$, $SO_2C_3H_7$-n, $SO_2C_3H_7$-i oder $SO_2N(CH_3)_2$ verschieden ist; und
(ii) wenn $R_{13}$ H ist, $R_{14}$ von H oder $SO_2C_3H_7$-n verschieden ist;

x) wenn Q Q-6 ist, A A-2 ist, $Y^1$ $CH_3$ ist, R H ist, $R_{15}$ $CH_3$ ist und $R_{13}$ $CO_2CH_3$ ist, dann $R_{14}$ von $CH_3$ verschieden ist;

y) wenn Q Q-4 ist, A A-2 ist, $Y^1$ $CH_3$ ist, R H ist, und $R_{10}$ und $R_{12}$ $CH_3$ sind, dann $R_{11}$ von Cl, $NO_2$ oder $SO_2N(CH_3)_2$ verschieden ist; und

z) wenn Q Q-5 ist, R, $R_{11}$ und $R_{12}$ H sind, und A A-2 ist, dann $Y^1$ von $CH_3$ verschieden ist;

und landwirtschaftlich geeigneter Salze davon, durch

(a) Inberührungbringen eines Aminoheterocyclus der Formel

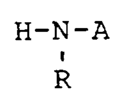

mit
i) einem Sulfonylisocyanat der Formel $QSO_2NCO$, oder mit
ii) einem N-Phenylsulfonylcarbamat der Formel

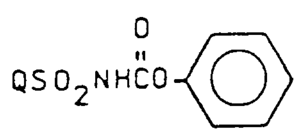

oder

(b) Inberührungbringen eines Sulfonamids der Formel $QSO_2NH_2$ mit
i) einem Methylcarbamat der Formel

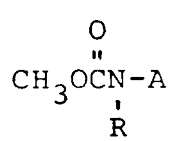

in Gegenwart einer äquimolaren Menge Trimethylaluminium, oder mit

ii) einem heterocyclischen Isocyanat der Formel

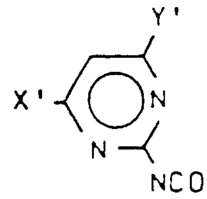

worin X' Cl ist und Y' Cl oder Br ist, gegebenenfalls gefolgt von der Reaktion mit einem Nukleophil, wie Methoxid- oder Ethoxidionen; oder mit

iii) einem N-heterocyclischen Carbamat der Formel

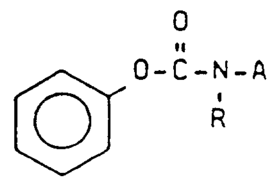

in Gegenwart einer Base.

**2.** Verfahren nach Anspruch 1,
worin Q Q-1, Q-2, Q-3, Q-4, Q-5, Q-6 ist;
A

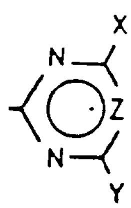 , 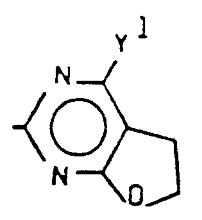 , 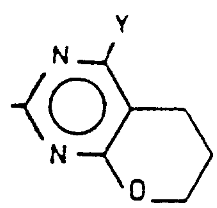

oder 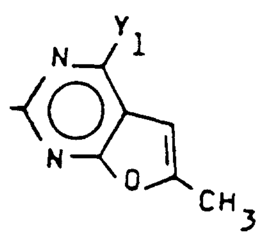

ist;

X $CH_3$, $OCH_3$ oder Cl ist;
Y $CH_3$, $C_2H_5$, $OCH_3$, $OC_2H_5$, $CH_2OCH_3$, $CH(OCH_3)_2$, $NH_2$, $NHCH_3$ oder $N(CH_3)_2$ ist;
Z CH oder N ist;
$Y_1$ H, Cl, $CH_3$ oder $OCH_3$ ist;
$X_2$ $OCH_3$ oder $CH_3$ ist; und
$Y_2$ $CH_3$ ist.

**3.** Verfahren nach Anspruch 1,
worin Q Q-7, Q-8 oder Q-9 ist;
$R_{26}$ H, $C_1$-$C_3$-Alkyl oder $CH_3C(O)NH$ ist;
$R_{27}$ H, $C_1$-$C_3$-Alkyl, $C_1$-$C_3$-Alkoxy, $C_1$-$C_3$-Alkylthio oder $C_1$-$C_3$-Alkoxycarbonyl ist;

$R_{28}$ H, $C_1$-$C_3$-Alkyl, $C_1$-$C_3$-Alkoxy, $C_1$-$C_3$-Alkylthio, $C_1$-$C_3$-Alkylsulfonyl, $C_1$-$C_3$-Alkoxycarbonyl, $NO_2$, Cl, Br oder $CF_3$ ist;
$R_{29}$ H, Cl oder $CH_3$ ist;
$R_{30}$ H, Cl oder $CH_3$ ist;
A

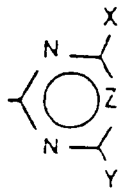

ist;
Y $CH_3$, $OCH_3$, $OCF_2H$, $OC_2H_5$ oder $CH_2OCH_3$ ist;
X $CH_3$, $OCH_3$ oder $OCF_2H$ ist; und
Z CH oder N ist.

**4.** Verfahren nach Anspruch 1,
worin Q Q-10 ist;
$R_{31}$ Cl, $C_1$-$C_3$-Alkoxycarbonyl oder $C_1$-$C_3$-Alkyl ist;
A

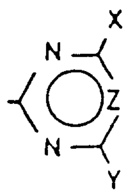

ist;
X $CH_3$, $OCH_3$ oder $OCF_2H$ ist;
Y $CH_3$, $OCH_3$, $OC_2H_5$, $OCF_2H$ oder $CH_2OCH_3$ ist; und
Z CH oder N ist.

**5.** Verfahren nach Anspruch 1, worin R H ist.

**6.** Verfahren nach Anspruch 1 oder 5, worin A A-1 ist und Z CH ist.

**7.** Verfahren nach Anspruch 1, 5 oder 6, worin Q Q-1 oder Q-6 ist.

**8.** Verfahren nach Anspruch 1, worin Q Q-4 ist, R H ist, $R_{10}$ $CH_3$ ist, $R_{12}$ H ist, $R_{11}$ $CO_2R_{24}$ ist, $R_{24}$ $C_1$-$C_3$-Alkyl ist, A A-1 ist, X und Y $OCH_3$ sind und Z CH ist.

**9.** Verfahren nach Anspruch 1, worin das Produkt N-[(4,6-Dimethylpyrimidin-2-yl)aminocarbonyl]-1-(1-methylethyl)-1H-imidazol-2-sulfonamid ist;
N-[(4,6-Dimethylpyrimidin-2-yl)aminocarbonyl]-1-ethyl-1H-imidazol-2-sulfonamid;
oder ein landwirtschaftlich geeignetes Salz von beiden ist.

**10.** Verfahren nach Anspruch 1, worin das Produkt eine Verbindung ist, ausgewählt aus:
N-[(4-Methoxy-6-methylpyrimidin-2-yl)aminocarbonyl]-1-(1-methylethyl)-1H-imidazol-2-sulfonamid;
N-[(4-Methoxy-6-methylpyrimidin-2-yl)aminocarbonyl]-1-ethyl-1H-imidazol-2-sulfonamid;
N-[(4-Methoxy-6-methylpyrimidin-2-yl)aminocarbonyl]-4-thiazolsulfonamid;
Methyl-4-[(4-methoxy-6-methylpyrimidin-2-yl)aminocarbonyl]aminosulfonyl-3-isothiazolcarboxylat;
oder ein landwirtschaftlich geeignetes Salz von einer davon.

**11.** Verfahren nach Anspruch 1, worin das Produkt eine Verbindung ist, ausgewählt aus:
N-[(4,6-Dimethoxypyrimidin-2-yl)aminocarbonyl]-1-(1-methylethyl)-1H-imidazol-2-sulfonamid;
N-[(4,6-Dimethoxypyrimidin-2-yl)aminocarbonyl]-1-ethyl-1H-imidazol-2-sulfonamid;
N-[(4,6-Dimethoxypyrimidin-2-yl)aminocarbonyl]-5-brom-1-methyl-1H-imidazol-4-sulfonamid;
N-[(4,6-Dimethoxypyrimidin-2-yl)aminocarbonyl]-4-thiazolsulfonamid;
Methyl-4-[(4,6-Dimethoxypyrimidin-2-yl)aminocarbonyl]aminosulfonyl-3-isothiazolcarboxylat;
oder ein landwirtschaftlich geeignetes Salz von einer davon.

**12.** Verfahren zur Bekämpfung des Wachstums unerwünschter Vegetation durch Anwendung einer landwirtschaftlich wirksamen Menge einer oder mehrerer Verbindungen der Formel (I), wie in einem der Ansprüche 1 bis 11 definiert, oder eines landwirtschaftlich geeigneten Salzes davon auf den Ort solcher Vegetation.

**13.** Verfahren nach Anspruch 14, worin eine Verbindung, wie in Anspruch 9, 10 oder 11 definiert, verwandt wird.

**Revendications**
**Revendications pour les Etats contractants suivants : CH, DE, FR, GB, IT, LI, NL**

**1.** Un composé de la formule

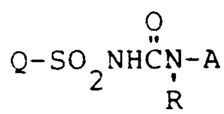

où

R est H ou $CH_3$ ;
Q est

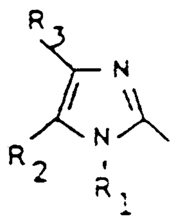 , 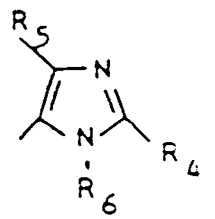 , 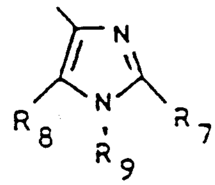 ,

Q-1 Q-2 Q-3

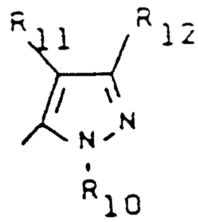 , 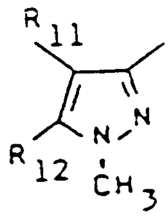 , 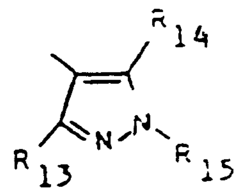 ;

Q-4 Q-5 Q-6

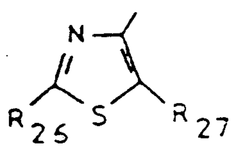 , 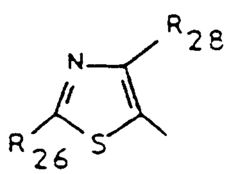 ,

Q-7 Q-8

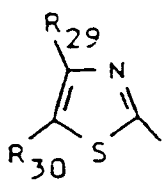 ou 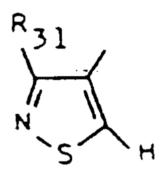 ;

Q-9 Q-10

$R_1$ est H, un groupe alkyle en $C_1$-$C_8$, alcényle en $C_3$-$C_6$, cycloalkyle en $C_5$-$C_6$, cycloalcényle en $C_5$-$C_6$, alcynyle en $C_3$-$C_6$, cycloalkylalkyle en $C_4$-$C_7$, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

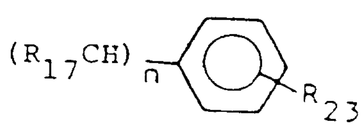 ;

ou alkyle en $C_1$-$C_6$ substitué par

a) 1-3 atomes de F, Br ou Cl ; ou par

b) $OR_{16}$ ;

$R_2$, $R_3$ et $R_4$ sont indépendamment H ou $CH_3$ ;

$R_5$ est H, un groupe alkyle en $C_1$-$C_4$, $-OR_6$, $NO_2$, F, Cl, Br, $CO_2R_{24}$, $S(O)_mR_{25}$ ou $SO_2NR_{20}R_{21}$ ;

$R_6$ est H, un groupe alkyle en $C_1$-$C_4$, alcényle en $C_3$-$C_4$, alcynyle en $C_3$-$C_4$, $CO_2R_{18}$, $SO_2NR_{20}R_{21}$, $SO_2R_{22}$ ou alkyle en $C_1$-$C_4$ substitué par a) 1-3 atomes de F, Cl, ou Br ; ou par b) $OCH_3$

;

$R_7$ est H ou $CH_3$ ;

$R_8$ est H, un groupe alkyle en $C_1$-$C_4$, -$OR_{16}$, $NO_2$, F, Cl, Br, $CO_2R_{24}$, $S(O)_mR_{25}$ ou $SO_2NR_{20}R_{21}$ ;

$R_9$ est $CH_3$ ou $C_2H_5$ ;

$R_{10}$ est H, un groupe alkyle en $C_1$-$C_4$, alcényle en $C_3$-$C_4$, alcynyle en $C_3$-$C_4$, $CO_2R_{24}$, $SO_2NR_{20}R_{21}$ ou $SO_2R_{22}$ ;

$R_{11}$ est H, un groupe alkyle en $C_1$-$C_3$, F, Cl, Br, $NO_2$, -$OR_{16}$, $CO_2R_{24}$, $S(O)_mR_{25}$ ou $SO_2NR_{20}R_{21}$ ;

$R_{12}$ est H ou $CH_3$ ;

$R_{13}$ et $R_{14}$ sont indépendamment H, un groupe alkyle en $C_1$-$C_3$, -$OR_{16}$, F, Cl, Br, $NO_2$, $CO_2R_{24}$, S-$(O)_mR_{25}$ ou $SO_2NR_{20}R_{21}$ ;

$R_{15}$ est H ou $CH_3$ ;

$R_{16}$ est un groupe alkyle en $C_1$-$C_3$ ;

$R_{17}$ est H ou $CH_3$ ;

$R_{18}$ est un groupe alkyle en $C_1$-$C_4$, alcényle en $C_3$-$C_4$, alcynyle en $C_3$-$C_4$, $CH_2CH_2Cl$ ou $CH_2CH_2OCH_3$ ;

$R_{19}$ est un groupe alkyle en $C_1$-$C_4$, alcényle en $C_3$-$C_4$, alcynyle en $C_3$-$C_4$ ou $CH_2CH_2OCH_3$ ;

$R_{20}$ et $R_{21}$ sont indépendamment $CH_3$ ou $C_2H_5$ ;

$R_{22}$ est un groupe alkyle en $C_1$-$C_3$ ou $CF_3$ ;

$R_{23}$ est H, Cl, Br, $CH_3$, F, $CF_3$, $OCH_3$ ou $NO_2$ ;

$R_{24}$ est un groupe alkyle en $C_1$-$C_3$ ou allyle ;

$R_{25}$ est un groupe alkyle en $C_1$-$C_3$ ;

m est 0, 1 ou 2 ;

n est 0 ou 1 ;

$R_{26}$ est H, un groupe alkyle en $C_1$-$C_3$ ou $CH_3C(O)NH$ ;

$R_{27}$ est H, un groupe alkyle en $C_1$-$C_3$, alcoxy en $C_1$-$C_3$, alkylthio en $C_1$-$C_3$ ou (alcoxy en $C_1$-$C_3$)-carbonyle ;

$R_{28}$ est H, un groupe alkyle en $C_1$-$C_3$, alcoxy en $C_1$-$C_3$, alkythio en $C_1$-$C_3$, (alcoxy en $C_1$-$C_3$)-carbonyle, $NO_2$, Cl, Br ou $CF_3$ ;

$R_{29}$ est H, Cl ou $CH_3$ ;

$R_{30}$ est H, Cl ou $CH_3$ ;

$R_{31}$ est Cl, un groupe (alcoxy en $C_1$-$C_3$)carbonyle ou alkyle en $C_1$-$C_3$ ;

A est

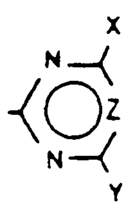 ,

A-1

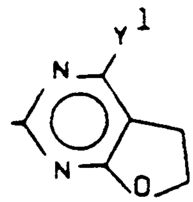 ,

A-2

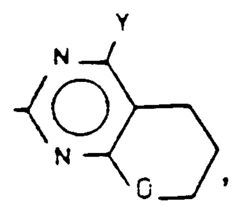 ,

A-3

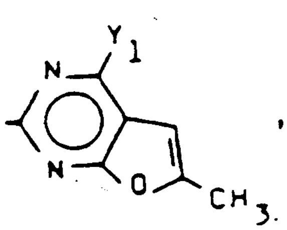 ,

A-4

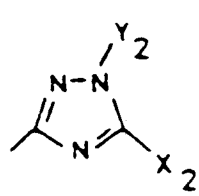 ou

A-5

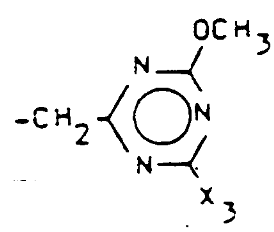 ;

A-6

X est $CH_3$, $OCH_3$, Cl, F, $OCF_2H$ ou $SCF_2H$ ;

Y est $CH_3$, $C_2H_5$, $OCH_3$, $OC_2H_5$, $CH_2OCH_3$,$CH(OCH_3)_2$, $OCH_2CF_3$, $OCF_3$, $NH_2$, $NHCH_3$, $N(CH_3)_2$ ou $GCF_2T$ où G est O ou S et T est H, CHClF, CHBrF, $CF_2H$ ou $CHFCF_3$ ;

Z est CH ou N ;
$Y_1$ est H, Cl, $CH_3$, $OCH_3$ ou $OCF_2H$ ;
$X_2$ est $OCH_3$, $CH_3$, $CH_2CH_3$, $OCH_2CH_3$, $SCH_3$ ou $SCH_2CH_3$ ;
$Y_2$ est $CH_3$, $CH_2CH_3$ ou $CH_2CF_3$ ;
$X_3$ est $OCH_3$ ou $CH_3$ ;

avec les conditions suivantes :

a) si Q est Q-7, Q-8, Q-9 ou Q-10, alors R est H ;
b) le nombre total d'atomes de carbone de $R_1$ est inférieur ou égal à 8 ;
c) si $R_1$ est autre chose qu'un groupe alkyle en $C_1$-$C_3$, alors $R_3$ doit être H ;
d) si $R_5$ est autre chose que H, $CH_3$, $OCH_3$ ou $NO_2$, alors $R_6$ est H ou $CH_3$ ;
e) si $R_6$ est $CO_2R_{18}$, $SO_2NR_{20}R_{21}$ ou $SO_2R_{22}$, alors $R_5$ est H, $CH_3$, $OCH_3$ ou $NO_2$ ;
f) si $R_{10}$ est autre chose qu'un groupe alkyle en $C_1$-$C_3$, alors $R_{11}$ est H, Cl, $OCH_3$, $NO_2$ ou $CH_3$ ;
g) si l'un de $R_{13}$ et $R_{14}$ est $CO_2R_{24}$, $S(O)_mR_{25}$ ou $SO_2NR_{20}R_{21}$, alors l'autre est H, Cl, $CH_3$, $OCH_3$ ou $NO_2$ ;
h) $R_{29}$ et $R_{30}$ ne sont pas simultanément Cl ;
i) si X est Cl ou F, alors Z est CH et Y est $OCH_3$, $NH_2$, $NHCH_3$ ou $N(CH_3)_2$ ;
j) si Q est Q-7, Q-8, Q-9 ou Q-10, alors A est A-1 ; X est $CH_3$, $OCH_3$ ou $OCF_2H$, Y est $CH_3$, $OCH_3$, $OC_2H_5$, $OCF_2H$ ou $CH_2OCH_3$ ; et
k) si l'un ou l'autre de X et Y est $OCF_2H$, alors Z est CH ;
   $k_1$) si Q est Q-2, R est H, Z est CH et si l'un de X et Y est $OCH_3$ alors l'autre n'est pas $-CH_3$ lorsque $R_4$, $R_5$ et $R_6$ sont H;
l) si Q est Q-6, R est H, Z est CH et Y est $CH_3$ ou $OCH_3$, alors X est autre chose que $CH_3$ ou $OCH_3$ si
   (1) $R_{13}$ et $R_{14}$ sont $CH_3$ et $R_{15}$ est H ou $CH_3$ ;
   (2) $R_{13}$ et $R_{15}$ sont $CH_3$ et $R_{14}$ est Br ;
   (3) $R_{13}$ est H, $R_{14}$ est Cl et $R_{15}$ est $CH_3$ ;
   (4) $R_{13}$ est $CH_2CH_2CH_3$ ou $CH(CH_3)_2$, $R_{14}$ est Cl et $R_{15}$ est $CH_3$ ;
   (5) $R_{13}$ est $CH_2CH_3$ ou $CH(CH_3)_2$, $R_{14}$ est $OCH_3$ et $R_{15}$ est $CH_3$ ;
   (6) $R_{13}$ et $R_{14}$ sont H et $R_{15}$ est H ou $CH_3$ ;
m) si Q est Q-6, R est H, Z est CH ou N et Y est $CH_3$ ou $OCH_3$, alors X est autre chose que $CH_3$ ou $OCH_3$ si
   (1) $R_{13}$ et $R_{15}$ sont $CH_3$ et $R_{14}$ est H, Cl ou $OCH_3$;
   (2) $R_{13}$ est H et $R_{14}$ et $R_{15}$ sont $CH_3$ ;
n) si Q est Q-6, R est H, Z est N et Y est $CH_3$ ou $OCH_3$, alors X est autre chose que $OCH_3$ si
   (1) $R_{13}$ et $R_{14}$ sont $CH_3$ et $R_{15}$ est H ou $CH_3$ ;
   (2) $R_{13}$ et $R_{15}$ sont $CH_3$ et $R_{14}$ est Br ;
   (3) $R_{13}$ est H, $R_{14}$ est Cl et $R_{15}$ est $CH_3$ ;
   (4) $R_{13}$ est $CH_2CH_3$ ou $CH(CH_3)_2$, $R_{14}$ est $OCH_3$ et $R_{15}$ est $CH_3$ ;
   (5) $R_{13}$ et $R_{14}$ sont H et $R_{15}$ est H ou $CH_3$ ;
o) si Q est Q-6, R est H, Z est CH et Y est $CH_3$ ou $OCH_3$, alors X est autre chose que $CH_3$ si
   (1) $R_{13}$ est $CH_2CH_3$, $R_{14}$ est H ou Cl et $R_{15}$ est $CH_3$ ;
   (2) $R_{13}$ est $CH_3$, $R_{14}$ est Cl et $R_{15}$ est H ;
   (3) $R_{13}$ et $R_{15}$ sont $CH_3$ et $R_{14}$ est $OCH_2CH_3$ ;
   (4) $R_{13}$ est $CH(CH_3)_2$, $R_{14}$ est H et $R_{15}$ est $CH_3$ ;
   (5) $R_{13}$ et $R_{14}$ sont $CH_2CH_3$ et $R_{15}$ est H ;
   (6) $R_{13}$ est H, $R_{14}$ est $CH_2CH_3$ ou $CH(CH_3)_2$ et $R_{15}$ est $CH_3$ ;
   (7) $R_{13}$ est $CH_2CH_3$ ou $CH(CH_3)_2$, $R_{14}$ est $OCH_3$ et $R_{15}$ est H ;
   (8) $R_{13}$ est $CH_2CH_2CH_3$, $R_{14}$ est $OCH_3$ et $R_{15}$ est $CH_3$ ;
p) si Q est Q-6, R est H, Z est CH et Y est $CH_2CH_3$ ; alors X est autre chose que $OCH_3$ si $R_{13}$ et $R_{15}$ sont $CH_3$ et $R_{14}$ est Cl ;
q) si Q est Q-6, R est H, Z est N et Y est $CH_3$, alors X est autre chose que $OCH_3$ si
   (1) $R_{13}$ est $CH_2CH_3$, $R_{14}$ est H ou Cl et $R_{15}$ est $CH_3$ ;
   (2) $R_{13}$ est $CH_3$ , $R_{14}$ est Cl et $R_{15}$ est H ;
   (3) $R_{13}$ est $CH(CH_3)_2$, $R_{14}$ est H et $R_{15}$ est $CH_3$ ;
   (4) $R_{13}$ et $R_{14}$ sont $CH_2CH_3$ et $R_{15}$ est H ;
   (5) $R_{13}$ et $R_{15}$ sont $CH_3$ et $R_{14}$ est $OCH_2CH_3$ ;
   (6) $R_{13}$ est H, $R_{14}$ est $CH_2CH_3$ ou $CH(CH_3)_2$ et $R_{15}$ est $CH_3$;
   (7) $R_{13}$ est $CH_2CH_3$ ou $CH(CH_3)_2$, $R_{14}$ est $OCH_3$ et $R_{15}$ est H ;

(8) $R_{13}$ est $CH_2CH_2CH_3$, $R_{14}$ est $OCH_3$ et $R_{15}$ est $CH_3$ ;

r) si Q est Q-6, R est H, Z est N et Y est $OCH_2CF_3$ alors X est autre chose que $OCH_3$ si $R_{13}$ et $R_{15}$ sont $CH_3$ et $R_{14}$ est $OCH_3$ ;

s) si Q est Q-6, R est H, Z est CH et Y est $CF_3$ ou $OCH_2CF_3$, alors X est autre chose que $CH_3$ si $R_{13}$ et $R_{15}$ sont $CH_3$ et $R_{14}$ est $CO_2CH_3$ ;

t) si Q est Q-6, R est H, Z est N et Y est $OCH_2CF_3$, alors X est autre chose que $CH_3$ si $R_{13}$ et $R_{15}$ sont $CH_3$ et $R_{14}$ est $SO_2CH_2CH_2CH_3$

u) si Q est Q-6, R est H et A est A-2, alors $Y_1$ est autre chose que $CH_3$ si $R_{13}$ et $R_{14}$ sont H et $R_{15}$ est $CH_3$ ;

v) si Q est Q-6, R est H, Z est N et Y est $CH_3$, alors X est autre chose que $CH_3$ si $R_{13}$ et $R_{14}$ sont H et $R_{15}$ est $CH_3$ ;

w) si Q est Q-3, R est H, Z est N et Y est $OCH_3$ alors X est autre chose que $CH_3$ si $R_7$ et $R_8$ sont H et $R_9$ est $CH_3$

x) si Q est Q-10, R est H, Z est N et Y est $OCH_3$ alors X est autre chose que $OCH_3$ si $R_{31}$ est $CH_3$ ;

y) si Q est Q-9, R est H, Z est CH et Y est $OCH_3$, alors X est autre chose que $CH_3$ si $R_{29}$ est $CH_3$ et $R_{30}$ est H ;

z) si Q est Q-1, R est H, Z est N et Y est $OCH_3$ alors X est autre chose que $CH_3$ si $R_1$ est $CH_3$ et $R_2$ et $R_3$ sont H ;

$a_1$) si Q est Q-1, R est H, Z est CH et Y est $CH_3$ ou $OCH_3$, alors X est autre chose que $CH_3$ ou $OCH_3$ si $R_1$ est $CH_3$ et $R_2$ et $R_3$ sont H ;

$b_1$) si Q est Q-1, R est H, Z est CH et Y est $OCHF_2$, alors X est autre chose que $CH_3$ si $R_1$ est $CH_3$ et $R_2$ et $R_3$ sont H ;

$c_1$) si Q est Q-4, R est H, Z est CH et Y est $CH_2CH_3$, alors X est autre chose que $CH_3$ si $R_{10}$, $R_{11}$ et $R_{12}$ sont H ;

$d_1$) si Q est Q-1, R est $CH_3$, Z est N et Y est $OCH_3$, alors X est autre chose que $CH_3$ si $R_1$, $R_2$ et $R_3$ sont H ;

$e_1$) si Q est Q-9, R est H, Z est N et Y est $CH_3$, alors X est autre chose que $CH_3$ si $R_{29}$ et $R_{30}$ sont H ;

$f_1$) si Q est Q-6, A est A-2, $Y^1$ est $CH_3$, R est H, $R_{15}$ et $R_{13}$ sont $CH_3$, alors $R_{14}$ est autre chose que Cl, $CO_2CH_3$, $CO_2CH_2CH_3$, $SO_2C_3H_7$-n, $SO_2C_3H_7$-i ou $SO_2N(CH_3)_2$ ;

$g_1$) si Q est Q-6, A est A-2, $Y^1$ est $CH_3$, R est H, $R_{15}$ est $CH_3$ et $R_{13}$ est H, alors $R_{14}$ est autre chose que H ou $SO_2C_3H_7$-n ;

$h_1$) si Q est Q-6, A est A-2, $Y^1$ est $CH_3$, R est H, $R_{15}$ est $CH_3$ et $R_{13}$ est $CO_2CH_3$, alors $R_{14}$ est autre chose que $CH_3$ ;

$i_1$) si Q est Q-4, A est A-2, $Y^1$ est $CH_3$, R est H et $R_{10}$ et $R_{12}$ sont $CH_3$, alors $R_{11}$ est autre chose que Cl, $NO_2$ ou $SO_2N(CH_3)_2$ ; et

$j_1$) si Q est Q-5, R, $R_{11}$ et $R_{12}$ sont H, et A est A-2, alors $Y^1$ est autre chose que $CH_3$ ; et ses sels convenant pour l'agriculture.

**2.** Un composé selon la revendication 1 où Q est Q-1, Q-2, Q-3, Q-4, Q-5 ou Q-6 ;
A est

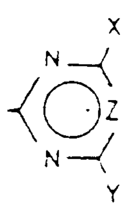 , 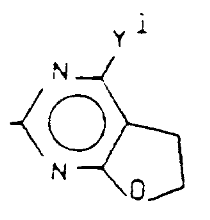 , 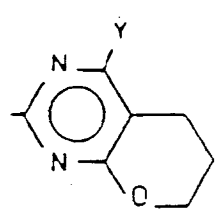

ou 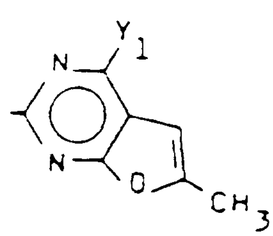 ;

X est $CH_3$, $OCH_3$ ou Cl ;
Y est $CH_3$, $C_2H_5$, $OCH_3$, $OC_2H_5$, $CH_2OCH_3$, $CH(OCH_3)_2$, $NH_2$, $NHCH_3$ ou $N(CH_3)_2$ ;
Z est CH ou N ;
$Y_1$ est H, Cl, $CH_3$ ou $OCH_3$ ;
$X_2$ est $OCH_3$ ou $CH_3$ ; et
$Y_2$ est $CH_3$.

**3.** Un composé selon la revendication 1 où
Q est Q-7, Q-8 ou Q-9 ;
$R_{26}$ est H, un groupe alkyle en $C_1$-$C_3$ ou $CH_3C(O)NH$ ;
$R_{27}$ est H, un groupe alkyle en $C_1$-$C_3$, alcoxy en $C_1$-$C_3$, alkylthio en $C_1$-$C_3$, ou (alcoxy en $C_1$-$C_3$)-carbonyle ;
$R_{28}$ est H, un groupe alkyle en $C_1$-$C_3$, alcoxy en $C_1$-$C_3$, alkylthio en $C_1$-$C_3$, (alkyle en $C_1$-$C_3$)-sulfonyle, (alcoxy en $C_1$-$C_3$)carbonyle, $NO_2$, Cl, Br ou $CF_3$ ;
$R_{29}$ est H, Cl ou $CH_3$ ;
$R_{30}$ est H, Cl ou $CH_3$ ;
A est

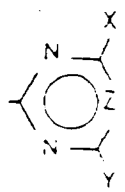 ;

Y est $CH_3$, $OCH_3$, $OCF_2H$, $OC_2H_5$ ou $CH_2OCH_3$ ;
X est $CH_3$, $OCH_3$ ou $OCF_2H$ ; et
Z est CH ou N.

**4.** Un composé selon la revendication 1 où
Q est Q-10 ;
$R_{31}$ est Cl, un groupe (alcoxy en $C_1$-$C_3$)carbonyle ou alkyle en $C_1$-$C_3$ ;
A est

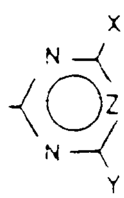 ;

X est $CH_3$, $OCH_3$ ou $OCF_2H$ ;
Y est $CH_3$, $OCH_3$, $OC_2H_5$, $OCF_2H$ ou $CH_2OCH_3$; et
Z est CH ou N.

**5.** Un composé selon la revendication 1 où R est H.

**6.** Un composé selon la revendication 1 ou 5 où A est A-1 et Z est CH.

**7.** Un composé selon la revendication 1, 5 et 6 où Q est Q-1 ou Q-6.

**8.** Un composé selon la revendication 1 où Q est Q-4, R est H, $R_{10}$ est $CH_3$, $R_{12}$ est H, $R_{11}$ est $CO_2R_{24}$, $R_{24}$ est un groupe alkyle en $C_1$-$C_3$, A est A-1, X et Y sont $OCH_3$ et Z est CH.

**9.** Un composé selon la revendication 1 qui est :
le N-[(4,6-diméthylpyrimidine-2-yl)aminocarbonyl]-1-(1-méthyléthyl)-1H-imidazole-2-sulfonamide ;

le N-[(4,6-diméthylpyrimidine-2-yl)aminocarbonyl]-1-éthyl-1H-imidazole-2-sulfonamide ; ou un sel convenant pour l'agriculture de l'un ou l'autre de ceux-ci.

**10.** Un composé selon la revendication 1 qui est choisi parmi
le N-[(4-méthoxy-6-méthylpyrimidine-2-yl)aminocarbonyl]-1-(1-méthyléthyl)-1H-imidazole-2-sulfonamide ;
le N-[(4-méthoxy-6-méthylpyrimidine-2-yl)aminocarbonyl]-1-éthyl-1H-imidazole-2-sulfonamide ;
le N-[(4-méthoxy-6-méthylpyrimidine-2-yl)aminocarbonyl]-4-thiazolesulfonamide ;
le 4-[[(4-méthoxy-6-méthylpyrimidine-2-yl)aminocarbonyl]aminosulfonyl]-3-isothiazolecarboxylate de méthyle ; ou un sel convenant pour l'agriculture de l'un quelconque de ceux-ci.

**11.** Un composé selon la revendication 1 qui est choisi parmi
le N-[(4,6diméthoxypyrimidine-2-yl)aminocarbonyl]-1-(1-méthyléthyl)-1H-imidazole-2-sulfonamide ;
le N-[(4,6-diméthoxypyrimidine-2-yl)aminocarbonyl]-1-éthyl-1H-imidazole-2-sulfonamide ;
le N-[(4,6-diméthoxypyrimidine-2-yl)aminocarbonyl]-5-bromo-1-méthyl-1H-imidazole-4-sulfonamide ;
le N-[(4,6-diméthoxypyrimidine-2-yl)aminocarbonyl]-4-thiazolesulfonamide ;
le 4-[[(4,6-diméthoxypyrimidine-2-yl)aminocarbonyl]aminosulfonyl]-3-isothiazolecarboxylate de méthyle ;
ou un sel convenant pour l'agriculture de l'un quelconque de ceux-ci.

**12.** Une composition pour l'agriculture comprenant une quantité à effet herbicide d'un ou plusieurs des composés de l'une quelconque des revendications 1-11 et au moins l'un des ingrédients suivant : (a) un agent tensio-actif et (b) un diluant solide ou liquide.

**13.** Un procédé pour combattre la croissance d'une végétation indésirable consistant à appliquer au lieu où se trouve cette végétation une quantité efficace pour l'agriculture d'un ou plusieurs composés de l'une quelconque des revendications 1-11.

**14.** Un procédé pour réguler la croissance de plantes qui consiste à appliquer au lieu où se trouvent ces plantes une quantité efficace mais sensiblement non phytotoxique d'un régulateur de la croissance des plantes choisi parmi les composés de l'une quelconque des revendications 1 à 11.

**15.** Un procédé pour préparer un composé de la revendication 1, caractérisé en ce qu'il consiste soit
(a) à mettre en contact un aminohétérocycle de formule

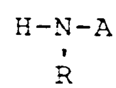

avec
i) un isocyanate de sulfonyle de formule $QSO_2NCO$, ou avec
ii) un N-sulfonylcarbamate de phényle de formule

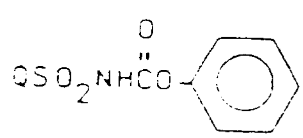 ;

soit
(b) à mettre en contact un sulfonamide de formule $QSO_2NH_2$ avec

i) un carbamate de méthyle de formule

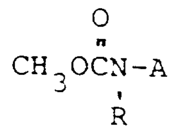

en présence d'une quantité équimolaire de triméthylaluminium, ou avec

ii) un isocyanate hétérocyclique de formule

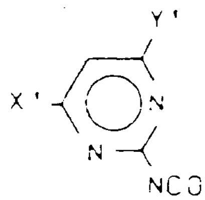

où X' est Cl et Y' est Cl ou Br, ceci étant éventuellement suivi d'une réaction avec une espèce nucléophile telle qu'un ion méthylate ou éthylate ; ou avec

iii) un carbamate N-hétérocyclique de formule

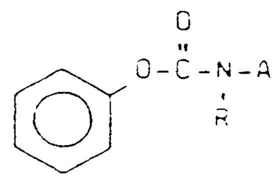

en présence d'une base.

**Revendications pour l'Etat contractant suivant : BE**

**1.** Un composé de la formule

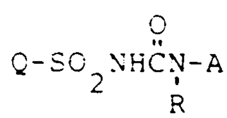

où

R est H ou $CH_3$ ;

Q est

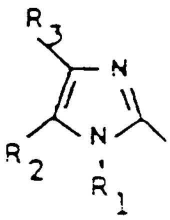 ,
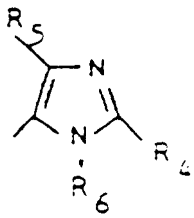 ,
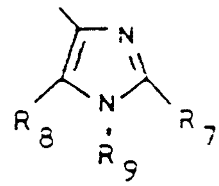 ,

Q-1 Q-2 Q-3

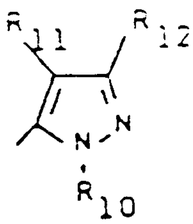 ,
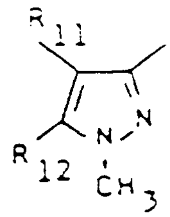 ,
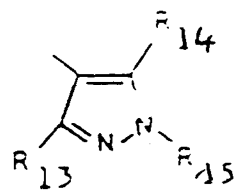 ;

Q-4 Q-5 Q-6

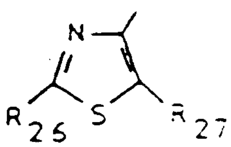 ,
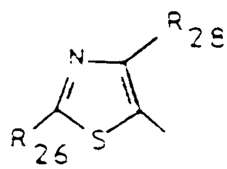 ,

Q-7 Q-8

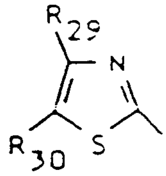 ou 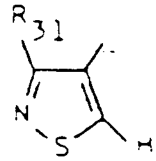 ;

Q-9 Q-10

$R_1$ est H, un groupe alkyle en $C_1$-$C_8$, alcényle en $C_3$-$C_6$, cycloalkyle en $C_5$-$C_6$, cycloalcényle en $C_5$-$C_6$, alcynyle en $C_3$-$C_6$, cycloalkylalkyle en $C_4$-$C_7$, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

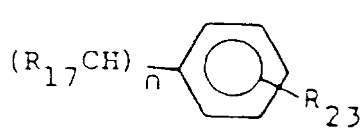 ;

ou alkyle en $C_1$-$C_6$ substitué par

a) 1-3 atomes de F, Br ou Cl ; ou par

b) $OR_{16}$ ;

$R_2$, $R_3$ et $R_4$ sont indépendamment H ou $CH_3$ ;

$R_5$ est H, un groupe alkyle en $C_1$-$C_4$, $-OR_6$, $NO_2$, F, Cl, Br, $CO_2R_{24}$, $S(O)_mR_{25}$ ou $SO_2NR_{20}R_{21}$ ;

$R_6$ est H, un groupe alkyle en $C_1$-$C_4$, alcényle en $C_3$-$C_4$, alcynyle en $C_3$-$C_4$, $CO_2R_{18}$, $SO_2NR_{20}R_{21}$, $SO_2R_{22}$ ou alkyle en $C_1$-$C_4$ substitué par a) 1-3 atomes de F, Cl, ou Br ; ou par b) $OCH_3$ ;

$R_7$ est H ou $CH_3$ ;

$R_8$ est H, un groupe alkyle en $C_1$-$C_4$, -$OR_{16}$, $NO_2$, F, Cl, Br, $CO_2R_{24}$, $S(O)_mR_{25}$ ou $SO_2NR_{20}R_{21}$ ;

$R_9$ est $CH_3$ ou $C_2H_5$ ;

$R_{10}$ est H, un groupe alkyle en $C_1$-$C_4$, alcényle en $C_3$-$C_4$, alcynyle en $C_3$-$C_4$, $CO_2R_{24}$, $SO_2NR_{20}R_{21}$ ou $SO_2R_{22}$ ;

$R_{11}$ est H, un groupe alkyle en $C_1$-$C_3$, F, Cl, Br, $NO_2$, -$OR_{16}$, $CO_2R_{24}$, $S(O)_mR_{25}$ ou $SO_2NR_{20}R_{21}$ ;

$R_{12}$ est H ou $CH_3$ ;

$R_{13}$ et $R_{14}$ sont indépendamment H, un groupe alkyle en $C_1$-$C_3$, -$OR_{16}$, F, Cl, Br, $NO_2$, $CO_2R_{24}$, S-$(O)_mR_{25}$ ou $SO_2NR_{20}R_{21}$ ;

$R_{15}$ est H ou $CH_3$ ;

$R_{16}$ est un groupe alkyle en $C_1$-$C_3$ ;

$R_{17}$ est H ou $CH_3$ ;

$R_{18}$ est un groupe alkyle en $C_1$-$C_4$, alcényle en $C_3$-$C_4$, alcynyle en $C_3$-$C_4$, $CH_2CH_2Cl$ ou $CH_2CH_2OCH_3$ ;

$R_{19}$ est un groupe alkyle en $C_1$-$C_4$, alcényle en $C_3$-$C_4$, alcynyle en $C_3$-$C_4$ ou $CH_2CH_2OCH_3$ ;

$R_{20}$ et $R_{21}$ sont indépendamment $CH_3$ ou $C_2H_5$ ;

$R_{22}$ est un groupe alkyle en $C_1$-$C_3$ ou $CF_3$ ;

$R_{23}$ est H, Cl, Br, $CH_3$, F, $CF_3$, $OCH_3$ ou $NO_2$ ;

$R_{24}$ est un groupe alkyle en $C_1$-$C_3$ ou allyle ;

$R_{25}$ est un groupe alkyle en $C_1$-$C_3$ ;

m est 0, 1 ou 2 ;

n est 0 ou 1 ;

$R_{26}$ est H, un groupe alkyle en $C_1$-$C_3$ ou $CH_3C(O)NH$ ;

$R_{27}$ est H, un groupe alkyle en $C_1$-$C_3$, alcoxy en $C_1$-$C_3$, alkylthio en $C_1$-$C_3$ ou (alcoxy en $C_1$-$C_3$)-carbonyle ;

$R_{28}$ est H, un groupe alkyle en $C_1$-$C_3$, alcoxy en $C_1$-$C_3$, alkythio en $C_1$-$C_3$, (alcoxy en $C_1$-$C_3$)-carbonyle, $NO_2$, Cl, Br ou $CF_3$ ;

$R_{29}$ est H, Cl ou $CH_3$ ;

$R_{30}$ est H, Cl ou $CH_3$ ;

$R_{31}$ est Cl, un groupe (alcoxy en $C_1$-$C_3$)carbonyle ou alkyle en $C_1$-$C_3$ ;

A est

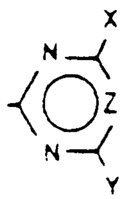
,
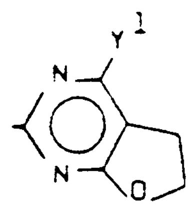
,
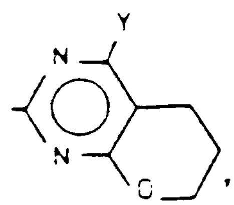
,

A-1 A-2 A-3

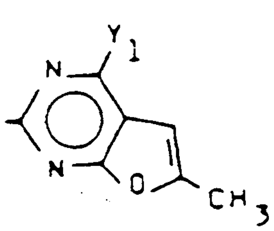
,
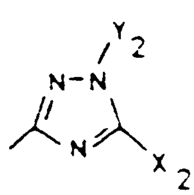
ou
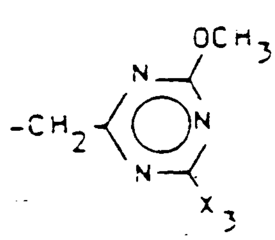
;

A-4 A-5 A-6

X est $CH_3$, $OCH_3$, Cl, F, $OCF_2H$ ou $SCF_2H$ ;

Y est $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ ou $GCF_2T$ où G est O ou S et T est H, CHClF, CHBrF, $CF_2H$ ou $CHFCF_3$ ;

Z est CH ou N ;

$Y_1$ est H, Cl, $CH_3$, $OCH_3$ ou $OCF_2H$ ;
$X_2$ est $OCH_3$, $CH_3$, $CH_2CH_3$, $OCH_2CH_3$, $SCH_3$ ou $SCH_2CH_3$ ;
$Y_2$ est $CH_3$, $CH_2CH_3$ ou $CH_2CF_3$ ;
$X_3$ est $OCH_3$ ou $CH_3$ ;

avec les conditions suivantes :

a) si Q est Q-7, Q-8, Q-9 ou Q-10, alors R est H ;
b) le nombre total d'atomes de carbone de $R_1$ est inférieur ou égal à 8 ;
c) si $R_1$ est autre chose qu'un groupe alkyle en $C_1$-$C_3$, alors $R_3$ doit être H ;
d) si $R_5$ est autre chose que H, $CH_3$, $OCH_3$ ou $NO_2$, alors $R_6$ est H ou $CH_3$ ;
e) si $R_6$ est $CO_2R_{18}$, $SO_2NR_{20}R_{21}$ ou $SO_2R_{22}$, alors $R_5$ est H, $CH_3$, $OCH_3$ ou $NO_2$ ;
f) si $R_{10}$ est autre chose qu'un groupe alkyle en $C_1$-$C_3$, alors $R_{11}$ est H, Cl, $OCH_3$, $NO_2$ ou $CH_3$ ;
g) si l'un de $R_{13}$ et $R_{14}$ est $CO_2R_{24}$, $S(O)_mR_{25}$ ou $SO_2NR_{20}R_{21}$, alors l'autre est H, Cl, $CH_3$, $OCH_3$ ou $NO_2$ ;
h) $R_{29}$ et $R_{30}$ ne sont pas simultanément Cl ;
i) si X est Cl ou F, alors Z est CH et Y est $OCH_3$, $NH_2$, $NHCH_3$ ou $N(CH_3)_2$ ;
j) si Q est Q-7, Q-8, Q-9 ou Q-10, alors A est A-1 ; X est $CH_3$, $OCH_3$ ou $OCF_2H$, Y est $CH_3$, $OCH_3$, $OC_2H_5$, $OCF_2H$ ou $CH_2OCH_3$ ; et
k) si l'un ou l'autre de X et Y est $OCF_2H$, alors Z est CH ;
  $k_1$) si Q est Q-2, R est H, Z est CH et si l'un de X et Y est $OCH_3$ alors l'autre n'est pas -$CH_3$ lorsque $R_4$, $R_5$ et $R_6$ sont H;
l) si Q est Q-6, R est H, Z est CH et Y est $CH_3$ ou $OCH_3$, alors X est autre chose que $CH_3$ ou $OCH_3$ si
  (1) $R_{13}$ et $R_{14}$ sont $CH_3$ et $R_{15}$ est H ou $CH_3$ ;
  (2) $R_{13}$ et $R_{15}$ sont $CH_3$ et $R_{14}$ est Br ;
  (3) $R_{13}$ est H, $R_{14}$ est Cl et $R_{15}$ est $CH_3$ ;
  (4) $R_{13}$ est $CH_2CH_2CH_3$ ou $CH(CH_3)_2$, $R_{14}$ est Cl et $R_{15}$ est $CH_3$ ;
  (5) $R_{13}$ est $CH_2CH_3$ ou $CH(CH_3)_2$, $R_{14}$ est $OCH_3$ et $R_{15}$ est $CH_3$ ;
  (6) $R_{13}$ et $R_{14}$ sont H et $R_{15}$ est H ou $CH_3$ ;
m) si Q est Q-6, R est H, Z est CH ou N et Y est $CH_3$ ou $OCH_3$, alors X est autre chose que $CH_3$ ou $OCH_3$ si
  (1) $R_{13}$ et $R_{15}$ sont $CH_3$ et $R_{14}$ est H, Cl ou $OCH_3$;
  (2) $R_{13}$ est H et $R_{14}$ et $R_{15}$ sont $CH_3$ ;
n) si Q est Q-6, R est H, Z est N et Y est $CH_3$ ou $OCH_3$, alors X est autre chose que $OCH_3$ si
  (1) $R_{13}$ et $R_{14}$ sont $CH_3$ et $R_{15}$ est H ou $CH_3$ ;
  (2) $R_{13}$ et $R_{15}$ sont $CH_3$ et $R_{14}$ est Br ;
  (3) $R_{13}$ est H, $R_{14}$ est Cl et $R_{15}$ est $CH_3$ ;
  (4) $R_{13}$ est $CH_2CH_3$ ou $CH(CH_3)_2$, $R_{14}$ est $OCH_3$ et $R_{15}$ est $CH_3$ ;
  (5) $R_{13}$ et $R_{14}$ sont H et $R_{15}$ est H ou $CH_3$ ;
o) si Q est Q-6, R est H, Z est CH et Y est $CH_3$ ou $OCH_3$, alors X est autre chose que $CH_3$ si
  (1) $R_{13}$ est $CH_2CH_3$, $R_{14}$ est H ou Cl et $R_{15}$ est $CH_3$ ;
  (2) $R_{13}$ est $CH_3$, $R_{14}$ est Cl et $R_{15}$ est H ;
  (3) $R_{13}$ et $R_{15}$ sont $CH_3$ et $R_{14}$ est $OCH_2CH_3$ ;
  (4) $R_{13}$ est $CH(CH_3)_2$, $R_{14}$ est H et $R_{15}$ est $CH_3$ ;
  (5) $R_{13}$ et $R_{14}$ sont $CH_2CH_3$ et $R_{15}$ est H ;
  (6) $R_{13}$ est H, $R_{14}$ est $CH_2CH_3$ ou $CH(CH_3)_2$ et $R_{15}$ est $CH_3$ ;
  (7) $R_{13}$ est $CH_2CH_3$ ou $CH(CH_3)_2$, $R_{14}$ est $OCH_3$ et $R_{15}$ est H ;
  (8) $R_{13}$ est $CH_2CH_2CH_3$, $R_{14}$ est $OCH_3$ et $R_{15}$ est $CH_3$ ;
p) si Q est Q-6, R est H, Z est CH et Y est $CH_2CH_3$ ; alors X est autre chose que $OCH_3$ si $R_{13}$ et $R_{15}$ sont $CH_3$ et $R_{14}$ est Cl ;
q) si Q est Q-6, R est H, Z est N et Y est $CH_3$, alors X est autre chose que $OCH_3$ si
  (1) $R_{13}$ est $CH_2CH_3$, $R_{14}$ est H ou Cl et $R_{15}$ est $CH_3$ ;
  (2) $R_{13}$ est $CH_3$ , $R_{14}$ est Cl et $R_{15}$ est H ;
  (3) $R_{13}$ est $CH(CH_3)_2$, $R_{14}$ est H et $R_{15}$ est $CH_3$ ;
  (4) $R_{13}$ et $R_{14}$ sont $CH_2CH_3$ et $R_{15}$ est H ;
  (5) $R_{13}$ et $R_{15}$ sont $CH_3$ et $R_{14}$ est $OCH_2CH_3$ ;
  (6) $R_{13}$ est H, $R_{14}$ est $CH_2CH_3$ ou $CH(CH_3)_2$ et $R_{15}$ est $CH_3$;
  (7) $R_{13}$ est $CH_2CH_3$ ou $CH(CH_3)_2$, $R_{14}$ est $OCH_3$ et $R_{15}$ est H ;
  (8) $R_{13}$ est $CH_2CH_2CH_3$, $R_{14}$ est $OCH_3$ et $R_{15}$ est $CH_3$ ;

r) si Q est Q-6, R est H, Z est N et Y est $OCH_2CF_3$ alors X est autre chose que $OCH_3$ si $R_{13}$ et $R_{15}$ sont $CH_3$ et $R_{14}$ est $OCH_3$ ;

s) si Q est Q-6, R est H, Z est CH et Y est $CF_3$ ou $OCH_2CF_3$, alors X est autre chose que $CH_3$ si $R_{13}$ et $R_{15}$ sont $CH_3$ et $R_{14}$ est $CO_2CH_3$ ;

t) si Q est Q-6, R est H, Z est N et Y est $OCH_2CF_3$, alors X est autre chose que $CH_3$ si $R_{13}$ et $R_{15}$ sont $CH_3$ et $R_{14}$ est $SO_2CH_2CH_2CH_3$

u) si Q est Q-6, R est H et A est A-2, alors $Y_1$ est autre chose que $CH_3$ si $R_{13}$ et $R_{14}$ sont H et $R_{15}$ est $CH_3$ ;

v) si Q est Q-6, R est H, Z est N et Y est $CH_3$, alors X est autre chose que $CH_3$ si $R_{13}$ et $R_{14}$sont H et $R_{15}$ est $CH_3$ ;

w) si Q est Q-6, A est A-2, $Y^1$ est $CH_3$, R est H et $R_{15}$ est $CH_3$, alors

(i) si $R_{13}$ est $CH_3$, $R_{14}$ est autre chose que Cl, $CO_2CH_3$, $CO_2CH_2CH_3$, $SO_2C_3H_7$-n, $SO_2C_3H_7$-i ou $SO_2N(CH_3)_2$ ; et

(ii) si $R_{13}$ est H, $R_{14}$ est autre chose que H ou $SO_2C_3H_7$-n ;

x) si Q est Q-6, A est A-2, $Y^1$ est $CH_3$, R est H, $R_{15}$ est $CH_3$ et $R_{13}$ est $CO_2CH_3$, alors $R_{14}$ est autre chose que $CH_3$;

y) si Q est Q-4, A est A-2, $Y^1$ est $CH_3$, R est H et $R_{10}$ et $R_{12}$ sont $CH_3$, alors $R_{11}$ est autre chose que Cl, $NO_2$ ou $SO_2N(CH_3)_2$ ; et

z) si Q est Q-5, R, $R_{11}$ et $R_{12}$ sont H, et A est A-2, alors $Y^1$ est autre chose que $CH_3$ ;

et ses sels convenant pour l'agriculture.

**2.** Un composé selon la revendication 1 où

Q est Q-1, Q-2, Q-3, Q-4, Q-5 ou Q-6 ;

A est

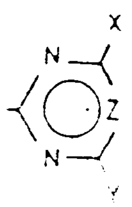 , 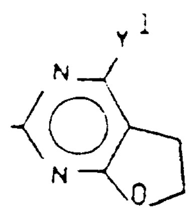 , 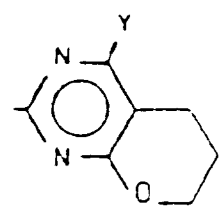

ou 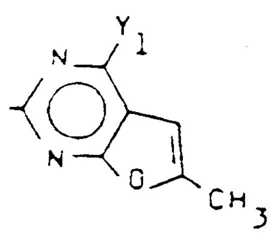 ;

X est $CH_3$, $OCH_3$ ou Cl ;

Y est $CH_3$, $C_2H_5$, $OCH_3$, $OC_2H_5$, $CH_2OCH_3$, $CH(OCH_3)_2$, $NH_2$, $NHCH_3$ ou $N(CH_3)_2$ ;

Z est CH ou N ;

$Y_1$ est H, Cl, $CH_3$ ou $OCH_3$ ;

$X_2$ est $OCH_3$ ou $CH_3$ ; et

$Y_2$ est $CH_3$.

**3.** Un composé selon la revendication 1 où

Q est Q-7, Q-8 ou Q-9 ;

$R_{26}$ est H, un groupe alkyle en $C_1$-$C_3$ ou $CH_3C(O)NH$ ;

$R_{27}$ est H, un groupe alkyle en $C_1$-$C_3$, alcoxy en $C_1$-$C_3$, alkylthio en $C_1$-$C_3$, ou (alcoxy en $C_1$-$C_3$)-carbonyle ;

$R_{28}$ est H, un groupe alkyle en $C_1$-$C_3$, alcoxy en $C_1$-$C_3$, alkylthio en $C_1$-$C_3$, (alkyle en $C_1$-$C_3$)-sulfonyle, (alcoxy en $C_1$-$C_3$)carbonyle, $NO_2$, Cl, Br ou $CF_3$ ;

$R_{29}$ est H, Cl ou $CH_3$ ;

$R_{30}$ est H, Cl ou $CH_3$ ;
A est

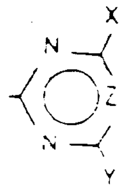

;

Y est $CH_3$, $OCH_3$, $OCF_2H$, $OC_2H_5$ ou $CH_2OCH_3$ ;
X est $CH_3$, $OCH_3$ ou $OCF_2H$ ; et
Z est CH ou N.

**4.** Un composé selon la revendication 1 où
Q est Q-10 ;
$R_{31}$ est Cl, un groupe (alcoxy en $C_1$-$C_3$)carbonyle ou alkyle en $C_1$-$C_3$ ;
A est

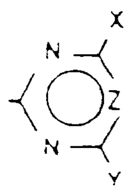

;

X est $CH_3$, $OCH_3$ ou $OCF_2H$ ;
Y est $CH_3$, $OCH_3$, $OC_2H_5$, $OCF_2H$ ou $CH_2OCH_3$; et
Z est CH ou N.

**5.** Un composé selon la revendication 1 où R est H.

**6.** Un composé selon la revendication 1 ou 5 où A est A-1 et Z est CH.

**7.** Un composé selon la revendication 1, 5 et 6 où Q est Q-1 ou Q-6.

**8.** Un composé selon la revendication 1 où Q est Q-4, R est H, $R_{10}$ est $CH_3$, $R_{12}$ est H, $R_{11}$ est $CO_2R_{24}$, $R_{24}$ est un groupe alkyle en $C_1$-$C_3$, A est A-1, X et Y sont $OCH_3$ et Z est CH.

**9.** Un composé selon la revendication 1 qui est :
le N-[(4,6-diméthylpyrimidine-2-yl)aminocarbonyl]-1-(1-méthyléthyl)-1H-imidazole-2-sulfonamide ;
le N-[(4,6-diméthylpyrimidine-2-yl)aminocarbonyl]-1-éthyl-1H-imidazole-2-sulfonamide ; ou un sel convenant pour l'agriculture de l'un ou l'autre de ceux-ci.

**10.** Un composé selon la revendication 1 qui est choisi parmi
le N-[(4-méthoxy-6-méthylpyrimidine-2-yl)aminocarbonyl]-1-(1-méthyléthyl)-1H-imidazole-2-sulfonamide ;
le N-[(4-méthoxy-6-méthylpyrimidine-2-yl)aminocarbonyl]-1-éthyl-1H-imidazole-2-sulfonamide ;
le N-[(4-méthoxy-6-méthylpyrimidine-2-yl)aminocarbonyl]-4-thiazolesulfonamide ;
le 4-[[(4-méthoxy-6-méthylpyrimidine-2-yl)aminocarbonyl]aminosulfonyl]-3-isothiazolecarboxylate de méthyle ; ou un sel convenant pour l'agriculture de l'un quelconque de ceux-ci.

**11.** Un composé selon la revendication 1 qui est choisi parmi
le N-[(4,6diméthoxypyrimidine-2-yl)aminocarbonyl]-1-(1-méthyléthyl)-1H-imidazole-2-sulfonamide ;
le N-[(4,6-diméthoxypyrimidine-2-yl)aminocarbonyl]-1-éthyl-1H-imidazole-2-sulfonamide ;
le N-[(4,6-diméthoxypyrimidine-2-yl)aminocarbonyl]-5-bromo-1-méthyl-1H-imidazole-4-sulfonamide ;
le N-[(4,6-diméthoxypyrimidine-2-yl)aminocarbonyl]-4-thiazolesulfonamide ;

le 4-[[(4,6-diméthoxypyrimidine-2-yl)aminocarbonyl]aminosulfonyl]-3-isothiazolecarboxylate de méthyle ;
ou un sel convenant pour l'agriculture de l'un quelconque de ceux-ci.

**12.** Une composition pour l'agriculture comprenant une quantité à effet herbicide d'un ou plusieurs des composés de l'une quelconque des revendications 1-11 et au moins l'un des ingrédients suivant : (a) un agent tensio-actif et (b) un diluant solide ou liquide.

**13.** Un procédé pour combattre la croissance d'une végétation indésirable consistant à appliquer au lieu où se trouve cette végétation une quantité efficace pour l'agriculture d'un ou plusieurs composés de l'une quelconque des revendications 1-11.

**14.** Un procédé pour réguler la croissance de plantes qui consiste à appliquer au lieu où se trouvent ces plantes une quantité efficace mais sensiblement non phytotoxique d'un régulateur de la croissance des plantes choisi parmi les composés de l'une quelconque des revendications 1 à 11.

**15.** Un procédé pour préparer un composé de la revendication 1, caractérisé en ce qu'il consiste soit
(a) à mettre en contact un aminohétérocycle de formule

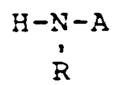

avec
i) un isocyanate de sulfonyle de formule $QSO_2NCO$, ou avec
ii) un N-sulfonylcarbamate de phényle de formule

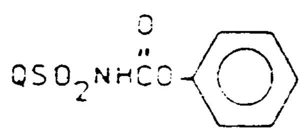

;

soit
(b) à mettre en contact un sulfonamide de formule $QSO_2NH_2$ avec
i) un carbamate de méthyle de formule

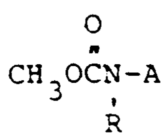

en présence d'une quantité équimolaire de triméthylaluminium, ou avec
ii) un isocyanate hétérocyclique de formule

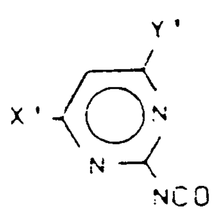

où X' est Cl et Y' est Cl ou Br, ceci étant éventuellement suivi d'une réaction avec une espèce nucléophile telle qu'un ion méthylate ou éthylate ; ou avec

iii) un carbamate N-hétérocyclique de formule

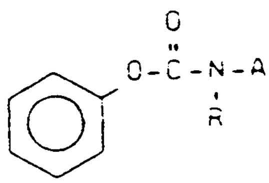

en présence d'une base.

**Revendications pour les Etats contractants suivants : LU, SE**

**1.** Un composé de la formule

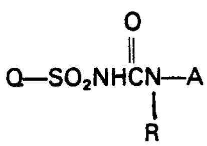

où

R est H ou $CH_3$;

Q est

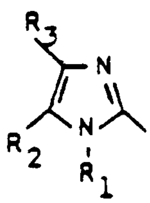 , 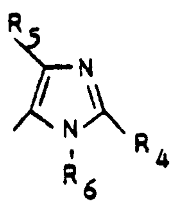 , 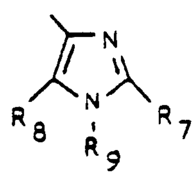 ,

Q-1 Q-2 Q-3

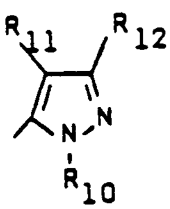 , 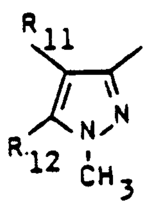 , 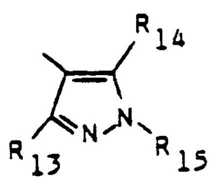 ;

Q-4 Q-5 Q-6

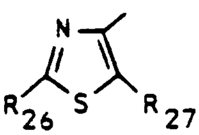 , 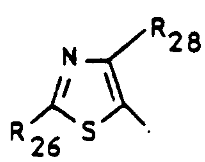 , 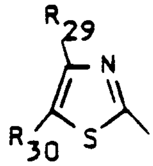 ou 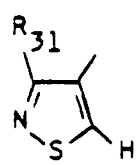 ;

Q-7 Q-8 Q-9 Q-10

$R_1$ est H, un groupe alkyle en $C_1$-$C_8$, alcényle en $C_3$-$C_6$ cycloalkyle en $C_5$-$C_6$, cycloalcényle en $C_5$-$C_6$,alcynyle en $C_3$-$C_6$, cycloalkylalkyle en $C_4$-$C_7$, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

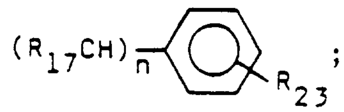

ou alkyle en $C_1$-$C_6$ substitué par a) 1-3 atomes de F, Br ou Cl; ou par b) $OR_{16}$;

$R_2$, $R_3$ est $R_4$ sont indépendamment H ou $CH_3$;

$R_5$ et H, un groupe alkyle en $C_1$-$C_4$, -$OR_6$, $NO_2$, F, Cl, Br, $CO_2R_{24}$, $S(O)_mR_{25}$ OU $SO_2NR_{20}R_{21}$;

$R_6$ est H, un groupe alkyle en $C_1$-$C_4$, alcényle en $C_3$-$C_4$, alcynyle en $C_3$-$C_4$, $CO_2R_{18}$, $SO_2NR_{20}R_{21}$, $SO_2R_{22}$ ou alkyle en $C_1$-$C_4$ substitué par a) 1-3 atomes de F, Cl, ou Br; ou par b) $OCH_3$;

$R_7$ est H ou $CH_3$;

$R_8$ est H, un groupe alkyle en $C_1$-$C_4$, -$OR_{16}$, $NO_2$, F, Cl, Br, $CO_2R_{24}$, $S(O)_mR_{25}$ OU $SO_2NR_{20}R_{21}$;

$R_9$ est $CH_3$ ou $C_2H_5$;

$R_{10}$ est H, un groupe alkyle en $C_1$-$C_4$, alcényle en $C_3$-$C_4$, alcynyle en $C_3$-$C_4$, $CO_2R_{24}$, $SO_2NR_{20}R_{21}$ ou $SO_2R_{22}$;

$R_{11}$ est H, un groupe alkyle en $C_1$-$C_3$, F, Cl, Br, $NO_2$, -$OR_{16}$, $CO_2R_{24}$, $S(O)_mR_{25}$ OU $SO_2NR_{20}R_{21}$;

$R_{12}$ est H ou $CH_3$;

$R_{13}$ et $R_{14}$ sont indépendamment H, un groupe alkyle en $C_1$-$C_3$, -$OR_{16}$, F, Cl, Br, $NO_2$, $CO_2R_{24}$, S-(O)$_mR_{25}$ ou $SO_2NR_{20}R_{21}$;

$R_{15}$ est H ou $CH_3$;

$R_{16}$ est un groupe alkyle en $C_1$-$C_3$;

$R_{17}$ est H ou $CH_3$;

$R_{18}$ est un groupe alkyle en $C_1$-$C_4$, alcényle en $C_3$-$C_4$, alcynyle en $C_3$-$C_4$, $CH_2CH_2Cl$ ou $CH_2CH_2OCH_3$;

$R_{19}$ est un groupe alkyle en $C_1$-$C_4$, alcényle en $C_3$-$C_4$, alcynyle en $C_3$-$C_4$ ou $CH_2CH_2OCH_3$;

$R_{20}$ et $R_{21}$ sont indépendamment $CH_3$ ou $C_2H_5$;

$R_{22}$ est un groupe alkyle en $C_1$-$C_3$ ou $CF_3$;

$R_{23}$ est H, Cl, Br, $CH_3$, F, $CF_3$, $OCH_3$ ou $NO_2$;

$R_{24}$ est un groupe alkyle en $C_1$-$C_3$ ou allyle;

$R_{25}$ est un groupe alkyle en $C_1$-$C_3$;

m est 0, 1 ou 2;

n est 0 ou 1;

$R_{26}$ est H, une groupe alkyle en $C_1$-$C_3$ ou $CH_3C(O)NH$;

$R_{27}$ est H, une groupe alkyle en $C_1$-$C_3$, alcoxy en $C_1$-$C_3$, alkylthio en $C_1$-$C_3$ ou (alcoxy en $C_1$-$C_3$)-carbonyle;

$R_{28}$ est H, une groupe alkyle en $C_1$-$C_3$, alcoxy en $C_1$-$C_3$, alkylthio en $C_1$-$C_3$, (alcoxy en $C_1$-$C_3$)-carbonyle, $NO_2$, Cl, Br ou $CF_3$;

$R_{29}$ est H, Cl, ou $CH_3$;

$R_{30}$ est H, Cl ou $CH_3$;

$R_{31}$ est Cl, un groupe (alcoxy en $C_1$-$C_3$)carbonyle ou alkyle en $C_1$-$C_3$;

A est

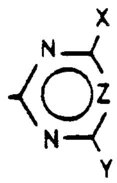 , 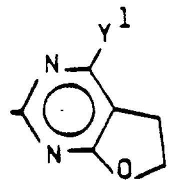 , 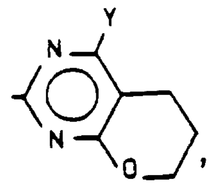 ,

A-1 A-2 A-3

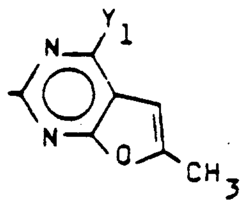 , 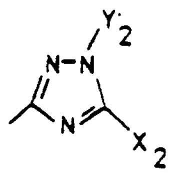 ou 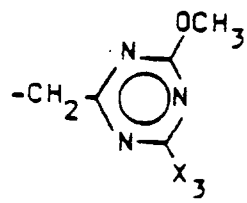 ;

A-4 A-5 A-6

X est $CH_3$, $OCH_3$, Cl, F, $OCF_2H$ ou $SCF_2H$;

Y est $CH_3$, $C_2H_5$, $OCH_3$, $OC_2H_5$, $CH_2OCH_3$, $CH(OCH_3)_2$, $OCH_2CF_3$, $OCF_3$, $NH_2$, $NHCH_3$, $N(CH_3)_2$ ou $GCF_2T$ où G est O ou S et T est H, CHClF, CHBrF, $CF_2H$ ou $CHFCF_3$;

Z est CH ou N;

$Y_1$ est H, Cl, $CH_3$, $OCH_3$ ou $OCF_2H$;

$X_2$ est $OCH_3$, $CH_3$, $CH_2CH_3$, $OCH_2CH_3$, $SCH_3$ ou $SCH_2CH_3$;

$Y_2$ est $CH_3$, $CH_2CH_3$ ou $CH_2CF_3$;

$X_3$ est $OCH_3$ ou $CH_3$;

avec les conditions suivantes:

a) si Q est Q-7, Q-8, Q-9 ou Q-10 alors R est H;

b) le nombre total d'atomes de carbone de $R_1$ est inférieur ou égal à 8;

c) si $R_1$ est autre chose qu'un groupe alkyle en $C_1$-$C_3$, alors $R_3$ doit être H;

d) si $R_5$ est autre chose que H, $CH_3$, $OCH_3$ ou $NO_2$, alors $R_6$ est H ou $CH_3$;

e) si $R_6$ est $CO_2R_{18}$, $SO_2NR_{20}R_{21}$ ou $SO_2R_{22}$, alors $R_5$ est H, $CH_3$, $OCH_3$ ou $NO_2$;

f) si $R_{10}$ est autre chose qu'un groupe alkyle en $C_1$-$C_3$, alors $R_{11}$ est H, Cl, $OCH_3$, $NO_2$ ou $CH_3$;

g) si l'un de $R_{13}$ est $R_{14}$ est $CO_2R_{24}$, $S(O)_mR_{25}$ OU $SO_2NR_{20}R_{21}$, alors l'autre est H, Cl, $CH_3$, $OCH_3$ ou $NO_2$;

h) $R_{29}$ et $R_{30}$ ne sont pas simultanément Cl;

i) si X est Cl ou F, alors Z est CH et Y est $OCH_3$, $NH_2$, $NHCH_3$ ou $N(CH_3)_2$;

i) si Q est Q-7, Q-8, Q-9 ou Q-10, alors A est A-1; X est $CH_3$, $OCH_3$ ou $OCF_2H$, Y est $CH_3$, $OCH_3$, $OC_2H_5$, $OCF_2H$ ou $CH_2OCH_3$; et

k) si l'un ou l'autre de X et Y est $OCF_2H$, alors Z est CH;

et ses sels convenant pour l'agriculture.

**2.** Un composé selon la revendication 1 où Q est Q-1, Q-2, Q-3, Q-4, Q-5 ou Q-6;

A est

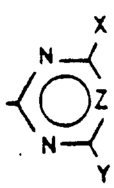 , 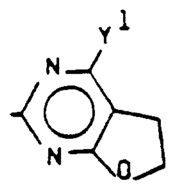 , 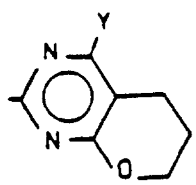 ou 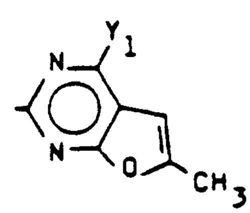 ;

X est $CH_3$, $OCH_3$ ou Cl;

Y est $CH_3$, $C_2H_5$, $OCH_3$, $OC_2H_5$, $CH_2OCH_3$, $CH(OCH_3)_2$, $NH_2$, $NHCH_3$ ou $N(CH_3)_2$;

Z est CH ou N;

$Y_1$ est H, Cl, $CH_3$ ou $OCH_3$;
$X_2$ est $OCH_3$ ou $CH_3$; et
$Y_2$ est $CH_3$.

**3.** Un composé selon la revendication 1 où Q est Q-7, Q-8 ou Q-9;
$R_{26}$ est H, un groupe alkyle en $C_1$-$C_3$ ou $CH_3C(O)NH$;
$R_{27}$ est H, un groupe alkyle en $C_1$-$C_3$, alcoxy en $C_1$-$C_3$, alkylthio en $C_1$-$C_3$, ou (alcoxy en $C_1$-$C_3$)-carbonyle;
$R_{28}$ est H, un groupe alkyle en $C_1$-$C_3$, alcoxy en $C_1$-$C_3$, alkylthio en $C_1$-$C_3$, (alkyle en $C_1$-$C_3$)-sulfonyle, (alcoxy en $C_1$-$C_3$)carbonyle, $NO_2$, Cl, Br ou $CF_3$;
$R_{29}$ est H, Cl ou $CH_3$;
$R_{30}$ est H, Cl ou $CH_3$;
A est

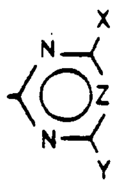 ;

Y est $CH_3$, $OCH_3$, $OCF_2H$, $OC_2H_5$ ou $CH_2OCH_3$;
X est $CH_3$, $OCH_3$ ou $OCF_2H$; et
Z est CH ou N.

**4.** Un composé selon la revendication 1 où
Q est Q-10;
$R_{31}$ est Cl, un groupe (alcoxy en $C_1$-$C_3$)carbonyle ou alkyle en $C_1$-$C_3$;
A est

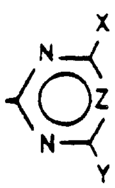 ;

X est $CH_3$, $OCH_3$ ou $OCF_2H$;
Y est $CH_3$, $OCH_3$, $OC_2H_5$, $OCF_2H$ ou $CH_2OCH_3$; et
Z est CH ou N.

**5.** Un composé selon la revendication 1 où R est H.

**6.** Un composé selon la revendication 1 ou 5 où A est A-1 et Z est CH.

**7.** Un composé selon la revendication 1, 5 et 6 où Q est Q-1 ou Q-6.

**8.** Un composé selon la revendication 1 où Q est Q-4, R est H, $R_{10}$ est $CH_3$, $R_{12}$ est H, $R_{11}$ est $CO_2R_{24}$ $R_{24}$ est un groupe alkyle en $C_1$-$C_3$, A est A-1, X et Y sont $OCH_3$ et Z est CH.

**9.** Un composé selon la revendication 1 qui est:
le N-[(4,6-diméthylpyrimidine-2-yl)aminocarbonyl]-1-(1-méthyléthyl)-1H-imidazole-2-sulfonamide;
le N-[(4,6-diméthylpyrimidine-2-yl)aminocarbonyl]-1-éthyl-1H-imidazole-2-sulfonamide; ou un sel convenant pour l'agriculture de l'un ou l'autre de ceux-ci.

**10.** Un composé selon la revendication 1 qui est choisi parmi
le N-[(4-méthoxy-6-méthylpyrimidine-2-yl)aminocarbonyl]-1-(1-méthyléthyl)-1H-imidazole-2-sulfonamide;

le N-[(4-méthoxy-6-méthylpyrimidine-2-yl)aminocarbonyl]-1-éthyl-1H-imidazole-2-sulfonamide;
le N-[(4-méthoxy-6-méthylpyrimidine-2-yl)aminocarbonyl]-4-thiazolesulfonamide;
le 4-[[(4-méthoxy-6-méthylpyrimidine-2-yl)aminocarbonyl]aminosulfonyl]-3-isothiazolecarboxylate de méthyle; ou un sel convenant pour l'agriculture de l'un quelconque de ceux-ci.

**11.** Un composé selon la revendication 1 qui est choisi parmi
le N-[(4,6-diméthoxypyrimidine-2-yl)aminocarbonyl]-1-(1-méthyléthyl)-1H-imidazole-2-sulfonamide;
le N-[(4,6-diméthoxypyrimidine-2-yl)aminocarbonyl]-1-(1-éthy-1H-imidazole-2-sulfonamide;
le N-[(4,6-diméthoxypyrimidine-2-yl)aminocarbonyl]-5-bromo-1-méthyl-1H-imidazole-4-sulfonamide;
le N-[(4,6-diméthoxypyrimidine-2-yl)aminocarbonyl]-4-thiazolesulfonamide;
le 4-[[(4,6-diméthoxypyrimidine-2-yl)aminocarbonyl]aminosulfonyl]-3-isothiazolecarboxylate de méthyle;
ou un sel convenant pour l'agriculture de l'une quelconque de ceux-ci.

**12.** Une composition pour l'agriculture comprenant une quantité à effet herbicide d'un ou plusieurs des composés de l'une quelconque des revendications 1-11 et au moins l'un des ingrédients suivant: (a) un agent tensio-actif et (b) un diluant solide ou liquide.

**13.** Un procédé pour combattre la croissance d'une végétation indésirable consistant à appliquer au lieu où se trouve cette végétation une quantité efficace pour l'agriculture d'un ou plusieurs composés de l'une quelconque des revendications 1-11.

**14.** Un procéde pour réguler le croissance de plantes qui consiste à appliquer au lieu où se trouvent ces plantes une quantité efficace mais sensiblement non phytotoxique d'un régulateur de la croissance des plantes choisi parmi les composés de l'une quelconque des revendications 1 à 11.

**15.** Un procédé pour préparer un composé de la revendication 1, caractérisé en ce qu'il consiste soit
(a) à mettre en contact un aminohétérocycle de formule

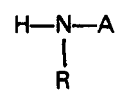

avec
i) un isocyanate de sulfonyle de formule $QSO_2NCO$, ou avec
ii) un N-sulfonylcarbamate de phényle de formule

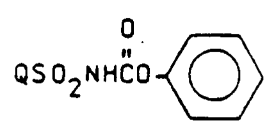 ;

soit
(b) à mettre en contact un sulfonamide de formule $QSO_2NH_2$ avec
i) un carbamate de méthyle de formule

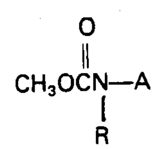

en présence d'une quantité équimolaire de triméthylaluminium, ou avec

ii) un isocyanate hétérocyclique de formule

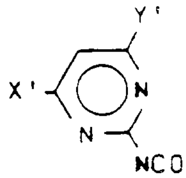

ou X' est Cl Y' est Cl ou Br, ceci étant éventuellement suivi d'une réaction avec une espèce nucléophile telle qu'un ion méthylate ou éthylate; ou avec

iii) un carbamate N-hétérocyclique de formule

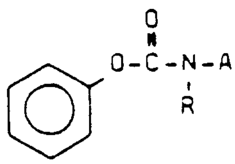

en présence d'une base.

**Revendications pour l'Etat contractant suivant : AT**

**1.** Un procédé pour la préparation d'un composé de la formule

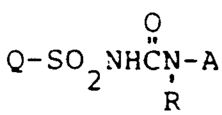

où

R est H ou $CH_3$ ;

Q est

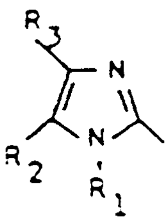 ,

Q-1

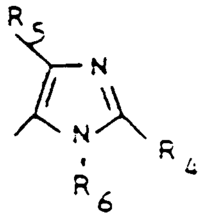 ,

Q-2

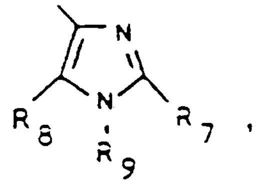 ,

Q-3

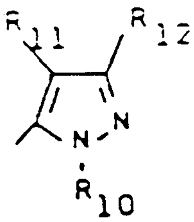 ,

Q-4

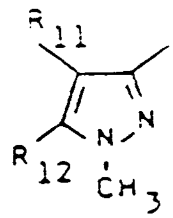 ,

Q-5

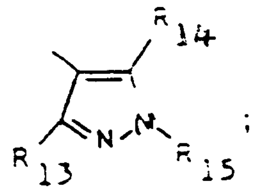 ;

Q-6

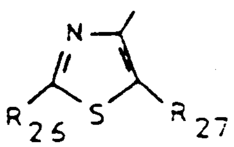 ,

Q-7

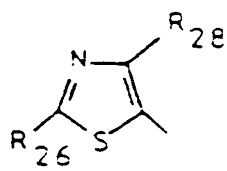 ,

Q-8

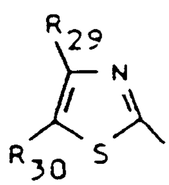

Q-9

ou

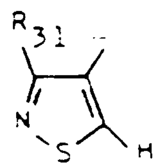 ;

Q-10

$R_1$ est H, un groupe alkyle en $C_1$-$C_8$, alcényle en $C_3$-$C_6$, cycloalkyle en $C_5$-$C_6$, cycloalcényle en $C_5$-$C_6$, alcynyle en $C_3$-$C_6$, cycloalkylalkyle en $C_4$-$C_7$, $(R_{17}CH)_nC(O)R_{16}$, $(R_{17}CH)_nCO_2R_{18}$, $(R_{17}CH)_nCOSR_{19}$, $(R_{17}CH)_nCONR_{20}R_{21}$, $(R_{17}CH)_nSO_2NR_{20}R_{21}$, $(R_{17}CH)_nSO_2R_{22}$,

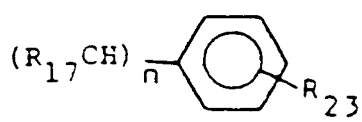 ;

ou alkyle en $C_1$-$C_6$ substitué par

a) 1-3 atomes de F, Br ou Cl ; ou par

b) $OR_{16}$ ;

$R_2$, $R_3$ et $R_4$ sont indépendamment H ou $CH_3$ ;

$R_5$ est H, un groupe alkyle en $C_1$-$C_4$, $-OR_6$, $NO_2$, F, Cl, Br, $CO_2R_{24}$, $S(O)_mR_{25}$ ou $SO_2NR_{20}R_{21}$ ;

$R_6$ est H, un groupe alkyle en $C_1$-$C_4$, alcényle en $C_3$-$C_4$, alcynyle en $C_3$-$C_4$, $CO_2R_{18}$, $SO_2NR_{20}R_{21}$, $SO_2R_{22}$ ou alkyle en $C_1$-$C_4$ substitué par a) 1-3 atomes de F, Cl, ou Br ; ou par b) $OCH_3$

;

$R_7$ est H ou $CH_3$ ;

$R_8$ est H, un groupe alkyle en $C_1$-$C_4$, -$OR_{16}$, $NO_2$, F, Cl, Br, $CO_2R_{24}$, $S(O)_mR_{25}$ ou $SO_2NR_{20}R_{21}$ ;

$R_9$ est $CH_3$ ou $C_2H_5$ ;

$R_{10}$ est H, un groupe alkyle en $C_1$-$C_4$, alcényle en $C_3$-$C_4$, alcynyle en $C_3$-$C_4$, $CO_2R_{24}$, $SO_2NR_{20}R_{21}$ ou $SO_2R_{22}$ ;

$R_{11}$ est H, un groupe alkyle en $C_1$-$C_3$, F, Cl, Br, $NO_2$, -$OR_{16}$, $CO_2R_{24}$, $S(O)_mR_{25}$ ou $SO_2NR_{20}R_{21}$ ;

$R_{12}$ est H ou $CH_3$ ;

$R_{13}$ et $R_{14}$ sont indépendamment H, un groupe alkyle en $C_1$-$C_3$, -$OR_{16}$, F, Cl, Br, $NO_2$, $CO_2R_{24}$, S-$(O)_mR_{25}$ ou $SO_2NR_{20}R_{21}$ ;

$R_{15}$ est H ou $CH_3$ ;

$R_{16}$ est un groupe alkyle en $C_1$-$C_3$ ;

$R_{17}$ est H ou $CH_3$ ;

$R_{18}$ est un groupe alkyle en $C_1$-$C_4$, alcényle en $C_3$-$C_4$, alcynyle en $C_3$-$C_4$, $CH_2CH_2Cl$ ou $CH_2CH_2OCH_3$ ;

$R_{19}$ est un groupe alkyle en $C_1$-$C_4$, alcényle en $C_3$-$C_4$, alcynyle en $C_3$-$C_4$ ou $CH_2CH_2OCH_3$ ;

$R_{20}$ et $R_{21}$ sont indépendamment $CH_3$ ou $C_2H_5$ ;

$R_{22}$ est un groupe alkyle en $C_1$-$C_3$ ou $CF_3$ ;

$R_{23}$ est H, Cl, Br, $CH_3$, F, $CF_3$, $OCH_3$ ou $NO_2$ ;

$R_{24}$ est un groupe alkyle en $C_1$-$C_3$ ou allyle ;

$R_{25}$ est un groupe alkyle en $C_1$-$C_3$ ;

m est 0, 1 ou 2 ;

n est 0 ou 1 ;

$R_{26}$ est H, un groupe alkyle en $C_1$-$C_3$ ou $CH_3C(O)NH$ ;

$R_{27}$ est H, un groupe alkyle en $C_1$-$C_3$, alcoxy en $C_1$-$C_3$, alkylthio en $C_1$-$C_3$ ou (alcoxy en $C_1$-$C_3$)-carbonyle ;

$R_{28}$ est H, un groupe alkyle en $C_1$-$C_3$, alcoxy en $C_1$-$C_3$, alkythio en $C_1$-$C_3$, (alcoxy en $C_1$-$C_3$)-carbonyle, $NO_2$, Cl, Br ou $CF_3$ ;

$R_{29}$ est H, Cl ou $CH_3$ ;

$R_{30}$ est H, Cl ou $CH_3$ ;

$R_{31}$ est Cl, un groupe (alcoxy en $C_1$-$C_3$)carbonyle ou alkyle en $C_1$-$C_3$ ;

A est

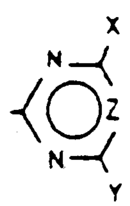 ,

A-1

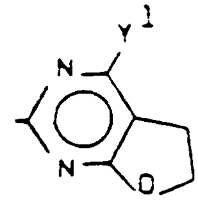 ,

A-2

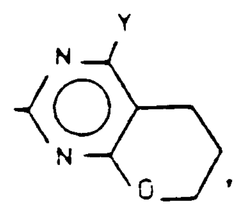 ,

A-3

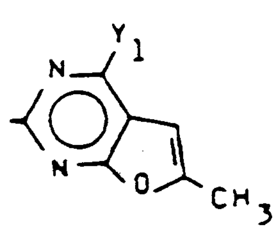 ,

A-4

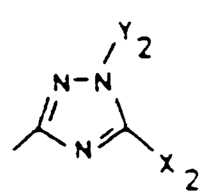 ou

A-5

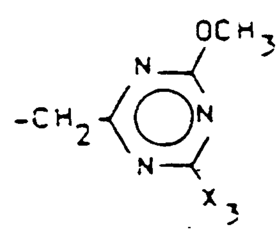 ;

A-6

X est $CH_3$, $OCH_3$, Cl, F, $OCF_2H$ ou $SCF_2H$ ;

Y est $CH_3$, $C_2H_5$, $OCH_3$, $OC_2H_5$, $CH_2OCH_3$,$CH(OCH_3)_2$, $OCH_2CF_3$, $OCF_3$, $NH_2$, $NHCH_3$, $N(CH_3)_2$ ou $GCF_2T$ où G est O ou S et T est H, CHClF, CHBrF, $CF_2H$ ou $CHFCF_3$ ;

Z est CH ou N ;

$Y_1$ est H, Cl, $CH_3$, $OCH_3$ ou $OCF_2H$ ;

$X_2$ est $OCH_3$, $CH_3$, $CH_2CH_3$, $OCH_2CH_3$, $SCH_3$ ou $SCH_2CH_3$ ;

$Y_2$ est $CH_3$, $CH_2CH_3$ ou $CH_2CF_3$ ;

$X_3$ est $OCH_3$ ou $CH_3$ ;

avec les conditions suivantes :

a) si Q est Q-7, Q-8, Q-9 ou Q-10, alors R est H ;

b) le nombre total d'atomes de carbone de $R_1$ est inférieur ou égal à 8 ;

c) si $R_1$ est autre chose qu'un groupe alkyle en $C_1$-$C_3$, alors $R_3$ doit être H ;

d) si $R_5$ est autre chose que H, $CH_3$, $OCH_3$ ou $NO_2$, alors $R_6$ est H ou $CH_3$ ;

e) si $R_6$ est $CO_2R_{18}$, $SO_2NR_{20}R_{21}$ ou $SO_2R_{22}$, alors $R_5$ est H, $CH_3$, $OCH_3$ ou $NO_2$ ;

f) si $R_{10}$ est autre chose qu'un groupe alkyle en $C_1$-$C_3$, alors $R_{11}$ est H, Cl, $OCH_3$, $NO_2$ ou $CH_3$ ;

g) si l'un de $R_{13}$ et $R_{14}$ est $CO_2R_{24}$, $S(O)_mR_{25}$ ou $SO_2NR_{20}R_{21}$, alors l'autre est H, Cl, $CH_3$, $OCH_3$ ou $NO_2$ ;

h) $R_{29}$ et $R_{30}$ ne sont pas simultanément Cl ;

i) si X est Cl ou F, alors Z est CH et Y est $OCH_3$, $NH_2$, $NHCH_3$ ou $N(CH_3)_2$ ;

j) si Q est Q-7, Q-8, Q-9 ou Q-10, alors A est A-1 ; X est $CH_3$, $OCH_3$ ou $OCF_2H$, Y est $CH_3$, $OCH_3$, $OC_2H_5$, $OCF_2H$ ou $CH_2OCH_3$ ; et

k) si l'un ou l'autre de X et Y est $OCF_2H$, alors Z est CH ;

$k_1$) si Q est Q-2, R est H, Z est CH et si l'un de X et Y est $OCH_3$ alors l'autre n'est pas -$CH_3$ lorsque $R_4$, $R_5$ et $R_6$ sont H;

l) si Q est Q-6, R est H, Z est CH et Y est $CH_3$ ou $OCH_3$, alors X est autre chose que $CH_3$ ou $OCH_3$ si

(1) $R_{13}$ et $R_{14}$ sont $CH_3$ et $R_{15}$ est H ou $CH_3$ ;

(2) $R_{13}$ et $R_{15}$ sont $CH_3$ et $R_{14}$ est Br ;

(3) $R_{13}$ est H, $R_{14}$ est Cl et $R_{15}$ est $CH_3$ ;

(4) $R_{13}$ est $CH_2CH_2CH_3$ ou $CH(CH_3)_2$, $R_{14}$ est Cl et $R_{15}$ est $CH_3$ ;

(5) $R_{13}$ est $CH_2CH_3$ ou $CH(CH_3)_2$, $R_{14}$ est $OCH_3$ et $R_{15}$ est $CH_3$ ;

(6) $R_{13}$ et $R_{14}$ sont H et $R_{15}$ est H ou $CH_3$ ;

m) si Q est Q-6, R est H, Z est CH ou N et Y est $CH_3$ ou $OCH_3$, alors X est autre chose que $CH_3$ ou $OCH_3$ si

(1) $R_{13}$ et $R_{15}$ sont $CH_3$ et $R_{14}$ est H, Cl ou $OCH_3$;

(2) $R_{13}$ est H et $R_{14}$ et $R_{15}$ sont $CH_3$ ;

n) si Q est Q-6, R est H, Z est N et Y est $CH_3$ ou $OCH_3$, alors X est autre chose que $OCH_3$ si

(1) $R_{13}$ et $R_{14}$ sont $CH_3$ et $R_{15}$ est H ou $CH_3$ ;

(2) $R_{13}$ et $R_{15}$ sont $CH_3$ et $R_{14}$ est Br ;

(3) $R_{13}$ est H, $R_{14}$ est Cl et $R_{15}$ est $CH_3$ ;

(4) $R_{13}$ est $CH_2CH_3$ ou $CH(CH_3)_2$, $R_{14}$ est $OCH_3$ et $R_{15}$ est $CH_3$ ;

(5) $R_{13}$ et $R_{14}$ sont H et $R_{15}$ est H ou $CH_3$ ;

o) si Q est Q-6, R est H, Z est CH et Y est $CH_3$ ou $OCH_3$, alors X est autre chose que $CH_3$ si

(1) $R_{13}$ est $CH_2CH_3$, $R_{14}$ est H ou Cl et $R_{15}$ est $CH_3$ ;

(2) $R_{13}$ est $CH_3$, $R_{14}$ est Cl et $R_{15}$ est H ;

(3) $R_{13}$ et $R_{15}$ sont $CH_3$ et $R_{14}$ est $OCH_2CH_3$ ;

(4) $R_{13}$ est $CH(CH_3)_2$, $R_{14}$ est H et $R_{15}$ est $CH_3$ ;

(5) $R_{13}$ et $R_{14}$ sont $CH_2CH_3$ et $R_{15}$ est H ;

(6) $R_{13}$ est H, $R_{14}$ est $CH_2CH_3$ ou $CH(CH_3)_2$ et $R_{15}$ est $CH_3$ ;

(7) $R_{13}$ est $CH_2CH_3$ ou $CH(CH_3)_2$, $R_{14}$ est $OCH_3$ et $R_{15}$ est H ;

(8) $R_{13}$ est $CH_2CH_2CH_3$, $R_{14}$ est $OCH_3$ et $R_{15}$ est $CH_3$ ;

p) si Q est Q-6, R est H, Z est CH et Y est $CH_2CH_3$ ; alors X est autre chose que $OCH_3$ si $R_{13}$ et $R_{15}$ sont $CH_3$ et $R_{14}$ est Cl ;

q) si Q est Q-6, R est H, Z est N et Y est $CH_3$, alors X est autre chose que $OCH_3$ si

(1) $R_{13}$ est $CH_2CH_3$, $R_{14}$ est H ou Cl et $R_{15}$ est $CH_3$ ;

(2) $R_{13}$ est $CH_3$ , $R_{14}$ est Cl et $R_{15}$ est H ;

(3) $R_{13}$ est $CH(CH_3)_2$, $R_{14}$ est H et $R_{15}$ est $CH_3$ ;

(4) $R_{13}$ et $R_{14}$ sont $CH_2CH_3$ et $R_{15}$ est H ;

(5) $R_{13}$ et $R_{15}$ sont $CH_3$ et $R_{14}$ est $OCH_2CH_3$ ;

(6) $R_{13}$ est H, $R_{14}$ est $CH_2CH_3$ ou $CH(CH_3)_2$ et $R_{15}$ est $CH_3$;

(7) $R_{13}$ est $CH_2CH_3$ ou $CH(CH_3)_2$, $R_{14}$ est $OCH_3$ et $R_{15}$ est H ;
(8) $R_{13}$ est $CH_2CH_2CH_3$, $R_{14}$ est $OCH_3$ et $R_{15}$ est $CH_3$ ;

r) si Q est Q-6, R est H, Z est N et Y est $OCH_2CF_3$ alors X est autre chose que $OCH_3$ si $R_{13}$ et $R_{15}$ sont $CH_3$ et $R_{14}$ est $OCH_3$ ;

s) si Q est Q-6, R est H, Z est CH et Y est $CF_3$ ou $OCH_2CF_3$, alors X est autre chose que $CH_3$ si $R_{13}$ et $R_{15}$ sont $CH_3$ et $R_{14}$ est $CO_2CH_3$ ;

t) si Q est Q-6, R est H, Z est N et Y est $OCH_2CF_3$, alors X est autre chose que $CH_3$ si $R_{13}$ et $R_{15}$ sont $CH_3$ et $R_{14}$ est $SO_2CH_2CH_2CH_3$

u) si Q est Q-6, R est H et A est A-2, alors $Y_1$ est autre chose que $CH_3$ si $R_{13}$ et $R_{14}$ sont H et $R_{15}$ est $CH_3$ ;

v) si Q est Q-6, R est H, Z est N et Y est $CH_3$, alors X est autre chose que $CH_3$ si $R_{13}$ et $R_{14}$ sont H et $R_{15}$ est $CH_3$ ;

w) si Q est Q-6, A est A-2, $Y^1$ est $CH_3$, R est H et $R_{15}$ est $CH_3$, alors
(i) si $R_{13}$ est $CH_3$, $R_{14}$ est autre chose que Cl, $CO_2CH_3$, $CO_2CH_2CH_3$, $SO_2C_3H_7$-n, $SO_2C_3H_7$-i ou $SO_2N(CH_3)_2$ ; et
(ii) si $R_{13}$ est H, $R_{14}$ est autre chose que H ou $SO_2C_3H_7$-n ;

x) si Q est Q-6, A est A-2, $Y^1$ est $CH_3$, R est H, $R_{15}$ est $CH_3$ et $R_{13}$ est $CO_2CH_3$, alors $R_{14}$ est autre chose que $CH_3$ ;

y) si Q est Q-4, A est A-2, $Y^1$ est $CH_3$, R est H et $R_{10}$ et $R_{12}$ sont $CH_3$, alors $R_{11}$ est autre chose que Cl, $NO_2$ ou $SO_2N(CH_3)_2$ ; et

z) si Q est Q-5, R, $R_{11}$ et $R_{12}$ sont H, et A est A-2, alors $Y^1$ est autre chose que $CH_3$ ; et d'un sel de celui-ci convenant pour l'agriculture ;

qui comprend soit

(a) la mise en contact d'un aminohétérocycle de formule

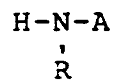

avec

i) un isocyanate de sulfonyle de formule $QSO_2NCO$, ou avec

ii) un N-sulfonylcarbamate de phényle de formule

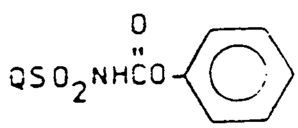 ;

soit

(b) la mise en contact d'un sulfonamide de formule $QSO_2NH_2$ avec

i) un carbamate de méthyle de formule

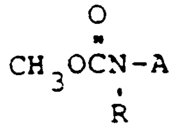

en présence d'une quantité équimolaire de triméthylaluminium, ou avec

ii) un isocyanate hétérocyclique de formule

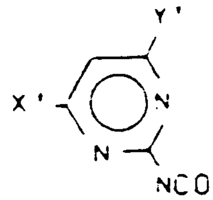

où X' est Cl et Y' est Cl ou Br, ceci étant éventuellement suivi d'une réaction avec une espèce nucléophile telle qu'un ion méthylate ou éthylate ; ou avec

iii) un carbamate N-hétérocyclique de formule

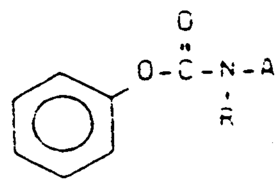

en présence d'une base.

**2.** Un procédé selon la revendication 1 où
Q est Q-1, Q-2, Q-3, Q-4, Q-5 ou Q-6 ;
A est

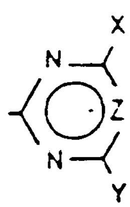 , 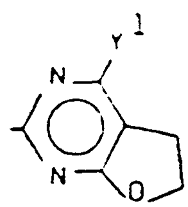 , 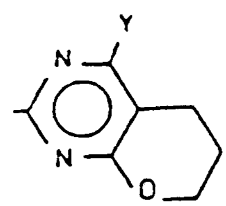

ou 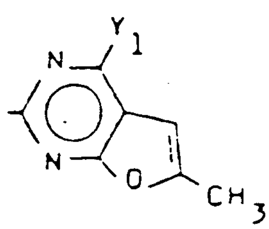 ;

X est $CH_3$, $OCH_3$ ou Cl ;
Y est $CH_3$, $C_2H_5$, $OCH_3$, $OC_2H_5$, $CH_2OCH_3$, $CH(OCH_3)_2$, $NH_2$, $NHCH_3$ ou $N(CH_3)_2$ ;
Z est CH ou N ;
$Y_1$ est H, Cl, $CH_3$ ou $OCH_3$ ;
$X_2$ est $OCH_3$ ou $CH_3$ ; et
$Y_2$ est $CH_3$.

**3.** Un procédé selon la revendication 1 où
Q est Q-7, Q-8 ou Q-9 ;
$R_{26}$ est H, un groupe alkyle en $C_1$-$C_3$ ou $CH_3C(O)NH$ ;
$R_{27}$ est H, un groupe alkyle en $C_1$-$C_3$, alcoxy en $C_1$-$C_3$, alkylthio en $C_1$-$C_3$, ou (alcoxy en $C_1$-$C_3$)-carbonyle ;
$R_{28}$ est H, un groupe alkyle en $C_1$-$C_3$, alcoxy en $C_1$-$C_3$, alkylthio en $C_1$-$C_3$, (alkyle en $C_1$-$C_3$)-

sulfonyle, (alcoxy en $C_1$-$C_3$)carbonyle, $NO_2$, Cl, Br ou $CF_3$ ;
$R_{29}$ est H, Cl ou $CH_3$ ;
$R_{30}$ est H, Cl ou $CH_3$ ;
A est

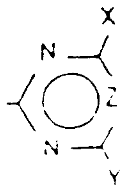 ;

Y est $CH_3$, $OCH_3$, $OCF_2H$, $OC_2H_3$ ou $CH_2OCH_3$ ;
X est $CH_3$, $OCH_3$ ou $OCF_2H$ ; et
Z est CH ou N.

**4.** Un procédé selon la revendication 1 où
Q est Q-10 ;
$R_{31}$ est Cl, un groupe (alcoxy en $C_1$-$C_3$)carbonyle ou alkyle en $C_1$-$C_3$ ;
A est

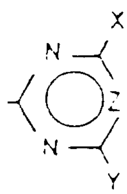 ;

X est $CH_3$, $OCH_3$ ou $OCF_2H$ ;
Y est $CH_3$, $OCH_3$, $OC_2H_5$, $OCF_2H$ ou $CH_2OCH_3$ ; et
Z est CH ou N.

**5.** Un procédé selon la revendication 1 où R est H.

**6.** Un procédé selon la revendication 1 ou 5 où A est A-1 et Z est CH.

**7.** Un procédé selon la revendication 1, 5 ou 6, où Q est Q-1 ou Q-6.

**8.** Un procédé selon la revendication 1 où Q est Q-4, R est H, $R_{10}$ est $CH_3$, $R_{12}$ est H, $R_{11}$ est $CO_2R_{24}$, $R_{24}$ est un groupe alkyle en $C_1$-$C_3$, A est A-1, X et Y sont $OCH_3$ et Z est CH.

**9.** Un procédé selon la revendication 1, dans lequel le produit est
le N-[(4,6-diméthylpyrimidine-2-yl)aminocarbonyl]-1-(1-méthyléthyl)-1H-imidazole-2-sulfonamide ;
le N-[(4,6-diméthylpyrimidine-2-yl)aminocarbonyl]-1-éthyl-1H-imidazole-2-sulfonamide ; ou un sel convenant pour l'agriculture de l'un ou l'autre de ceux-ci.

**10.** Un procédé selon la revendication 1, dans lequel le produit est un composé choisi parmi
le N-[(4-méthoxy-6-méthylpyrimidine-2-yl)aminocarbonyl]-1-(1-méthyléthyl)-1H-imidazole-2-sulfonamide ;
le N-[(4-méthoxy-6-méthylpyrimidine-2-yl)aminocarbonyl]-1-éthyl-1H-imidazole-2-sulfonamide ;
le N-[(4-méthoxy-6-méthylpyrimidine-2-yl)aminocarbonyl]-4-thiazolesulfonamide ;
le 4-[[(4-méthoxy-6-méthylpyrimidine-2-yl)aminocarbonyl]aminosulfonyl]-3-isothiazolecarboxylate de méthyle ; ou un sel convenant pour l'agriculture de l'une quelconque de ceux-ci.

**11.** Un procédé selon la revendication 1, dans lequel le produit est un composé choisi parmi
le N-[(4,6-diméthoxypyrimidine-2-yl)aminocarbonyl]-1-(1-méthyléthyl)-1H-imidazole-2-sulfonamide ;

le N-[(4,6-diméthoxypyrimidine-2-yl)aminocarbonyl]-1-éthyl-1H-imidazole-2-sulfonamide ;
le N-[(4,6-diméthoxypyrimidine-2-yl)aminocarbonyl]-5-bromo-1-méthyl-1H-imidazole-4-sulfonamide ;
le N-[(4,6-diméthoxypyrimidine-2-yl)aminocarbonyl]-4-thiazolesulfonamide ;
le 4-[[(4,6-diméthoxypyrimidine-2-yl)aminocarbonyl]-aminosulfonyl]-3-isothiazolecarboxylate de méthyle; ou un sel convenant pour l'agriculture de l'un quelconque de ceux-ci.

**12.** Un procédé pour combattre la croissance d'une végétation indésirable consistant à appliquer au lieu où se trouve cette végétation une quantité efficace pour l'agriculture d'un ou plusieurs composés de formule I tel que défini à l'une quelconque des revendications 1-11, ou d'un sel de celui-ci convenant pour l'agriculture.

**13.** Un procédé selon la revendication 12, dans lequel on utilise un composé tel que défini dans la revendication 9, 10 ou 11.